(19) Europäisches Patentamt
European Patent Office
Office européen des brevets

(11) EP 3 733 663 B1

(12) EUROPEAN PATENT SPECIFICATION

(45) Date of publication and mention of the grant of the patent:
**17.05.2023 Bulletin 2023/20**

(21) Application number: **18890726.5**

(22) Date of filing: **19.12.2018**

(51) International Patent Classification (IPC):
*C07D 403/12* (2006.01) *C07D 413/14* (2006.01)
*C07D 401/14* (2006.01) *C07D 405/14* (2006.01)
*C07D 403/14* (2006.01) *C07D 409/14* (2006.01)
*C07D 471/04* (2006.01) *C07D 451/02* (2006.01)
*C07D 498/04* (2006.01) *A61K 31/517* (2006.01)
*A61P 35/00* (2006.01)

(52) Cooperative Patent Classification (CPC):
**C07D 401/14; A61P 35/00; C07D 401/12; C07D 403/12; C07D 403/14; C07D 405/14; C07D 409/14; C07D 413/14; C07D 451/02; C07D 471/04; C07D 471/08; C07D 498/04**

(86) International application number:
**PCT/CN2018/122016**

(87) International publication number:
**WO 2019/120213 (27.06.2019 Gazette 2019/26)**

(54)

# 1-[6-[[4-(PHENYLAMINO)-6-QUINAZOLINYL]OXY]-3-AZABICYCLO[3.1.1]HEPT-3-YL]-2-PROPEN-1-ONE DERIVATIVES AND RELATED COMPOUNDS AS IRREVERSIBLE INHIBITORS OF PAN-HER TYROSINE KINASE FOR THE TREATMENT OF CANCER

1-[6-[[4-(PHENYLAMINO)-6-QUINAZOLINYL]OXY]-3-AZABICYCLO[3.1.1]HEPT-3-YL]-2-PROPEN-1-ON-DERIVATE UND ÄHNLICHE VERBINDUNGEN ALS IRREVERSIBLE INHIBITOREN DER PAN-HER TYROSINE KINASE ZUR BEHANDLUNG VON KREBS

DÉRIVÉS DE 1-[6-[[4-(PHÉNYLAMINO)-6-QUINAZOLINYL]OXY]-3-AZABICYCLO[3.1.1]HEPT-3-YL]-2-PROPEN-1-ONE ET COMPOSÉS SIMILAIRES EN TANT QU'INHIBITEURS IRRÉVERSIBLES DE TYROSINE KINASE PAN-HER POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2017 CN 201711376757**
**19.03.2018 CN 201810225742**
**05.06.2018 CN 201810576529**

(43) Date of publication of application:
**04.11.2020 Bulletin 2020/45**

(73) Proprietor: **CHENGDU JINRUI FOUNDATION BIOTECH CO., LTD.**
**Chengdu, Sichuan 610000 (CN)**

(72) Inventors:
- **CHEN, Kevin X**
  **Shanghai 200131 (CN)**
- **WEI, XiaWei**
  **Chengdu, Sichuan 610041 (CN)**
- **ZHANG, Li**
  **Shanghai 200131 (CN)**
- **YU, Yanxin**
  **Shanghai 200131 (CN)**
- **ZHOU, Kai**
  **Shanghai 200131 (CN)**
- **HU, Boyu**
  **Shanghai 200131 (CN)**
- **CHEN, Zhaoguo**
  **Shanghai 200131 (CN)**
- **ZHANG, Huiyu**
  **Shanghai 200131 (CN)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHEN, Shuhui**
  **Shanghai 200131 (CN)**

(74) Representative: **De Clercq & Partners**
**Edgard Gevaertdreef 10a**
**9830 Sint-Martens-Latem (BE)**

(56) References cited:
**WO-A1-2015/154725**
**WO-A1-2017/107985**
**CN-A- 101 679 384**
**CN-A- 104 513 229**
**CN-A- 106 279 128**

## Description

### REFERENCE TO RELATED APPLICATIONS

This application claims the priority of:

CN201711376757.0, with the application date of 2017-12-19;
CN201810225742.2, with the application date of 2018-03-19; and
CN201810576529.6, with the application date of 2018-06-05.

### TECHNICAL FIELD

The present disclosure relates to a series of quinazoline compounds, especially compounds of Formula (I), stereoisomers thereof or pharmaceutically acceptable salts thereof, the pharmaceutical composition containing the same and those for use in a method of treatment of cancer associated with Pan-HER tyrosine kinase.

### BACKGROUND OF THE INVENTION

Human epidermal growth factor receptor (HER, EGFR) is a member of the protein tyrosine kinase family. It is widely distributed on the cell membrane of various human tissues, and can regulate the proliferation, growth, metastasis and apoptosis of cells. Its structure is composed of three parts: extracellular ligand binding region, transmembrane region and intracellular tyrosine kinase region. According to the structural differences of receptors, HER can be divided into four isoforms, i.e., HER1 (EGFR, ErbB-1), HER2 (ErbB-2), HER3 (ErbB-3) and HER4 (ErbB-4). Research disclosed that there is over expression or abnormal activation of HER in various tumor cells such as breast cancer, non-small cell lung cancer, gastric cancer, pancreatic cancer, ovarian cancer, colorectal cancer, head and neck squamous cell carcinoma, malignant glioma and prostate cancer. In addition, research shows that the over expression or abnormal activation of HER is closely associated with the degree of tumor differentiation, the degree of malignancy and prognosis (Baselga. J., Oncologist 2002, 7, 2-8). Therefore, the inhibition of HER has become a hot topic in the research of anti-tumor drugs.

At present, targeted HER inhibitors on the market include Gefitinib, Erlotinib and Lapatinib, etc. However, these marketed drugs have a low effective response rate, are susceptiple to drug resistance, and have some toxic and side effects. Therefore, there is an urgent need to develop other anti-tumor drugs that have excellent anti-tumor effects, can overcome drug resistance, and are well tolerated.

Irreversible inhibitors of Pan-HER tyrosine kinase simultaneously inhibit HER1, HER2 and HER4. Research reveals that this irreversible inhibition of HER family receptors can not only enhance the activity of the drug, but also reduce the occurrence of drug resistance. At the same time, it has a significant inhibitory effect on some tumor cell lines with drug resistance, such as the H1975 cell line that is resistant to Erlotinib. At present, the marketed irreversible inhibitors of Pan-HER tyrosine kinase are only Afatinib and Neratinib. Many inhibitors are in clinical research, such as Poziotinib, Dacomitinib and Canertinib, and there are still unmet market demands.

Therefore, it is necessary to further develop irreversible inhibitors of Pan-HER tyrosine kinase for the treatment of cancer.

Poziotinib (control compound 1) is Pan-HER inhibitor developed by WO2008150118, with the following structure:

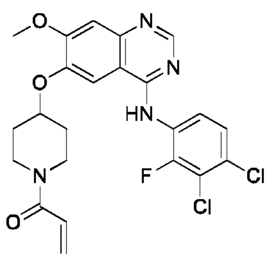

.

The patent application WO2015043515 discloses control compound 2, which has inhibitory effects on EGFR and HER2, and has the following structure:

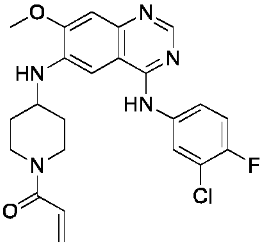

Control compound 2

Further inhibitors with various structures are disclosed in the below patent applications: CN104513229A, CN101679384A, CN106279128A, WO2017107985A1 and WO2015154725A1.

## SUMMARY OF THE INVENTION

The present disclosure provides compounds of Formula (I), stereoisomers thereof or pharmaceutically acceptable salts thereof,

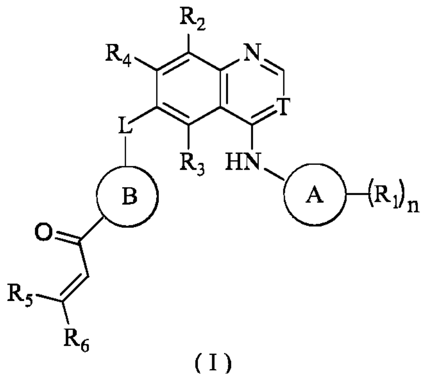

(I)

wherein,

T is selected from the group consisting of N and CR';

each $R_1$ is independently selected from the group consisting of H, F, Cl, Br, I, OH, $NH_2$, CN, $C_{1-3}$ alkyl and $C_{1-3}$ alkoxy, wherein the $C_{1-3}$ alkyl or $C_{1-3}$ alkoxy is optionally substituted with 1, 2 or 3 substituents independently selected from the group consisting of F, Cl, Br, I, OH, $NH_2$ and CN;

$R_2$ and $R_3$ are each independently selected from the group consisting of H, F, Cl, Br, I, OH, $NH_2$ and $C_{1-3}$ alkyl, wherein the $C_{1-3}$ alkyl is optionally substituted with 1, 2 or 3 substituents independently selected from the group consisting of F, Cl, Br and I;

$R_4$ is selected from the group consisting of $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O-, wherein the $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O- are each independently optionally substituted with 1, 2, or 3 R;

$R_5$ and $R_6$ are each independently selected from the group consisting of H, $C_{1-3}$ alkyl and 3- to 7-membered heterocycloalkyl, wherein the $C_{1-3}$ alkyl and 3- to 7-membered heterocycloalkyl are optionally substituted with 1, 2, or 3 R;

L is selected from the group consisting of -O-, $-NR^a-$, $-CR^{b1}R^{b2}-$, $-CR^{b1}R^{b2}-O-$, and $-CR^{b1}R^{b2}-NH-$;

$R^a$ is H;

alternatively, $R_4$ is connected to $R^a$ to form a 5- to 7-membered heterocycloalkyl group optionally substituted with 1, 2, 3 or 4 R';

ring A is selected from the group consisting of phenyl and 5- to 10-membered heteroaryl;

ring B is selected from the group consisting of

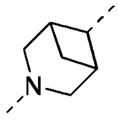, 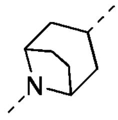, 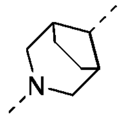,

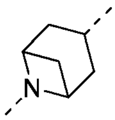, 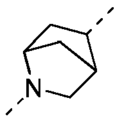, 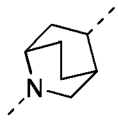, 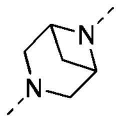 and 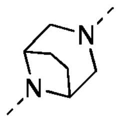;

n is selected from the group consisting of 1, 2, 3, 4 and 5;
each R is independently selected from the group consisting of F, Cl, Br, I, OH, $NH_2$, CN, $NR^{c1}R^{c2}$, $C_{1-3}$ alkyl, $C_{1-3}$ alkoxy, $C_{1-3}$ alkyl-NH-C(=O)-, $C_{1-3}$ alkylthio and 3- to 7-membered heterocycloalkyl, wherein the $C_{1-3}$ alkyl, $C_{1-3}$ alkoxy, $C_{1-3}$ alkyl-NH-C(=O)-, $C_{1-3}$ alkylthio and 3- to 7-membered heterocycloalkyl are optionally substituted with 1, 2, or 3 R';
R' is each independently selected from the group consisting of F, Cl, Br, I, OH, $NH_2$, CN and $C_{1-3}$ alkyl;
$R^{b1}$ and $R^{b2}$ are each independently selected from the group consisting of H, F, Cl, Br, I and $C_{1-3}$ alkyl;
$R^{c1}$ and $R^{c2}$ are each independently selected from the group consisting of H and $C_{1-3}$ alkyl;
the 5- to 7-membered heterocycloalkyl, 5- to 10-membered heteroaryl and 3- to 7-membered heterocycloalkyl groups each contain 1, 2, 3 or 4 heteroatoms or heteroatom groups independently selected from the group consisting of N, -O-, -S- and -NH-.

In some embodiments disclosed herein, the above R is each independently selected from the group consisting of F, Cl, Br, I, OH, $NH_2$, CN,

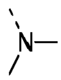,

Me, Et,

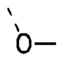, 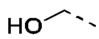,

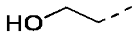, 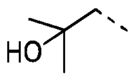, 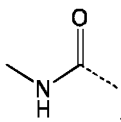,

piperidinyl, pyrrolidinyl, azetidinyl, morpholinyl and oxetanyl, wherein the Me, Et, 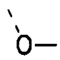, piperidinyl, pyrrolidinyl, azetidinyl, morpholinyl and oxetanyl are optionally substituted with 1, 2 or 3 substituents independently selected from the group consisting of F, Cl, Br, I, OH, $NH_2$ and $C_{1-3}$ alkyl, and other variables are as defined herein.

In some embodiments disclosed herein, the above R is each independently selected from the group consisting of F, Cl, Br, I, OH, $NH_2$, CN,

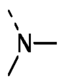,

Me, Et,

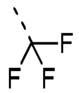, 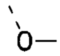,

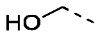, 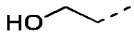, 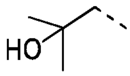, 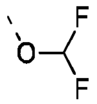, 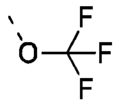, 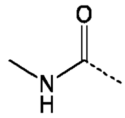, 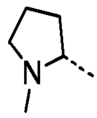, 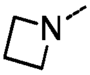,

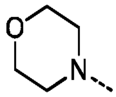 and 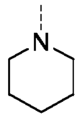,

and other variables are as defined herein.

In some embodiments disclosed herein, the above $R_1$ is each independently selected from the group consisting of H, F, Cl, Br, I, OH, $NH_2$, CN, Me and 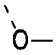, wherein the Me and 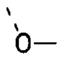 are optionally substituted with 1, 2 or 3 substituents independently selected from the group consisting of F, Cl, Br, I, OH, $NH_2$ and CN, and other variables are as defined herein.

In some embodiments disclosed herein, the above $R_1$ is each independently selected from the group consisting of H, F, Cl, Br, I, OH, $NH_2$, CN, Me, $CH_2F$, $CHF_2$, $CF_3$, 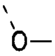 and

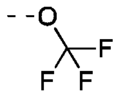,

and other variables are as defined herein.

In some embodiments disclosed herein, the above $R_2$ and $R_3$ are each independently selected from the group consisting of H, F, Cl, Br, I, OH, $NH_2$, Me and $CF_3$, and other variables are as defined herein.

In some embodiments disclosed herein, the above $R_4$ is selected from the group consisting of

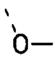, 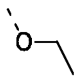, 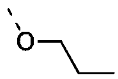,

oxetanyl-O-, piperidinyl-$C_{1-3}$ alkyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O-, wherein the

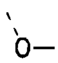, 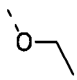,

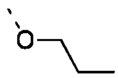,

oxetanyl-O-, piperidinyl-$C_{1-3}$ alkyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O- are optionally substituted with 1, 2 or 3 R, and other variables are as defined herein.

In some embodiments disclosed herein, the above $R_4$ is selected from the group consisting of

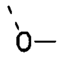, 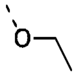, 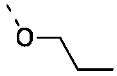, 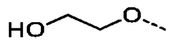, 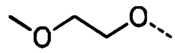, 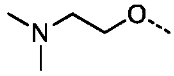,

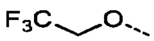, 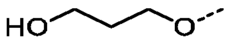, 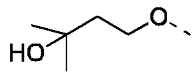, 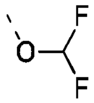, 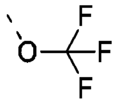, 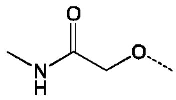,

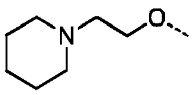, 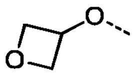, 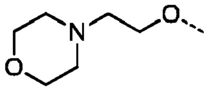, 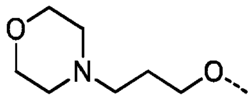, 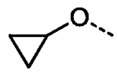,

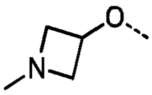, 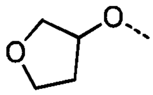, 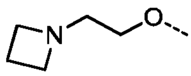 and 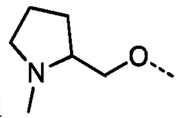,

and other variables are as defined herein.

In some embodiments disclosed herein, the above $R_5$ and $R_6$ are each independently selected from the group consisting of H, Me, Et, and

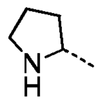

wherein the Me, Et and

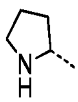

are optionally substituted with 1, 2 or 3 R, and other variables are as defined herein.

In some embodiments disclosed herein, the above $R_5$ and $R_6$ are each independently selected from the group consisting of H,

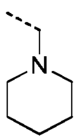, 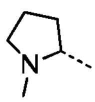 and 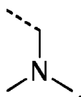,

and other variables are as defined herein.

In some embodiments disclosed herein, the above T is selected from the group consisting of N and C (CN), and other variables are as defined herein.

In some embodiments disclosed herein, the above L is selected from the group consisting of -O-, -NH-, $-CH_2-$, $-CH_2-O-$ and $-CH_2-NH-$, and other variables are as defined herein.

In some embodiments disclosed herein, the above moiety

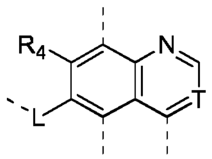

is selected from the group consisting of

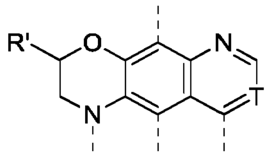 , 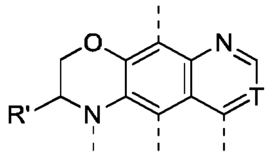 ,

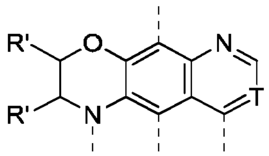 , 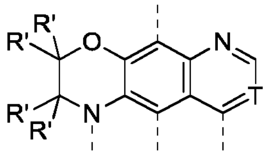 and 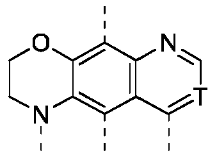 ,

and other variables are as defined herein.

In some embodiments disclosed herein, the above moiety

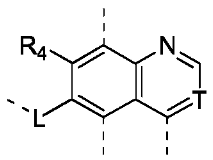

is

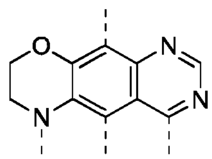 ,

and other variables are as defined herein.

In some embodiments disclosed herein, the above ring A is selected from the group consisting of phenyl, azaindenyl, benzo[b]thienyl, imidazo[1,2-a]pyridyl and benzo[d]isoxazolyl, and other variables are as defined herein.

In some embodiments disclosed herein, the above moiety

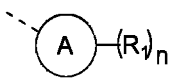

is selected from the group consisting of

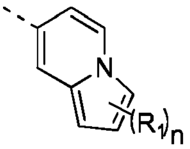, 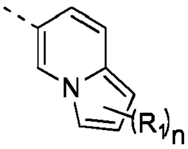, 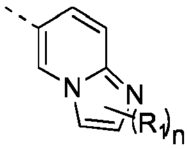,

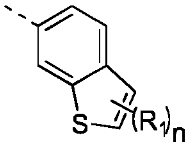, 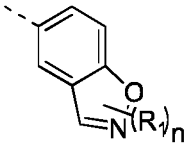 and 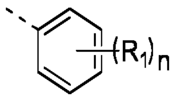,

and other variables are as defined herein.

In some embodiments disclosed herein, the above moiety

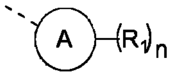

is selected from the group consisting of

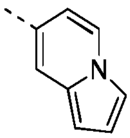, 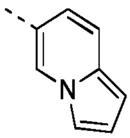, 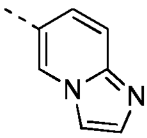, 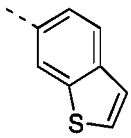,

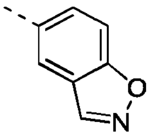, 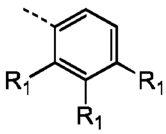, 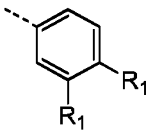 and 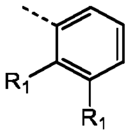,

and other variables are as defined herein.

In some embodiments disclosed herein, the above moiety

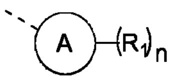

is selected from the group consisting of

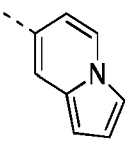, 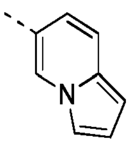, 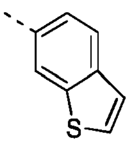, 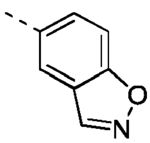,

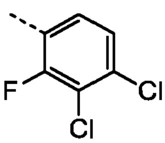, 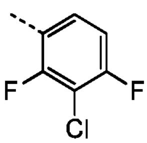, 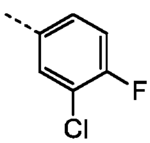, 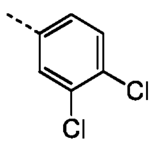, 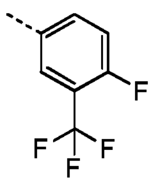, 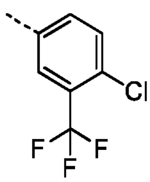,

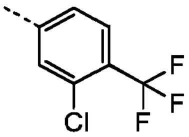, 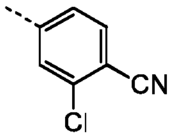, 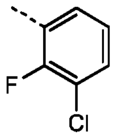 and 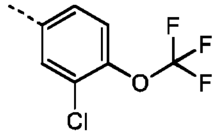

, and other variables are as defined herein.

In some embodiments disclosed herein, the above ring B is selected from the group consisting of

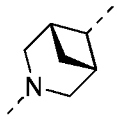, 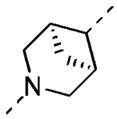, 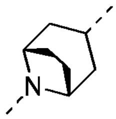, 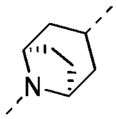, 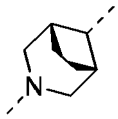, 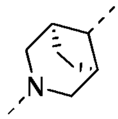, 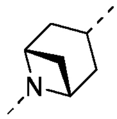,

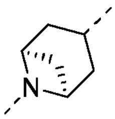, 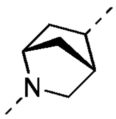, 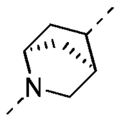, 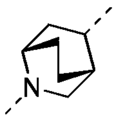, 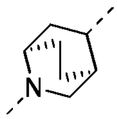, 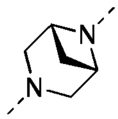, 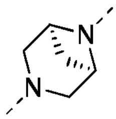, 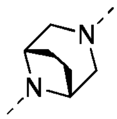

and

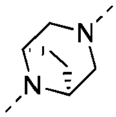

, and other variables are as defined herein.

In some embodiments disclosed herein, the above moiety

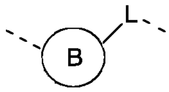

is selected from the group consisting of

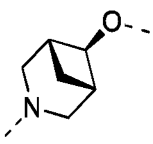, 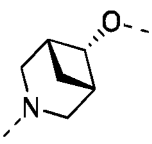, 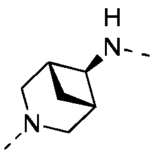, 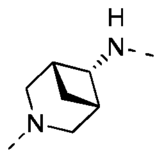,

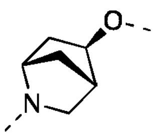, 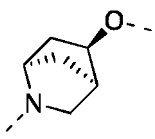, 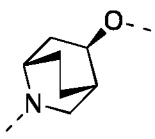, 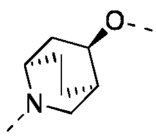, 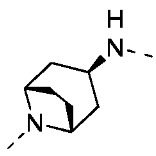, 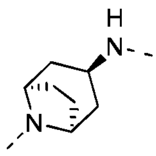,

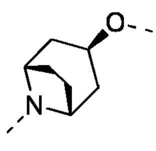 and 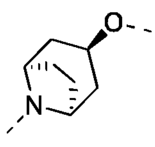,

and other variables are as defined herein.

The present disclosure also provides compounds of Formula (I), stereoisomers thereof or pharmaceutically acceptable salts thereof,

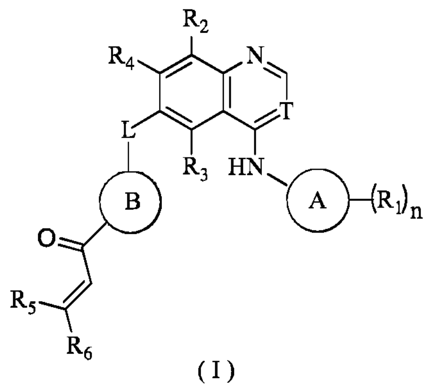

(I)

wherein,

- T is selected from the group consisting of N and CR';
- $R_1$ is each independently selected from the group consisting of H, F, Cl, Br, I, OH, $NH_2$, CN, $C_{1-3}$ alkyl and $C_{1-3}$ alkoxy, wherein the $C_{1-3}$ alkyl or $C_{1-3}$ alkoxy is optionally substituted with 1, 2 or 3 substituents independently selected from the group consisting of F, Cl, Br, I, OH, $NH_2$ and CN;
- $R_2$ and $R_3$ are each independently selected from the group consisting of H, F, Cl, Br, I, OH, $NH_2$ and $C_{1-3}$ alkyl, wherein the $C_{1-3}$ alkyl is optionally substituted with 1, 2 or 3 substituents independently selected from the group consisting of F, Cl, Br, and I;
- $R_4$ is selected from the group consisting of $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O-, wherein the $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O- are each independently optionally substituted with 1, 2, or 3 R;
- $R_5$ and $R_6$ are each independently selected from the group consisting of H, $C_{1-3}$ alkyl and 3- to 7-membered heterocycloalkyl, wherein the $C_{1-3}$ alkyl and 3- to 7-membered heterocycloalkyl are optionally substituted with 1, 2, or 3 R;
- L is selected from the group consisting of -O-, -$NR^a$-, -$CR^{b1}R^{b2}$-, -$CR^{b1}R^{b2}$-O-, and -$CR^{b1}R^{b2}$-NH-;
- $R^a$ is H;
- alternatively, $R_4$ is connected to $R^a$ to form a 5- to 7-membered heterocycloalkyl optionally substituted with 1, 2, 3 or 4 R';
- ring A is selected from the group consisting of phenyl and 5- to 10-membered heteroaryl;
- ring B is selected from the group consisting of

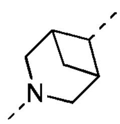, 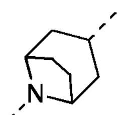, 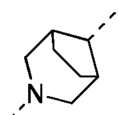,

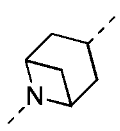, 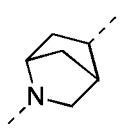, 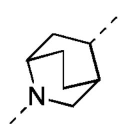, 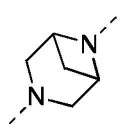 and 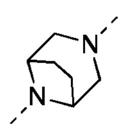;

- n is selected from the group consisting of 1, 2, 3, 4 and 5;
- each R is independently selected from the group consisting of F, Cl, Br, I, OH, $NH_2$, CN, $NR^{c1}R^{c2}$, $C_{1-3}$ alkyl, $C_{1-3}$ alkoxy, $C_{1-3}$ alkylthio and 3- to 7-membered heterocycloalkyl, wherein the $C_{1-3}$ alkyl, $C_{1-3}$ alkoxy, $C_{1-3}$ alkylthio and 3- to 7-membered heterocycloalkyl are optionally substituted with 1, 2, or 3 R';
- R' is each independently selected from the group consisting of F, Cl, Br, I, OH, $NH_2$, CN and $C_{1-3}$ alkyl;
- $R^{b1}$ and $R^{b2}$ are each independently selected from the group consisting of H, F, Cl, Br, I and $C_{1-3}$ alkyl;
- $R^{c1}$ and $R^{c2}$ are each independently selected from the group consisting of H and $C_{1-3}$ alkyl;

the 5- to 7-membered heterocycloalkyl, 5- to 10-membered heteroaryl, and 3- to 7-membered heterocycloalkyl each contains 1, 2, 3 or 4 heteroatoms or heteroatom groups independently selected from the group consisting of N, -O-, -S- and -NH-.

In some embodiments disclosed herein, the above R is each independently selected from the group consisting of F, Cl, Br, I, OH, $NH_2$, CN,

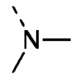,

Me, Et, 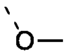, piperidinyl, pyrrolidinyl, azetidinyl, morpholinyl and oxetanyl, wherein the Me, Et,

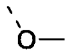,

piperidinyl, pyrrolidinyl, azetidinyl, morpholinyl and oxetanyl are optionally substituted with 1, 2 or 3 substituents independently selected from the group consisting of F, Cl, Br, I, OH, $NH_2$ and $C_{1-3}$ alkyl, and other variables are as defined herein.

In some embodiments disclosed herein, the above R is each independently selected from the group consisting of F, Cl, Br, I, OH, $NH_2$, CN,

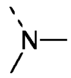,

Me,

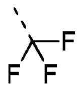, 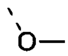,

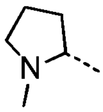, 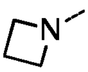, 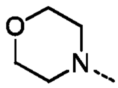 and 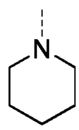,

and other variables are as defined herein.

In some embodiments disclosed herein, the above $R_1$ are each independently selected from the group consisting of H, F, Cl, Br, I, OH, $NH_2$, CN, Me and 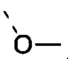,, wherein the Me and 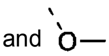 are optionally substituted with 1, 2 or 3 substituents independently selected from the group consisting of F, Cl, Br, I, OH, $NH_2$ and CN, and other variables are as defined herein.

In some embodiments disclosed herein, the above $R_1$ are each independently selected from the group consisting of H, F, Cl, Br, I, OH, $NH_2$, CN, Me,

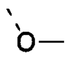 and 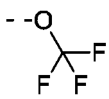,

and other variables are as defined herein.

In some embodiments disclosed herein, the above $R_2$ and $R_3$ are each independently selected from the group

consisting of H, F, Cl, Br, I, OH, $NH_2$, Me and $CF_3$, and other variables are as defined herein.

In some embodiments disclosed herein, the above $R_4$ is selected from the group consisting of

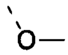, 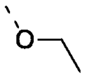, 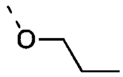,

oxetanyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O-, wherein the

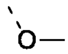, 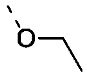, 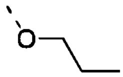,

oxetanyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O- are optionally substituted with 1, 2, or 3 R, and other variables are as defined herein.

In some embodiments disclosed herein, the above $R_4$ is selected from the group consisting of

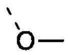, 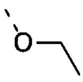, 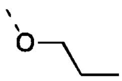, 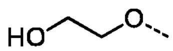, 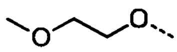, 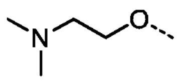,

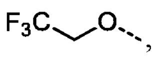, 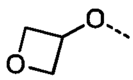, 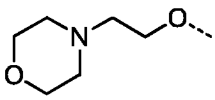, 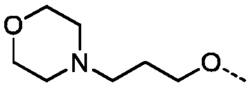, 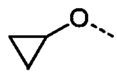, 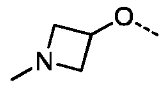,

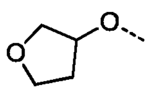, 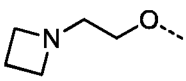 and 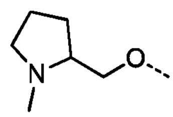,

and other variables are as defined herein.

In some embodiments disclosed herein, the above $R_5$ and $R_6$ are each independently selected from the group consisting of H, Me, Et and

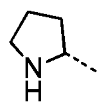,

wherein the Me, Et and

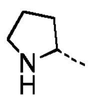

are optionally substituted with 1, 2, or 3 R, and other variables are as defined herein.

In some embodiments disclosed herein, the above $R_5$ and $R_6$ are each independently selected from the group consisting of H,

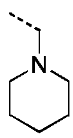, 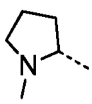 and 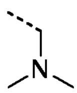,

and other variables are as defined herein.

In some embodiments disclosed herein, the above T is selected from the group consisting of N and C(CN), and other variables are as defined herein.

In some embodiments disclosed herein, the above L is selected from the group consisting of -O-, -NH-, $-CH_2-$ $-CH_2-O-$ and $-CH_2-NH-$, and other variables are as defined herein.

In some embodiments disclosed herein, the above moiety

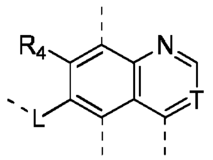

is selected from the group consisting of

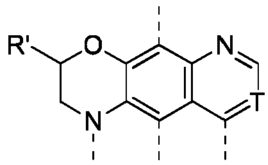
,
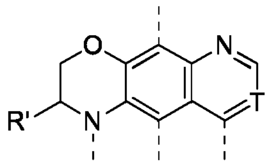
,

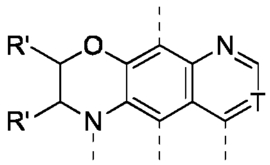
,
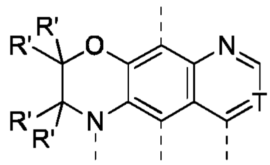
and
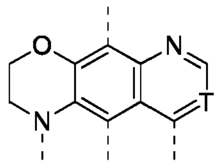
,

and other variables are as defined herein.

In some embodiments disclosed herein, the above moiety

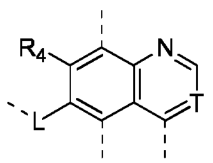

is

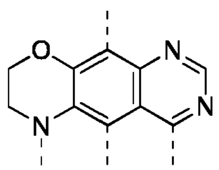
,

and other variables are as defined herein.

In some embodiments disclosed herein, the above ring A is selected from the group consisting of phenyl, azaindenyl, benzo[b]thienyl and benzo[d]isoxazolyl, and other variables are as defined herein.

In some embodiments disclosed herein, the above moiety

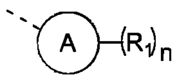

is selected from the group consisting of

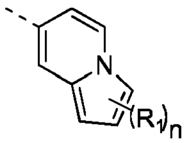 , 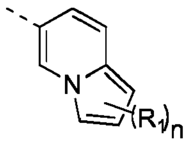 , 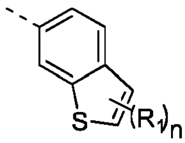 ,

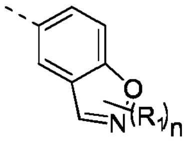 and 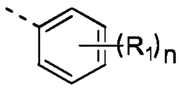 ,

and other variables are as defined herein.

In some embodiments disclosed herein, the above moiety

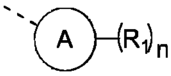

is selected from the group consisting of

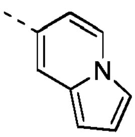 , 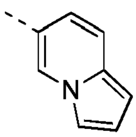 , 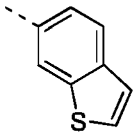 , 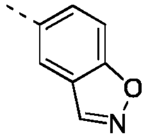 ,

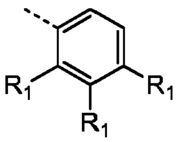 , 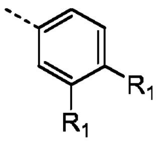 and 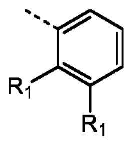 ,

and other variables are as defined herein.

In some embodiments disclosed herein, the above moiety

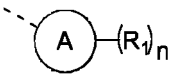

is selected from the group consisting of

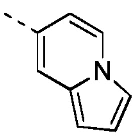 , 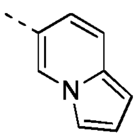 , 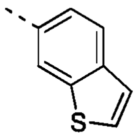 , 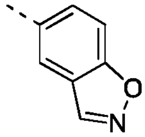 ,

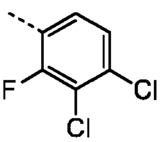 , 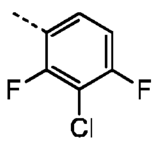 , 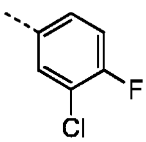 , 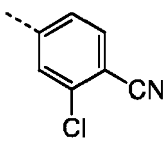 , 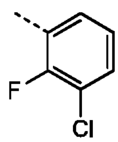

and

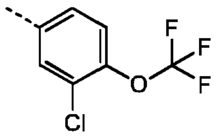,

and other variables are as defined herein.

In some embodiments disclosed herein, the above ring B is selected from the group consisting of

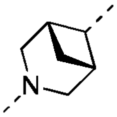, 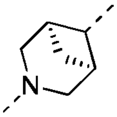, 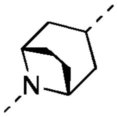, 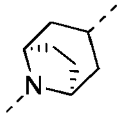, 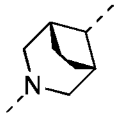, 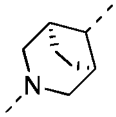,

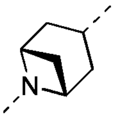, 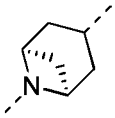, 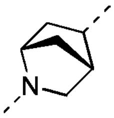, 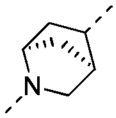, 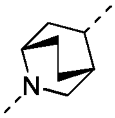, 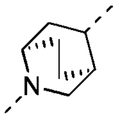, 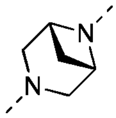, 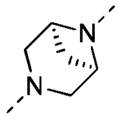,

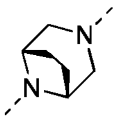 and 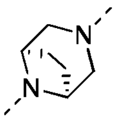,

and other variables are as defined herein.

In some embodiments disclosed herein, the above moiety

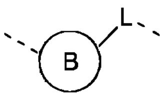

is selected from the group consisting of

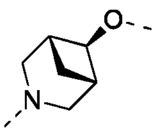, 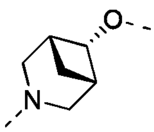, 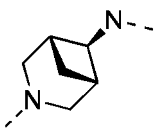, 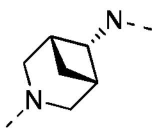,

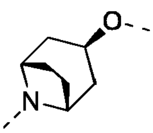 and 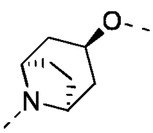,

and other variables are as defined herein.

The present disclosure also includes some embodiments that are obtained by combination of any above definitions of the above variables.

In some embodiments disclosed herein, the above compounds, stereoisomers thereof or pharmaceutically acceptable salts thereof are selected from the group consisting of:

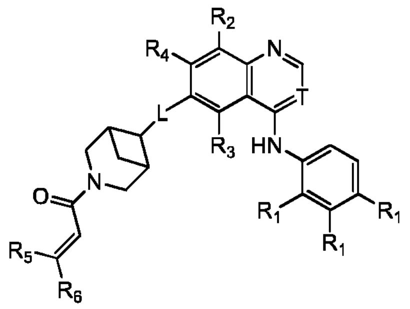

(Ⅱ -1) ,

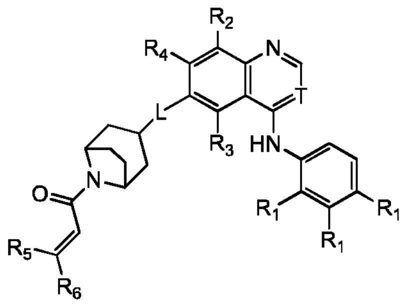

(Ⅱ -2) ,

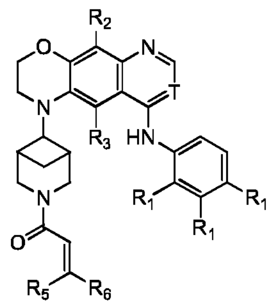

(Ⅱ -3) ,

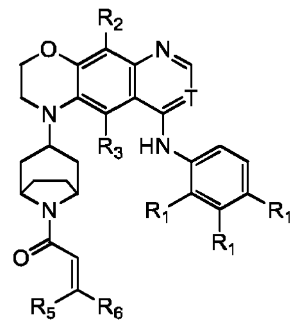

(Ⅱ -4) ,

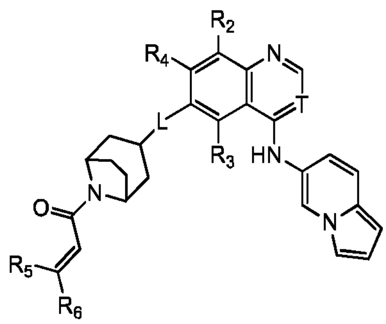

(Ⅱ -5) ,

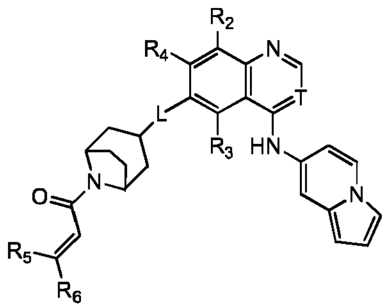

(Ⅱ -6) ,

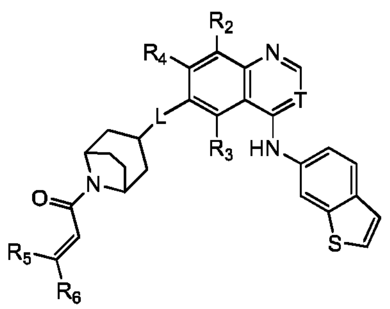

(Ⅱ -7) ,

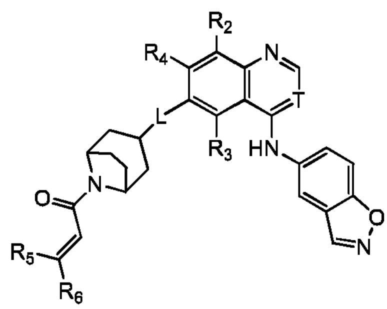

(Ⅱ -8) ,

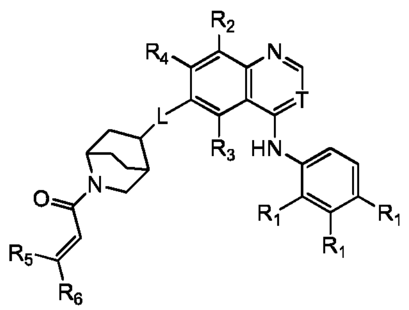

(Ⅱ -9) ,

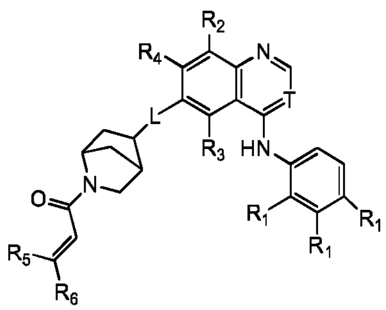

(Ⅱ -10)

and

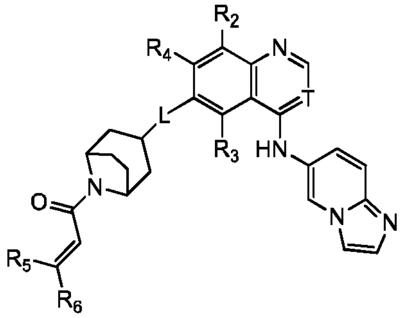

(II -11) ,

wherein T, L, $R_1$, $R_2$, $R_3$, $R_4$, $R_5$, and $R_6$ are as defined herein.

In some embodiments disclosed herein, the above compounds, stereoisomers thereof or pharmaceutically acceptable salts thereof are selected from the group consisting of:

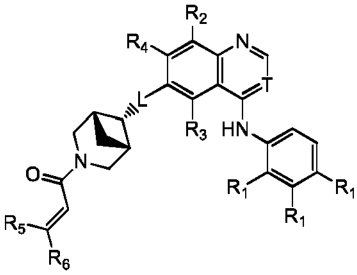

( III-1) ,

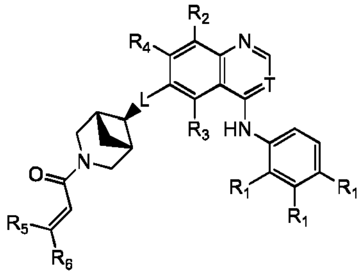

( III-2)

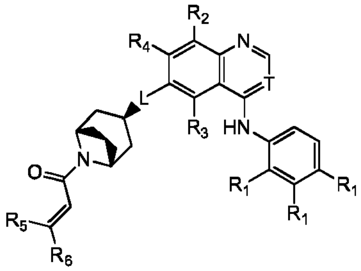

( III-3) ,

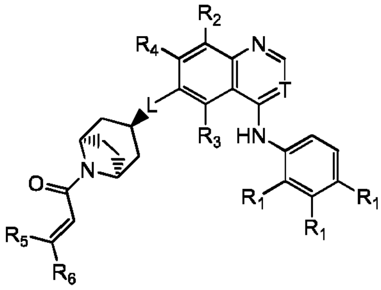

( III-4) ,

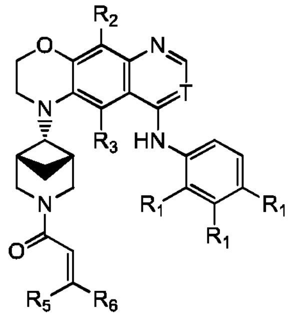

(III-5) ,

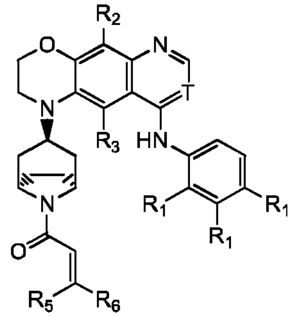

( III-6) ,

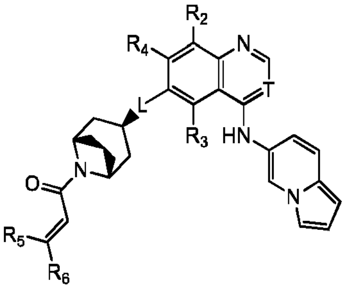

(III-7) ,

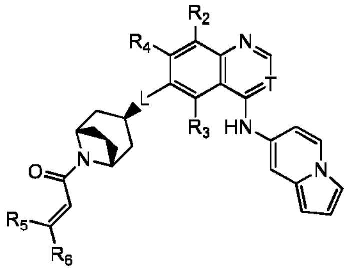

(III-8) ,

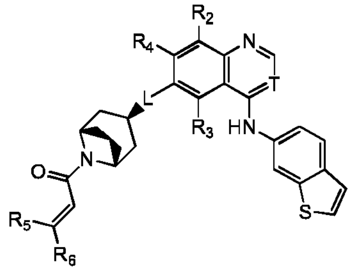

(III-9) ,

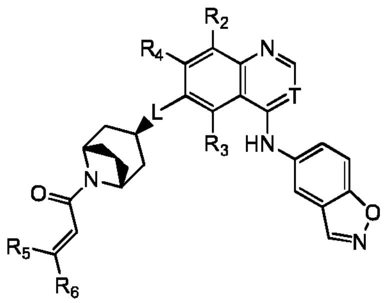

(III-10) ,

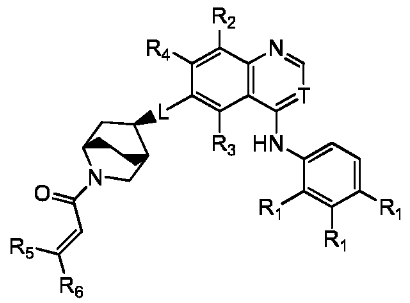

(III-11) ,

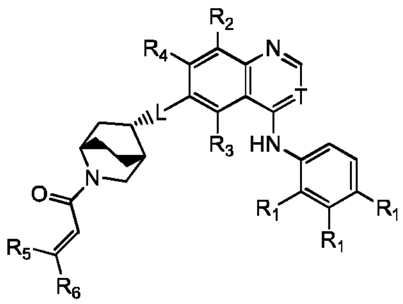

(III-12)

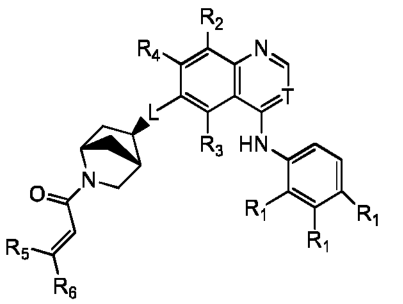

(III-13) ,

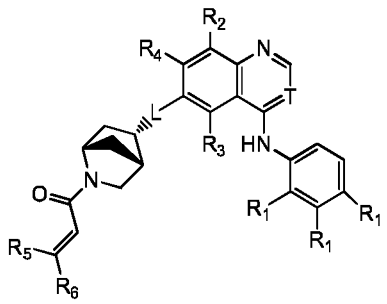

(III-14) ,

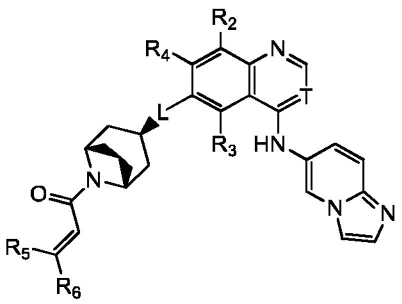

(III-15)

and

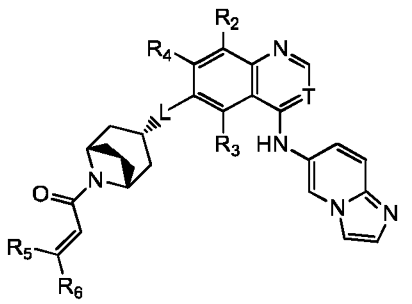

(III-16)

wherein T, L, $R_1$, $R_2$, $R_3$, $R_4$, $R_5$ and $R_6$ are as defined herein.

The present disclosure also provides the following compounds, stereoisomers thereof or pharmaceutically acceptable salts thereof:

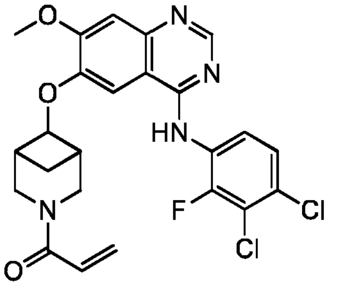

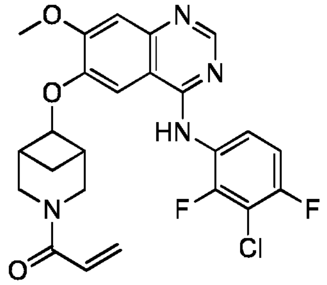

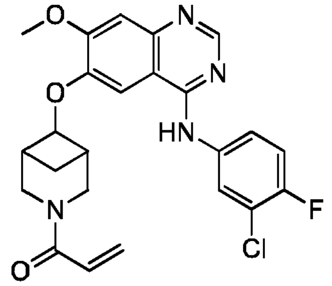

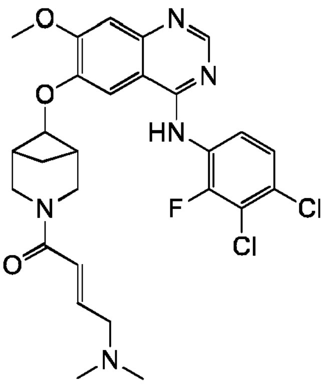

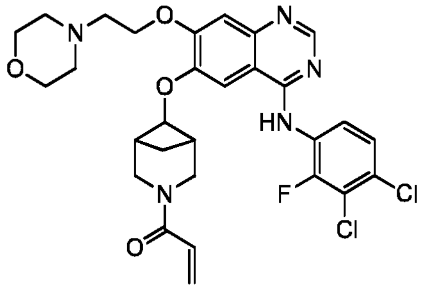

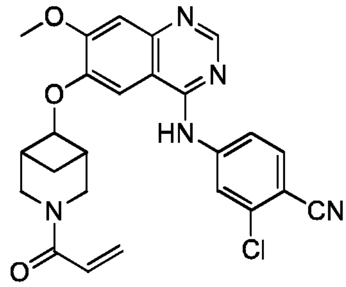

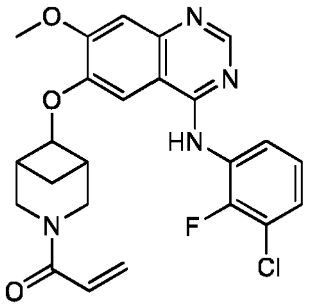

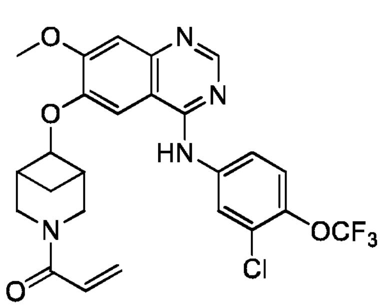

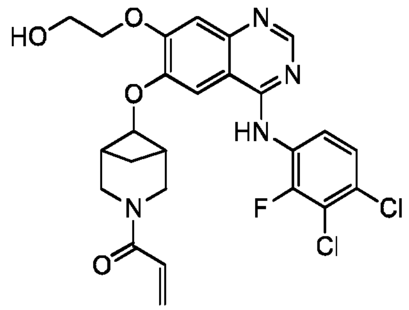

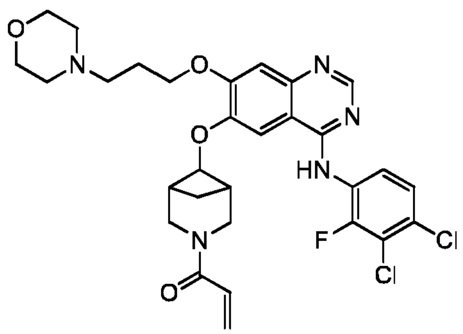

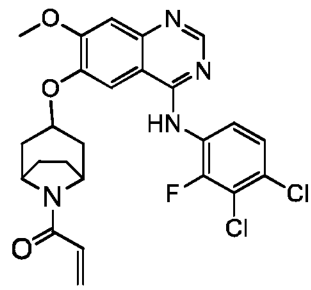

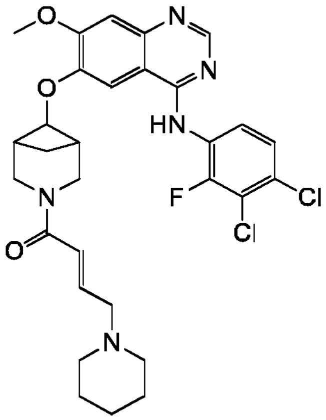

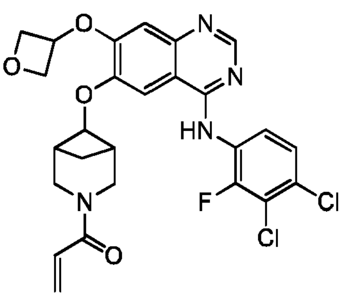

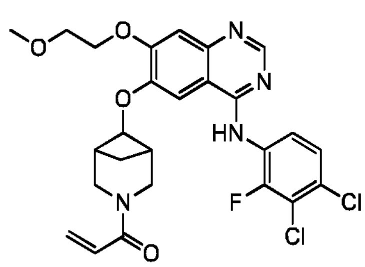

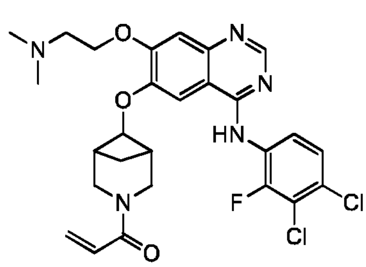

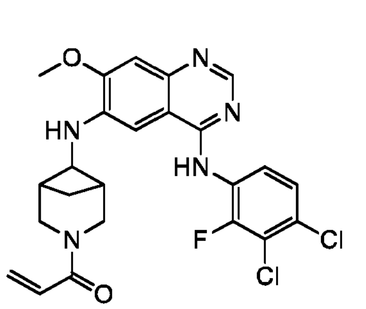
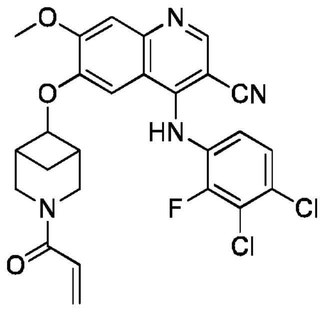
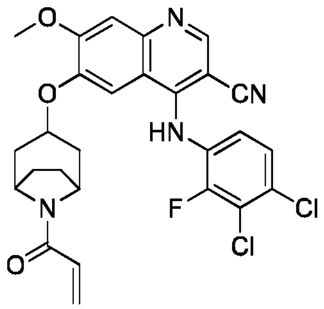
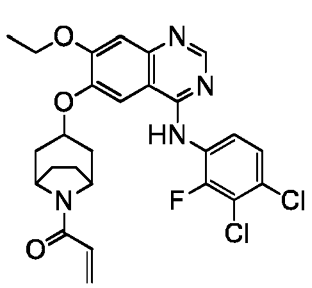
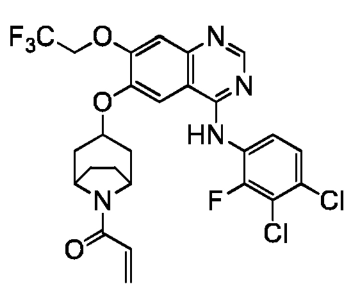
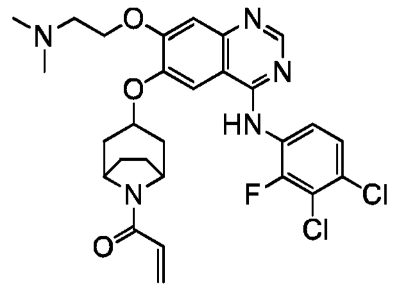
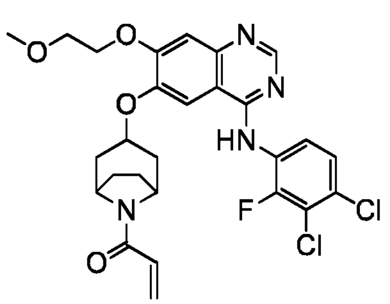
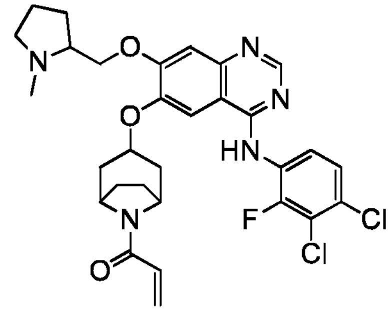
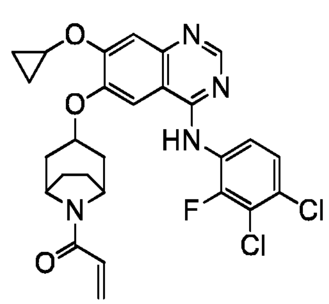
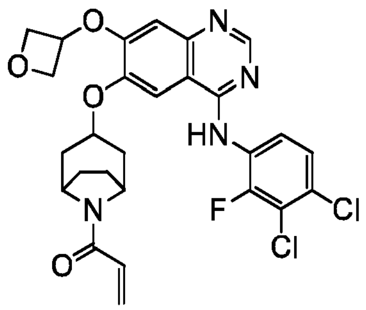
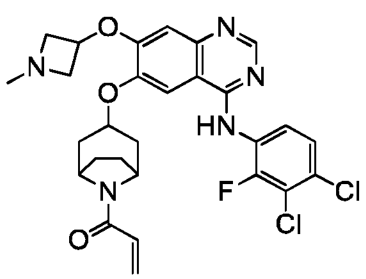
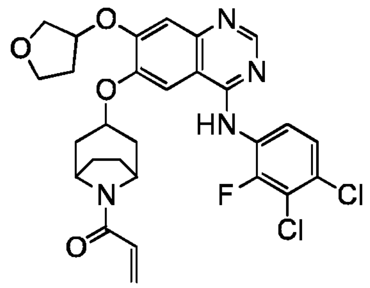
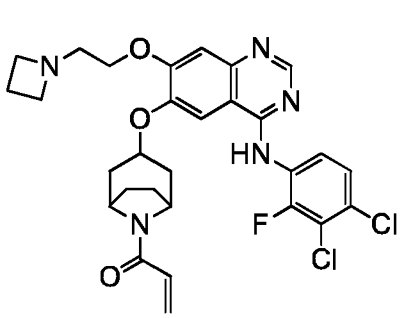
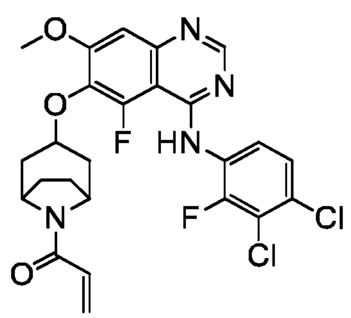
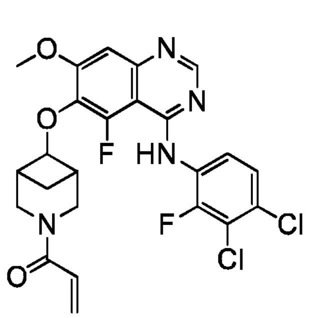

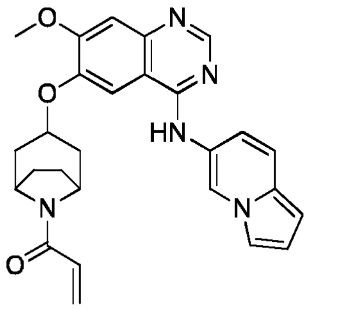
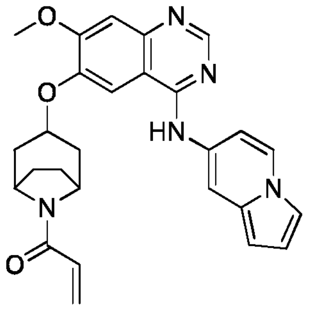
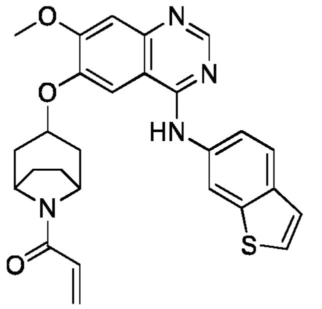

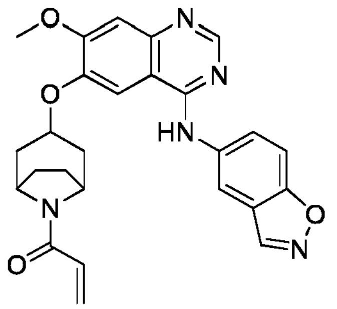
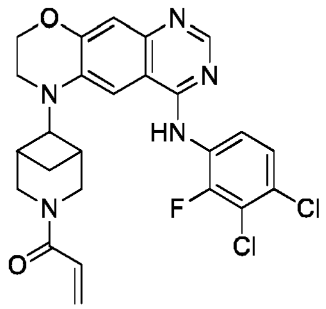
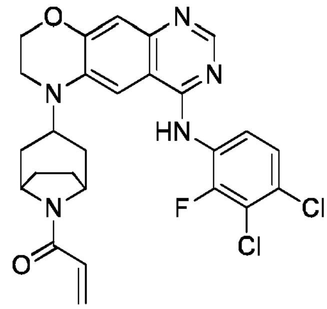

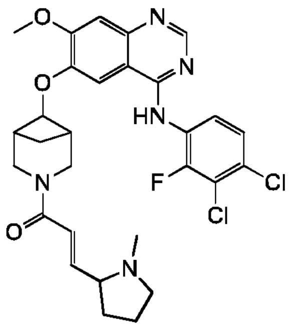
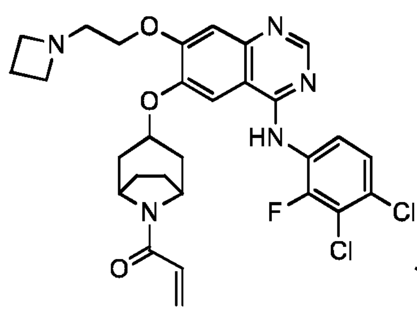
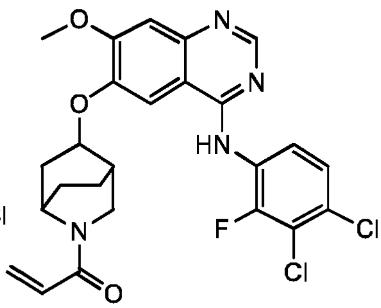

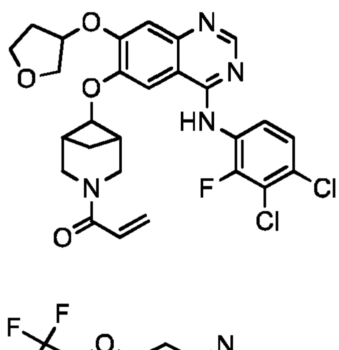
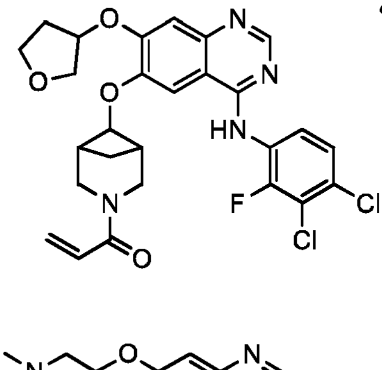
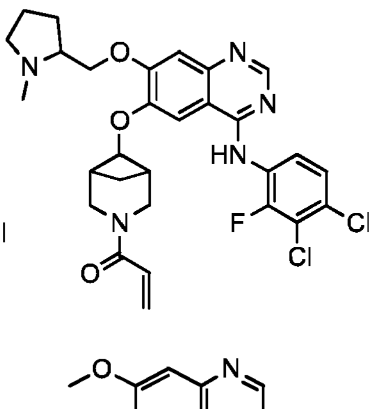

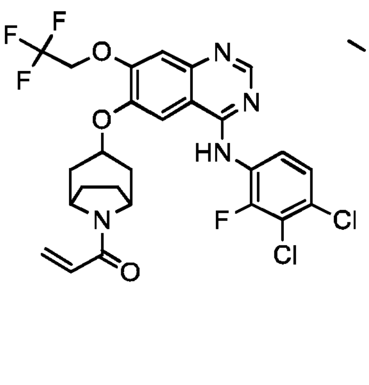
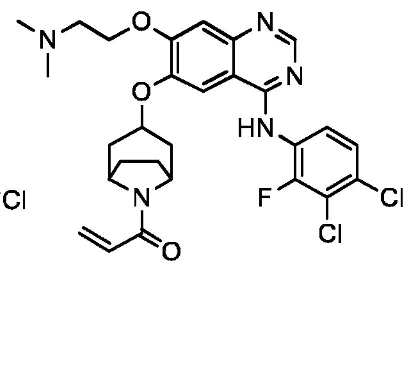
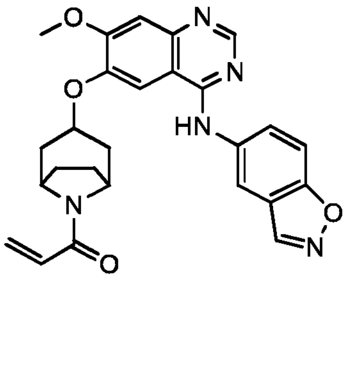

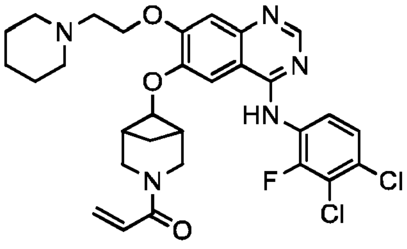
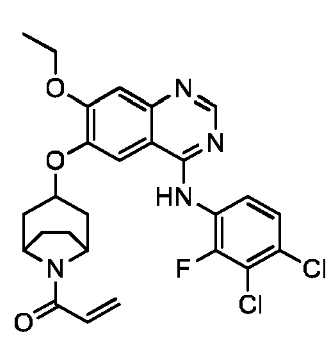
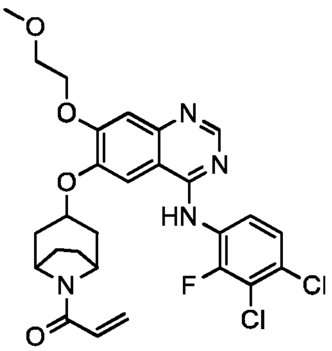

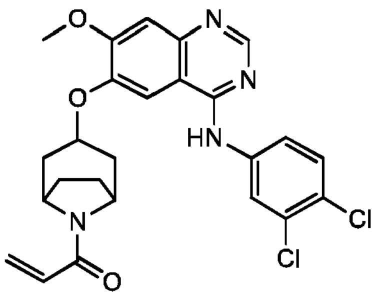
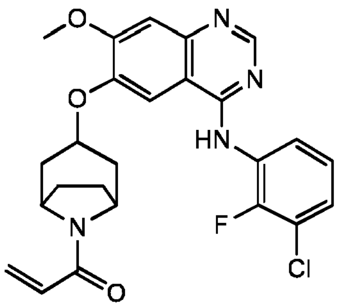
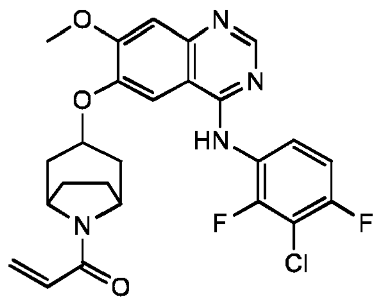

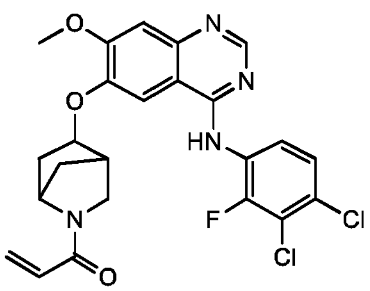
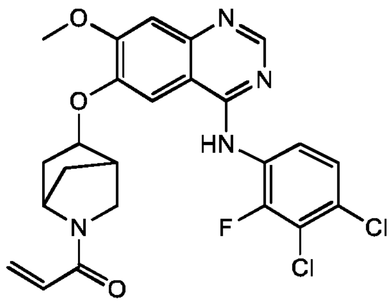
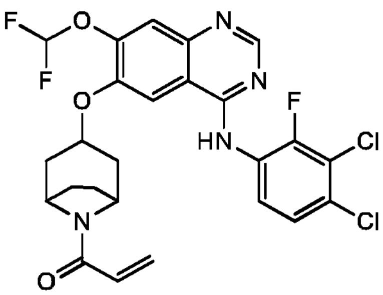

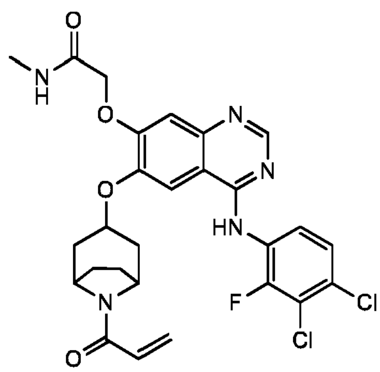
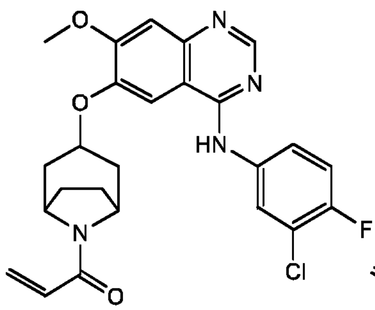
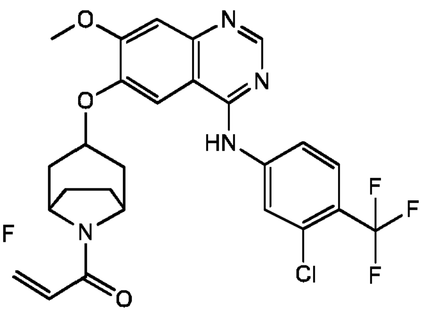

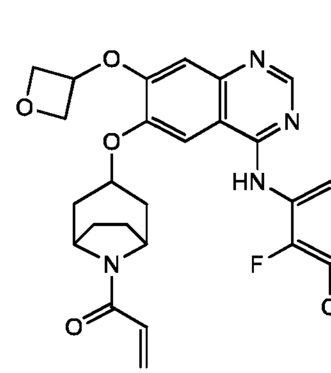

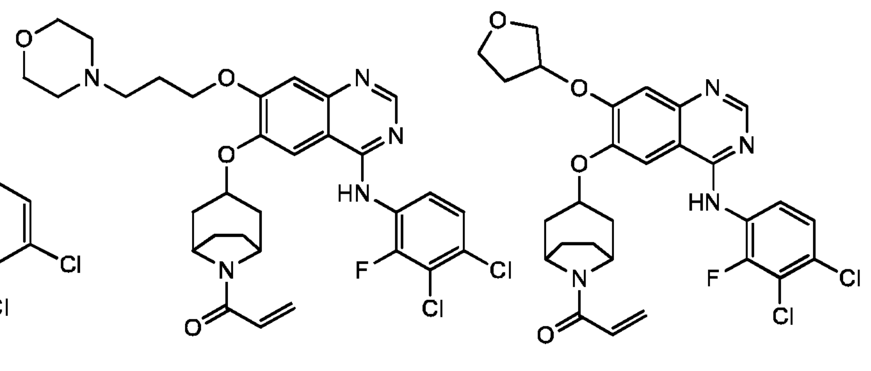

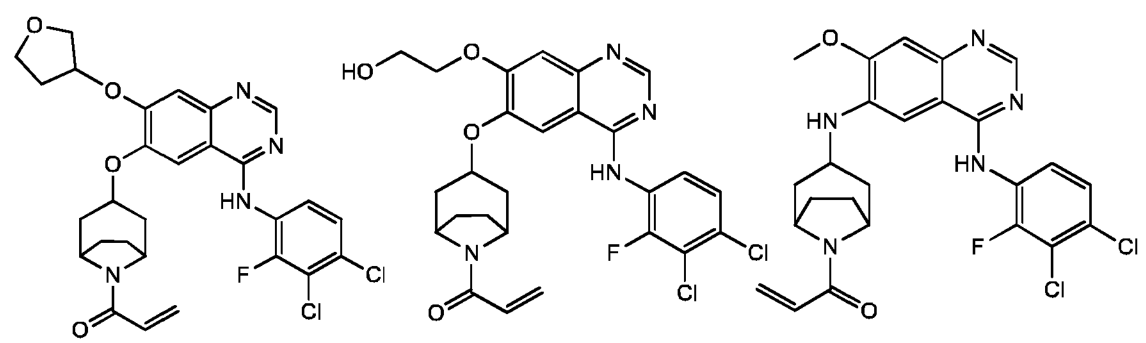

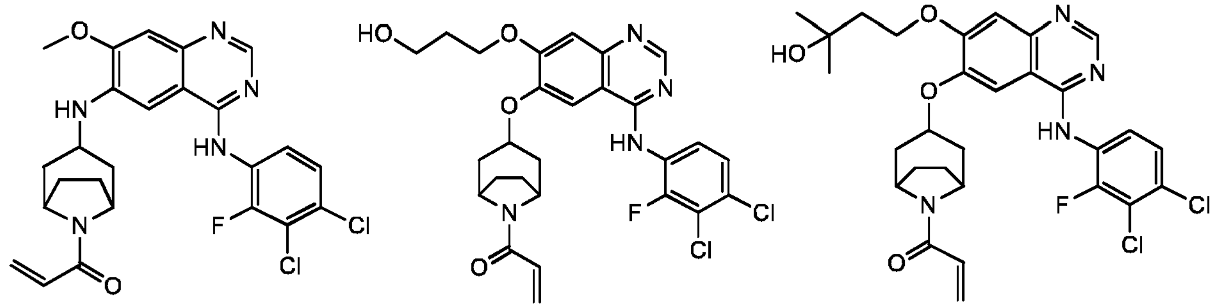

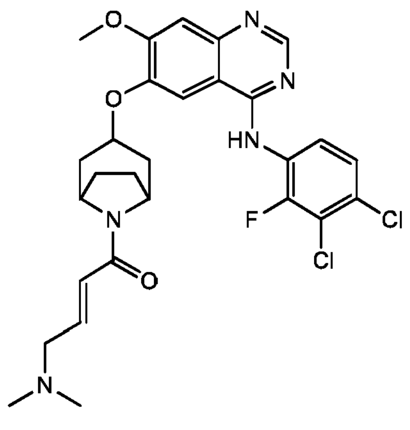

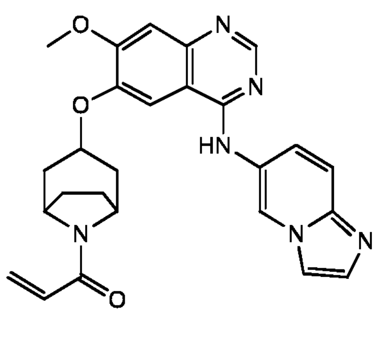

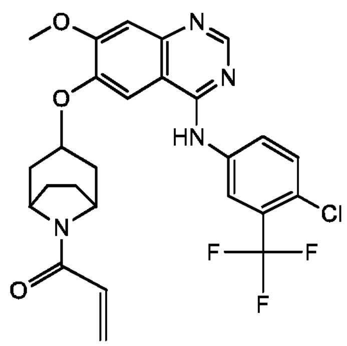

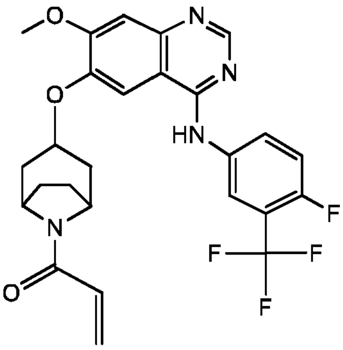 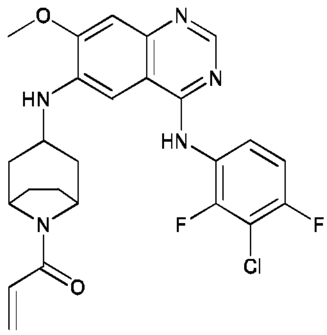

In some embodiments disclosed herein, the above compounds are selected from the group consisting of:

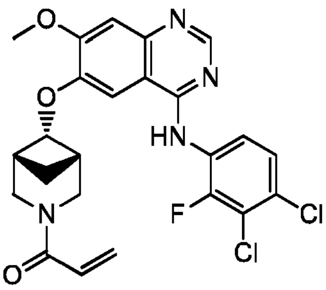 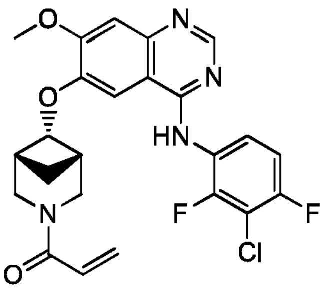 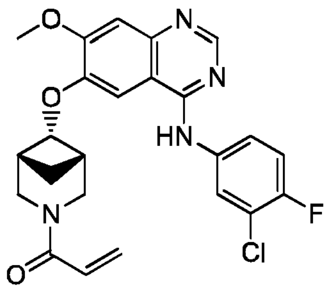

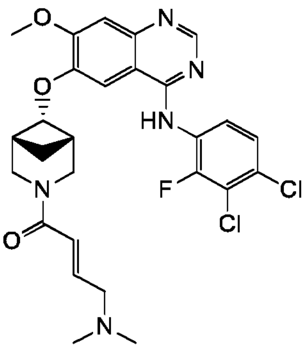 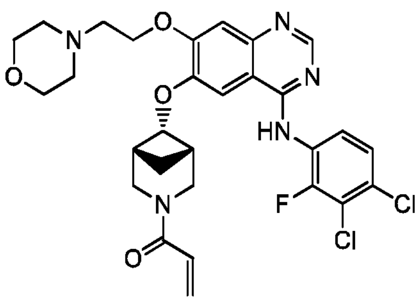 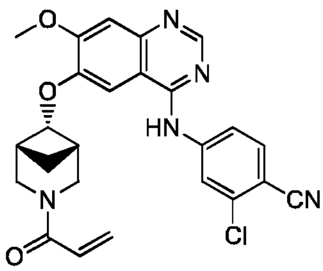

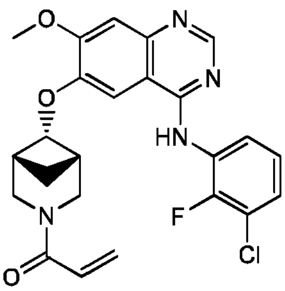 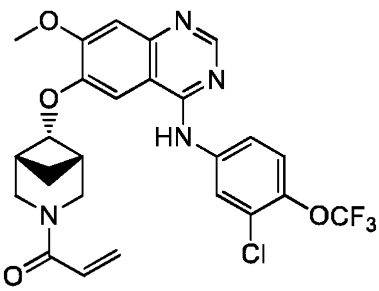 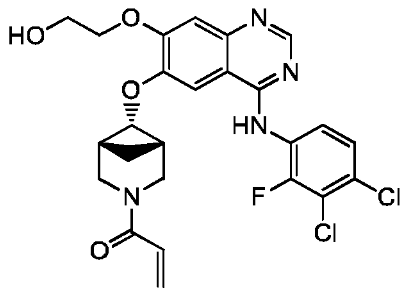

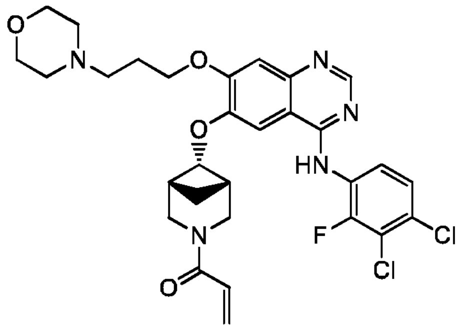

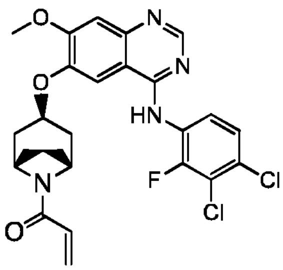

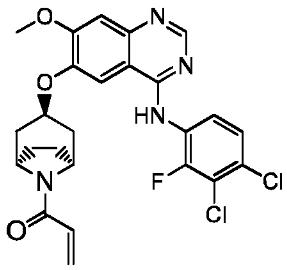

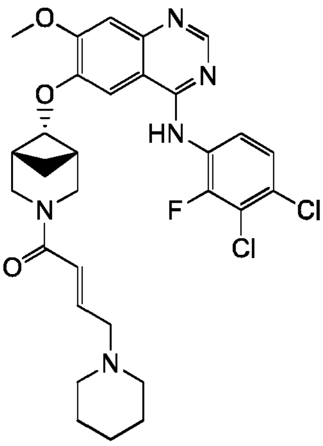

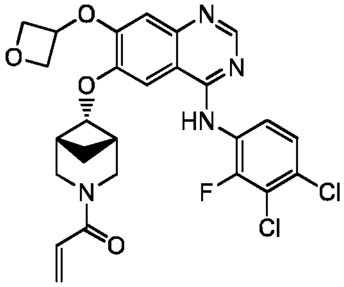

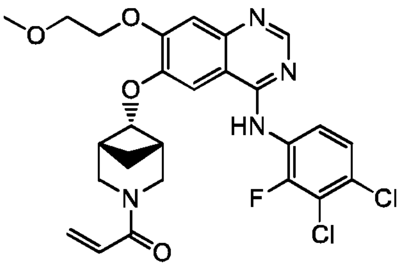

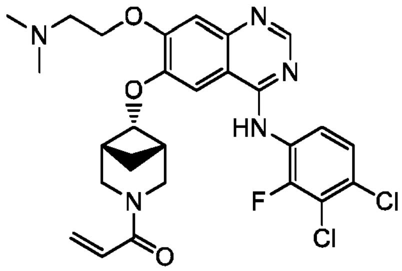

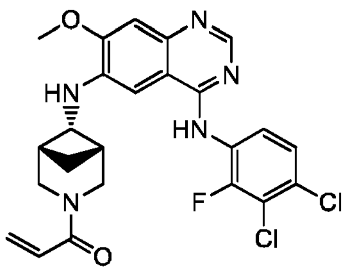

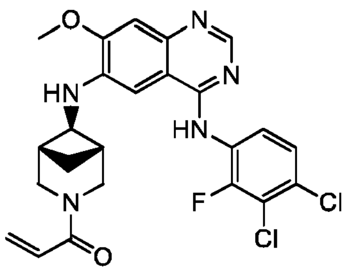

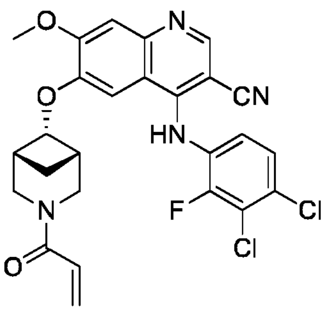

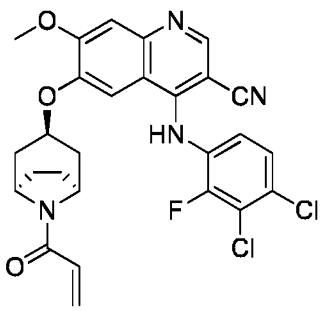

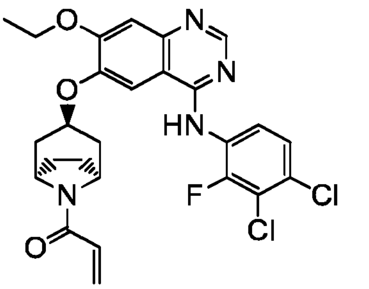

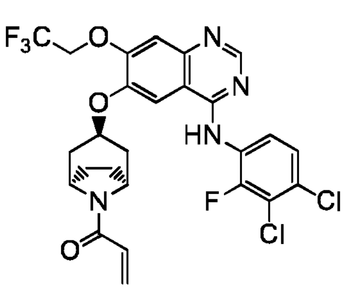

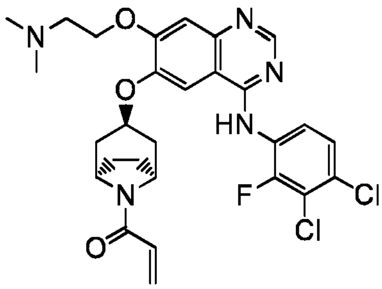

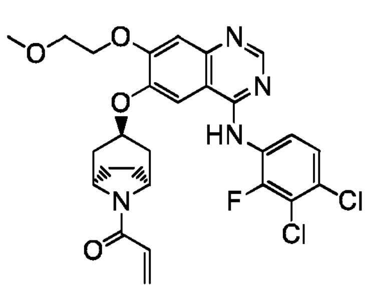

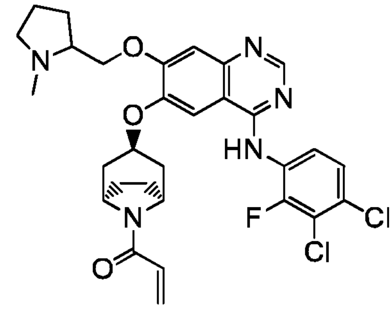

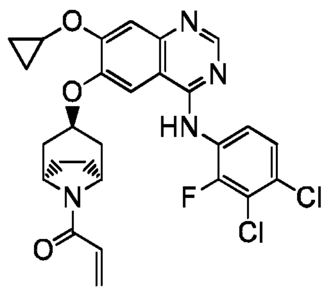

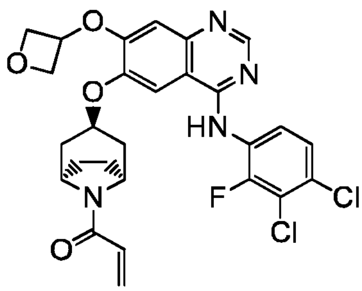

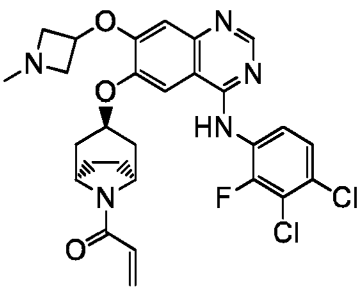

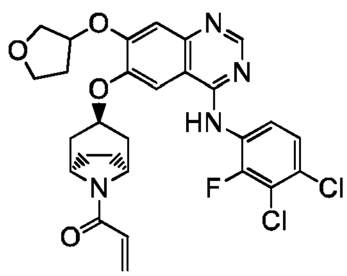

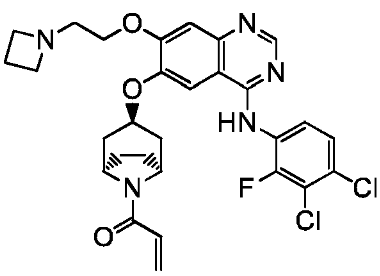

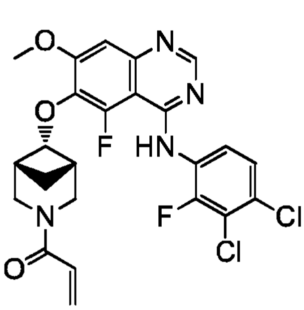

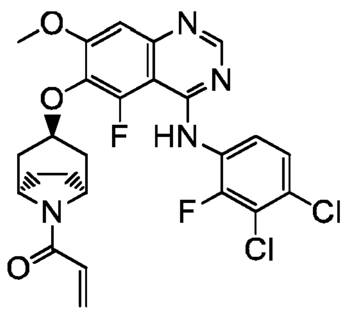

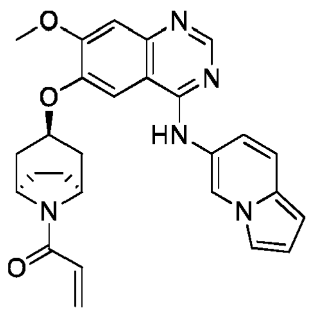

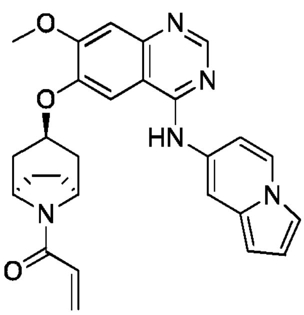

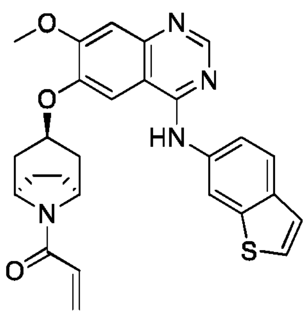

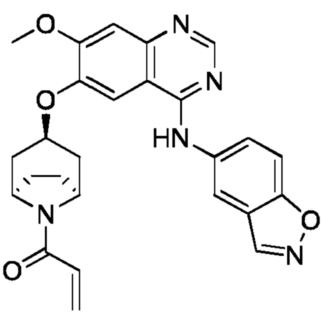

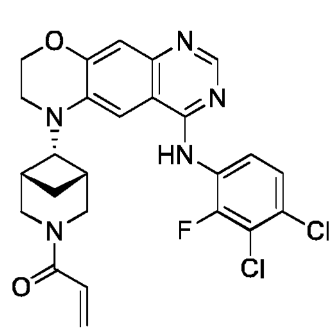
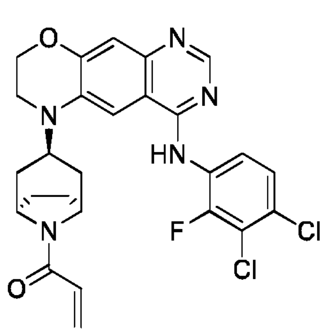
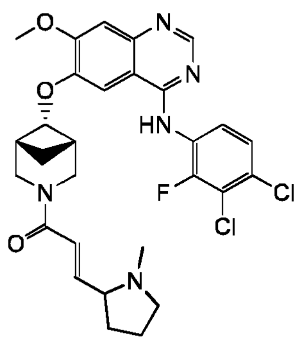

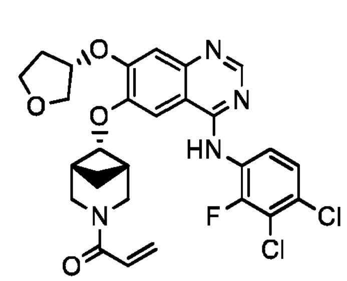
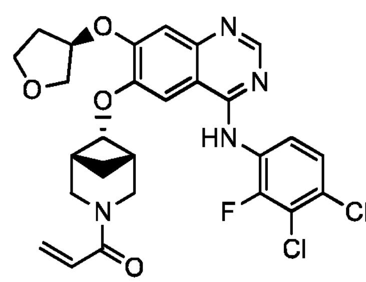
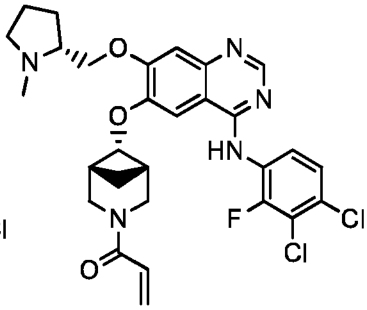

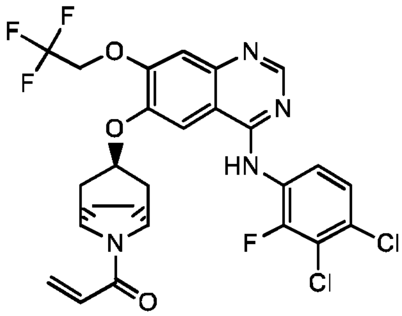
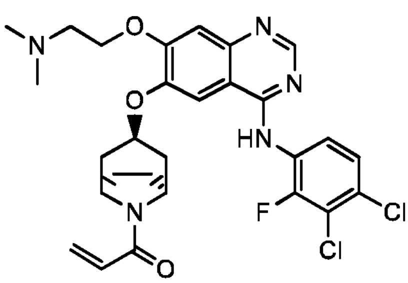
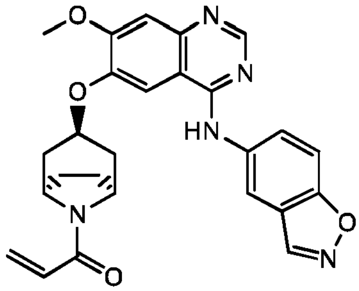

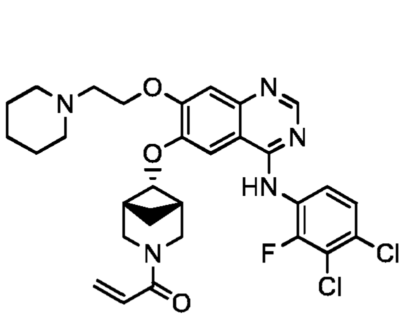
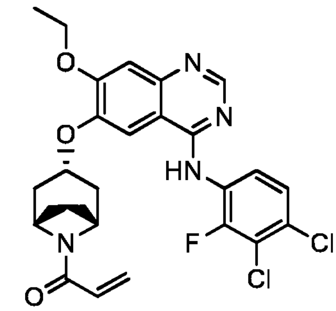
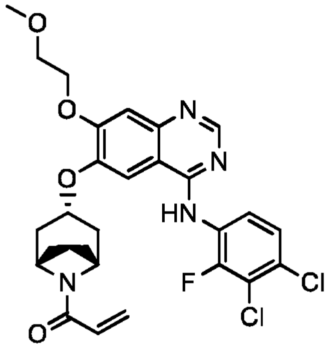

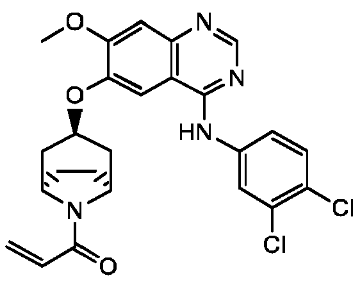
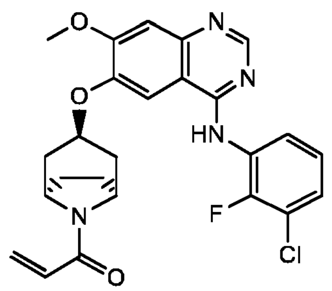
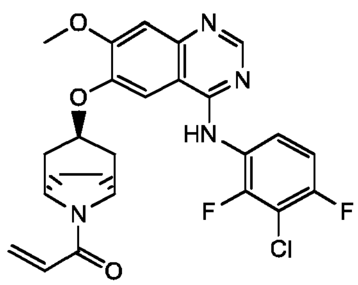

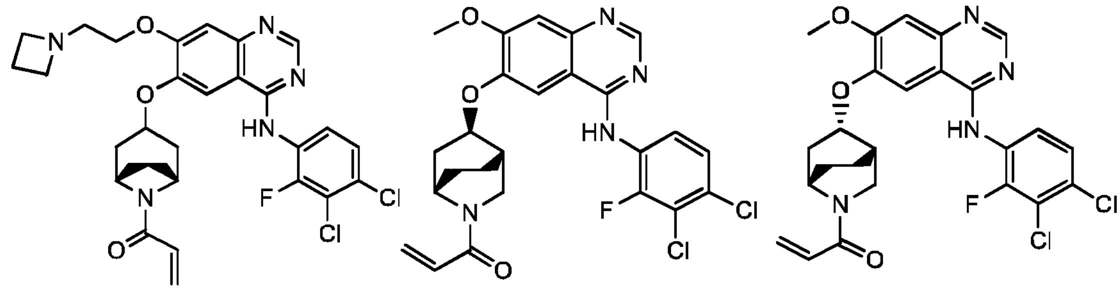

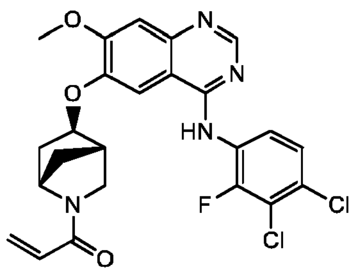

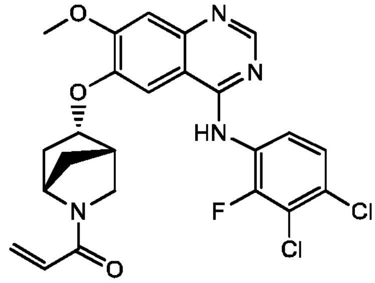

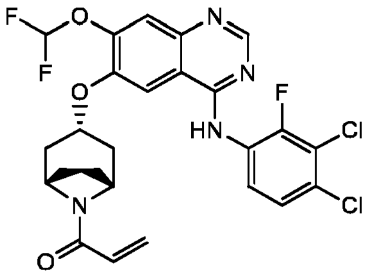

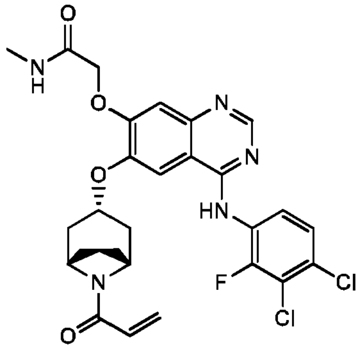

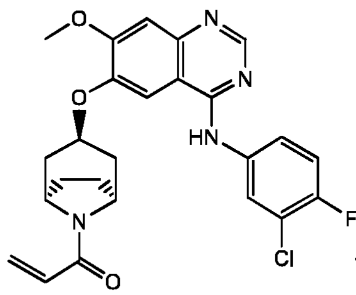

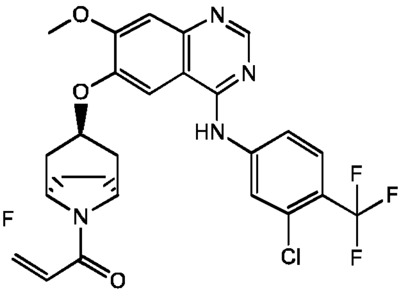

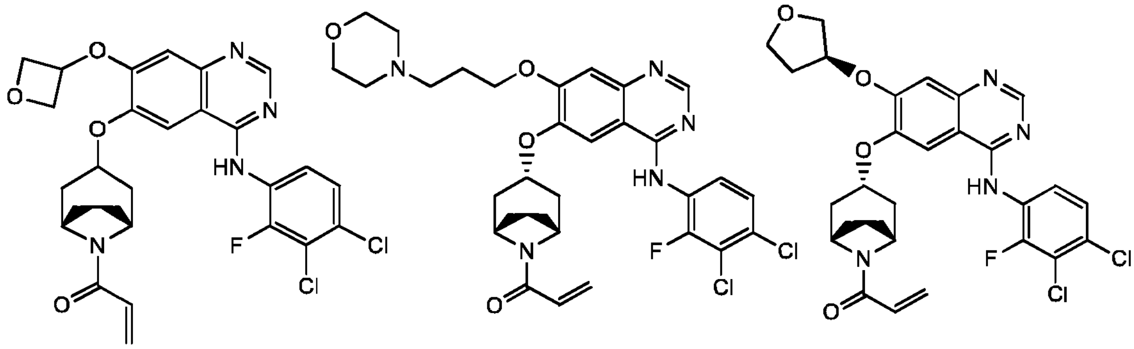

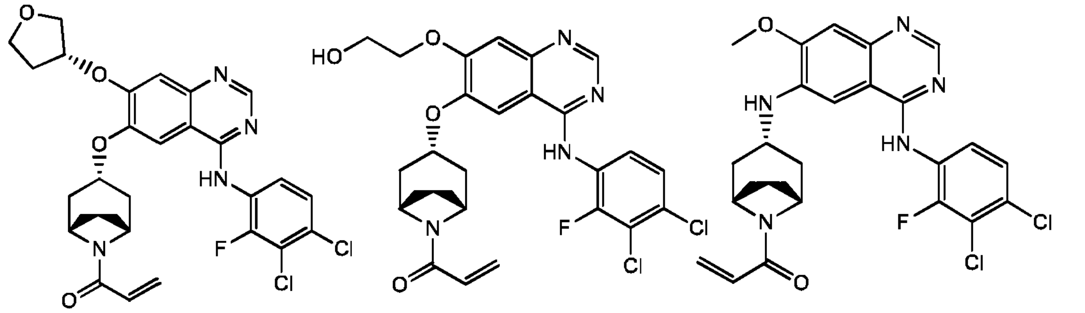

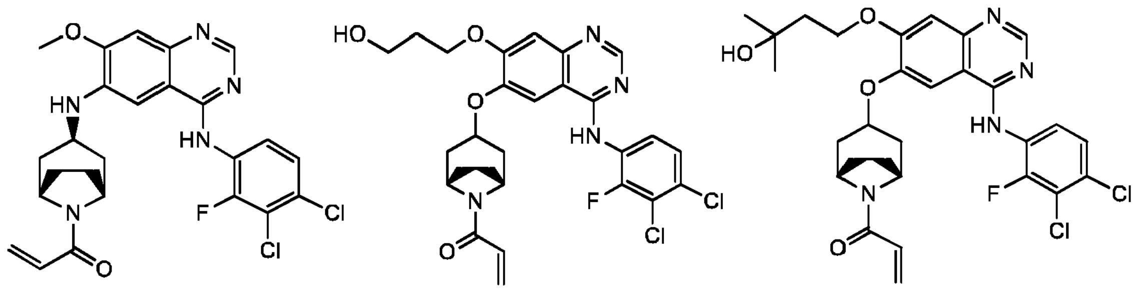

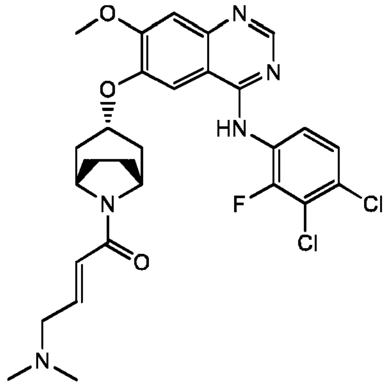

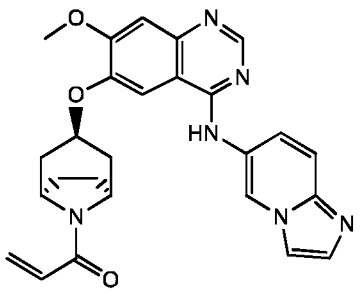

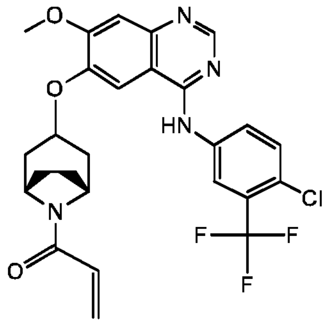

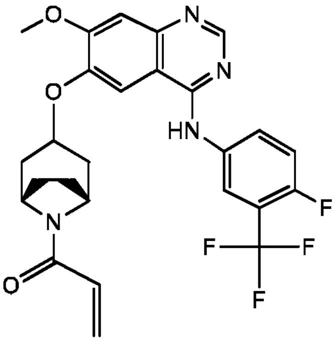

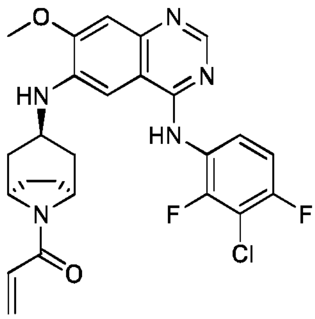

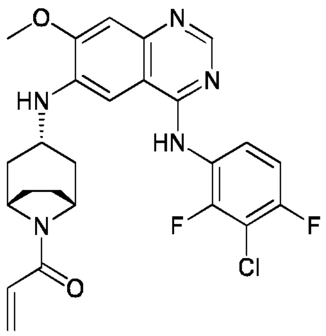

.

The present disclosure also provides a pharmaceutical composition, comprising a therapeutically effective amount of the above compound or a pharmaceutically acceptable salt thereof as an active ingredient, and pharmaceutically acceptable carrier(s).

The present disclosure also provides use of the above compound or a pharmaceutically acceptable salt thereof or the above composition in the manufacture of a medicament associated with a Pan-HER tyrosine kinase inhibitor.

**Definitions and Terms**

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" refers to a salt of the compound disclosed herein that is prepared by reacting the compound having a specific substituent disclosed herein with a relatively non-toxic acid or base. When the compound disclosed herein contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound disclosed herein contains a relatively basic

functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compound disclosed herein may be present in a specific geometric or stereoisomeric form. The present disclosure contemplates all such compounds, including cis and trans isomer, (-)- and (+)-enantiomer, (R)- and (S)-enantiomer, diastereoisomer, (D)-isomer, (L)-isomer, and racemic mixture and other mixtures, for example, an enantiomer or diastereoisomer enriched mixture, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" refers to stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" refers to a stereoisomer in which two or more chiral centers of are contained in a molecule and is in a non-mirrored relationship between molecules.

Unless otherwise specified, "(D)" or "(+)" means dextroisomer, "(L)" or "(-)" means levoisomer, and "(DL)" or "(±)" means racemate.

Unless otherwise specified, a wedged solid bond (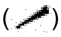) and a wedged dashed bond (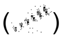) indicate the absolute configuration of a stereocenter; a straight solid bond (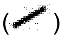) and a straight dashed bond (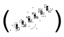) indicate the relative configuration of a stereocenter; a wavy line (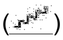) indicates a wedged solid bond (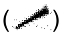) or a wedged dashed bond (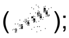); or a wavy line (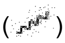) indicates a straight solid bond (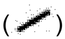) and a straight dashed bond (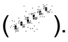).

The compounds disclosed herein may be present in a particular form. Unless otherwise specified, the terms "tautomer" or "tautomeric form" means that different functional groups are in dynamic equilibrium at room temperature and can be rapidly converted into each other. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, 98% or more, 99% or more, 99.5% or more, 99.6% or more, 99.7% or more, 99.8% or more, or 99.9% or more.

Unless otherwise specified, the term "isomer excess" or "enantiomeric excess" refers to the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (R)- and (S)-isomer, or D and L isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase

and optionally combines with a chemical derivative method (for example, carbamate generated from amine).

The compounds disclosed herein may contain an unnatural proportion of atomic isotopes at one or more of the atoms that make up the compounds. For example, a compound may be labeled with a radioisotope such as tritium ($^3H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "pharmaceutically acceptable carrier" refers to any formulation or carrier medium that is capable of delivering an effective amount of an active substance disclosed herein, which does not interfere with the biological activity of an active substance, and has no toxic side effects to the host or patient. Representative carriers include water, oil, vegetables, minerals, cream bases, lotion bases, ointment bases and the like. These bases include suspending agents, tackifiers, transdermal enhancers, etc. Their formulations are well known to those skilled in cosmetic or topical pharmaceutical arts.

"Optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e. =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary as long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variable is a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When an enumerative substituent does not indicate through which atom it is linked to the substituted group, such substituent can be bonded through any of its atoms. For example, a pyridyl group as a substituent may be linked to the substituted group through any one of carbon atoms on the pyridine ring. When an enumerative linking group does not indicate its linking direction, its linking direction is arbitrary. For example, when the linking group L in

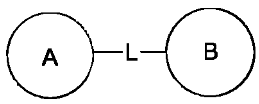

is -M-W-, the -M-W- can be linked to the ring A and the ring B in the same direction as the reading order from left to right to constitute

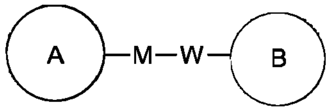

,

or can be linked to the ring A and the ring B in the reverse direction as the reading order from left to right to constitute

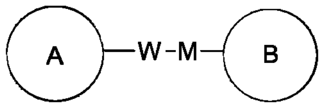

.

A combination of the linking groups, substituents and/or variants thereof is allowed only when such combination can result in a stable compound.

Unless otherwise specified, the term "hetero" represents a heteroatom or a heteroatom group (e.g., an atom group containing a heteroatom), including the atom except carbon (C) and hydrogen (H) and the atom group containing the above heteroatom, for example, including oxygen (O), nitrogen (N), sulfur (S), silicon (Si), germanium (Ge), aluminum (Al), boron (B), -O-, -S-, =O, =S, -C(=O)O-, -C(=O)-, -C(=S)-, -S(=O), $-S(=O)_2-$, and the group consisting of -C(=O)N(H)-, -N(H)-, -C(=NH)-, $-S(=O)_2$ N(H)- and -S(=O)N(H)-, each of which is optionally substituted.

Unless otherwise specified, the term "ring" refers to a substituted or unsubstituted cycloalkyl, heterocycloalkyl, cycloalkenyl, heterocycloalkenyl, cycloalkynyl, heterocycloalkynyl, aryl or heteroaryl. The so-called ring includes a single ring, a ring assembly, a spiral ring, a fused ring or a bridged ring. The number of the atom on the ring is usually defined as the member number of the ring, for example, a "5-7 membered ring" means that 5 to 7 atoms are arranged on a ring. Unless otherwise specified, the ring optionally contains 1 to 3 heteroatoms. Therefore, a "5-7 membered ring" includes, for example, phenyl, pyridyl and piperidyl; on the other hand, the term "5-7 membered heterocycloalkyl ring" includes pyridyl and piperidyl, but excluding phenyl. The term "ring" also includes a ring system containing at least one ring, wherein each ring independently meets the above definition.

Unless otherwise specified, the term "heterocycle" or "heterocyclyl" refers to a stable monocyclic, bicyclic or tricyclic ring containing a heteroatom or a heteroatom group, which can be saturated, partially unsaturated or unsaturated (aromatic) and can contain carbon atoms and 1, 2, 3 or 4 ring heteroatoms independently selected from the group consisting of N, O and S, wherein any of the above heterocycle can be fused to a benzene ring to form a bicyclic ring. Nitrogen and sulfur heteroatoms can optionally be oxidized (i.e., NO and $S(O)_p$, p is 1 or 2). Nitrogen atom can be substituted or unsubstituted (i.e., N or NR, wherein R is H or other substituents already defined herein). The heterocycle can be attached to the pendant group of any heteroatom or carbon atom to form a stable structure. If the resulting compound is stable, the heterocycle described herein may have a substitution at a carbon or nitrogen position. Nitrogen atom on the heterocycle is optionally quaternized. In a preferred embodiment, when the total number of S and O atom of the heterocycle is more than 1, the heteroatom is not adjacent to each other. In another preferred embodiment, the total number of S and O atom of the heterocycle is not more than 1. As used herein, the term "aromatic heterocyclic group" or "heteroaryl" refers to a stable 5-, 6- or 7-membered monocyclic or bicyclic or 7-, 8-, 9- or 10-membered bicyclic heterocyclic aromatic ring which contains carbon atoms and 1, 2, 3 or 4 ring heteroatoms independently selected from the group consisting of N, O and S. Nitrogen atom can be substituted or unsubstituted (i.e., N or NR, wherein R is H or other substituents already defined herein). Nitrogen and sulfur heteroatoms may optionally be oxidized (i.e., NO and $S(O)_p$, p is 1 or 2). It is worth noting that the total number of S and O atom of an aromatic heterocycle is not more than one. The bridged ring is also included in the definition of the heterocycle. A bridged ring is formed when one or more than one atom (i.e, C, O, N or S) link two non-adjacent carbon or nitrogen atoms. A preferred bridged ring includes, but not limited to one carbon atom, two carbon atoms, one nitrogen atom, two nitrogen atoms and one carbon-nitrogen group. It is worth noting that a bridge always converts a monocyclic ring to a tricyclic ring. In a bridged ring, the substituent on the ring may also be present on the bridge.

Examples of the heterocyclic compound include, but are not limited to: azetidinyl, azocinyl, benzimidazolyl, benzofuryl, benzomercaptofuryl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-b]tetrahydrofuryl, furyl, furazanyl, imidazolidinyl, imidazolinyl, imidazolyl, 1*H*-indazolyl, indolenyl, indolinyl, indolizinyl, indolyl, 3*H*-indolyl, isobenzofuryl, isoindolyl, isoindolinyl, isoquinolinyl, isothiazolyl, isoxazolyl, methylenedioxyphenyl, morpholinyl, naphthyridinyl, octahydro-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, hydroxindolyl, pyrimidinyl, phenanthridinyl, phenanthrolinyl, phenazine, phenothiazine, benzoxanthinyl, phenoloxazinyl, phthalazinyl, piperazinyl, piperidyl, piperidonyl, 4-piperidonyl, piperonyl, pteridinyl, purinyl, pyranyl, pyrazinyl, pyrazolidinyl, pyrazolinyl, pyrazolyl, pyridazinyl, pyrido-oxazolyl, pyrido-imidazolyl, pyrido-thiazolyl, pyridyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuryl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thieno-oxazolyl, thieno-thiazolyl, thieno-imidazolyl, thienyl, triazinyl, 1H-1,2,3-triazolyl, 2H-1,2,3-triazolyl, 1H-1,2,4-triazolyl, 4H-1,2,4-triazolyl and xanthenyl. Fused-ring compounds and spiro compounds are also included.

Unless otherwise specified, the term "hydrocarbyl" or its hyponyms (e.g. alkyl, alkenyl, alkynyl, and aryl, etc.), by itself or as part of another substituent, refers to a linear, branched chain or cyclic hydrocarbon radical or any combination thereof. They can be fully saturated (e.g. alkyl), mono- or polyunsaturated (e.g. alkenyl, alkynyl, and aryl), can be mono-, di- or poly-substituted, can be monovalent (e.g. methyl), divalent (e.g. methylene) or multivalent (e.g. methenyl), can also include a divalent or multivalent group, have a specified number of carbon atom (for example, $C_1$-$C_{12}$ indicates 1 to 12 carbon atoms, $C_{1-12}$ is selected from the group consisting of $C_1$, $C_2$, $C_3$, $C_4$, $C_5$, $C_6$, $C_7$, $C_8$, $C_9$, $C_{10}$, $C_{11}$ and $C_{12}$; $C_{3-12}$ is selected from the group consisting of $C_3$, $C_4$, $C_5$, $C_6$, $C_7$, $C_8$, $C_9$, $C_{10}$, $C_{11}$ and $C_{12}$). The term "hydrocarbyl"

includes, but is not limited to aliphatic hydrocarbyl and aromatic hydrocarbyl. The aliphatic hydrocarbyl includes linear and cyclic hydrocarbyl, specifically includes but not limited to alkyl, alkenyl, and alkynyl. The aromatic hydrocarbyl includes but is not limited to 6-12 membered aromatic hydrocarbyl such as phenyl, naphthyl and the like. In some embodiments, the term "hydrocarbyl" refers to a linear or branched group or a combination thereof which can be fully saturated, mono- or polyunsaturated, and can include a divalent or multivalent group. Examples of the saturated hydrocarbyl group include, but are not limited to, methyl, ethyl, n-propyl, isopropyl, n-butyl, tert-butyl, isobutyl, sec-butyl, isobutyl, cyclohexyl, (cyclohexyl)methyl, cyclopropylmethyl, and the homolog or isomer of n-amyl, n-hexyl, n-heptyl, n-octyl and other atom groups. The unsaturated hydrocarbyl has one or more than one double or triple bonds. Examples of the unsaturated alkyl include but are not limited to, vinyl, 2-propenyl, butenyl, crotyl, 2-isopentenyl, 2-(butadienyl), 2,4-pentadienyl, 3-(1,4-pentadienyl), ethynyl, 1- and 3-propynyl, 3-butynyl, and more higher homologs and isomers.

Unless otherwise specified, the term "heterohydrocarbyl" or the specific term thereof (such as heteroalkyl, heteroalkenyl, heteroalkynyl, and heteroaryl, etc.), by itself or as part of another substituent, refers to a stable linear, branched or cyclic hydrocarbon radical or any combination thereof, which has a specified number of carbon atoms and at least one heteroatom. In some embodiments, the term "heteroalkyl" by itself or in combination with another term refers to a stable, linear or branched hydrocarbon radical or a combination thereof, which has a specified number of carbon atoms and at least one heteroatom. In a typical embodiment, a heteroatom is selected from the group consisting of B, O, N and S, wherein nitrogen and sulfur atoms are optionally oxidized and the nitrogen atom is optionally quaternized. The heteroatom or heteroatom group can be located at any interior position of a heterohydrocarbyl, including the position where the hydrocarbyl attaches to the rest part of the molecule. But the terms "alkoxy", "alkylamino" and "alkylthio" are used by the conventional meaning and refer to an alkyl group connected to the rest part of the molecule via an oxygen atom, an amino or a sulfur atom respectively. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-CH_2-CH=N-OCH_3$ and-$CH=CH-N(CH_3)-CH_3$. Up to two consecutive heteroatoms can be present, such as, $-CH_2-NH-OCH_3$.

Unless otherwise specified, the term "cyclohydrocarbyl", "heterocyclohydrocarbyl" or hyponyms thereof (such as aryl, heteroaryl, cycloalkyl, heterocycloalkyl, cycloalkenyl, heterocycloalkenyl, cycloalkynyl, heterocycloalkynyl, etc.) by itself or in combination with another term refers to cyclized "hydrocarbyl" or "heterohydrocarbyl". Furthermore, for heterohydrocarbyl or heterocyclohydrocarbyl (e.g. heteroalkyl, and heterocycloalkyl), one heteroatom can occupy the position where the heterocycle attaches to the remainder position of the molecule. Examples of the cyclohydrocarbyl include, but are not limited to, cyclopentyl, cyclohexyl, 1-cyclohexenyl, 3-cyclohexenyl, cycloheptyl and the like. Non-limiting examples of heterocyclohydrocarbyl include 1-(1,2,5,6-tetrahydropyridyl), 1-piperidyl, 2-piperidyl, 3-piperidyl, 4-morpholinyl, 3-morpholinyl, tetrahydrofuran-2-yl, tetrahydrofuran-3-yl, tetrahydrothiophen-2-yl, tetrahydrothiophen-3-yl, 1-piperazinyl and 2-piperazinyl.

Unless otherwise specified, the term "heterocycloalkyl" by itself or in combination with other terms means cyclized "heteroalkyl". In addition, as far as the "heterocycloalkyl" is concerned, the heteroatom may occupy the connection position of the heterocycloalkyl to the rest of the molecule. In some embodiments, the heterocycloalkyl is a 4- to 6-membered heterocycloalkyl; in other embodiments, the heterocycloalkyl is a 5- to 6-membered heterocycloalkyl. Examples of heterocycloalkyl include, but are not limited to, azetidinyl, oxetanyl, thietanyl, pyrrolidinyl, pyrazolidinyl, imidazolidinyl, tetrahydrothienyl, tetrahydrofuranyl, tetrahydropyranyl, piperidinyl, piperazinyl, morpholinyl, dioxanyl, dithianyl, isoxazolidinyl, isothiazolidinyl, 1,2-oxazinyl, 1,2-thiazinyl, hexahydropyridazinyl, homopiperazinyl, homopiperidinyl or oxepanyl.

Unless otherwise specified, the term "alkyl" refers to a linear chain or branched saturated hydrocarbon group, can be mono-substituted (e.g. $-CH_2F$) or poly-substituted (e.g. $-CF_3$), can be monovalent (e.g. methyl), divalent (e.g. methylene) or multivalent (e.g. methenyl). Examples of alkyl include methyl (Me), ethyl (Et), propyl (such as n-propyl and isopropyl), butyl (such as n-butyl, isobutyl, s-butyl, t-butyl), pentyl (such as n-pentyl, isopentyl, neopentyl) and the like.

Unless otherwise specified, cycloalkyl includes any stable cyclic or polycyclic hydrocarbyl, and any carbon atom thereof is saturated. Cycloalkyl can be mono-substituted or poly-substituted, and can be monovalent, divalent or multivalent. Examples of cycloalkyl include, but are not limited to, cyclopropyl, norbornanyl, [2.2.2]bicyclooctane, [4.4.0] bicyclodecanyl and the like.

Unless otherwise specified, the term "halo" or "halogen" by itself or as part of another substituent refers to fluorine, chlorine, bromine or iodine atom. Furthermore, the term "haloalkyl" is meant to include monohaloalkyl and polyhaloalkyl. For example, the term "halo($C_1$-$C_4$)alkyl" is meant to include, but not limited to, trifluoromethyl, 2,2,2-trifluoroethyl, 4-chlorobutyl, 3-bromopropyl and the like. Examples of haloalkyl include, but not limited to trifluoromethyl, trichloromethyl, pentafluoroethyl and pentachloroethyl.

The term "alkoxy" represents any alkyl defined above having a specified number of carbon atoms attached by an oxygen bridge. Unless otherwise specified, $C_{1-6}$ alkoxy includes $C_1$, $C_2$, $C_3$, $C_4$, $C_5$ and $C_6$ alkoxy. Examples of alkoxy include, but not limited to methoxy, ethoxy, n-propoxy, isopropoxy, n-butoxy, sec-butoxy, tert-butoxy, n-pentyloxy and s-pentoxy.

Unless otherwise specified, the term "aryl" refers to a polyunsaturated aromatic substituent, can be mono-, di- or poly-substituted, can be a monovalent, divalent or multivalent, can be a single ring or a multiple ring (e.g. one to three rings; wherein at least one ring is aromatic), which are fused together or connected covalently. The term "heteroaryl" refers to an aryl (or ring) containing one to four heteroatoms. In an illustrative example, the heteroatom is selected from the group consisting of B, O, N and S, wherein nitrogen and sulfur atoms are optionally oxidized and nitrogen atom is optionally quaternized. A heteroaryl may attach to the rest part of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furyl, thienyl, pyridyl, pyrimidinyl, benzothiazolyl, purinyl, benzimidazolyl, indolyl, isoquinolinyl, quinoxalinyl, quinolinyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolinyl, 5-isoquinolinyl, 2-quinoxalinyl, 5-quinoxalinyl, 3-quinolinyl and 6-quinolinyl. The substituent of any of the above aryl and heteroaryl ring system is selected from the group consisting of the acceptable substituents described below.

Unless otherwise specified, when combined with other terms (such as aryloxy, arylthio, arylalkyl), the aryl includes the aryl and heteroaryl ring as defined above. Thus, the term "aralkyl" is meant to include the group (e.g. benzyl, phenethyl, pyridylmethyl, etc.) where an aryl is attached to an alkyl, including an alkyl where the carbon atom (e.g. methylene) has been replaced by an atom such as oxygen, for example, phenoxymethyl, 2-pyridyloxymethyl, 3-(1-naphthyloxy)propyl, and the like.

The term "leaving group" refers to a functional group or atom which can be replaced by another functional group or atom through a substitution reaction (such as affinity substitution reaction). For example, representative leaving groups include triflate; chlorine, bromine and iodine; sulfonate group, such as mesylate, tosylate, p-bromobenzenesulfonate, p-toluenesulfonate and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g. acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl such as methyl, ethyl and tert-butyl; acyl such as alkanoyl (e.g. acetyl); arylmethyl such as benzyl (Bn), p-methoxybenzyl (PMB), 9-fluorenylmethyl (Fm), and diphenylmethyl (benzhydryl, DPM); silyl such as trimethylsilyl (TMS) and tert-butyl dimethyl silyl (TBS) and the like.

The compound disclosed herein can be prepared by a variety of synthetic methods well known to the skilled in the art, including the following enumerative embodiment, the embodiment formed by the following enumerative embodiment in combination with other chemical synthesis methods and the equivalent replacement well known to the skilled in the art. The preferred embodiment includes, but is not limited to the embodiment disclosed herein.

The compounds disclosed herein may have various uses or indications, including but not limited to the specific uses or indications listed herein.

All of the solvents used in the present disclosure are commercially available. The present disclosure employs the following abbreviations: aq represents water; HATU represents O-(7-azabenzotriazol-1-yl)-N,N,N',N'-tetramethyluronium hexafluorophosphate; EDC represents N-(3-dimethylaminopropyl)-N'-ethylcarbodiimide hydrochloride; m-CPBA represents 3-chloroperoxybenzoic acid; eq represents equivalent or equivalence; CDI represents carbonyl diimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl, which is an amino protecting group; BOC represents tert-butyloxycarbonyl, which is an amino protecting group; HOAc represents acetic acid; $NaCNBH_3$ represents sodium cyanoborohydride; r.t. represents room temperature; ON represents overnight; THF represents tetrahydrofuran; $Boc_2O$ represents di-tert-butyldicarbonate; TFA represents trifluoroacetic acid; DIPEA represents diisopropylethylamine; $SOCl_2$ represents thionyl chloride; $CS_2$ represents carbon disulfide; TsOH represents p-toluenesulfonic acid; NFSI represents N-fluoro-N-(phenylsulfonyl)benzenesulfonamide; NCS represents 1-chloropyrrolidine-2,5-dione; $n$-$Bu_4NF$ represents tetrabutylammonium fluoride; iPrOH represents 2-propanol; mp represents melting point; LDA represents lithium diisopropylamide; DEA represents diethylamine; ACN represents acetonitrile; HATU represents 2-(7-Azabenzotriazol-1-yl)-N,N,N',N'-tetramethyluronium hexafluorophosphate; DIEA represents N,N-diisopropylethylamine; PBS represents phosphate buffer solution; and Matrigel represents matrix gelatin.

Compounds are named according to conventional naming principles in the art or using ChemDraw® software, and names in supplier catalog are used for commercially available compounds.

**Technical** effect: The compounds disclosed herein have obvious inhibitory activity against HER1, HER2 and HER4, and have obvious inhibitory activity against the proliferation of NCI-N87 cells and BT-474 cells. The results from the in vivo pharmacodynamics study in BALB/c nude mouse model with subcutaneous xenograft tumor of human gastric cancer NCI-N87 cells showed that the compounds disclosed herein have a significant effect of inhibiting tumor growth without significantly affecting the weight of mice at the effective dose, and thus they are safe.

## BRIEF DESCRIPTION OF THE DRAWINGS

Figure 1: relative weight change (%) of BALB/c nude mouse model with subcutaneous xenograft tumor of human esophageal cancer OE21 cells, wherein the relative weight change is calculated based on the animal's body weight at the start of dosing. Data points represent the percentage in average body weight change within the group, and error lines represent standard error (SEM).

PO: oral administration; QD: once a day; D1-D7: day 1 to day 7 of administration; D8-D21: day 8 to day 21 of administration; D1-D2: day 1 to day 2 of administration; D3-D21: day 3 to day 21 of administration; n = 6: 6 mice per group.

## DETAILED DESCRIPTION OF THE EMBODIMENTS

Reference Example 1

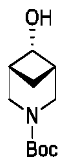

A-2

Synthetic route:

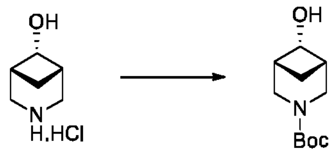

A-1 A-2

Compound A-1 (500 mg, 4.42 mmol) and triethylamine (1.12 g, 11.05 mmol) were dissolved in dichloromethane (20 mL), and to the mixture was added di-tert-butyl dicarbonate (1.01 g, 4.64 mmol). The reaction solution was stirred at 10 °C for 16 hours. The reaction solution was washed twice with saturated ammonium chloride solution (20 mL), dried over anhydrous sodium sulfate, and filtered. The filtrate was concentrated under reduced pressure, and the residue was slurried with petroleum ether (3 mL) to afford compound A-2. $^1$H NMR (400 MHz, $CDCl_3$) δ 4.05-4.15 (m, 1H), 3.50-3.62 (m, 4H), 2.40-2.60 (m, 2H), 2.04 (d, *J* = 7.2 Hz, 1H), 1.52-1.60 (m, 1H),1.47 (s, 9H), 1.30-1.40 (m, 1H).

Example 1

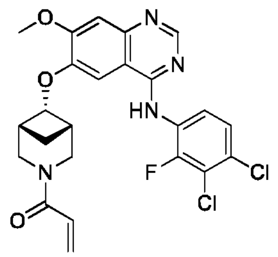

Synthetic route:

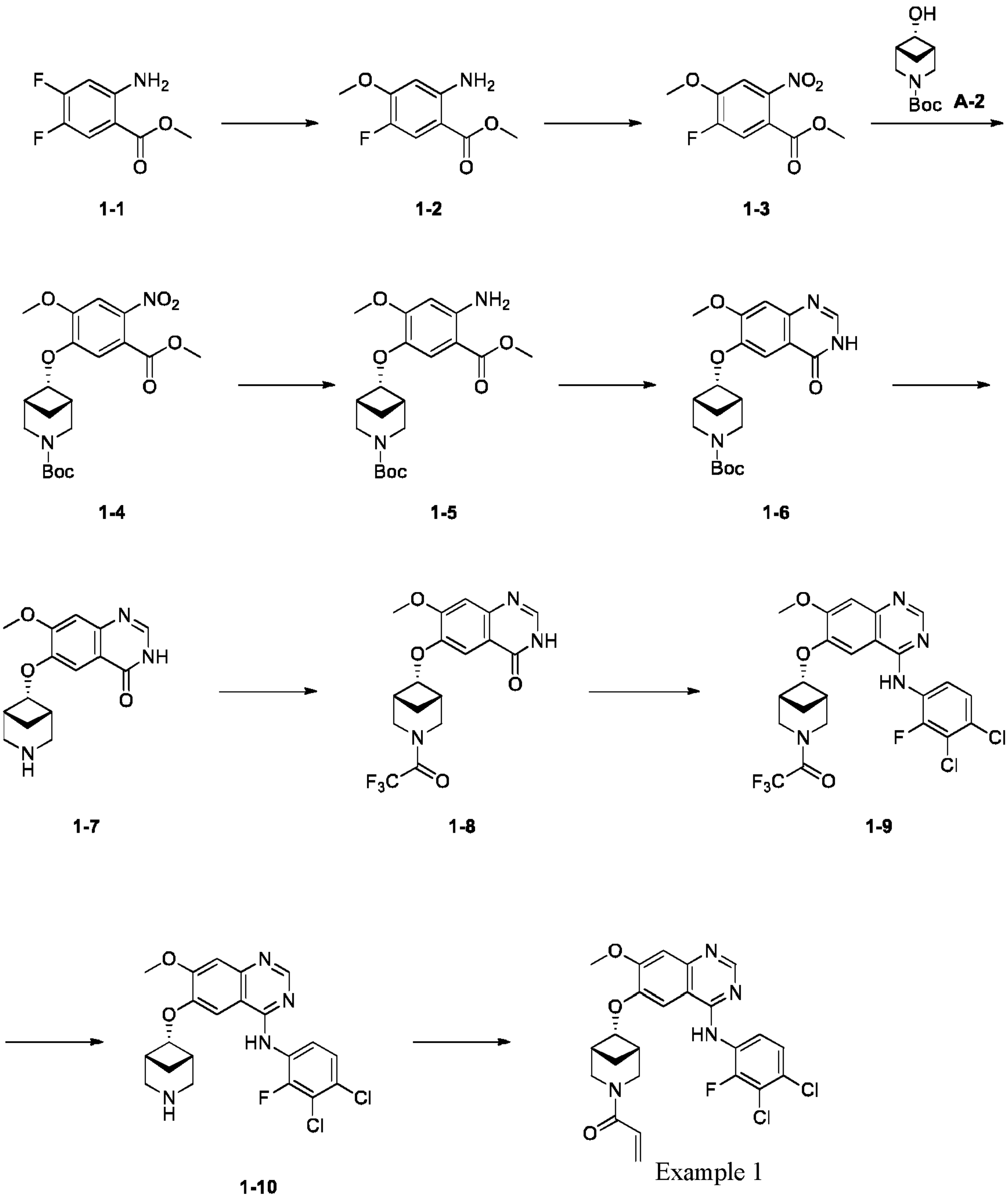


1-1 1-2 1-3

1-4 1-5 1-6

1-7 1-8 1-9

1-10 Example 1

The first step

Sodium metal (1.84 g, 80.15 mmol) was added to anhydrous methanol (100 mL) and stirred under nitrogen at 30 °C for 30 minutes. The reaction solution was cooled to 0 °C, and compound **1-1** (10 g, 53.44 mmol) was added. The reaction solution was stirred at 70 °C for 16 hours, and then cooled to 0 °C. To the mixture was added 1 N dilute hydrochloric acid (100 mL), diluted with water (200 mL), and extracted with dichloromethane (200 mL x 2). The organic phases were combined, dried over anhydrous sodium sulfate, and filtered. The filtrate was concentrated under reduced pressure, and the residue was isolated by silica gel column chromatography (ethyl acetate:petroleum ether = 1:8) to afford compound **1-2.** $^1$H NMR (400 MHz, $CDCl_3$) $\delta$ 7.53 (d, *J* = 12.4 Hz, 1H), 6.15 (d, *J* = 6.8 Hz, 1H), 5.69 (brs, 2H), 3.87 (s, 3H), 3.84 (s, 3H). LC-MS: *m/z* = 199.9 $[M+H]^+$.

The second step

A solution of compound **1-2** (5.00 g, 25.10 mmol) in dichloroethane (100 mL) was slowly added to a solution of metachloroperbenzoic acid (85%, 20.39 g, 100.41 mmol) in dichloroethane (200 mL) dropwise at 90 °C. The reaction solution was stirred at 90 °C for two hours, and then cooled to 0 °C, and quenched with saturated aqueous sodium sulfite solution (300 mL). The mixture was extracted with dichloromethane (300 mL x 2). The organic phases were combined, washed sequentially with saturated sodium bicarbonate solution (300 mL x 2), water (300 mL x 2) and saturated brine (300 mL x 2), dried over anhydrous sodium sulfate, and filtered. The filtrate was concentrated under reduced pressure, and the residue was isolated by silica gel column chromatography (ethyl acetate:petroleum ether = 1:8) to afford compound **1-3.** $^1$H NMR (400 MHz, $CDCl_3$) $\delta$ 7.47 (d, *J* = 10.4 Hz, 1H), 7.43 (d, *J* = 7.2 Hz, 1H), 3.98 (s, 3H), 3.89 (s, 3H).

The third step

Compound **1-3** (730.0 mg, 3.19 mmol) was dissolved in N,N-dimethylformamide (10 mL). To the mixture were added cesium carbonate (2.08 g, 6.37 mmol) and compound **A-2** (679.0 mg, 3.19 mmol). The reaction solution was heated to 65 °C, and stirred for 3 hours. The reaction solution was cooled to 20 °C, diluted with water (30.0 mL) and extracted with ethyl acetate (30 mL x 2). The organic phases were combined, washed sequentially with water (40 mL x 2) and saturated brine (40 mL x 2), dried over anhydrous sodium sulfate, and filtered. The filtrate was concentrated under reduced pressure, and the residue was isolated by silica gel column chromatography (petroleum ether:ethyl acetate = 8:1) to afford compound **1-4.** $^1$H NMR (400 MHz, $CDCl_3$) $\delta$ 7.44 (s, 1H), 7.03 (s, 1H), 4.54 (t, *J* = 5.6 Hz, 1H), 3.92 (s, 3H), 3.89 (s, 3H), 3.89 (td, $J_1$= 12.0 Hz, $J_2$= 3.2 Hz, 2H), 3.51 (t, *J* = 11.2 Hz, 2H), 2.80-2.95 (m, 2H), 1.75-1.85 (m, 1H), 1.55-1.65 (m, 1H), 1.47 (s, 9H). LC-MS: *m/z* = 445.2 $[M+Na]^+$.

The fourth step

Compound **1-4** (740.0 mg, 1.75 mmol) was dissolved in methanol (100 mL), and to the mixture was added wet palladium/carbon (10%, 150 mg). The reaction solution was stirred at 20 °C under hydrogen atmosphere (hydrogen balloon) for 0.5 hour. The reaction solution was filtered through celite, and the filtrate was concentrated under reduced pressure to afford compound **1-5.** $^1$H NMR (400 MHz, $CDCl_3$) $\delta$ 7.29 (s, 1H), 6.12 (s, 1H), 4.30-4.40 (m, 1H), 3.83 (s, 3H), 3.81 (s, 3H), 3.55-3.65 (m, 4H), 2.70-2.85 (m, 2H), 1.65-1.75 (m, 1H), 1.50-1.55 (m, 1H), 1.47 (s, 9H).

The fifth step

Compound **1-5** (687.0 mg, 1.75 mmol) and ammonium acetate (675.0 mg, 8.75 mmol) were added to trimethyl orthoformate (17.09 g, 161.02 mmol). The reaction solution was heated to 70 °C and stirred for 10 hours. The reaction solution was cooled to 30 °C, and concentrated under reduced pressure. The residue was slurried with water (10 mL) and filtered. The filter cake was dried to afford compound **1-6.** $^1$H NMR (400 MHz, DMSO-$d_6$) $\delta$ 12.08 (brs, 1H), 7.99 (s, 1H), 7.47 (s, 1H), 7.15 (s, 1H), 4.65 (t, *J* = 5.6 Hz, 1H), 3.89 (s, 3H), 3.45-3.60 (m, 2H), 3.10-3.20 (m, 2H), 2.75-2.90 (m, 2H), 1.75-1.85 (m, 1H), 1.40-1.50 (m, 1H), 1.40 (s, 9H). LC-MS: *m/z* = 410.1 $[M+Na]^+$.

The sixth step

Compound **1-6** (640.0 mg, 1.65 mmol) was added to a solution of hydrogen chloride in dioxane (4 N, 10 mL). The reaction solution was stirred at 0 °C for 0.4 hour, and then concentrated under reduced pressure to obtain the hydrochloride salt of compound 1-7. $^1$H NMR (400 MHz, DMSO-$d_6$) $\delta$ 9.72 (brs, 1H), 9.02 (brs, 1H), 8.34 (s, 1H), 7.56 (s, 1H), 7.27 (s, 1H), 4.76 (t, *J* = 6.0 Hz, 1H), 3.96 (s, 3H), 3.35- 3.45 (m, 2H), 3.20-3.35 (m, 2H), 2.89-2.95 (m, 2H), 2.01-2.08 (m, 1H), 1.90-2.00 (m, 1H).

The seventh step

The hydrochloride salt of **1-7** (640.0 mg, 1.98 mmol) and triethylamine (2.00 g, 19.77 mmol) were dissolved in dichloromethane (10 mL) at 0 °C, and trifluoroacetic anhydride (830.0 mg, 3.95 mmol) was added to the mixture dropwise. The reaction solution was stirred at 20 °C for 2 hours. The reaction solution was quenched by adding saturated aqueous sodium bicarbonate solution (10 mL), and extracted with dichloromethane (10 mL x 2). The organic phases were combined, dried over anhydrous sodium sulfate, and filtered. The filtrate was concentrated under reduced pressure to afford compound 1-8. $^1$H NMR (400 MHz, DMSO-$d_6$) δ 12.10 (s, 1H), 8.00 (s, 1H), 7.52 (s, 1H), 7.15 (s, 1H), 4.84 (t, *J* = 5.6 Hz, 1H), 3.90- 4.00 (m, 1H), 3.87 (s, 3H), 3.65-3.85 (m, 2H), 3.45-3.55 (m, 1H), 2.90-3.00 (m, 2H), 1.90-2.00 (m, 1H), 1.50-1.60 (m, 1H).

The eighth step

Compound **1-8** (270.0 mg, 704.37 μmol) was dissolved in thionyl chloride (13.1 mL, 180.28 mmol), and N,N-dimethylformamide (26.0 μL, 338.10 μmol) was added to the mixtuire. The reaction solution was heated to 80 °C and stirred for 1 hour. The reaction solution was concentrated under reduced pressure. To the residue was added a solution of 3,4-dichloro-2-fluoroaniline (392.0 mg, 2.18 mmol) in isopropanol (10 mL) at room temperature. The mixture was heated to 90 °C, and stirred for 1 hour. The reaction solution was cooled to 20 °C, quenched by adding saturated sodium bicarbonate solution (10 mL), and extracted with ethyl acetate (10 mL x 2). The organic phases were combined, dried over anhydrous sodium sulfate, and filtered. The filtrate was concentrated under reduced pressure, and the residue was isolated by silica gel column chromatography (ethyl acetate:petroleum ether = 2:1) to afford compound 1-9. $^1$H NMR (400 MHz, DMSO-$d_6$) δ 9.68 (s, 1H), 8.41 (s, 1H), 7.80 (s, 1H), 7.60 (s, 2H), 7.24 (s, 1H), 4.82 (t, *J* = 6.0 Hz, 1H), 3.95-4.05 (m, 1H), 3.91 (s, 3H), 3.70-3.95 (m, 2H), 3.50-3.60 (m, 1H), 3.05-3.15 (m, 2H), 1.85-1.95 (m, 1H), 1.55-1.65 (m, 1H). LC-MS: *m*/*z* = 545.0 $[M+H]^+$.

The ninth step

Compound **1-9** (300.0 mg, 551.14 umol) was dissolved in methanol (15 mL), and to the mixture was added potassium carbonate (380.9 mg, 2.76 mmol). The reaction solution was stirred at 40 °C for 14 hours. The mixture was concentrated under reduced pressure, diluted with water (10 mL), and extracted with ethyl acetate (40 mL x 4). The organic phases were combined, dried over anhydrous sodium sulfate, and filtered. The filtrate was concentrated under reduced pressure to afford compound 1-10. $^1$H NMR (400 MHz, DMSO-$d_6$) δ 9.81 (s, 1H), 8.41 (s, 2H), 7.76 (s, 1H), 7.59 (s, 2H), 7.29 (s, 1H), 4.73 (t, *J* = 6.0 Hz, 1H), 3.99 (s, 3H), 3.20-3.30 (m, 4H), 2.95-3.05 (m, 2H), 1.95-2.05 (m, 1H), 1.75-1.85 (m, 1H). LC-MS: *m*/*z* = 449.1 $[M+H]^+$.

The tenth step

Compound **1-10** (240.0 mg, 534.16 μmol) was dissolved in a mixed solvent of tetrahydrofuran (6 mL) and water (6 mL) at 0 °C, and to the mixture was added sodium bicarbonate (134.6 mg, 1.60 mmol). After the addition of sodium bicarbonate, acryloyl chloride (48.4 mg, 534.16 μmol) was added slowly dropwise to the mixture. The reaction solution was further stirred at 0 °C for 0.5 hour. The reaction solution was quenched by adding methanol (1 mL) and concentrated under reduced pressure. The residue was isolated by preparative thin-layer chromatography (dichloromethane:methanol =10:1) to obtain the **compound of example 1.** $^1$H NMR (400 MHz, $CDCl_3$) δ 8.68 (s, 1H), 8.43 (t, *J* = 8.8 Hz, 1H), 7.34-7.45 (m, 2H), 7.10 (s, 1H), 6.60 (dd, $J_1$ = 16.8 Hz, $J_2$ = 9.6 Hz, 1H), 5.71 (dd, $J_1$ = 16.8 Hz, $J_2$ = 2.0, Hz, 1H), 6.60 (dd, $J_1$ = 9.6 Hz, $J_2$ = 2.0 Hz, 1H), 4.66 (t, *J* = 5.6 Hz, 1H), 3.96 (s, 3H), 3.85-3.95 (m, 2H), 3.65-3.75 (m, 2H), 2.95-3.05 (m, 2H), 1.85-1.95 (m, 1H), 1.55-1.65 (m, 1H). LC-MS: *m*/*z* = 503.1 $[M+H]^+$.

Example 2

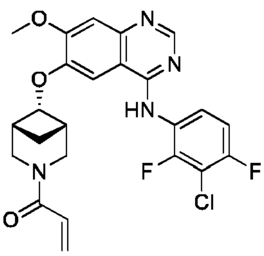

Synthetic route:

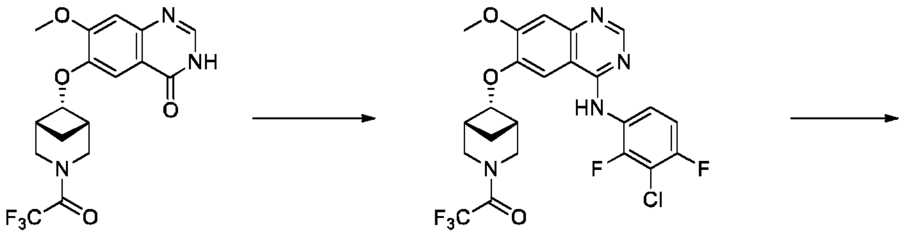

1-8 2-2

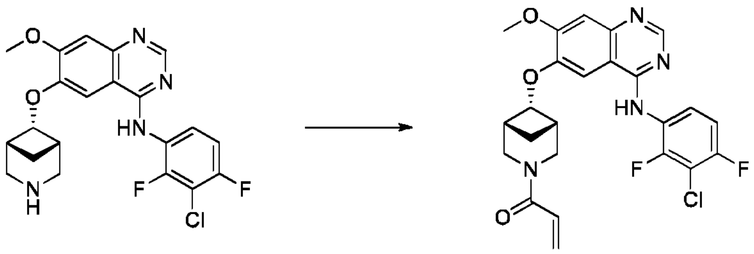

2-3 Example 2

The first step

Compound **2-2** was obtained by referring to the eighth step of Example 1. $^1$H NMR (400 MHz, DMSO-$d_6$) δ 11.79 (s, 1H), 8.44 (s, 1H), 8.37 (s, 1H), 7.60-7.70 (m, 1H), 7.45-7.55 (m, 1H), 7.35 (s, 1H), 4.95 (t, *J* = 5.6 Hz, 1H), 4.00-4.05 (m, 1H), 3.97 (s, 3H), 3.70-3.90 (m, 3H), 3.10-3.20 (m, 2H), 1.80-1.90 (m, 1H), 1.61 (d, *J* = 10.4 Hz, 1H). LC-MS: *m*/*z* = 529.1 $[M+H]^+$.

The second step

Compound **2-2** (70.0 mg, 132.61 μmol) was dissolved in methanol (2 mL), and to the mixture was added potassium carbonate (91.6 mg, 663.04 μmol). The reaction solution was stirred at 50 °C for 14 hours. The reaction solution was concentrated under reduced pressure, diluted with water (10 mL) and extracted with ethyl acetate (20 mL x 4). The organic phases were combined, dried over anhydrous sodium sulfate, and filtered. The filtrate was concentrated under reduced pressure to afford compound **2-3.** $^1$H NMR (400 MHz, DMSO-$d_6$) δ 9.78 (s, 1H), 8.39 (s, 2H), 7.79 (s, 1H), 7.50-7.60 (m, 1H), 7.38-7.48 (m, 1H), 7.29 (s, 1H), 4.74 (t, *J* = 6.0 Hz, 1H), 3.99 (s, 3H), 3.40-3.50 (m, 4H), 2.95-3.05 (m, 2H), 1.98 (d, *J* = 11.2 Hz,1H), 1.80-1.90 (m, 1H). LC-MS: *m*/*z* = 433.1 $[M+H]^+$.

The third step

**Compound of example 2** was obtained by referring to the tenth step of Example 1. $^1$H NMR (400 MHz, DMSO-$d_6$) δ 9.66 (s, 1H), 8.37 (s, 1H), 7.79 (s, 1H), 7.55-7.65 (m, 1H), 7.41 (t, *J* = 8.4 Hz, 1H), 7.22 (s, 1H), 6.75 (dd, $J_1$ = 16.8 Hz, $J_2$ = 10.4 Hz, 1H), 6.16 (d, *J* = 16.8, Hz, 1H), 5.68 (d, *J* = 9.6 Hz, 1H), 4.65-4.75 (m, 1H), 3.90-3.95 (m, 1H), 3.90 (s, 3H), 3.65-3.85 (m, 2H), 3.45-3.55 (m, 1H), 3.02-3.12 (m, 2H), 1.75-1.85 (m, 1H), 1.51 (d, *J* = 10.0 Hz,1H). LC-MS:

*m*/*z* = 487.0 $[M+H]^+$.

Example 3

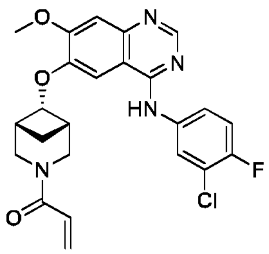

Synthetic route:

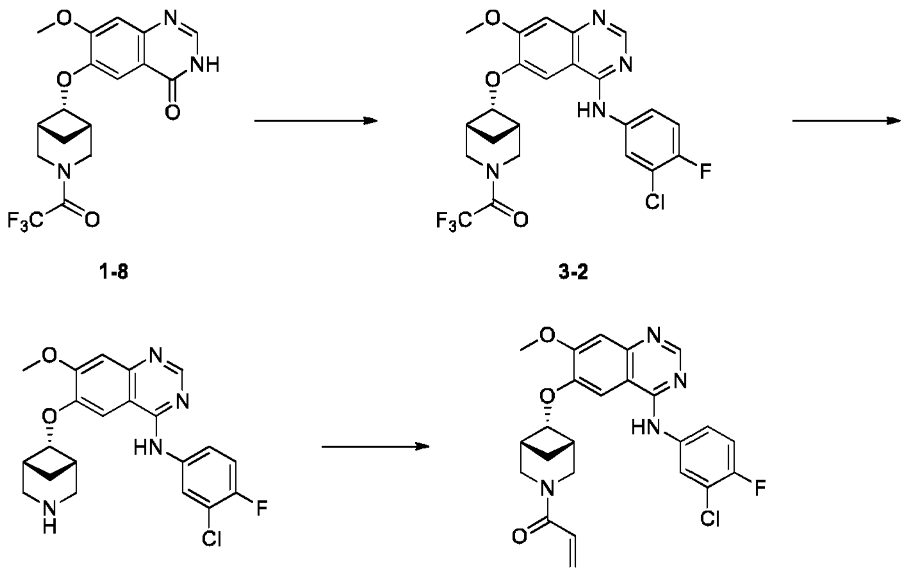

**1-8** **3-2**

**3-3** Example 3

The first step

Compound **3-2** was obtained by referring to the eighth step of Example 1. $^1H$ NMR (400 MHz, DMSO-$d_6$) δ 11.45 (s, 1H), 8.87 (s, 1H), 8.35 (s, 1H), 8.01-8.07 (m, 1H), 7.70-7.80 (m, 1H), 7.57 (t, *J* = 8.8 Hz, 1H), 7.31(s, 1H), 4.98(t, *J* = 5.6 Hz, 1H), 3.96-4.44 (m, 1H), 3.96 (s, 3H), 3.70-3.85 (m, 2H), 3.50-3.55 (m, 1H), 1.80-1.90 (m, 1H), 1.62 (d, *J* = 10.4 Hz, 1H). LC-MS: *m*/*z* = 511.1 $[M+H]^+$.

The second step

Compound **3-3** was obtained by referring to the second step of Example 2. $^1H$ NMR (400 MHz, DMSO-$d_6$) δ 9.60 (s, 1H), 8.50 (s, 1H), 8.05-8.12 (m, 1H), 7.70-7.85 (m, 2H), 7.46 (t, *J* = 9.2 Hz, 1H), 7.26 (s, 1H), 4.75 (t, *J* = 5.6 Hz, 1H), 3.98 (s, 3H), 3.15-3.30 (m, 2H), 2.95-3.10 (m, 2H), 2.88-2.95 (m, 2H), 1.90-2.00 (m, 1H), 1.70-1.80 (m, 1H). LC-MS: *m*/*z* = 415.1 $[M+H]^+$.

The third step

Compound 3-3 (40.0 mg, 96.42 μmol) was dissolved in a mixed solvent of tetrahydrofuran (4 mL) and water (4 mL) at 0 °C. To the mixture was added sodium bicarbonate (24.3 mg, 289.25 μmol), and added slowly acryloyl chloride

(10.57 mg, 115.70 μmol) dropwise. The reaction solution was further stirred at 0 °C for 0.5 hour. The reaction solution was quenched by adding methanol (1 mL), and extracted with ethyl acetate (20 mL x 2). The organic phases were combined, dried over anhydrous sodium sulfate, and filtered. The filtrate was concentrated under reduced pressure, and the residue was isolated by preparative thin-layer chromatography (dichloromethane:methanol = 10:1) to obtain the **compound of example 3.** $^1H$ NMR (400 MHz, DMSO-$d_6$) δ 9.59 (s, 1H), 8.49 (s, 1H), 8.08-8.12 (m, 1H), 7.75-7.85 (m, 2H), 7.46 (t, *J* = 9.2 Hz, 1H), 7.22 (s, 1H), 6.74 (dd, $J_1$ = 16.4 Hz, $J_2$= 10.0 Hz, 1H), 6.17 (d, *J* = 12.0 Hz, 1H), 5.69 (d, *J* = 10.0 Hz, 1H), 4.73 (t, *J* = 5.6, Hz, 1H), 3.95-4.00 (m, 1H), 3.89 (s, 3H), 3.65-3.80 (m, 2H), 3.40-3.50 (m, 1H), 3.05-3.10 (m, 2H), 1.75-1.90 (m, 1H), 1.51 (d, *J* = 10.0 Hz,1H). LC-MS: *m*/*z* = 469.1 [M+H]$^+$.

Example 4

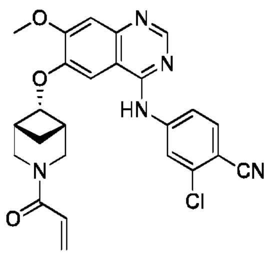

Synthetic route:

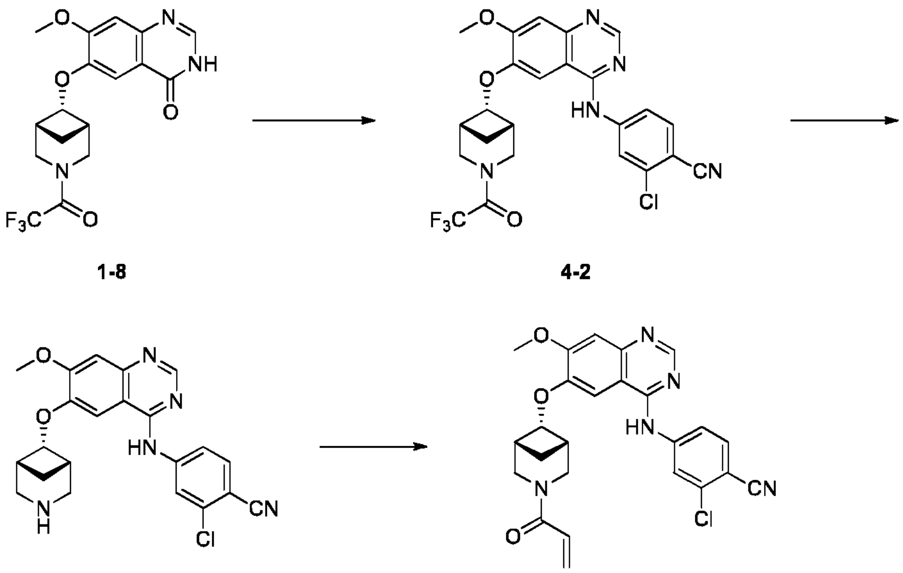

1-8

4-2

4-3

Example 4

The first step

Compound **4-2** was obtained by referring to the eighth step of Example 1. $^1H$ NMR (400 MHz, DMSO-$d_6$) δ 11.50 (s, 1H), 8.94 (s, 1H), 8.47 (s, 1H), 8.39 (d, *J* = 2.0 Hz 1H), 8.10-8.15 (m, 1H), 8.05-8.10 (m, 1H), 7.35(s, 1H), 5.06 (t, *J* = 5.6 Hz, 1H), 3.97 (s, 3H), 3.70-3.90 (m, 4H), 3.10-3.20 (m, 2H), 1.80-1.90 (m, 1H), 1.61 (d, *J* = 10.4 Hz, 1H). LC-MS: *m*/*z* = 518.1 [M+H]$^+$.

The second step

Compound **4-3** was obtained by referring to the ninth step of Example 1. $^1H$ NMR (400 MHz, DMSO-$d_6$) δ 9.90

(s, 1H), 8.64 (s, 1H), 8.37 (s, 1H), 8.05-8.15 (m, 1H), 7.90-8.00 (m, 1H), 7.72 (s, 1H), 7.30 (s, 1H), 4.70-4.80 (m, 1H), 3.99 (s, 3H), 3.02-3.10 (m, 2H), 2.75-2.85 (m, 4H), 1.93 (d, $J$ = 9.6 Hz, 1H), 1.60-1.70 (m, 1H). LC-MS: $m/z$ = 422.1 $[M+H]^+$.

The third step

**Compound of example 4** was obtained by referring to the third step of **Example 3.** $^1$H NMR (400 MHz, DMSO-$d_6$) δ 9.91 (s, 1H), 8.65 (s, 1H), 8.39 (s, 1H), 8.05-8.15 (m, 1H), 7.92-8.00 (m, 1H), 7.85 (s, 1H), 7.28 (s, 1H), 6.74 (dd, 16.4 Hz, $J_2$= 10.4 Hz, 1H), 6.18 (dd, $J_1$ = 16.4 Hz, $J_2$= 2.4 Hz, 1H), 5.60 (dd, $J_1$= 10.4 Hz, $J_2$= 2.4 Hz, 1H), 4.77 (t, $J$ = 5.6 Hz, 1H), 3.92 (s, 3H), 3.85-3.90 (m, 1H), 3.65-3.80 (m, 2H), 3.40-3.50 (m, 1H), 3.00-3.10 (m, 2H), 1.80-1.90 (m, 1H), 1.52 (d, $J$ = 10.0 Hz,1H). LC-MS: $m/z$ = 476.0 $[M+H]^+$.

Example 5

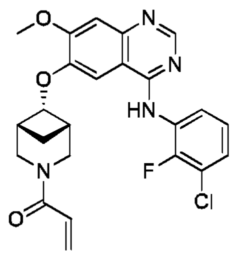

Synthetic route:

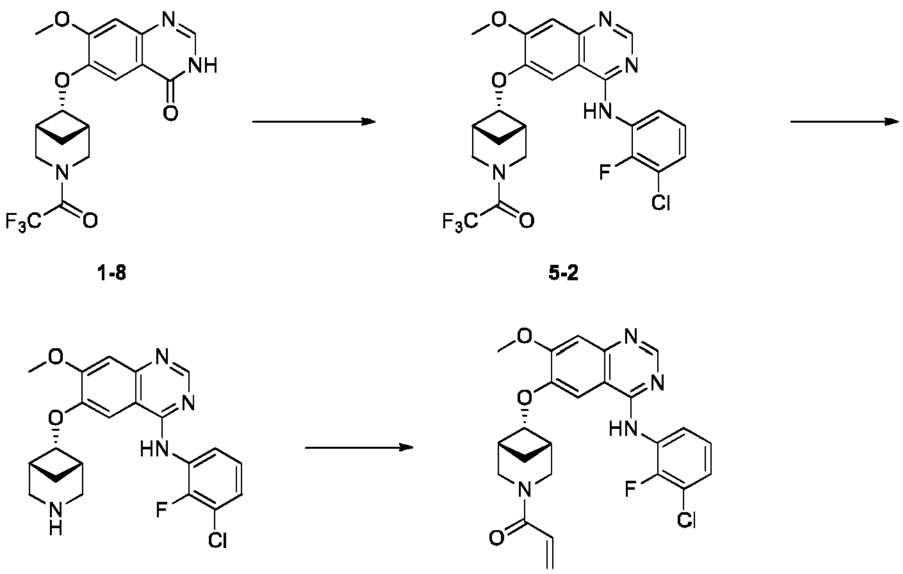

1-8

5-2

5-3

Example 5

The first step

Compound **5-2** was obtained by referring to the eighth step of Example 1. $^1$H NMR (400 MHz, DMSO-$d_6$) δ 11.92 (s, 1H), 8.87 (s, 1H), 8.41 (s, 1H), 7.65-7.75 (m, 1H), 7.50-7.65 (m, 1H), 7.35-7.45 (m, 2H), 4.97 (t, $J$ = 5.6 Hz, 1H), 3.98-4.00 (m, 1H), 3.98 (s, 3H), 3.70-3.90 (m, 3H), 3.10-3.20 (m, 2H), 1.80-1.90 (m, 1H), 1.62 (d, $J$ = 10.0 Hz, 1H). LC-MS: $m/z$ = 511.2 $[M+H]^+$.

The second step

Compound **5-3** was obtained by referring to the ninth step of Example 1. $^1$H NMR (400 MHz, DMSO-$d_6$) δ 9.87 (s, 1H), 8.40 (s, 1H), 7.85 (s, 1H), 7.45-7.60 (m, 1H), 7.20-7.40 (m, 2H), 4.60 (t, $J$ = 5.6 Hz1H), 3.99 (s, 3H), 3.30-3.50 (m, 4H), 3.00-3.15 (m, 2H), 2.00-2.10 (m,1H), 1.80-1.90 (m, 1H). LC-MS: $m/z$ = 415.1 [M+H]$^+$.

The third step

**Compound of example 5** was obtained by referring to the third step of **Example 3.** $^1$H NMR (400 MHz, DMSO-$d_6$) δ 9.67 (s, 1H), 8.38 (s, 1H), 7.76 (s, 1H), 7.50-7.60 (m, 2H), 7.25-7.35 (m, 1H), 7.22 (s, 1H), 6.75 (dd, $J_1$= 16.4, $J_2$= 10.4 Hz, 1H), 6.18 (dd, 16.4 Hz, $J_2$= 2.0 Hz, 1H), 5.69 (dd, 10.4 Hz, $J_2$= 2.4 Hz, 1H), 4.71 (t, $J$ = 5.6 Hz, 1H), 3.95-4.00 (m, 1H), 3.92 (s, 3H), 3.65-3.80 (m, 2H), 3.42-3.50 (m, 1H), 3.00-3.10 (m, 2H), 1.75-1.85 (m, 1H), 1.52 (d, $J$ = 10.0 Hz,1H). LC-MS: $m/z$ = 469.0 [M+H]$^+$.

Example 6

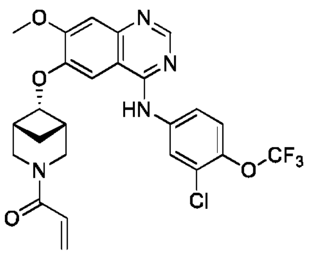

Synthetic route:

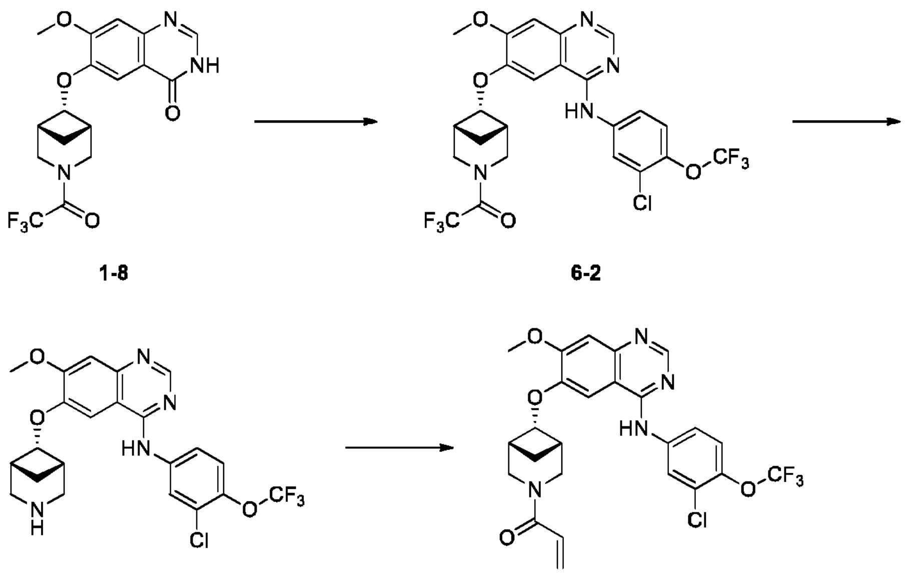

1-8

6-2

6-3

Example 6

The first step

Compound **6-2** was obtained by referring to the eighth step of Example 1. $^1$H NMR (400 MHz, DMSO-$d_6$) δ 9.77 (s, 1H), 8.56 (s, 1H), 8.27 (s, 1H), 7.90-7.80 (m, 2H), 7.55-7.65 (m, 1H), 7.25 (s, 1H), 4.90 (t, $J$ = 5.6 Hz, 1H), 3.92-4.00 (m, 1H), 3.95 (s, 3H), 3.80-3.90 (m, 1H), 3.70-3.80 (m, 1H), 3.50-3.60 (m, 1H), 3.00-3.10 (m, 2H), 1.80-1.90 (m, 1H), 1.60 (d, $J$ = 10.0 Hz, 1H). LC-MS: $m/z$ = 577.1 [M+H]$^+$.

The second step

Compound **6-3** was obtained by referring to the second step of Example 2. $^1$H NMR (400 MHz, DMSO-$d_6$) $\delta$ 10.01 (s, 1H), 8.57 (s, 1H), 8.33 (s, 1H), 8.00-8.10 (m, 2H), 7.55-7.70 (m, 1H), 7.32 (s, 1H), 4.80-4.90 (m, 1H), 4.00 (s, 3H), 3.00-3.20 (m, 4H), 1.80-2.10 (m, 2H), 1.10-1.30 (m, 2H). LC-MS: m/z = 481.1 $[M+H]^+$.

The third step

**Compound of example 6** was obtained by referring to the tenth step of Example 1. $^1$H NMR (400 MHz, DMSO-$d_6$) $\delta$ 9.68 (s, 1H), 8.54 (s, 1H), 8.23 (s, 1H), 7.90-8.00 (m, 1H), 7.81 (s, 1H), 7.55-7.65 (m, 1H), 7.24 (s, 1H), 6.65-6.80 (m, 1H), 6.17 (d, *J* = 15.2 Hz, 1H), 5.68 (d, *J* = 8.8 Hz, 1H), 4.70-4.80 (m, 1H), 3.90 (s, 3H), 3.80-3.90 (m, 1H), 3.50-3.80 (m, 3H), 3.00-3.10 (m, 2H), 1.80-1.90 (m, 1H), 1.45-1.55 (m,1H). LC-MS: *m*/*z* = 535.1 $[M+H]^+$

Example 7

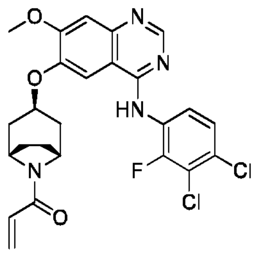

Synthetic route:

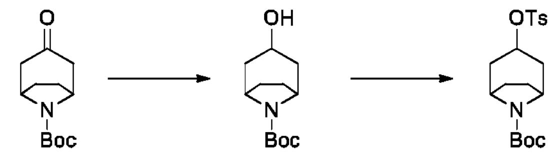

B-1 B-2 B-3

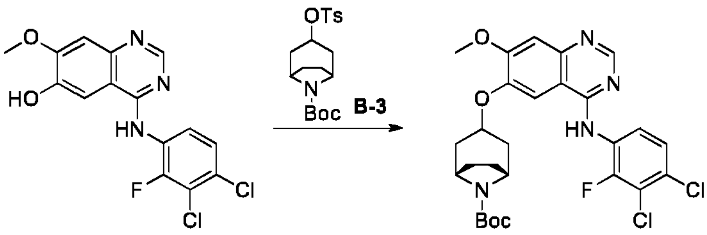


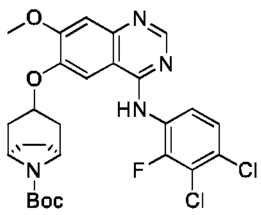

7-1 7-2 8-1

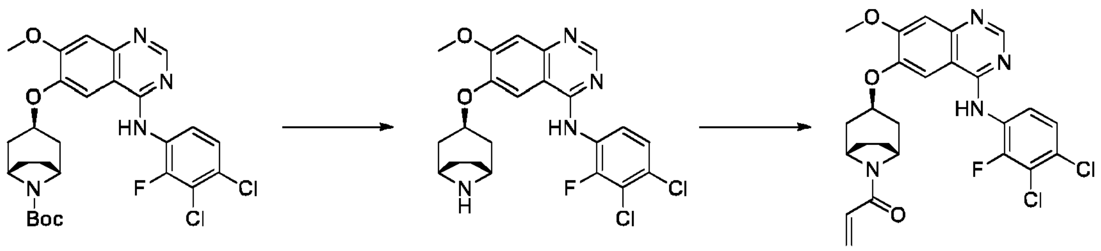

7-2 7-3 Example 7

The first step

Compound **B-1** (2.60 g, 11.54 mmol) was dissolved in ethanol (30 mL), and to the mixture was added sodium borohydride (1.09 g, 28.85 mmol). The reaction solution was stirred at 30 °C for 2 hours. The reaction solution was quenched by adding saturated ammonium chloride solution (30 mL) and extracted with dichloromethane (40 mL x 2). The organic phases were combined, washed with saturated brine (40 mL x 2), dried over anhydrous sodium sulfate, and filtered. The filtrate was concentrated under reduced pressure to afford compound **B-2.** $^1$H NMR (400 MHz, $CDCl_3$) δ 4.00-4.32 (m, 3H), 2.10-2.20 (m, 1H), 1.90-2.00 (m, 3H), 1.60-1.90 (m, 4H), 1.40-1.50 (m, 9H).

The second step

Compound **B-2** (2.60 g, 11.44 mmol), triethylamine (3.2 mL, 22.88 mmol), and 4-dimethylaminopyridine (1.68 g, 13.73 mmol) were dissolved in dichloromethane (60 mL), and to the mixture was added p-toluenesulfonyl chloride (2.62 g, 13.73 mmol). The reaction solution was stirred at 25 °C for 16 hours, and then quenched by adding saturated ammonium chloride aqueous solution (60 mL x 3). The organic phase was washed with saturated brine (60 mL), dried over anhydrous sodium sulfate, and filtered. The filtrate was concentrated under reduced pressure to afford compound **B-3**. $^1$H NMR (400 MHz, $CDCl_3$) δ 7.77 (d, *J* = 8.0 Hz, 2H), 7.33 (d, *J* = 8.0 Hz, 2H), 4.75-4.90 (m, 1H), 4.05-4.25 (m, 2H), 2.45 (s, 3H), 1.50-1.90 (m, 4H), 1.85-2.20 (m, 4H), 1.30-1.45 (s, 9H). LC-MS: *m/z* = 404.1 $[M+Na]^+$.

The third step

Compound **7-1** (2.00 g, 5.65 mmol) and potassium carbonate (1.56 g, 11.29 mmol) were added to N,N-dimethylacetamide (20 mL), and then to the mixture was added compound **B-3** (4.20 g, 8.47 mmol). The reaction was heated to 70 °C, and stirred for 16 hours. The reaction solution was cooled to 30 °C, poured into water (30 mL), and extracted with ethyl acetate (20 mL x 3). The organic phases were combined, washed sequentially with water (30 mL x 3) and saturated brine (30 mL x 3), dried over anhydrous sodium sulfate, and filtered. The filtrate was concentrated under reduced pressure, and the residue was isolated by silica gel column chromatography (petroleum ether:ethyl acetate =

1:1) to afford compounds **7-2** and **8-1.**

Compound **7-2:** $^1$H NMR (400 MHz, $CDCl_3$) δ 8.67 (s, 1H), 8.42 (t, *J* = 8.4 Hz, 1H), 7.26-7.40 (m, 3H), 6.96 (s, 1H), 4.80-4.90 (m, 1H), 4.15-4.35 (m, 2H), 4.00 (s, 3H), 2.10-2.35 (m, 4H), 1.90-2.10 (m, 4H), 1.48 (s, 9H). According to two-dimensional nuclear magnetic NOE identification: the single hydrogen on the carbon of the piperidine ring connected to the oxygen atom is not observed to be associated with the hydrogen of methylene on the bridge ring, and it is determined to be a cis structure. LC-MS: *m*/*z* 563.2 [M+H]$^+$.

Compound **8-1:** $^1$H NMR (400 MHz, $CDCl_3$) δ 8.70 (s, 1H), 8.53 (t, *J* = 8.4 Hz, 1H), 7.26-7.40 (m, 3H), 7.21 (s, 1H), 4.70-4.80 (m, 1H), 4.20-4.40 (m, 2H), 4.01 (s, 3H), 2.10-2.20 (m, 4H), 1.85-2.00 (m, 2H), 1.65-1.80 (m, 2H), 1.50 (s, 9H). According to two-dimensional nuclear magnetic NOE identification: the single hydrogen on the carbon of the piperidine ring connected to the oxygen atom is observed to be associated with the hydrogen of methylene on the bridge ring, and it is determined to be a trans structure. LC-MS: *m*/*z* 563.2 [M+H]$^+$.

The fourth step

Compound **7-2** (400.0 mg, 709.92 μmol) was dissolved in a solution of hydrogen chloride in dioxane (4 N, 6 mL). The reaction solution was reacted at 0 °C for 0.3 hour. The reaction solution was concentrated under reduced pressure, diluted with water (10 mL), and washed with ethyl acetate (10 mL). The aqueous phase was adjusted to pH of 10 with the solid potassium carbonate and extracted with ethyl acetate (10 mL x 3). The organic phases were combined, and dried over anhydrous sodium sulfate, and the filtrate was concentrated under reduced pressure. The residue was isolated by preparative thin-layer chromatography (methanol:dichloromethane = 1:10) to afford compound **7-3.** $^1$H NMR (400 MHz, DMSO-*d*$_6$) δ 9.96 (s, 1H), 8.39 (s, 1H), 7.96 (s, 1H), 7.55-7.65 (m, 2H), 7.26 (s, 1H), 4.95-5.00 (m, 1H), 3.90-4.05 (m, 5H), 2.45-2.50 (m, 2H), 2.25-2.35 (m, 2H), 2.10-2.20 (m, 2H), 1.90-2.05 (m, 2H).

The fifth step

Compound **7-3** (230.0 mg, 496.41 μmol) was dissolved in a mixed solvent of tetrahydrofuran (6 mL) and water (6 mL) at 0 °C. To the mixture was added sodium bicarbonate (125.1 mg, 1.49 mmol), and then added slowly acryloyl chloride (35.9 mg, 397.12 μmol) dropwise. The reaction solution was further stirred at 0 °C for 0.5 hour. The reaction solution was quenched by adding methanol (1 mL), and concentrated under reduced pressure. The residue was isolated by preparative thin-layer chromatography (dichloromethane:methanol = 10:1) to obtain the **compound of example** 7. $^1$H NMR (400 MHz, DMSO-*d*$_6$) δ 9.63 (s, 1H), 8.39 (s, 1H), 7.74 (s, 1H), 7.50-7.60 (m, 2H), 7.26 (s, 1H), 6.75 (dd, 16.4 Hz,$J_2$ =10.0 Hz, 1H), 6.19 (dd, $J_1$ = 16.4 Hz, $J_2$ = 2.4 Hz, 1H), 5.70 (dd, $J_1$ = 10.0 Hz, $J_2$ = 2.4 Hz, 1H), 4.88-4.90 (m, 1H), 4.50-4.61 (m, 2H), 3.97 (s, 3H), 2.20-2.30 (m, 2H), 1.95-2.15 (m, 5H), 1.80-1.95 (m, 1H). LC-MS: *m*/*z* = 517.1 [M+H]$^+$.

Example 8

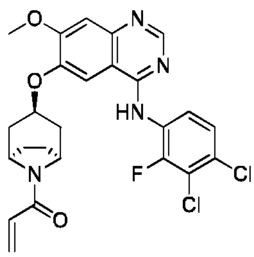

Synthetic route:

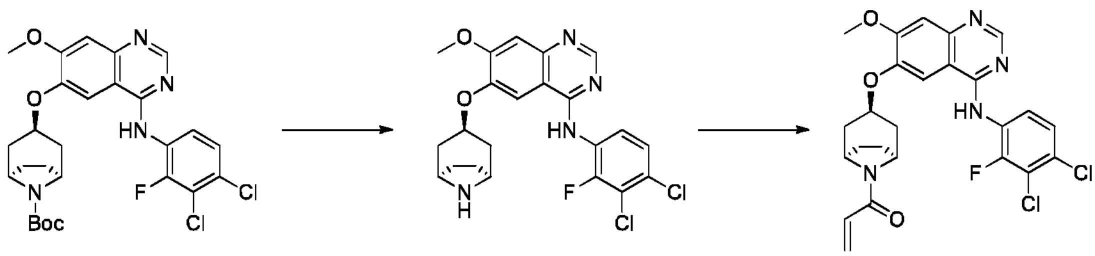

**8-1** **8-2** Example 8

The first step

Compound **8-2** was obtained by referring to the fourth step of **Example** 7. $^1$H NMR (400 MHz, DMSO-$d_6$) δ 10.13 (s, 1H), 8.39 (s, 1H), 8.10 (s, 1H), 7.55-7.70 (m, 2H), 7.23 (s, 1H), 4.90-5.00 (m, 1H), 3.90-4.05 (m, 2H), 3.94 (s, 3H), 2.30-2.40 (m, 2H), 2.10-2.20 (m, 2H), 1.80-2.00 (m, 4H).

The second step

**Compound of example 8** was obtained by referring to the fifth step of **Example 7.** $^1$H NMR (400 MHz, DMSO-$d_6$) δ 9.67 (s, 1H), 8.39 (s, 1H), 7.80 (s, 1H), 7.55-7.70 (m, 2H), 7.26 (s, 1H), 6.77 (dd, 16.4 Hz, $J_2$ = 10.4 Hz, 1H), 6.23 (dd, $J_1$ = 16.4 Hz, $J_2$ = 2.4 Hz, 1H), 5.72 (dd, $J_1$ = 10.4 Hz, $J_2$ = 2.4 Hz, 1H), 4.90-5.10 (m, 1H), 4.55-4.60 (m, 2H), 3.92 (s, 3H), 2.20-2.40 (m, 2H), 1.80-2.10 (m, 4H), 1.66 (t, $J$ = 10.4 Hz, 1H), 1.50 (t, $J$ = 10.4 Hz, 1H). LC-MS: $m/z$ = 517.1 [M+H]$^+$.

Example 9

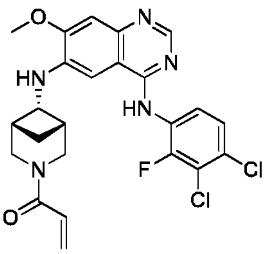

Synthetic route:

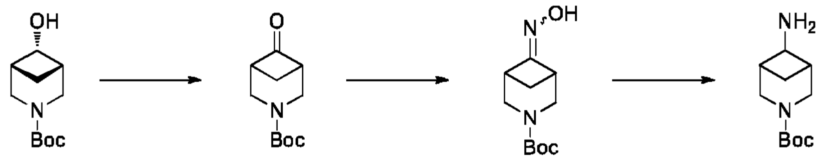

A-2 C-2 C-3 C-4

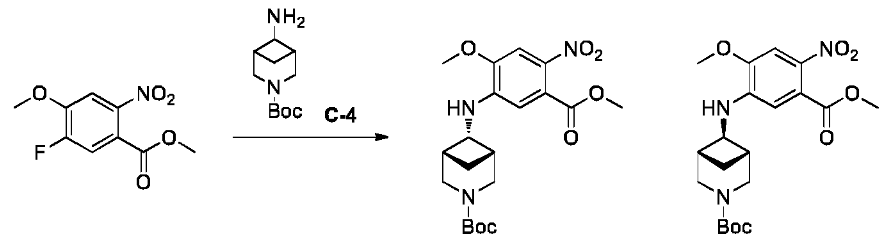

C-4

9-1 9-2 10-1

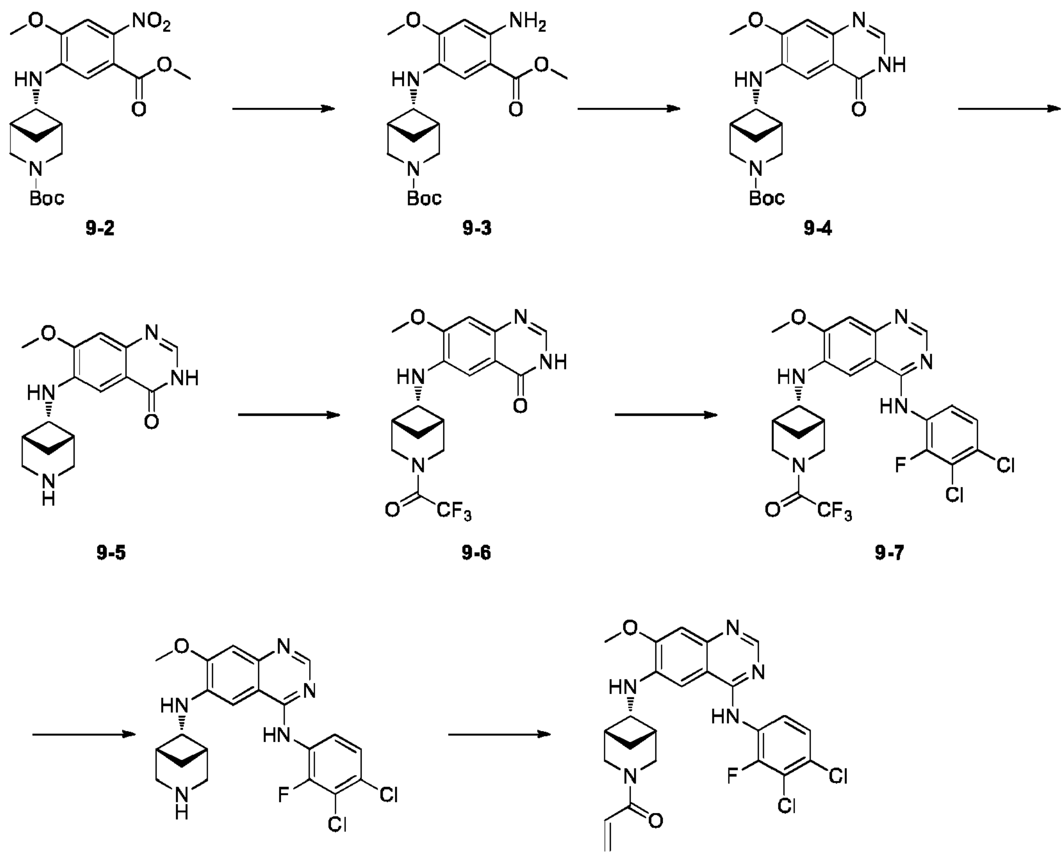

9-2 9-3 9-4

9-5 9-6 9-7

9-8 Example 9

The first step

Compound **A-2** (2.00 g, 9.38 mmol) was dissolved in dichloromethane (50 mL), and to the mixture was added Dess-Martin periodinane (4.77 g, 11.25 mmol). The reaction solution was stirred at 25 °C for 2 hours. The reaction solution was quenched by adding saturated aqueous sodium bicarbonate solution (30 mL), and extracted with dichloromethane (30 mL x 2). The organic phases were combined, dried over anhydrous sodium sulfate, and filtered. The filtrate was concentrated under reduced pressure, and the residue was isolated by silica gel column chromatography (ethyl acetate:petroleum ether = 1:4) to afford compound **C-2.** $^{1}H$ NMR (400 MHz, $CDCl_3$) δ 3.95-4.10 (m, 2H), 3.85-3.95 (m, 2H), 3.10-3.20 (m, 2H), 2.10-2.20 (m, 1H), 1.75-1.80(m, 1H), 1.45 (s, 9H).

The second step

Compound **C-2** (1.70 g, 8.05 mmol) was dissolved in methanol (20 mL), and to the mixture were added hydroxylamine hydrochloride (671.0 mg, 9.66 mmol) and sodium carbonate (511.7 mg, 4.83 mmol). The reaction solution was stirred at 25 °C for 16 hours. The reaction solution was concentrated under reduced pressure, diluted by adding dichloromethane (30 mL), washed with saturated aqueous sodium bicarbonate solution (20 mL), dried over anhydrous sodium sulfate, and filtered. The filtrate was concentrated under reduced pressure to afford compound **C-3.** $^1$H NMR (400 MHz, $CDCl_3$) δ 7.40-7.50 (m, 1H), 3.70-3.90 (m, 4H), 3.45-3.60 (m, 1H), 3.05-3.20 (m, 1H), 2.10-2.20 (m, 1H), 1.60-1.65 (m, 1H), 1.46 (s, 9H)

The third step

Compound **C-3** (2.00 g, 8.84 mmol) was dissolved in methanol (20 mL), and to the mixture was added Raney nickel (0.50 g). The reaction solution was stirred at 20 °C under hydrogen atmosphere (hydrogen balloon) for 16 hours, and then filtered through celite. The filtrate was concentrated under reduced pressure and dried to afford compound C-4. $^1$H NMR (400 MHz, $CDCl_3$) δ 3.85-4.10(m, 1H), 3.45-3.75 (m, 4H), 2.60-2.80 (m, 2H), 1.85-1.95 (m, 1H), 1.50-1.60 (m, 1H), 1.47 (s, 9H).

The fourth step

Compound **9-1** (900.0 mg, 3.93 mmol) was dissolved in N,N-dimethylformamide (20 mL), and to the mixture were added potassium carbonate (1.09 g, 7.85 mmol) and compound **C-4** (1.50 g, 7.07 mmol). The reaction solution was stirred at 90 °C for 16 hours. The reaction solution was cooled to 20 °C, diluted with water (30 mL) and extracted with ethyl acetate (30 mL x 2). The organic phases were combined, washed sequentially with water (40 mL x 2) and saturated brine (40 mL x 2), dried over anhydrous sodium sulfate, and filtered. The filtrate was concentrated under reduced pressure, and the residue was isolated by preparative thin-layer chromatography (petroleum ether: ethyl acetate = 3:1) to afford compounds **9-2** and **10-1.**

Compound **10-1:** $^1$H NMR (400 MHz, $CDCl_3$) δ 7.51 (s, 1H), 6.27 (s, 1H), 5.36 (d, *J* = 4 Hz, 1H), 3.97 (s, 3H), 3.93 (s, 3H), 3.80-3.90 (m, 1H), 3.55-3.70 (m, 2H), 3.45-3.53 (m, 1H), 3.35-3.45 (m, 1H), 2.65-2.85 (m, 2H), 1.90-2.00 (m, 1H), 1.45-1.55 (m, 1H), 1.51 (s, 9H). According to two-dimensional nuclear magnetic NOE identification: the single hydrogen on the carbon of the piperidine ring connected to the phenylamine is strongly associated with the methylene group next to the amino group of the piperidine ring, and not associated with the hydrogen of methylene on the bridge ring, and it is determined to be a cis structure. LC-MS: *m/z* = 444.2 $[M+Na]^+$.

Compound **9-2:** $^1$H NMR (400 MHz, $CDCl_3$) δ 7.47 (s, 1H), 6.56 (s, 1H), 5.15-5.25 (m, 1H), 3.97 (s, 3H), 3.91 (s, 3H), 3.65-3.80 (m, 4H), 3.40-3.50 (m, 1H), 2.45-2.60 (m, 2H), 2.40-2.45 (m, 1H), 1.50-1.55 (m, 1H), 1.51 (s, 9H). LC-MS: *m/z* = 444.2 $[M+Na]^+$. The structure of compound **9-2** was deduced by the results of structural identification of compound **10-2.**

The fifth step

Compound **9-2** (580.0 mg, 1.38 mmol) was dissolved in methanol (100 mL), and to the mixture was added wet palladium/carbon (10%, 150 mg). The reaction solution was stirred at 20 °C under hydrogen atmosphere (hydrogen balloon) for 0.5 hour, and then filtered through celite. The filtrate was concentrated under reduced pressure, and dried to afford compound **9-3.** $^1$H NMR (400 MHz, $CDCl_3$) δ 7.04 (s, 1H), 6.10 (s, 1H), 3.83 (s, 3H), 3.82 (s, 3H), 3.70-3.80 (m, 1H), 3.40-3.60 (m, 4H), 2.60-2.70 (m, 2H), 1.85-1.95 (m, 1H), 1.45 (s, 9H), 1.40-1.45 (m, 1H).

The sixth step

Compound **9-3** (500.0 mg, 1.28 mmol) and ammonium acetate (984.6 mg, 12.77 mmol) were added to trimethyl orthoformate (12.47 g, 117.48 mmol). The reaction solution was stirred at 70 °C for 10 hours. The reaction solution was cooled to 20 °C, and concentrated under reduced pressure. The residue was slurried with water (10 mL), filtered, and dried to afford compound **9-4.** $^1$H NMR (400 MHz, DMSO-$d_6$) δ 7.87 (s, 1H), 7.06 (s, 1H), 7.04 (s, 1H), 5.18 (d, *J* = 5.2 Hz, 1H), 3.94 (s, 3H), 3.75-3.80 (m, 1H), 3.35-3.55 (m, 4H), 2.65-2.75 (m, 2H), 1.92-2.00 (m, 1H), 1.40 (s, 9H), 1.30-1.40 (m, 1H). LC-MS: *m/z* = 387.2 $[M+Na]^+$.

The seventh step

Compound **9-4** (400.0 mg, 1.04 mmol) was added to a solution of hydrogen chloride in dioxane (4 N, 10 mL).

The reaction solution was stirred at 0 °C for 0.4 hour. The reaction solution was concentrated under reduced pressure to obtain the hydrochloride salt of compound **9-5.** LC-MS: *m/z* = 287.0 $[M+H]^+$.

The eighth step

At 0 °C, the hydrochloride salt of **9-5** (340.0 mg, 1.19mmol) and triethylamine (1.7 mL, 11.87 mmol) were dissolved in dichloromethane (10 mL), and to the mixture was added trifluoroacetic anhydride (498.8 mg, 2.37 mmol) dropwise. The reaction solution was stirred at 20 °C for 1 hour, and then concentrated under reduced pressure. The residue was slurried with water (40 mL), filtered, and dried to afford compound **9-6.** $^1$H NMR (400 MHz, DMSO-$d_6$) δ 11.93 (s, 1H), 7.87 (s, 1H), 7.02-7.88 (m, 2H), 4.95-5.05 (m, 1H), 3.98 (s, 3H), 3.80-3.95 (m, 3H), 3.60-3.70 (m, 1H), 3.51-3.60 (m, 1H), 2.80-2.90 (m, 2H), 2.00-2.10 (m, 1H), 1.52 (d, *J* = 10.0 Hz, 1H). LC-MS: *m/z* = 383.1 $[M+H]^+$.

The ninth step

Compound **9-6** (280.0 mg, 732.34 μmol) was dissolved in phosphorus oxychloride (20 mL, 215.22 mmol), and to the mixture was added N,N-dimethylformamide (53.5mg, 732.34 μmol). The reaction solution was stirred at 100 °C for 5 hours, and then concentrated under reduced pressure. To the residue was added a solution of 3,4-dichloro-2-fluoroaniline (263.65 mg, 1.46 mmol) in isopropanol (10 mL). The reaction solution was stirred at 90 °C for 1 hour. The reaction solution was cooled to 20 °C, quenched by adding saturated aqueous sodium bicarbonate solution (10 mL), and extracted with ethyl acetate (10 mL x 2). The organic phases were combined, dried over anhydrous sodium sulfate, and filtered. The filtrate was concentrated under reduced pressure, and the residue was isolated by silica gel column chromatography (ethyl acetate:petroleum ether = 2:1) to afford compound **9-7**. $^1$H NMR (400 MHz, DMSO-$d_6$) δ 9.46 (s, 1H), 8.29 (s, 1H), 7.52-7.65 (m, 2H), 7.12-7.20 (m, 2H), 5.64 (d, *J* = 3.6 Hz, 1H), 3.98 (s, 3H), 3.82-3.98 (m, 3H), 3.65-3.75 (m, 1H), 3.55-3.60 (m, 1H), 2.95-3.05 (m, 2H), 2.00-2.10 (m, 1H), 1.56 (d, *J* = 10.0 Hz, 1H). LC-MS: *m/z* = 544.2 $[M+H]^+$.

The tenth step

Compound **9-7** (290.0 mg, 533.74 μmol) was dissolved in methanol (15 mL), and to the mixture was added potassium carbonate (369.0 mg, 2.67 mmol). The reaction solution was stirred at 55 °C for 14 hours. The reaction solution was concentrated under reduced pressure, quenched by adding water (10 mL), and extracted with ethyl acetate (20 mL x 4). The organic phases were combined, dried over anhydrous sodium sulfate, and filtered. The filtrate was concentrated under reduced pressure to afford compound **9-8**. $^1$H NMR (400 MHz, DMSO-$d_6$) δ 9.50 (s, 1H), 8.28 (s, 1H), 7.50-7.65 (m, 2H), 7.16 (s, 1H), 7.14 (s, 1H), 5.91 (d, *J* = 4.8 Hz, 1H), 4.01 (s, 3H), 3.85-3.95 (m, 1H), 3.15-3.20 (m, 2H), 2.80-2.90 (m, 2H), 1.90-2.00 (m, 2H), 1.70-1.80 (m, 1H), 1.10 (d, *J* = 6.4 Hz, 1H). LC-MS: *m/z* = 448.2 $[M+H]^+$.

The eleventh step

Compound **9-8** (200.0 mg, 446.11 μmol) was dissolved in a mixed solvent of tetrahydrofuran (6 mL) and water (6 mL) at 0 °C. To the mixture was added sodium bicarbonate (112.4 mg, 1.34 mmol), and added slowly acryloyl chloride (36.3 mg, 401.50 μmol) dropwise. The reaction solution was further stirred at 0 °C for 0.5 hour. The reaction solution was quenched by adding MeOH (1 mL), and concentrated under reduced pressure. The residue was isolated by preparative thin-layer chromatography (dichloromethane:methanol = 10:1) to obtain the **compound of example** 9. $^1$H NMR (400 MHz, DMSO-$d_6$) δ 9.45 (s, 1H), 8.28 (s, 1H), 7.55-7.65 (m, 2H), 7.17 (s, 1H), 7.12 (s, 1H), 6.75 (dd, $J_1$ = 16.4 Hz, $J_2$ = 10.4 Hz, 1H), 6.18 (dd, $J_1$ = 16.4 Hz, $J_2$=2.4 Hz, 1H), 5.68 (dd, $J_1$ = 10.4 Hz, $J_2$ = 2.4 Hz, 1H), 5.40 (d, *J* = 4.8 Hz, 1H), 3.96 (s, 3H), 3.80-3.90 (m, 2H), 3.65-3.75 (m, 2H), 3.45-3.52 (m, 1H), 2.90-3.05 (m, 2H), 1.90-2.00 (m, 1H), 1.47 (d, *J* = 9.6 Hz, 1H). LC-MS: *m/z* = 502.1 $[M+H]^+$.

Example 10

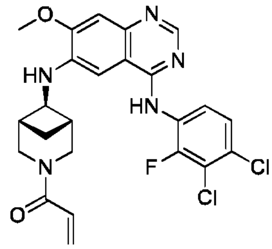

Synthetic route:

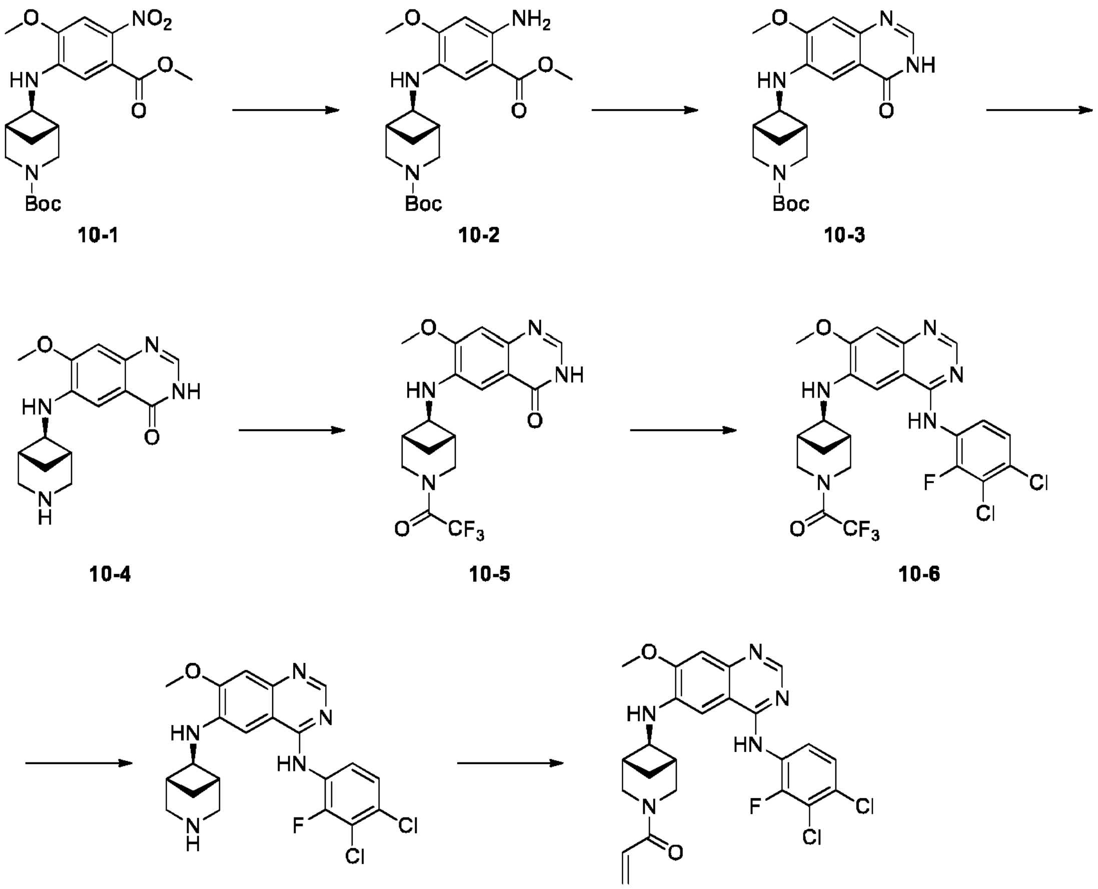

10-1 10-2 10-3

10-4 10-5 10-6

10-7 Example 10

The first step

Compound **10-2** was obtained by referring to the fifth step of **Example 9**. $^1$H NMR (400 MHz, $CDCl_3$) δ 6.78 (s, 1H), 6.13 (s, 1H), 3.86 (s, 3H), 3.84 (s, 3H), 3.60-3.80 (m, 4H), 3.32-3.36 (m, 1H), 2.50-2.60 (m, 1H), 2.40-2.48 (m, 1H), 2.30-2.40 (m, 1H), 1.51 (s, 9H), 1.40-1.45 (m, 1H).

The second step

Compound **10-3** was obtained by referring to the sixth step of **Example 9**. $^1$H NMR (400 MHz, DMSO-$d_6$) δ 7.85 (s, 1H), 7.05 (s, 1H), 6.72 (s, 1H), 5.95 (d, $J$ = 3.2 Hz, 1H), 3.97 (s, 3H), 3.55-3.80 (m, 4H), 3.25-3.30 (m, 1H), 2.51-2.60 (m, 2H), 1.75-1.80 (m, 1H), 1.46 (s, 9H), 1.35-1.40 (m, 1H). LC-MS: $m/z$ = 387.2 [M+Na]$^+$.

The third step

The hydrochloride salt of compound **10-4** was obtained by referring to the seventh step of **Example 9**. LC-MS: *m*/*z* = 287.0 $[M+H]^+$.

The fourth step

Compound **10-5** was obtained by referring to the eighth step of **Example 9**. $^1H$ NMR (400 MHz, DMSO-$d_6$) δ 11.92 (s, 1H), 7.85 (d, *J* = 3.2 Hz, 1H), 7.06 (s, 1H), 6.76 (s, 1H), 6.02 (d, *J* = 3.2 Hz, 1H), 4.05-4.15 (m, 2H), 3.98 (s, 3H), 3.85-3.95 (m, 1H), 3.75-3.80 (m, 1H), 3.45-3.50 (m, 1H), 2.55-2.70 (m, 3H), 1.50-1.60 (m, 1H). LC-MS: *m*/*z* = 383.1 $[M+H]^+$.

The fifth step

Compound **10-6** was obtained by referring to the ninth step of **Example 9**. $^1H$ NMR (400 MHz, DMSO-$d_6$) δ 9.35 (s, 1H), 8.29 (s, 1H), 7.60-7.70 (m, 1H), 7.55-7.60 (m, 1H), 7.15 (s, 1H), 6.81 (s, 1H), 6.18 (d, *J*= 3.6 Hz, 1H), 4.10-4.25 (m, 2H), 4.03 (s, 3H), 3.80-4.00 (m, 2H), 3.50-3.63 (m, 1H), 2.65-2.75 (m, 2H), 2.55-2.60 (m, 1H), 1.50-1.56 (m, 1H). LC-MS: *m*/*z* = 544.2 $[M+H]^+$.

The sixth step

Compound **10-7** was obtained by referring to the tenth step of **Example 9**. $^1H$ NMR (400 MHz, DMSO-$d_6$) δ 9.69 (s, 1H), 8.26 (s, 1H), 7.50-7.65 (m, 2H), 7.12 (s, 1H), 6.95 (d, *J* = 8.0 Hz, 1H), 4.10-4.20 (m, 1H), 4.01 (s, 3H), 3.60-3.70 (m, 2H), 3.20-3.30 (m, 2H), 2.40-2.50 (m, 3H), 1.60-1.70 (m, 1H). LC-MS: *m*/*z* = 448.2 $[M+H]^+$.

The seventh step

**Compound of example 10** was obtained by referring to the eleventh step of **Example 9**. $^1H$ NMR (400 MHz, DMSO-$d_6$) δ 9.38 (s, 1H), 8.27 (s, 1H), 7.55-7.65 (m, 2H), 7.14 (s, 1H), 6.75-6.85 (m, 2H), 6.18 (dd, $J_1$ = 16.8 Hz, $J_2$=2.4 Hz, 1H), 6.14 (d, $J_1$ = 4.0 Hz, 1H), 5.73 (dd, $J_1$ = 10.4 Hz, $J_2$ = 2.4 Hz, 1H), 4.10-4.20 (m, 1H), 4.02 (s, 3H), 3.85-3.95 (m, 2H), 3.75-3.80 (m, 1H), 3.50-3.55 (m, 1H), 2.50-2.65 (m, 3H), 1.40-1.50 (m, 1H). LC-MS: *m*/*z* = 502.1 $[M+H]^+$.

Example 11

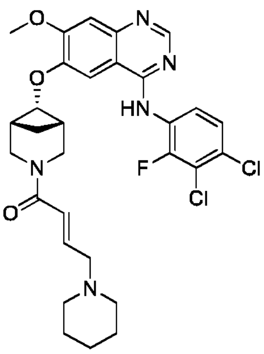

Synthetic route:

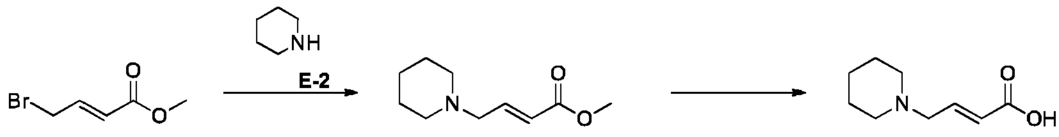


E-1 E-3 E-4

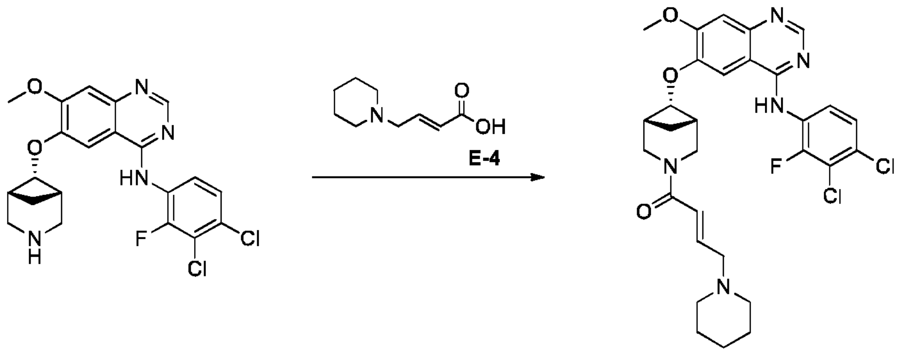


1-10 Example 11

The first step

Compound **E-1** (500.0 mg, 2.79 mmol) was added to dichloromethane (6 mL), and compound **E-2** (475.7 mg, 5.59 mmol) was added under nitrogen protection. The reaction solution was stirred at 25 °C for 2 hours. The reaction solution was concentrated under reduced pressure, and the residue was isolated by silica gel column chromatography (petroleum ether:ethyl acetate = 3:1) to afford compound **E-3**. $^1$H NMR (400 MHz, $CDCl_3$) $\delta$ 7.03-6.95 (m, 1H), 5.89-6.03 (m, 1H), 3.72 (s, 3H), 3.09 (dd, $J_1$ = 6.4 Hz, $J_2$ = 1.6 Hz, 2H), 2.20-2.50 (m, 4H), 1.63-1.56 (m, 4H), 1.38-1.46 (m, 2H). LC-MS: $m/z$ = 184.0 $[M+H]^+$.

The second step

To a solution of compound **E-3** (367.0 mg, 2.00 mmol) dissolved in tetrahydrofuran (10 mL) and water (10 mL) was added lithium hydroxide monohydrate (168.1 mg, 4.01 mmol). The reaction solution was stirred at 25 °C for 1 hour, and then extracted with ethyl acetate (10 mL). The aqueous phase was adjusted to pH of 3 with dilute hydrochloric acid and lyophilized to afford compound **E-4**. $^1$H NMR (400 MHz, DMSO-$d_6$) $\delta$ 11.28 (brs, 1H), 7.10-6.86 (m, 1H), 6.17 (d, $J$ = 15.6 Hz, 1H), 3.85 (d, $J$ = 6.8 Hz, 2H), 3.31-3.20 (m, 4H), 1.61-1.89 (m, 6H). LC-MS: $m/z$ = 170.2 $[M+H]^+$.

The third step

Compound **1-10** (50.0 mg, 111.28 μmol), HATU (63.5 mg, 166.92 μmol) and DIEA (36.0 mg, 278.21 μmol) were dissolved in N,N-dimethylformamide (8 mL), and to the mixture was added compound **E-4** (56.5 mg, 333.85 μmol). The reaction solution was stirred at 25 °C for 16 hours. The reaction solution was concentrated under reduced pressure, and the residue was isolated by preparative high performance liquid chromatography (basic) to afford **compound of example 11**. $^1$H NMR (400MHz, DMSO-$d_6$) $\delta$ 9.69 (brs, 1H), 8.39 (s, 1H), 7.74 (s, 1H), 7.60 (s, 1H), 7.23 (s, 1H), 6.45-6.71 (m, 2H), 4.69 (s, 1H), 3.80-4.00 (m, 4H), 3.62-3.80 (m, 3H), 2.90-3.10 (m, 4H), 2.25-2.35 (m, 3H), 1.78-1.85 (m, 1H), 1.33-1.52 (m, 7H). LC-MS: $m/z$ = 600.2 $[M+H]^+$.

Example 12

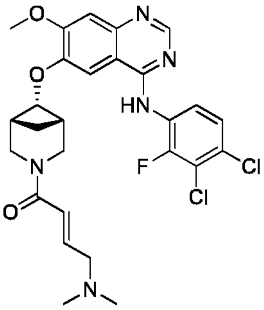

Synthetic route:

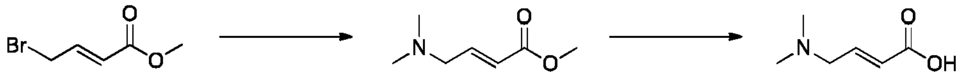

**F-1** **F-2** **F-3**

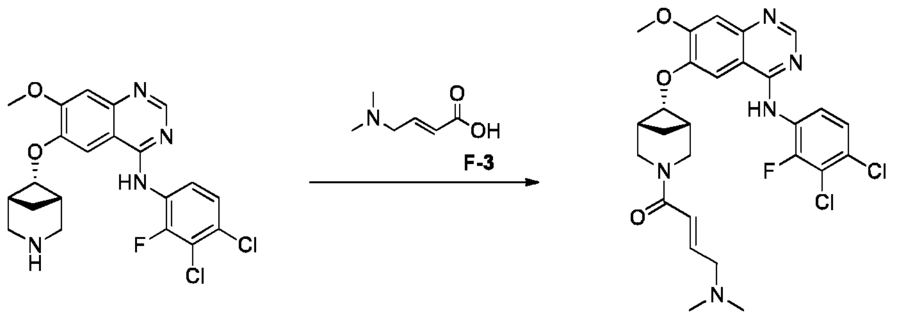


**1-10** Example 12

The first step

Compound **F-1** (1.00 g, 5.59 mmol) and DIEA (1.44 g, 11.17 mmol) were dissolved in tetrahydrofuran (10 mL), and to the mixture was added an aqueous solution of dimethylamine (33%, 1.53 g, 11.17 mmol) dropwise. The reaction solution was stirred at 70 °C for 1 hour. The reaction solution was cooled to room temperature, quenched with water (10 mL), and extracted with ethyl acetate (15 mL x 2). The organic phases were combined and concentrated under reduced pressure. The residue was isolated by preparative thin-layer chromatography (petroleum ether: ethyl acetate = 0:1) to afford compound **F-2**. $^1$H NMR (400MHz, $CDCl_3$) δ 7.00-6.80 (m, 1H), 6.00-5.85 (m, 1H), 3.67 (s, 3H), 3.07-2.93 (m, 2H), 2.18 (s, 6H).

The second step

Compound **F-3** was obtained by referring to the second step of Example 11. $^1$H NMR (400 MHz, DMSO-$d_6$) δ 11.42 (brs, 1H), 6.78-6.94 (m, 1H), 6.19 (d, *J* = 15.6 Hz, 1H), 3.87-3.91 (m, 2H), 2.73-2.60 (m, 6H).

The third step

**Compound of example 12** was obtained by referring to the third step of Example 11. $^1$H NMR (400 MHz, DMSO-$d_6$) δ 8.35 (s, 1H), 7.73 (s, 1H), 7.51-7.63 (m, 2H), 7.20 (s, 1H), 6.59-6.69 (m, 1H), 6.47-6.56 (m, 1H), 4.68 (t, *J* = 5.6 Hz, 1H), 3.84-3.94 (m, 4H), 3.64-3.78 (m, 2H), 3.50-3.45 (m, 1H), 2.98-3.10 (m, 4H), 2.14 (s, 6H), 1.74-1.91 (m, 1H), 1.50 (d, *J* = 10.0 Hz, 1H).

Example 13

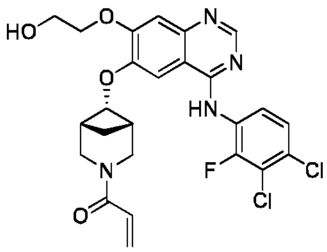

Synthetic route:

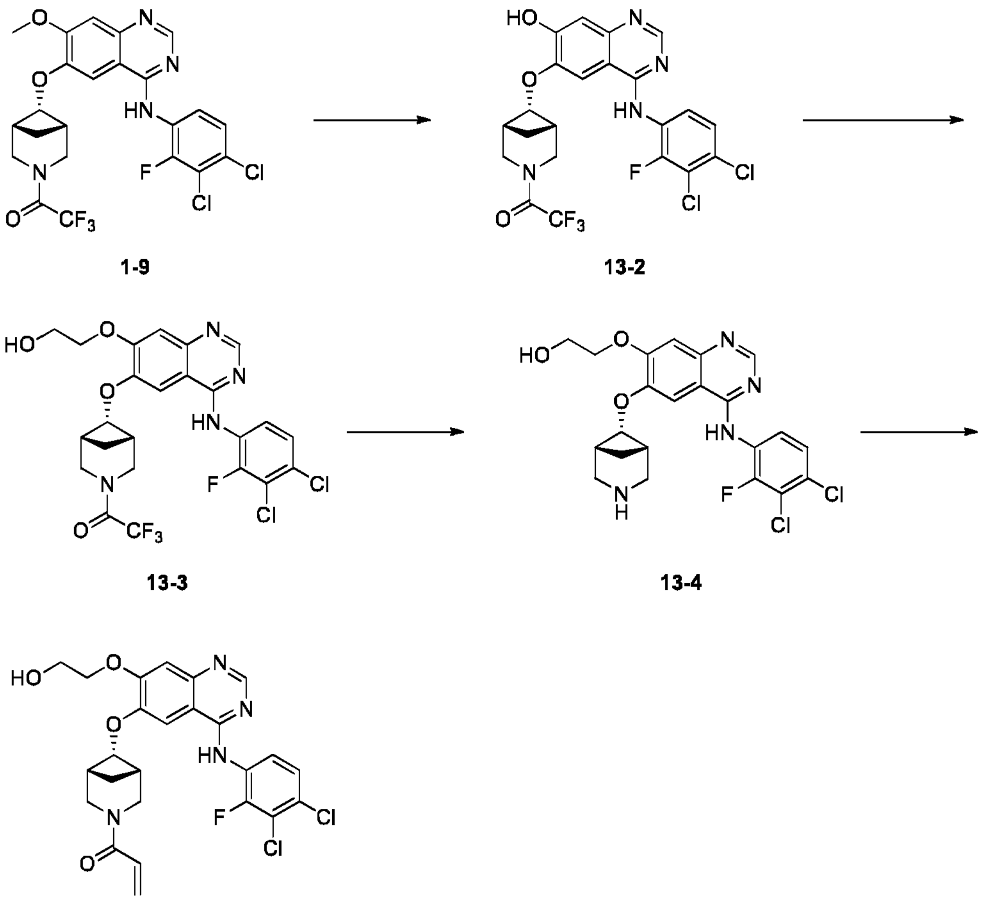

1-9

13-2

13-3

13-4

Example 13

The first step

Compound **1-9** (6.80 g, 10.62 mmol) was added to pyridine hydrochloride (57.94 g, 501.35 mmol). The reaction solution was stirred at 180-185 °C for 1 hour. The reaction solution was cooled to room temperature, slurried with water (30 mL) and ethyl acetate (30 mL) at 20-25 °C for 1 hour, filtered, and dried to afford compound **13-2**. LC-MS: *m/z* 530.9 $[M+H]^+$.

The second step

Compound **13-2** (500.0 mg, 941.11 μmol) was dissolved in N,N-dimethylformamide (5mL), and to the mixture

were added 1,3-dioxolan-2-one (165.8 mg, 1.88 mmol) and potassium fluoride (54.7 mg, 941.11 μmol). The reaction solution was stirred at 110 °C for 18 hours, and potassium fluoride (164.0 mg, 2.82 mmol) and 1,3-dioxolan-2-one (414.4 mg, 4.71 mmol) were added again. The reaction solution was stirred at 110 °C for 3 hours. The reaction solution was concentrated under reduced pressure, and the residue was isolated by preparative thin-layer chromatography (dichloromethane:methanol = 15:1) to afford compound **13-3**. LC-MS: *m/z* = 575.2 $[M+H]^+$.

The third step

Compound **13-3** (170.0 mg, 295.48 μmol) was dissolved in methanol (10 mL), and to the mixture was added potassium carbonate (163.4 mg, 1.18 mmol). The reaction solution was stirred at 80 °C for 3 hours. The reaction solution was concentrated under reduced pressure, and the residue was isolated by preparative thin-layer chromatography (dichloromethane:methanol = 15:1) to afford compound **13-4**. LC-MS: *m/z* = 479.2$[M+H]^+$.

The fourth step

**Compound of example 13** was obtained by referring to the tenth step of Example 1. $^1H$ NMR (400 MHz, DMSO-$d_6$) δ 9.69 (s, 1H), 8.39 (s, 1H), 7.78 (s, 1H), 7.64-7.57 (m, 2H), 7.24 (s, 1H), 6.74 (dd, $J_1$ = 16.8 Hz, $J_2$ = 10.4 Hz, 1H), 6.16 (dd, $J_1$ = 16.8 Hz, $J_2$ = 2.4 Hz, 1H), 5.68 (dd, $J_1$ = 10.4 Hz, $J_2$ = 2.4 Hz, 1H), 4.84 (t, $J$ = 5.2 Hz, 1H), 4.73-4.71 (m, 1H), 4.11 (t, $J$ = 5.2 Hz, 2H), 4.00-3.90 (m, 1H), 3.76-3.70 (m, 4H), 3.49 (d, $J$ = 13.2 Hz, 1H), 3.05 (brs, 2H), 1.86-1.77 (m, 1H), 1.51 (d, $J$ = 10.0 Hz, 1H). LC-MS: *m/z* = 533.3 $[M+H]^+$.

Example 14

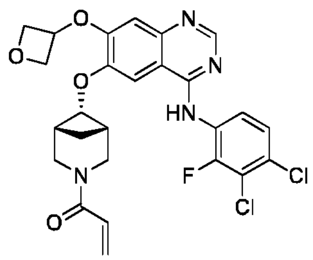

Synthetic route:

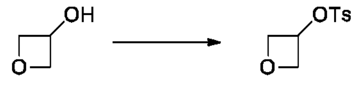

G-1 G-2

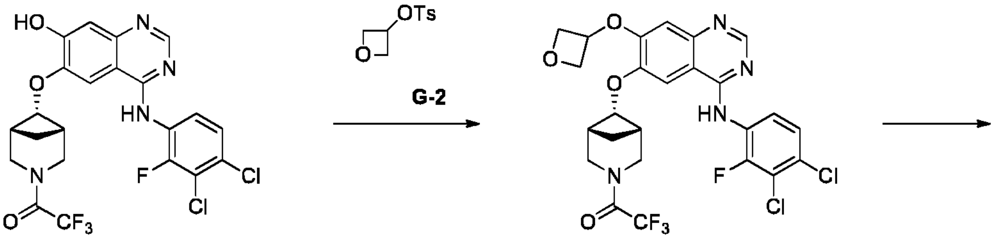


13-2 14-2

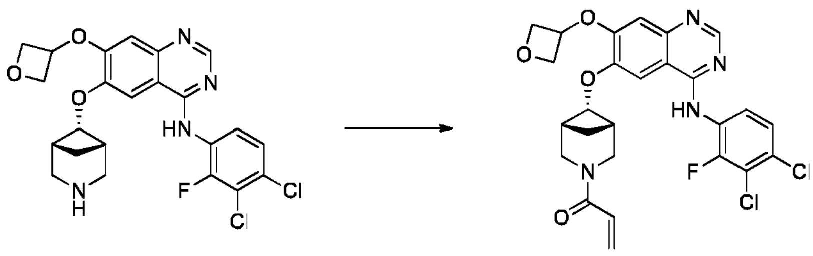

14-3 Example 14

The first step

Compound **G-1** (3.00 g, 40.50 mmol) was dissolved in dichloromethane (30 mL), and to the mixture were added 4-methylbenzenesulfonyl chloride (7.72g, 40.50 mmol), triethylamine (10.24 g, 101.24 mmol) and 4-dimethylaminopyridine (247.4 mg, 2.02 mmol). The reaction solution was stirred at room temperature for 8 hours, concentrated under reduced pressure, slurried with water (150 mL), and filtered. The filter cake was washed with water and dried to afford compound **G-2**. $^1$H NMR (400 MHz, $CDCl_3$) δ 7.79 (d, *J* = 8.0 Hz, 2H), 7.37 (d, *J* = 8.4 Hz, 2H), 5.38-5.23 (m, 1H), 4.80-4.60 (m, 4H), 2.47 (s, 3H).

The second step

Compound **13-2** (200.0 mg, 376.44 μmol) was dissolved in N,N-dimethylformamide (5 mL), and to the mixture were added potassium fluoride (65.6 mg, 1.13 mmol) and compound **G-2** (257.8 mg, 1.13 mmol). The reaction solution was stirred at 110 °C for 18 hours. To the mixture were added potassium fluoride (65.6 mg, 1.13 mmol), potassium carbonate (52.0 mg, 376.44 μmol) and compound **G-2** (257.8 mg, 1.13 mmol). The reaction solution was stirred at 110 °C for 5 hours. To the mixture were added compound **G-2** (100.0 mg, 438.09 μmol) and potassium carbonate (30.0 mg, 217.07 μmol). The reaction solution was concentrated under reduced pressure, and the residue was isolated by preparative thin-layer chromatography (dichloromethane:methanol = 15:1) to afford compound **14-2**. LC-MS: *m/z* = 587.2 $[M+H]^+$.

The third step

Compound **14-3** was obtained by referring to the third step of Example 13. LCMS: *m/z* = 587.2 $[M+H]^+$.

The fourth step

**Compound of example 14** was obtained by referring to the tenth step of Example 1. $^1$H NMR (400 MHz, DMSO-$d_6$) δ 9.74 (s, 1H), 8.39 (s, 1H), 7.86 (s, 1H), 7.60 (s, 2H), 6.88 (s, 1H), 6.75 (dd, $J_1$ = 16.8 Hz, $J_2$ = 10.4 Hz, 1H), 6.18 (dd, $J_1$ = 16.8 Hz, $J_2$ = 2.4 Hz, 1H), 5.68 (dd, $J_1$ = 10.4 Hz, $J_2$ = 2.4 Hz, 1H), 5.51-5.40 (m, 1H), 5.02-4.90 (m, 2H), 4.85-4.75 (m, 1H), 4.57-4.47 (m, 2H), 3.96-3.85 (m, 1H), 3.77-3.66 (m, 2H), 3.56-3.46 (m, 1H), 3.06 (m, 2H), 1.89-1.78

(m, 1H), 1.53 (d, $J$ = 10.4 Hz, 1H). LC=MS: $m/z$ = 545.3 $[M+H]^+$.

Example 15

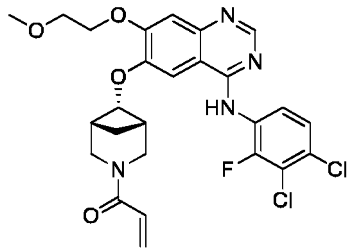

Synthetic route:

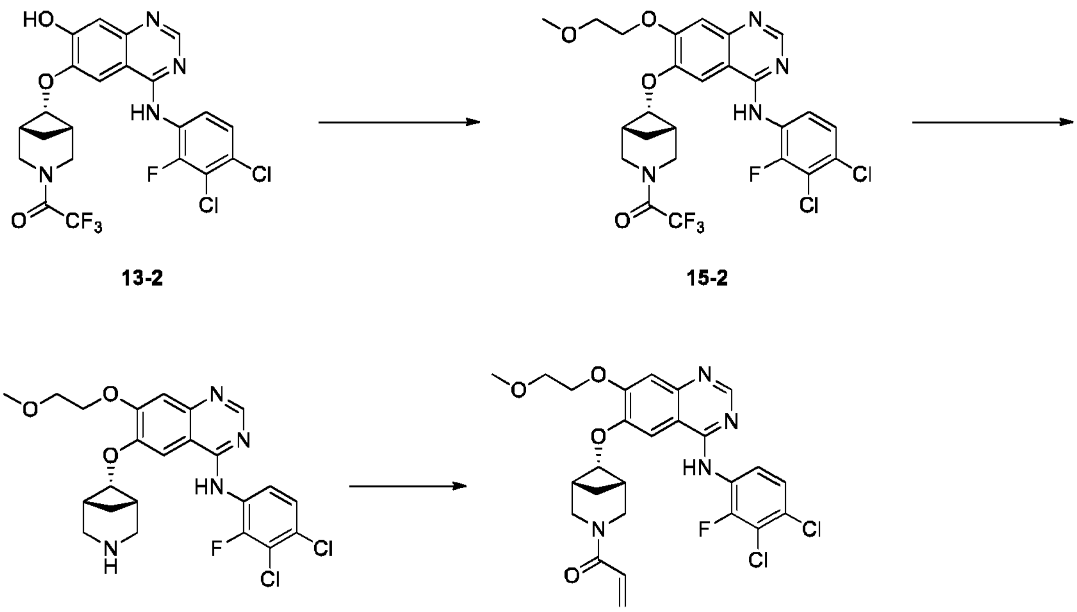

**13-2** **15-2**

**15-3** Example 15

The first step

Compound **13-2** (200.0 mg, 376.44 μmol) was dissolved in N,N-dimethylformamide (2 mL), and then to the mixture were added sodium iodide (112.9 mg, 752.89 μmol), potassium fluoride (87.5 mg, 1.51 mmol) and 1-bromo-2-methoxyethane (130.8 mg, 941.11 μmol) at room temperature. The reaction solution was stirred at 110 °C for 5 hours. To the mixture were added potassium fluoride (87.5 mg, 1.51 mmol) and 1-bromo-2-methoxyethane (157.0 mg, 1.13 mmol) again, and the reaction solution was further stirred at 110 °C for 18 hours. The reaction solution was concentrated under reduced pressure, and isolated by preparative thin-layer chromatography (dichloromethane:methanol = 7:1) to afford compound **15-2**. LC-MS: $m/z$ = 589.3$[M+H]^+$.

The second step

Compound **15-3** was obtained by referring to the third step of Example 13.

The third step

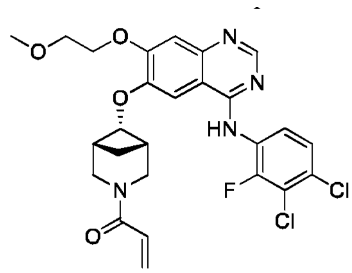

**Compound of example 15** was obtained by referring to the tenth step of Example 1. $^1$H NMR (400 MHz, DMSO-$d_6$) δ 7.89 (s, 1H), 7.64 (s, 1H), 7.33 (d, $J$ = 8.4 Hz, 1H), 7.03 (s, 1H), 6.91 (s, 1H), 6.74 (dd, $J_1$ = 16.8 Hz, $J_2$ =10.4 Hz, 1H), 6.16 (dd, $J_1$ = 16.8 Hz, $J_2$ = 2.4 Hz, 1H), 5.71-5.65 (m, 1H), 4.70-4.63 (m, 1H), 4.15-4.25 (m, 2H), 3.80-3.90 (m, 2H), 3.65-3.75 (m, 1H), 3.59 (t, $J$ = 4.8 Hz, 2H), 3.51-3.46 (m, 1H), 3.23 (s, 3H), 2.84-2.90 (m, 2H), 1.80-1.85 (m, 1H), 1.47-1.40 (m, 1H); LC-MS: $m/z$ = 547.2[M+H]$^+$.

Example 16

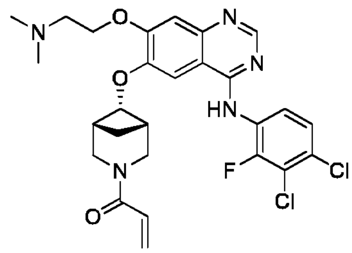

Synthetic route:

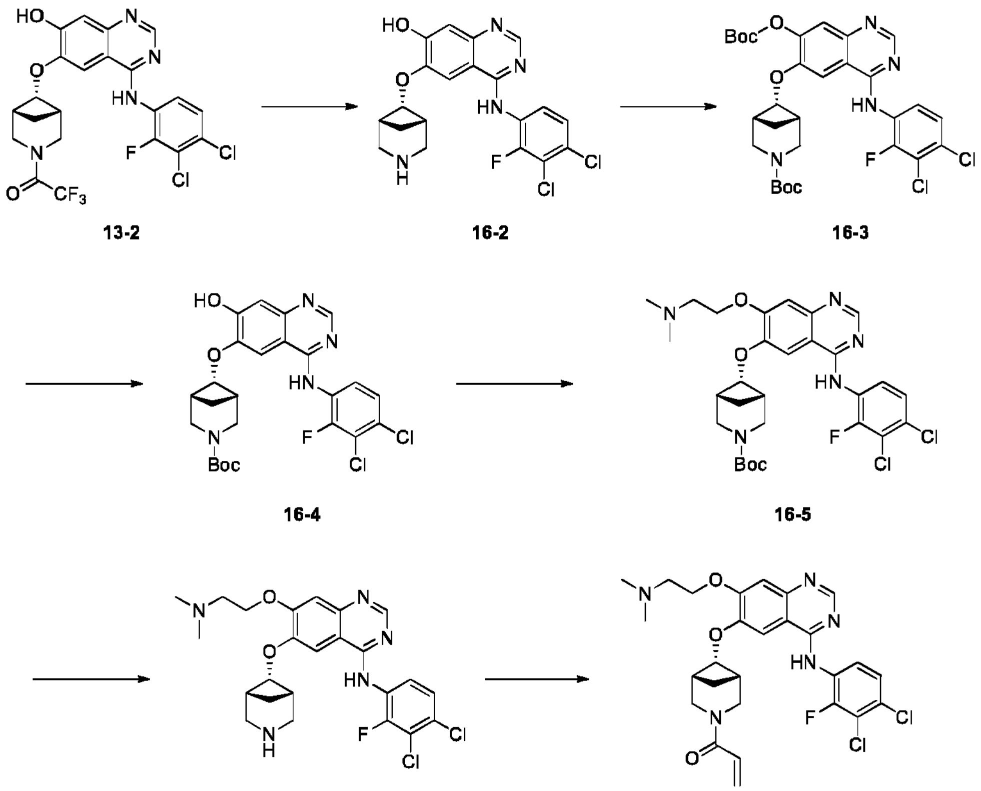

13-2 16-2 16-3

16-4 16-5

16-6 Example 16

The first step

Compound **13-2** (1.00 g, 1.88 mmol) was dissolved in methanol (20 mL), and then to the mixture was added potassium carbonate (1.30 g, 9.41 mmol) in one portion at 20-25 °C. The reaction solution was stirred at 20-25 °C for 18 hours, and then heated to and reacted at 50 °C for 5 hours. The reaction solution was filtered, and the filtrate was concentrated under reduced pressure. The residue was diluted with saturated aqueous citric acid solution (50 mL) and extracted with ethyl acetate (50 mL x 3). The organic phases were combined, washed with saturated brine (50 mL), dried over anhydrous sodium sulfate, and filtered. The filtrate was concentrated under reduced pressure to afford compound **16-2**.

The second step

Compound **16-2** (850.0 mg, 1.95 mmol) was dissolved in methanol (10 mL), and to the mixture were added triethylamine (988.0 mg, 9.76 mmol) and di-tert-butyl dicarbonate (1.28 g, 5.86 mmol) at 20 °C. The reaction solution was stirred at 20-25 °C for 2 hours. The reaction solution was concentrated under reduced pressure, slurried with water (20 mL) for 0.5 hour, and filtered. The filter cake was dried to afford compound **16-3**. LC-MS: *m*/*z* = 635.3 $[M+H]^+$.

The third step

Compound **16-3** (1.00 g, 1.57 mmol) was dissolved in methanol (15 mL), and then to the mixture was added potassium carbonate (1.09 g, 7.87 mmol) in one portion at 20-25 °C. The reaction solution was stirred at 20-25 °C for 18 hours. The reaction solution was concentrated under reduced pressure, and the residue was adjusted to pH of 5 with saturated citric acid aqueous solution. The reaction solution was stirred at 20-25 °C for 0.5 hour and then filtered. The filter cake was dried to afford compound **16-4**. LC-MS: *m*/*z* = 535.3$[M+H]^+$.

The fourth step

Compound **16-4** (150.0 mg, 280.17 μmol) was dissolved in N,N-dimethylformamide (3 mL), and then to the mixture was added sodium hydride (60%, 28.0 mg, 700.42 μmol) in one portion at 0 °C. The reaction solution was stirred at 0 °C for 0.5 hour, and then 2-bromo-N,N-dimethylethylamine (55.4 mg, 364.22 μmol) was added. The reaction solution

was stirred at 20 °C for 2 hours, quenched by adding water (2 mL), and extracted with ethyl acetate (3 mL x 3). The organic phases were combined, dried over anhydrous sodium sulfate, and filtered. The filtrate was concentrated under reduced pressure, and the residue was isolated by preparative thin-layer chromatography (dichloromethane:methanol = 5:1) to afford compound **16-5**. LC-MS: *m*/*z* = 606.4 $[M+H]^+$.

The fifth step

Compound **16-5** (60.0 mg, 98.93 μmol) was dissolved in a solution of hydrogen chloride in dioxane (4 N, 4 mL), and stirred at 20-25 °C for 0.5 hour. The reaction solution was concentrated under reduced pressure to obtain the hydrochloride salt of compound **16-6**. LC-MS: *m*/*z* = 506.3 $[M+H]^+$.

The sixth step

The hydrochloride salt of compound **16-6** (50.0 mg, 92.10 μmol) was dissolved in tetrahydrofuran (0.5 mL) and water (0.5 mL). To the mixture was added sodium bicarbonate (38.7 mg, 460.52 μmol) at 0 °C, and added slowly acryloyl chloride (3.3 mg, 36.84 μmol) dropwise. The reaction solution was stirred at 0 °C for 0.5 hour and at 20-25 °C for 18 hours. Acryloyl chloride (2.2 mg, 23.95 μmol) was added again, and the reaction solution was further stirred at 20-25 °C for 2 hours. The reaction solution was concentrated under reduced pressure, and the residue was isolated by preparative thin-layer chromatography (dichloromethane:methanol = 3:1) to afford **compound of example 16**. $^1$H NMR (400MHz, $CD_3OD$) δ 8.34 (s, 1H), 7.58 (s, 1H), 7.50-7.47 (m, 2H), 6.69 (s, 1H), 6.21-6.12 (m, 1H), 6.11-6.04 (m, 1H), 5.75(dd, $J_1$ = 16.8 Hz, $J_2$=2.4 Hz, 1H), 5.58-5.51 (m, 1H), 4.73-4.65 (m, 1H), 4.39 (t, *J* = 6.2 Hz, 2H), 4.19 (d, *J* = 10.8 Hz, 1H), 3.96-3.83 (m, 2H), 3.66-3.58 (m, 1H), 3.22-3.13 (m, 1H), 3.05-3.10 (m, 1H), 2.79 (t, *J* = 6.2 Hz, 2H), 2.32 (s, 6H), 2.00-1.91 (m, 1H), 1.59 (d, *J* = 10.0 Hz, 1H). LC-MS: *m*/*z* = 560.4 $[M+H]^+$.

Example 17

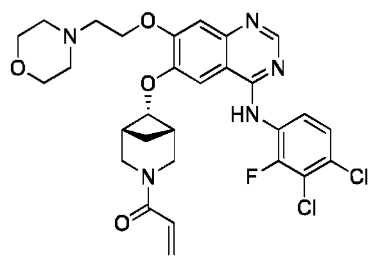

Synthetic route:

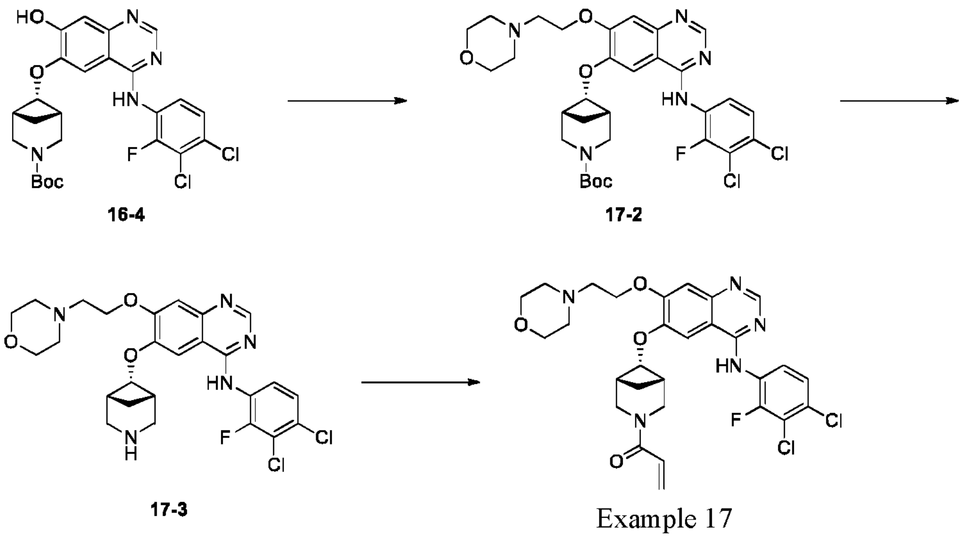

16-4

17-2

17-3

Example 17

The first step

Compound 16-4 (150.0 mg, 280.17 μmol) was dissolved in N,N-dimethylformamide (3 mL), and to the mixture was added sodium hydride (60%, 44.82 mg, 1.12 mmol) in one portion at 0 °C. The reaction solution was stirred at 0 °C for 0.5 hour, and 4-(2-bromoethyl)morpholine (163.1 mg, 840.50 μmol) was added. The reaction solution was further stirred at 0 °C for 2 hours. The reaction solution was quenched with water (20 mL) and extracted with ethyl acetate (20 mL x 3). The organic phases were combined, washed with saturated brine (30 mL), dried over anhydrous sodium sulfate, and filtered. The filtrate was concentrated under reduced pressure, and the residue was isolated by preparative thin-layer chromatography (dichloromethane:methanol = 10:1) to afford compound **17-2**. LC-MS: *m/z* = 648.4[M+H]$^+$.

The second step

Compound **17-3** was obtained by referring to the fifth step of Example 16. LC-MS: *m/z* = 548.3 [M+H]$^+$.

The third step

**Compound of example 17** was obtained by referring to the tenth step of Example 1. $^1$H NMR (400MHz, $CD_3OD$) δ 8.39 (s, 1H), 7.77 (s, 1H), 7.67-7.55 (m, 1H), 7.49-7.45 (m, 1H), 7.22 (s, 1H), 6.79 (dd, $J_1$ = 16.8 Hz, $J_2$ = 10.4 Hz, 1H), 6.34 (dd, $J_1$ = 16.8 Hz, $J_2$ = 2.4 Hz, 1H), 5.80 (dd, $J_1$ = 10.4 Hz, $J_2$ = 2.4 Hz, 1H), 4.40-4.25 (m, 2H), 3.92-3.81 (m, 2H), 3.75-3.68 (m, 6H), 3.60-3.56 (m, 2H), 3.17-3.05 (m, 2H), 2.89 (t, $J$ = 5.2 Hz, 2H), 2.69-2.62 (m, 3H), 2.07-1.90 (m, 1H), 1.62 (d, $J$= 10.4 Hz, 1H). LC-MS: *m/z* = 602.4 [M+H]$^+$.

Example 18

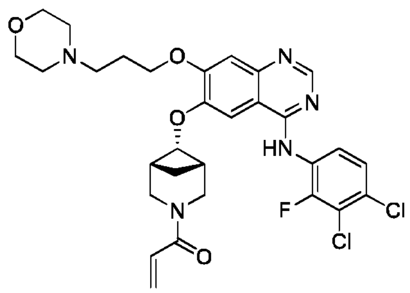

Synthetic route:

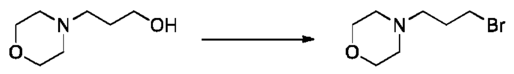

H-1 H-2

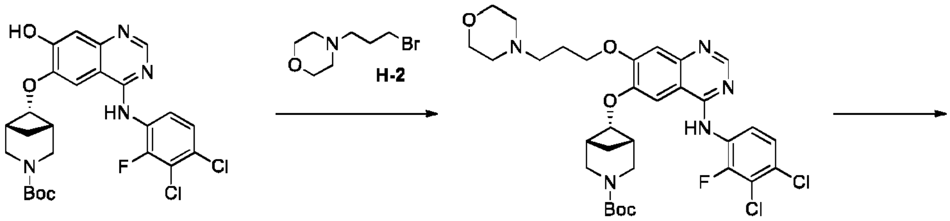


16-4 18-2

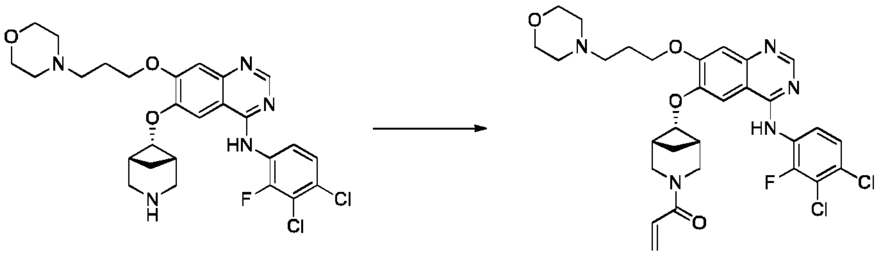

18-3 Example 18

The first step

**H-1** (2.00 g, 13.77 mmol) was dissolved in tetrahydrofuran (20 mL), and to the mixture was added triphenylphosphine (3.97 g, 15.15 mmol). The reaction solution was cooled to 0 °C. Under nitrogen protection, carbon tetrabromide (5.02 g, 15.15 mmol) was added to the reaction solution in batches. The reaction solution was warmed to room temperature and stirred for 10 hours. The reaction solution was concentrated under reduced pressure, diluted with water (50 mL), and extracted with ethyl acetate (30 mL x 3). The aqueous phase was adjusted to pH of 11 with 4N sodium hydroxide solution, and concentrated to remove water. Methanol (100 mL) was added to the solid. The mixture was stirred at room temperature for 15 minutes, and filtered. The filtrate was concentrated under reduced pressure, and to the residue was added dichloromethane (50 mL). The mixture was further stirred for 15 minutes, and filtered. The filtrate was concentrated under reduced pressure to afford compound **H-2**. $^1H$ NMR (400MHz, $CDCl_3$) $\delta$ 3.76-3.66 (m, 4H), 3.48 (t, *J* = 6.4 Hz, 2H), 2.51-2.46 (m, 2H), 2.46-2.41 (m, 4H), 2.11-1.97 (m, 2H).

The second step

Compound **16-4** (200.0 mg, 373.56 $\mu$mol) was dissolved in N,N-dimethylformamide (3 mL), and to the mixture was added sodium hydride (60%, 44.8 mg, 1.12 mmol) in one portion at 0 °C. The reaction solution was stirred for 0.5 hour, and then compound **H-2** (155.5 mg, 747.11 $\mu$mol) was added. The reaction solution was stirred at 0 °C for 1.5 hours. The reaction solution was diluted with water (5 mL) and extracted with ethyl acetate (15 mL x 3). The organic phases were combined, washed with saturated brine (20 mL), dried over anhydrous sodium sulfate, and filtered. The filtrate was concentrated under reduced pressure, and the residue was isolated by preparative thin-layer chromatography (dichloromethane:methanol = 10:1) to afford compound **18-2**. LC-MS: *m/z* = 662.5 [M+H]

The third step

The hydrochloride salt of compound **18-3** was obtained by referring to the fifth step of Example 16. LC-MS: *m/z* = 562.3 [M+H]

The fourth step

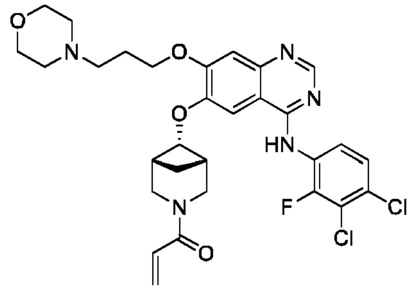

**Compound of example 18** was obtained by referring to the tenth step of Example 1. $^1$H NMR (400MHz, $CD_3OD$) δ 8.39 (s, 1H), 7.75 (s, 1H), 7.61-7.59 (m, 1H), 7.49-7.46 (m, 1H), 7.21 (s, 1H), 6.79 (dd, $J_1$ = 16.8 Hz, $J_2$ = 10.4 Hz, 1H), 6.32 (dd, $J_1$ = 16.8 Hz, $J_2$ = 2.0 Hz , 1H), 5.78 (dd, $J_1$ = 10.4 Hz, $J_2$ = 2.0 Hz, 1H), 4.27-4.17 (m, 2H), 4.03-3.94 (m, 1H), 3.92-3.83 (m, 2H), 3.77-3.60 (m, 5H), 3.37-3.35 (m, 1H), 3.10 (t, $J$ = 5.4 Hz, 2H), 2.65-2.44 (m, 6H), 2.12-1.94 (m, 3H), 1.63 (d, $J$ = 10.0 Hz, 1H). LC-MS: $m/z$ = 616.4 $[M+H]^+$.

Example 19

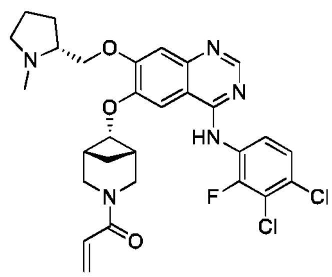

Synthetic route:

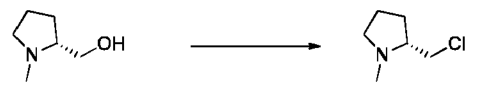

I-1 I-2

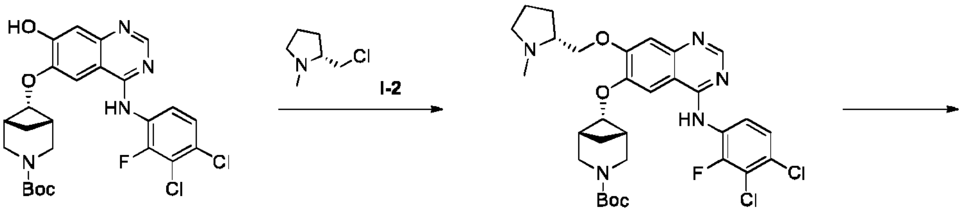


16-4 19-2

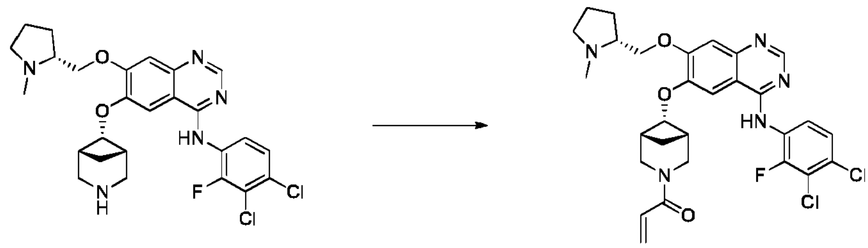

19-3 Example 19

The first step

Compound **I-1** (300.0 mg, 2.60 mmol) was added to toluene (5 mL), and to the mixture was added thionyl chloride (309.8 mg, 2.60 mmol). The mixture was stirred at 70 °C for 3 hours. The reaction solution was concentrated under reduced pressure to afford compound **I-2**. $^1$H NMR (400MHz, DMSO-$d_6$) δ 4.16 (dd, $J_1$ = 12.0 Hz, $J_2$ = 6.0 Hz, 1H), 4.00 (dd, $J_1$ = 12.0 Hz, $J_2$ = 6.0 Hz, 1H), 3.70 (m, 1H), 3.56 (m, 1H), 3.09 (m, 1H), 2.87 (s, 3H), 2.36-2.21 (m, 1H), 2.07-1.72 (m, 4H).

The second step

Compound **16-4** (100.0 mg, 186.78 μmol) was added to N,N-dimethylformamide (1.0 mL). To the mixture was added sodium hydride (60%, 29.8 mg, 747.12 μmol) at 0 °C. After stirring for 0.2 hour, the reaction solution was warmed to 16 °C. Compound **I-2** (49.9 mg, 373.56 μmol) was added and stirred at 40 °C for 0.5 hour. The reaction solution was quenched with water (10 mL) and extracted with ethyl acetate (10 mL x 3). The organic phases were combined, washed sequentially with water (10 mL) and saturated brine (10 mL), dried over anhydrous sodium sulfate, and filtered. The filtrate was concentrated under reduced pressure, and the residue was isolated by preparative high performance liquid chromatography (basic) to afford compound **19-2**. LC-MS: m/z = 632.5 [M+H]$^+$.

The third step

Compound **19-2** (33.0 mg, 52.17 μmol) was added to a solution of hydrogen chloride in ethyl acetate (2.0 mL). The reaction solution was stirred at 16 °C for 0.5 hour. The reaction solution was concentrated under reduced pressure to obtain the hydrochloride salt of compound **19-3**. LC-MS: m/z = 532.4 [M+H]$^+$.

The fourth step

The hydrochloride salt of compound **19-3** (30.0 mg, 56.34 μmol) was added to a mixed solvent of anhydrous tetrahydrofuran (0.5 mL) and water (0.5 mL), and to the mixture was added sodium bicarbonate (14.2 mg, 169.03 μmol). The reaction solution was stirred at 0 °C for 0.05 hour, and acryloyl chloride (3.8 mg, 42.26 μmol) was added slowly. The reaction solution was stirred at 0 °C for 0.2 hour. The reaction solution was concentrated under reduced pressure, diluted with water (5 mL), and extracted with ethyl acetate (10 mL x 3). The organic phases were combined, dried over anhydrous sodium sulfate, and filtered. The filtrate was concentrated under reduced pressure, and the residue was

isolated by preparative high performance liquid chromatography (basic) to obtain **example 19**. $^1$H NMR (400MHz, $CDCl_3$) δ 8.71-8.68 (m, 1H), 8.54-8.45 (m, 1H), 7.35 (m, 1H), 7.08-7.00 (m, 1H), 6.66-6.53 (m, 1H), 6.47-6.36 (m, 1H), 5.71 (m, 1H), 4.77-4.68 (m, 1H), 4.13 (m, 3H), 4.02-3.85 (m, 3H), 3.76-3.66 (m, 1H), 3.50 (s, 1H), 3.00 (m, 2H), 2.97 (s, 1H), 2.89 (s, 1H), 2.55 (m, 1H), 2.31 (s, 2H), 2.05 (s, 3H), 1.93 (m, 2H), 1.86 (m, 2H); LC-MS: m/z = 586.4 [M+H]$^+$.

Example 20

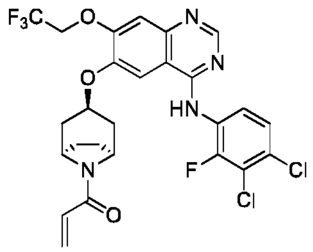

Synthetic route:

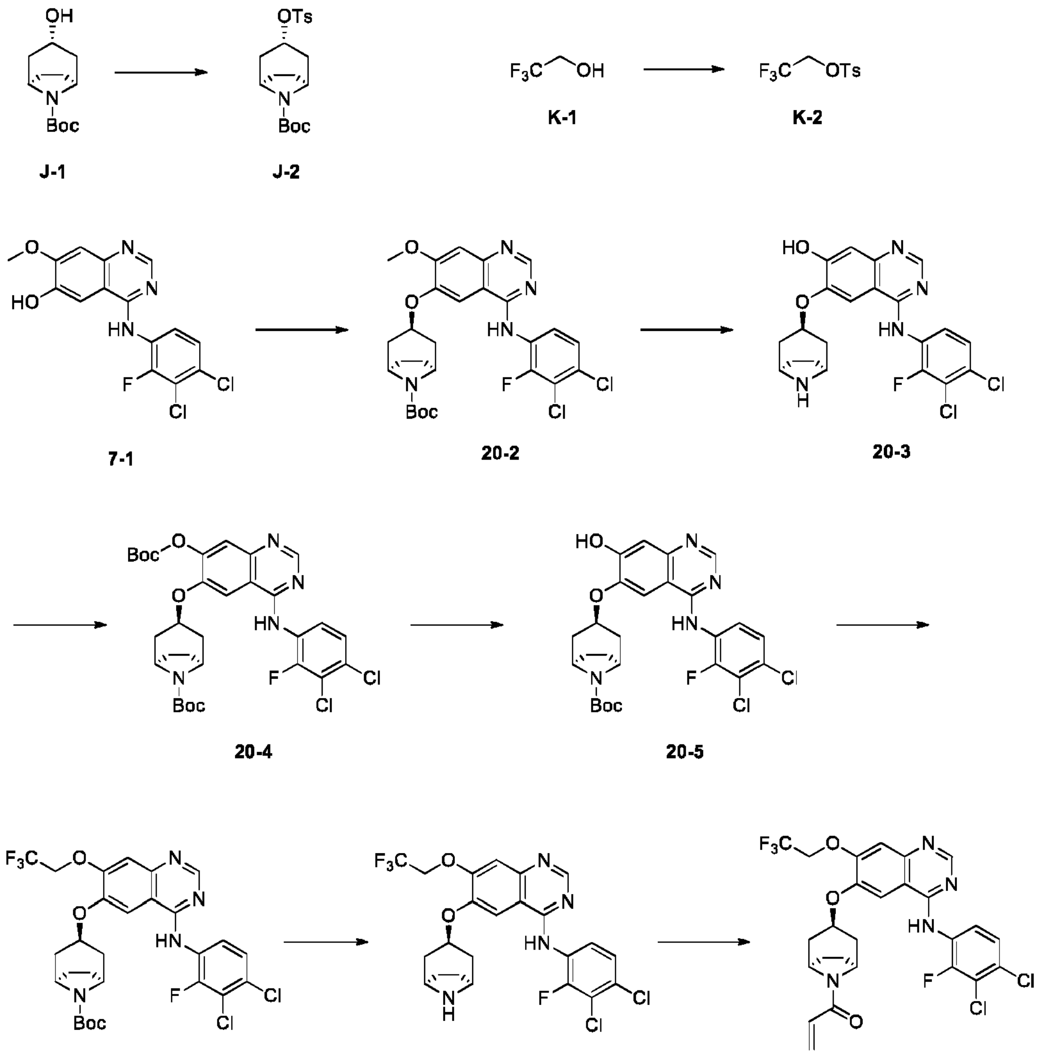

J-1 J-2 K-1 K-2

7-1 20-2 20-3

20-4 20-5

20-6 20-7 Example 20

The first step

Compound **J-1** (6.40 g, 28.16 mmol) was dissolved in pyridine (20 mL), and to the mixture was added p-toluenesulfonyl chloride (8.05 g, 42.24 mmol). The mixture was stirred at 10 °C for 16 hours. The reaction solution was concentrated under reduced pressure, diluted with ethyl acetate (300 mL), washed sequentially with 1 N dilute hydrochloric acid (100 mL x 2) and saturated brine (100 mL), dried over anhydrous sodium sulfate, and filtered. The filtrate was concentrated under reduced pressure, and the residue was isolated by silica gel column chromatography (ethyl acetate:petroleum ether = 1:8) to afford compound **J-2**. $^1$H NMR (400 MHz, $CDCl_3$) δ 7.77 (d, *J* = 8.4 Hz, 2H), 7.34 (d, *J* = 8.4 Hz, 2H), 5.69 (t, *J* = 4.8 Hz, 1H), 4.05-4.30 (m, 2H), 2.45 (s, 3H), 1.90-2.15 (m, 6H), 1.70-1.90 (m, 2H), 1.43 (s, 9H). LC-MS: m/z = 282.0 $[M+H-100]^+$.

The second step

Compound **K-1** (1.00 g, 10.00 mmol) was dissolved in pyridine (5 mL) and to the mixture was added p-toluenesulfonyl chloride (2.86 g, 14.99 mmol). The reaction solution was stirred at 10 °C for 16 hours, and then concentrated under reduced pressure. The residue was dissolved in ethyl acetate (40 mL), washed successively with saturated ammonium chloride solution (60 mL x 3), saturated brine (60 mL), dried over anhydrous sodium sulfate, and filtered. The filtrate was concentrated under reduced pressure, and the residue was isolated by silica gel column chromatography (ethyl acetate:petroleum ether = 1:8) to afford compound **K-2**. $^1$H NMR (400 MHz, $CDCl_3$) δ 7.82 (d, *J* = 8.4 Hz, 2H), 7.38 (d, *J* = 8.0 Hz, 2H), 4.34 (q, *J* = 8.8 Hz, 2H), 2.47 (s, 3H).

The third step

Compound **7-1** (4.00 g, 11.29 mmol) was dissolved in N,N-dimethylacetamide (20 mL), and to the mixture were added potassium carbonate (2.34 g, 16.94 mmol) and compound **J-2** (5.60 g, 14.68 mmol). The reaction solution was stirred at 75 °C for 16 hours. The reaction solution was cooled to 30 °C, diluted with water (60 mL), and extracted with ethyl acetate (60 mL x 3). The organic phases were combined, washed successively with aqueous solution (100 mL x 3) and saturated brine (60 mL x 2), dried over anhydrous sodium sulfate, and filtered. The filtrate was concentrated under reduced pressure, and the residue was isolated by silica gel column chromatography (ethyl acetate:petroleum ether = 1:1) to afford compound **20-2**. $^1$H NMR (400 MHz, $CDCl_3$) δ 8.64 (s, 1H), 8.49 (t, *J* = 8.4 Hz, 1H), 7.26-7.40 (m, 3H), 7.13 (s, 1H), 4.60-4.75 (m, 1H), 4.15-4.40 (m, 2H), 3.94 (s, 3H), 1.75-2.20 (m, 6H), 1.55-1.70 (m, 2H),1.43 (s, 9H). LC-MS: m/z = 563.1 $[M+H]^+$.

The fourth step

A mixture of compound **20-2** (2.70 g, 4.80 mmol) and pyridine hydrochloride (10.80 g, 93.46 mmol) was heated to 170 °C under nitrogen protection, and stirred for 3 hours to afford compound **20-3**. LC-MS: m/z = 449.0 $[M+H]^+$.

The fifth step

Compound **20-3**(13.00 g, 4.63 mmol) and triethylamine (7.00 g, 69.18 mmol) were dissolved in methanol (20 mL), and to the mixture was added di-tert-butyl dicarbonate (9.63 g, 44.12 mmol). The reaction solution was stirred at 10 °C for 1 hour. The reaction solution was concentrated under reduced pressure, and the residue was slurried with water (20 mL) and filtered. The obtained solid was washed successively with saturated ammonium chloride solution (10 mL x 3) and petroleum ether (10 mL), and dried to afford compound **20-4**. LC-MS: m/z = 649.1 $[M+H]^+$.

The sixth step

Compound **20-4** (3.00 g, 4.62 mmol) was dissolved in methanol (50 mL), and to the mixture was added potassium carbonate (3.19 g, 23.09 mmol). The reaction solution was stirred at 40 °C for 14 hours, and then water (30 mL) was added. The mixture was adjusted to pH of 6 with acetic acid and filtered. The solid was washed with water (30 mL) and dried in the air to afford compound **20-5**. $^1$H NMR (400 MHz, DMSO-$d_6$) δ 9.54 (brs, 1H), 8.22 (s, 1H), 7.68 (s, 1H), 7.61 (t, *J* = 8.4 Hz, 1H), 7.50-7.60 (m, 1H), 6.94 (s, 1H), 4.90-5.00 (m, 1H), 4.10-4.20 (m, 2H), 2.10-2.20 (m, 2H), 1.70-2.00 (m, 4H), 1.60-1.70 (m, 2H),1.44 (s, 9H). LC-MS: *m/z* = 549.1 $[M+H]^+$.

The seventh step

Compound **20-5** (70.0 mg, 127.41 μmol) was dissolved in N,N-dimethylformamide (3 mL), and to the mixture

were added potassium carbonate (35.2 mg, 254.81 μmol) and compound **K-2** (64.78 mg, 254.81 μmol). The mixture was stirred at 70 °C for 16 hours. The reaction solution was cooled to 30 °C, and water (10 mL) was added. The mixture was extracted with ethyl acetate (10 mL x 3). The organic phases were combined, washed sequentially with water (30 mL x 2) and saturated brine (30 mL), dried over anhydrous sodium sulfate, and filtered. The filtrate was concentrated under reduced pressure, and the residue was isolated by silica gel column chromatography (ethyl acetate:petroleum ether = 1:1) to afford compound **20-6**. $^1$H NMR (400 MHz, $CDCl_3$) δ 9.77 (s, 1H), 8.43 (s, 1H), 7.97 (s, 1H), 7.55-7.65 (m, 2H), 7.43 (s, 1H), 4.95-5.05 (m, 2H), 4.75-4.90 (m, 1H), 4.10-4.20 (m, 2H), 2.10-2.20 (m, 2H), 1.90-2.20 (m, 2H), 1.70-1.80 (m, 2H), 1.55-1.70 (m, 2H), 1.44 (s, 9H). LC-MS: m/z = 631.1 $[M+H]^+$.

The eighth step

Compound **20-6** (35.0 mg, 55.43 μmol) was dissolved in a solution of hydrogen chloride in dioxane (4 N, 5 mL). The reaction solution was stirred at 10 °C for 0.3 hour. The reaction solution was concentrated under reduced pressure to obtain the hydrochloride salt of compound **20-7**. LC-MS: m/z = 531.0 $[M+H]^+$.

The ninth step

The hydrochloride salt of compound **20-7** (30.0 mg, 52.84 μmol) was dissolved in a mixed solvent of tetrahydrofuran (3 mL) and water (3 mL) at 0 °C. To the mixture was added sodium bicarbonate (13.3 mg, 158.51 μmol), and added slowly acryloyl chloride (3.8 mg, 42.27 μmol) dropwise. The reaction solution was further stirred at 0 °C for 0.5 hour. The reaction solution was quenched by adding methanol (1 mL) and concentrated under reduced pressure. The residue was isolated by preparative thin-layer chromatography (dichloromethane:methanol = 10:1) to obtain **example 20**. $^1$H NMR (400 MHz, DMSO-$d_6$) δ 9.78 (s, 1H), 8.42 (s, 1H), 7.93 (s, 1H), 7.60-7.70 (m, 2H), 7.43 (s, 1H), 6.77 (dd, $J_1$ = 16.8 Hz, $J_2$ = 10.4 Hz, 1H), 6.19 (dd, $J_1$ = 16.8 Hz, $J_2$ = 2.0 Hz, 1H), 5.70 (dd, $J_1$ = 10.4 Hz, $J_2$ = 2.0 Hz, 1H), 4.95-5.05 (m, 2H), 4.85-4.95 (m, 1H), 4.55-4.65 (m, 2H), 2.20-2.35 (m, 2H), 1.80-2.10 (m, 4H), 1.60-1.75 (m, 1H), 1.50-1.60 (m, 1H). LC-MS: m/z = 585.1 $[M+H]^+$.

Example 21

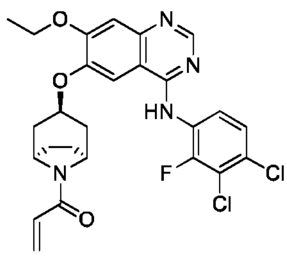

Synthetic route:

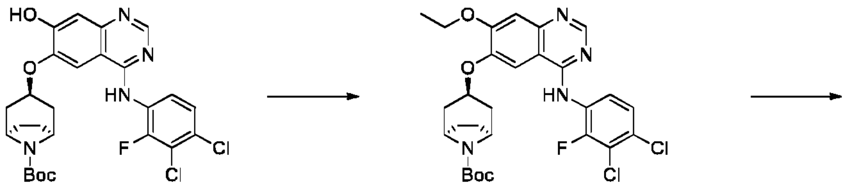

20-5 21-2

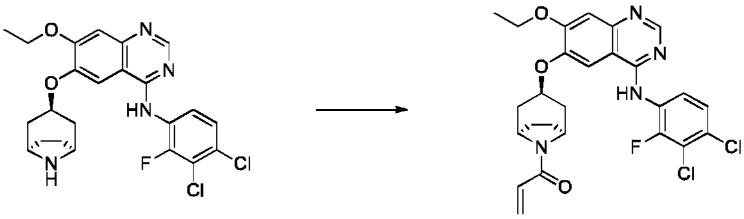

21-3 Example 21

The first step

Compound **20-5** (150.0 mg, 273.0 μmol) was added to N,N-dimethylformamide (3 mL), and to the mixture was added sodium hydride (60%, 32.8 mg, 819.1 μmol) under nitrogen protection. After stirring at 0 °C for 30 minutes, bromoethane (74.4 mg, 682.54 μmol) was added. The mixture was warmed to 15 °C and further stirred for 30 minutes. The reaction solution was quenched by adding water (5 mL) and extracted with ethyl acetate (10 mL x 3). The organic phases were combined, washed sequentially with water (10 mL x 3) and saturated brine (10 mL x 1), dried over sodium sulfate, and filtered. The filtrate was concentrated under reduced pressure, and the residue was isolated by silica gel column chromatography (ethyl acetate:petroleum ether = 1:0) to afford compound **21-2**. $^1$H NMR (400MHz, DMSO-$d_6$) δ 9.62 (br s, 1H), 8.39 (s, 1H), 7.82 (s, 1H), 7.68-7.55 (m, 1H), 7.21 (s, 1H), 4.82 (m, 1H), 4.25-4.19 (m, 2H), 4.18-4.13 (m, 2H), 2.19 (m, 2H), 1.92 (m, 2H), 1.79 (m, 2H), 1.65 (m, 2H), 1.45 (s, 9H), 1.43-1.41 (m, 3H). LC-MS: *m*/*z* = 577.3 [M+H]$^+$.

The second step

Compound **21-2** (50.0 mg, 86.6 μmol) was added to ethyl acetate (0.5 mL), and to the mixture was added a solution of hydrogen chloride in ethyl acetate (4 N, 1.7 mL) at room temperature. The mixture was stirred at 15 °C for 10 minutes. The reaction solution was concentrated under reduced pressure to obtain the hydrochloride salt of compound **21-3**. LC-MS: *m*/*z* = 477.1 [M+H]$^+$.

The third step

**Compound of example 21** was obtained by referring to the ninth step of example 20. $^1$H NMR (400MHz, DMSO-$d_6$) δ 9.65 (s, 1H), 8.39 (s, 1H), 7.80 (s, 1H), 7.67-7.55 (m, 2H), 7.21 (s, 1H), 6.78 (dd, $J_1$ = 16.8 Hz, $J_2$ = 10.4 Hz, 1H), 6.22 (dd, $J_1$ = 16.8 Hz, $J_2$ = 2.4 Hz, 1H), 5.73 (dd, $J_1$ = 10.4 Hz, $J_2$ = 2.4 Hz, 1H), 5.00-4.85 (m, 1H), 4.62 (m, 2H), 4.20 (q, $J$ = 6.8 Hz, 2H), 3.17 (d, $J$ = 5.2 Hz, 3H), 2.39-2.30 (m, 1H), 2.25 (m, 1H), 2.06-1.93 (m, 2H), 1.89 (m, 2H), 1.67 (m, 1H), 1.52 (m, 1H). LC-MS: *m*/*z* = 531.2 [M+H]$^+$.

Example 22

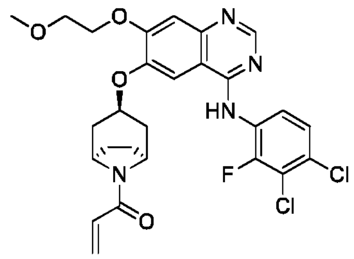

Synthetic route:

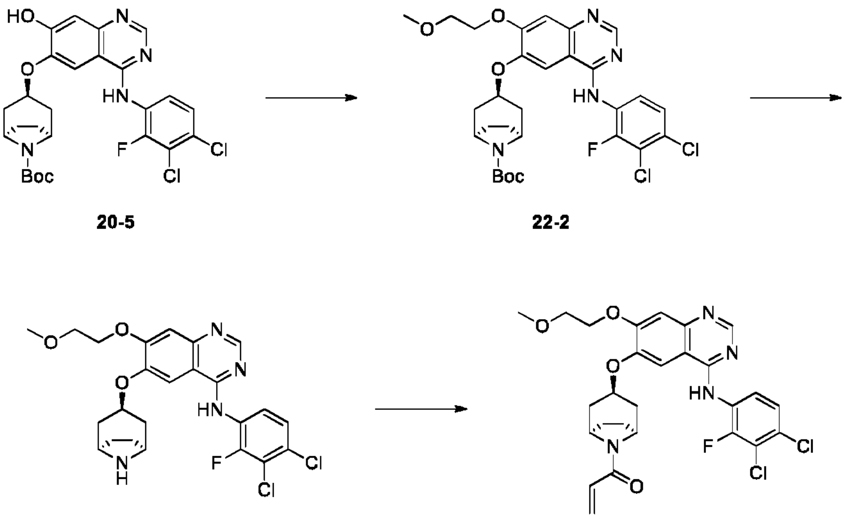

**20-5** **22-2**

**22-3** Example 22

The first step

Compound **22-2** was obtained by referring to the first step of Example 21. $^1$H NMR (400MHz, DMSO-$d_6$) δ 9.63 (s, 1H), 8.40 (s, 1H), 7.86 (s, 1H), 7.63-7.57 (m, 2H), 7.26 (s, 1H), 4.92-4.74 (m, 1H), 4.34-4.26 (m, 2H), 4.19-4.14 (m, 2H), 3.79-3.72 (m, 2H), 3.36 (s, 3H), 2.18 (m, 2H), 1.96-1.86 (m, 3H), 1.63 (m, 3H), 1.44 (s, 9H). LC-MS: *m/z* = 607.3

$[M+H]^+$.

The second step

The hydrochloride salt of compound **22-3** was obtained by referring to the first step of example 21. LC-MS: *m/z* = 507.1 $[M+H]^+$.

The third step

**Compound of example 22** was obtained by referring to the ninth step of Example 20. $^1$H NMR (400MHz, DMSO-$d_6$) δ 9.68 (s, 1H), 8.40 (s, 1H), 7.85 (s, 1H), 7.69-7.55 (m, 2H), 7.26 (s, 1H), 6.77 (dd, $J_1$ = 16.8 Hz, $J_2$ = 10.4 Hz, 1H), 6.22 (dd, $J_1$ = 16.8 Hz, $J_2$ = 2.4 Hz, 1H), 5.80-5.68 (m, 1H), 5.00-4.84 (m, 1H), 4.61 (m, 2H), 4.34-4.23 (m, 2H), 3.79-3.70 (m, 2H), 3.35 (s, 3H), 2.36-2.15 (m, 2H), 2.04-1.82 (m, 4H), 1.68 (m, 1H), 1.52 (m, 1H). LC-MS: *m/z* =561.3 $[M+H]^+$.

Example 23

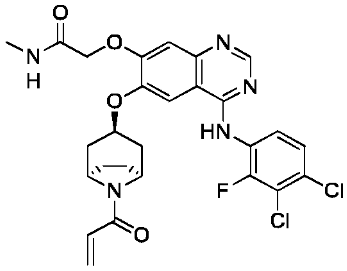

Synthetic route:

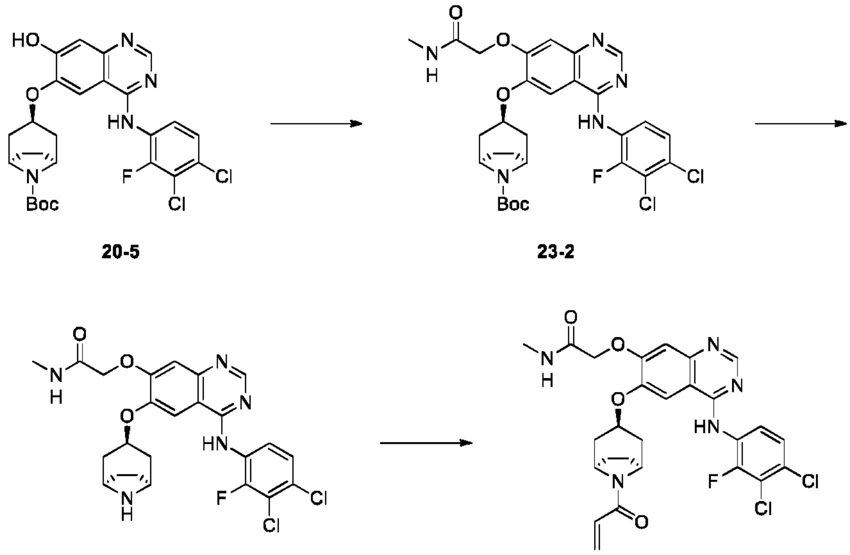

**20-5** **23-2**

**23-3** Example 23

The first step

Compound **20-5** (200.0 mg, 364.02 μmol) was added to N,N-dimethylformamide (5 mL) at room temperature, and then to the mixture were added cesium carbonate (474.4 mg, 1.46 mmol), 2-chloro-N-methylacetamide (47.0 mg, 436.82 μmol) and potassium iodide (60.4 mg, 364.02 μmol). The mixture was heated to 50 °C and stirred for 3 hours. The reaction solution was cooled to room temperature, quenched with water (10 mL), and extracted with ethyl acetate (10 mL x 3). The organic phases were combined, washed sequentially with water (5 mL x 3) and saturated brine (5 mL), dried over anhydrous sodium sulfate, and filtered. The filtrate was concentrated under reduced pressure, and the residue was isolated by preparative thin-layer chromatography (dichloromethane:methanol = 20: 1) to afford compound **23-2**. LC-MS: m/z = 620.3 $[M+H]^+$.

The second step

The hydrochloride salt of compound **23-3** was obtained by referring to the first step of example 21. LC-MS: *m/z* = 520.2 $[M+H]^+$.

The third step

**Compound of example 22** was obtained by referring to the ninth step of example 20. $^1H$ NMR (400MHz, MeOD-$d_4$) δ 8.38 (s, 1H), 7.82 (s, 1H), 7.61 (t, $J$ = 8.4 Hz, 1H), 7.47 (dd, $J_1$ = 8.8 Hz, $J_2$ = 1.6 Hz, 1H), 7.19 (s, 1H), 6.77 (dd, $J_1$ = 16.8 Hz, $J_2$ = 10.4 Hz, 1H), 6.36 (dd, $J_1$ = 16.8 Hz, $J_2$ = 2.0 Hz, 1H), 5.82 (dd, $J_1$ = 10.8 Hz, $J_2$ = 1.6 Hz, 1H), 5.22-5.09 (m, 1H), 4.79 (m, 1H), 4.73 (s, 2H), 4.69-4.64 (m, 1H), 2.85 (s, 3H), 2.53-2.31 (m, 2H), 2.20-2.08 (m, 2H), 2.07-2.01 (m, 2H), 1.96-1.86 (m, 1H), 1.80 (m, 1H). LC-MS: *m/z* = 574.1 $[M+H]^+$.

Example 24

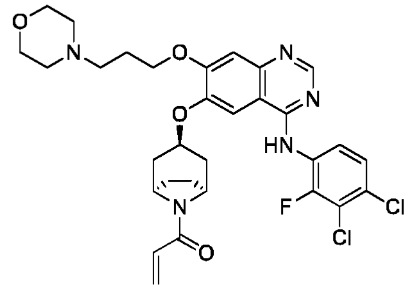

Synthetic route:

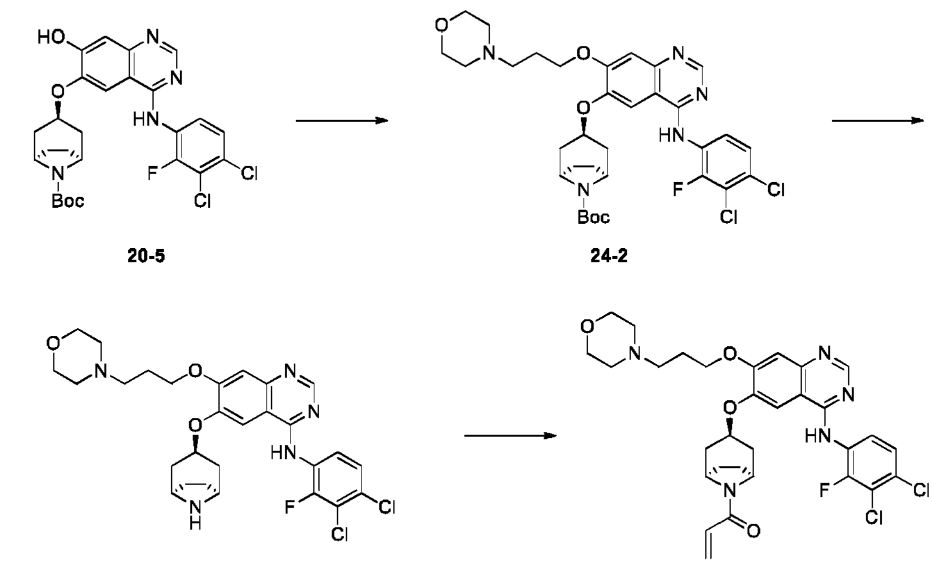

20-5

24-2

24-3

Example 24

The first step

Compound **24-2** was obtained by referring to the first step of Example 23. LC-MS: $m/z$ = 676.3 $[M+H]^+$.

The second step

The hydrochloride salt of compound **24-3** was obtained by referring to the first step of Example 21. LC-MS: $m/z$ = 576.1 $[M+H]^+$.

The third step

**Compound of example 24** was obtained by referring to the ninth step of example 20. $^1$H NMR (400MHz, MeOD-$d_4$) δ 8.38 (s, 1H), 7.79 (s, 1H), 7.62 (t, $J$ = 8.4 Hz, 1H), 7.46 (dd, $J_1$ = 8.8 Hz, $J_2$ = 1.6 Hz, 1H), 7.22 (s, 1H), 6.77 (dd, $J_1$ = 16.8 Hz, $J_2$ = 10.4 Hz, 1H), 6.36 (dd, $J_1$ = 16.4 Hz, 1.6 Hz, 1H), 5.82 (dd, $J_1$ = 10.4 Hz, $J_2$ = 1.6 Hz, 1H), 5.13-4.98 (m, 1H), 4.80-4.78 (m, 1H), 4.68-4.61 (m, 1H), 4.24 (t, $J$ = 6.0 Hz, 2H), 3.77-3.67 (m, 4H), 2.68-2.58 (m, 2H), 2.53 (m, 4H), 2.42 (m, 1H), 2.37-2.30 (m, 1H), 2.20-1.99 (m, 6H), 1.91-1.82 (m, 1H), 1.74 (m, 1H). LC-MS: $m/z$ = 630.2 $[M+H]^+$.

Example 25

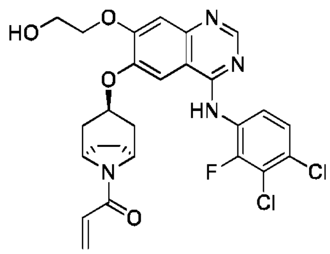

Synthetic route:

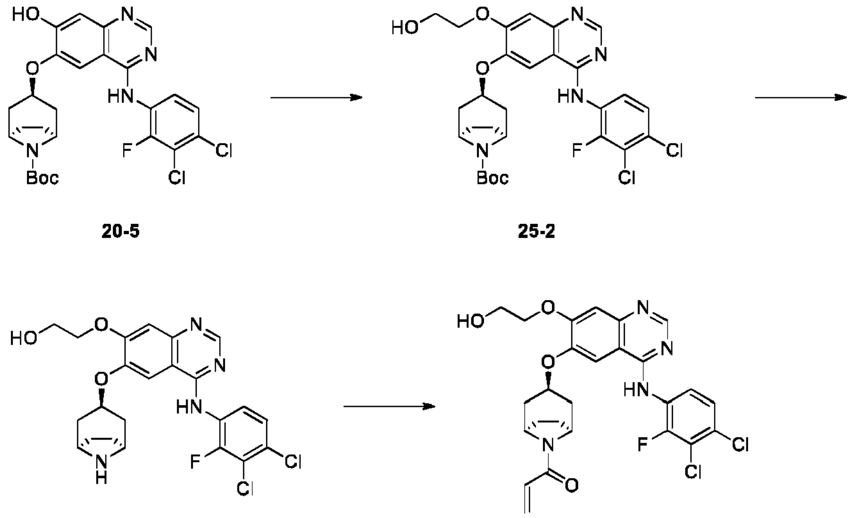

20-5

25-2

25-3

Example 25

The first step

Compound **20-5** (150.0 mg, 273.02 μmol) was added to N,N-dimethylformamide (5 mL), and then to the mixture were added potassium fluoride (47.6 mg, 819.05 μmol) and ethylene carbonate (72.1 mg, 819.05 μmol). The reaction solution was heated to 110 °C and stirred for 3 hours. The reaction solution was cooled to room temperature, quenched with water (20 mL), and extracted with ethyl acetate (10 mL x 3). The organic phases were combined, washed sequentially with water (5 mL x 3) and saturated brine (5 mL), dried over sodium sulfate, and filtered. The filtrate was concentrated under reduced pressure, and the residue was isolated by preparative thin-layer chromatography (dichloromethane:methanol = 20:1) to afford compound **25-2**. LC-MS: m/z = 593.2 $[M+H]^+$.

The second step

The hydrochloride salt of compound **25-3** was obtained by referring to the first step of example 21. LC-MS: *m/z* = 493.0 $[M+H]^+$.

The third step

**Compound of example 25** was obtained by referring to the ninth step of example 20. $^1H$ NMR (400MHz, MeOD-$d_4$) δ 8.27 (s, 1H), 7.69 (s, 1H), 7.50 (t, $J$ = 8.0 Hz, 1H), 7.35 (br d, $J$ = 8.8 Hz, 1H), 7.12 (s, 1H), 6.65 (dd, $J_1$ = 16.8 Hz, $J_2$ = 10.8 Hz, 1H), 6.24 (dd, $J_1$ = 16.8 Hz, $J_2$ = 1.6 Hz, 1H), 5.70 (dd, $J_1$ = 10.4 Hz, $J_2$ = 1.6 Hz, 1H), 5.03-4.90 (m, 1H), 4.66 (m, 1H), 4.52 (m, 1H), 4.14 (m, 2H), 3.94-3.83 (m, 2H), 2.38-2.27 (m, 1H), 2.23 (m, 1H), 2.07-1.93 (m, 2H), 1.89 (m, 2H), 1.77 (m, 1H), 1.66 (m, 1H). LC-MS: *m/z* = 547.1 $[M+H]^+$.

Example 26

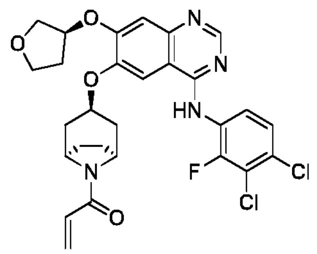

Synthetic route:

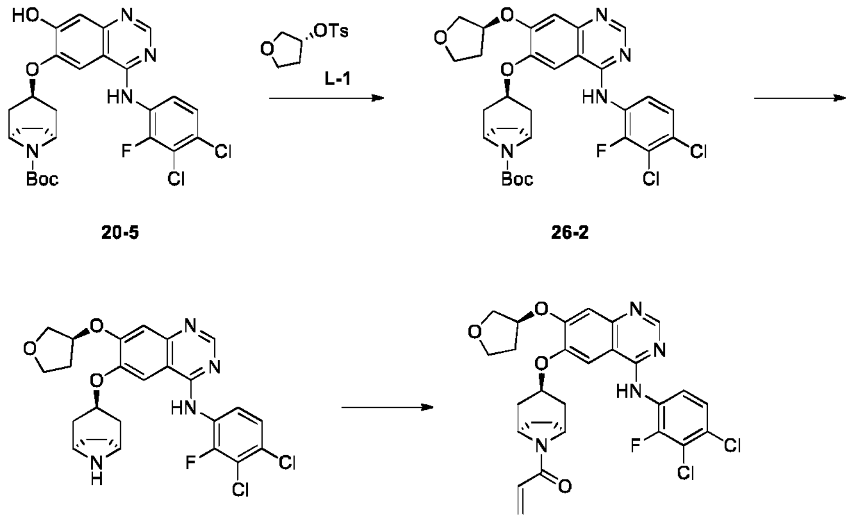


20-5 26-2

26-3 Example 26

The first step

Compound **20-5** (200.0 mg, 364.02 umol) was added to N,N-dimethylformamide (5 mL) at room temperature, and then to the mixture were added cesium carbonate (55.8 mg, 1.09 mmol) and compound **L-1** (97.0 mg, 400.42 μmol). The reaction solution was heated to 100 °C, and stirred for 3 hours. The reaction solution was cooled to room temperature, quenched with water (10 mL), and extracted with ethyl acetate (10 mL x 3). The organic phases were combined, washed sequentially with water (5 mL x 3) and saturated brine (5 mL), dried over sodium sulfate, and filtered. The filtrate was concentrated under reduced pressure, and the residue was isolated by preparative thin-layer chromatography (dichloromethane:methanol = 20:1) to afford compound **26-2**. LC-MS: $m/z$ = 619.2 $[M+H]^+$.

The second step

The hydrochloride salt of compound **26-3** was obtained by referring to the first step of Example 21. LC-MS: $m/z$ = 519.0 $[M+H]^+$.

The third step

**Compound of example 26** was obtained by referring to the ninth step of example 20. $^1$H NMR (400MHz, MeOD-$d_4$) δ 8.39 (s, 1H), 7.81 (s, 1H), 7.62 (t, $J$ = 8.4 Hz, 1H), 7.45 (dd, $J_1$ = 9.2 Hz, $J_2$ = 2.0 Hz, 1H), 7.17 (s, 1H), 6.76 (dd, $J_1$ = 16.8 Hz, $J_2$ = 10.4 Hz, 1H), 6.36 (dd, $J_1$ = 16.8 Hz, $J_2$ = 2.0 Hz, 1H), 5.81 (dd, $J_1$ = 10.4 Hz, $J_2$ = 1.6 Hz, 1H), 5.22 (m, 1H), 5.07-4.94 (m, 1H), 4.80-4.73 (m, 1H), 4.67-4.60 (m, 1H), 4.05 (s, 2H), 4.04-3.98 (m, 1H), 3.93 (m, 1H), 2.46-2.35 (m, 2H), 2.32 (m, 1H), 2.26-2.18 (m, 1H), 2.17-2.09 (m, 1H), 2.06-1.93 (m, 3H), 1.92-1.82 (m, 1H), 1.75 (m,

1H); LC-MS: $m/z$ = 573.3 $[M+H]^+$.

Example 27

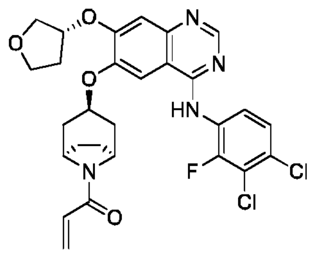

Synthetic route:

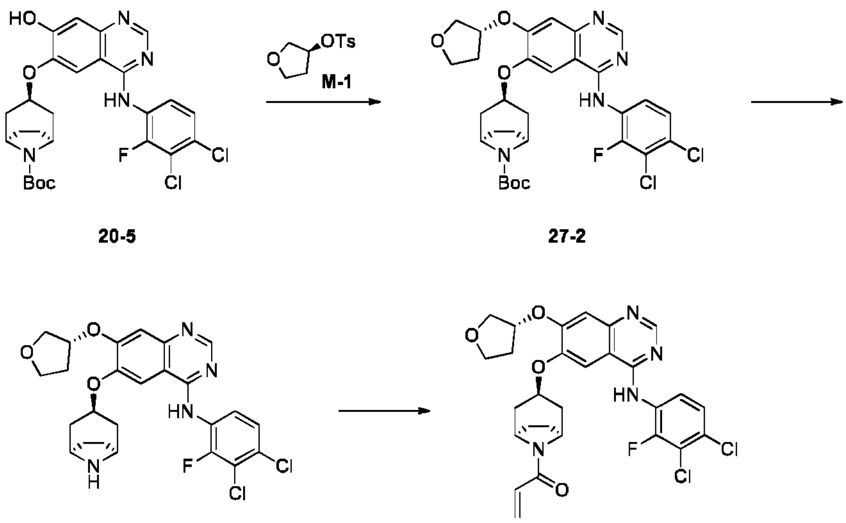


20-5

27-2

27-3

Example 27

The first step

Compound **27-2** was obtained by referring to the first step of example 26. LC-MS: $m/z$ = 619.2 $[M+H]^+$.

The second step

The hydrochloride salt of compound **27-3** was obtained by referring to the first step of example 21. LC-MS: $m/z$ = 519.0 $[M+H]^+$.

The third step

**Compound of example 27** was obtained by referring to the ninth step of example 20. $^1$H NMR (400MHz, MeOD-$d_4$) δ 8.39 (s, 1H), 7.82 (s, 1H), 7.62 (t, $J$ = 8.4 Hz, 1H), 7.46 (dd, $J_1$ = 8.8 Hz, $J_2$ = 1.6 Hz, 1H), 7.19 (s, 1H), 6.76 (dd, $J_1$ = 16.4 Hz, $J_2$ = 10.4 Hz, 1H), 6.36 (dd, $J_1$ = 16.8 Hz, $J_2$ = 1.6 Hz, 1H), 5.81 (dd, $J_1$ = 10.4 Hz, $J_2$ = 1.6 Hz, 1H), 5.23 (m, 1H), 5.08-4.94 (m, 1H), 4.78 (m, 1H), 4.68-4.61 (m, 1H), 4.06 (s, 2H), 4.04-3.99 (m, 1H), 3.93 (m, 1H), 2.46-2.36 (m, 2H), 2.32 (m, 1H), 2.26-2.19 (m, 1H), 2.18-2.10 (m, 1H), 2.06-1.94 (m, 3H), 1.87 (m, 1H), 1.76 (m, 1H). LC-MS: $m/z$ = 573.3 $[M+H]^+$.

Example 28

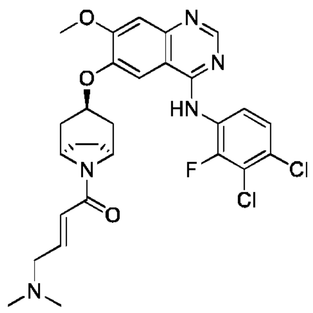

Synthetic route:

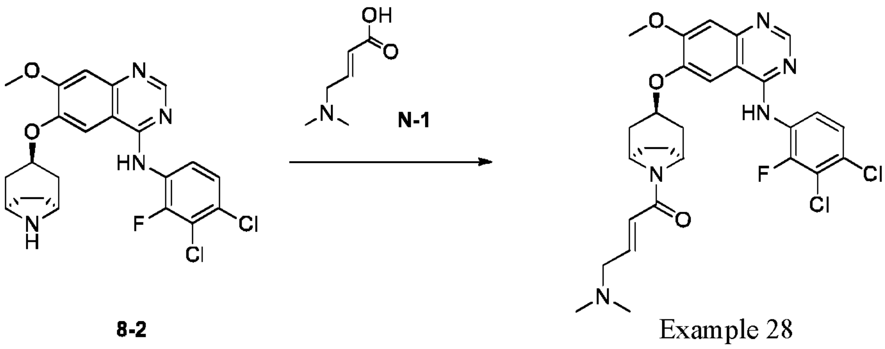


**8-2** Example 28

The first step

Compound **8-2** (83.6 mg, 647.49 μmol) was dissolved in N,N-dimethylformamide (1 mL), and to the mixture was added HATU (123.1 mg, 323.74 μmol). The reaction solution was stirred at 16 °C for 0.5 hour, and DIEA (69.7 mg, 539.57 μmol) and compound **N-1** (100.0 mg, 215.83 μmol) were added. The mixture was further stirred for 16 hours. The reaction solution was filtered, the filtrate was concentrated under reduced pressure, and the residue was isolated by preparative high-performance liquid chromatography (neutral) to obtain the **compound of example 28**. $^1$H NMR (400MHz, MeOD-$d_4$) δ 8.37 (s, 1H), 7.76 (s, 1H), 7.60 (t, $J$ = 8.3 Hz, 1H), 7.45 (dd, $J_1$ = 8.8 Hz , $J_2$ = 1.6 Hz, 1H), 7.20 (s, 1H), 6.90 (m, 1H), 6.60 (d, $J$ = 15.2 Hz, 1H), 5.11-5.01 (m, 1H), 4.75 (m, 1H), 4.63 (m, 1H), 3.99 (s, 3H), 3.19 (d, J = 6.0 Hz, 2H), 2.44 - 2.37 (m, 1H), 2.33 (m, 1H), 2.30 (s, 6H), 2.19-2.10 (m, 1H), 2.09-1.96 (m, 3H), 1.85 (m, 1H), 1.75 (m, 1H). LC-MS: m/z = 574.4 [M+H]$^+$.

Example 29

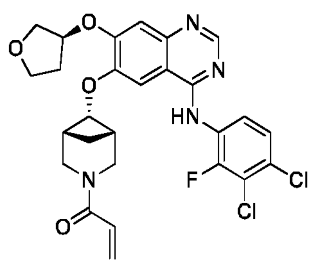

Synthetic route:

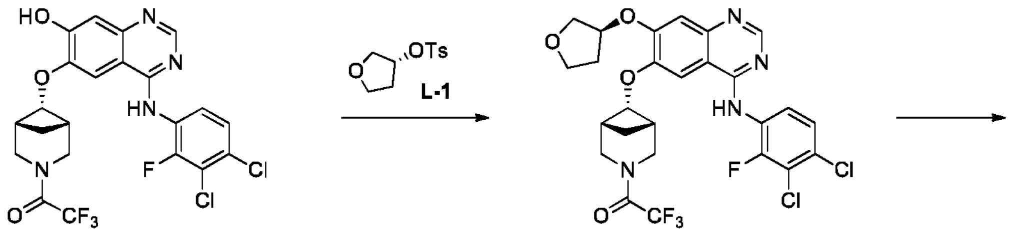


**13-2** **29-2**

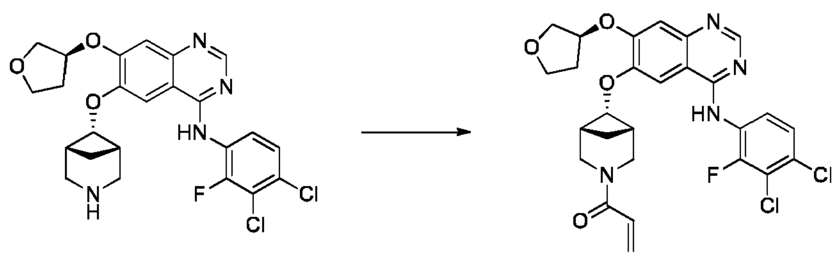

**29-3** Example 29

The first step

Compound 13-2 (250.0 mg, 470.56 μmol) was dissolved in N,N-dimethylformamide (4 mL). To the reaction solution were added potassium fluoride (65.6 mg, 1.13 mmol), potassium carbonate (130.07 mg, 941.11 μmol) and compound **L-1** (171.0 mg, 705.83 μmol). The reaction solution was stirred at 118 °C for 18 hours. The reaction solution was concentrated under reduced pressure to remove the solvent, and the residue was isolated by preparative thin-layer chromatography (ethyl acetate) to afford compound **29-2**. LC-MS: m/z = 601.3 $[M+H]^+$.

The second step

Compound **29-2** (147.0 mg, 244.44 μmol) was dissolved in methanol (10 mL), and to the mixture was added potassium carbonate (168.9 mg, 1.22 mmol). The reaction solution was stirred at 50 °C for 5 hours. The reaction solution was concentrated under reduced pressure, and the residue was dissolved in methanol (5 mL). Potassium carbonate (33.8 mg, 244.44 μmol) was added, and the reaction solution was stirred at 50 °C for 5 hours. The reaction solution was concentrated under reduced pressure, and the residue was isolated by preparative thin-layer chromatography (dichloromethane:methanol = 10:1) to afford compound **29-3**. LC-MS: m/z = 505.3 $[M+H]^+$.

The third step

**Compound of example 29** was obtained by referring to the ninth step of example 20. $^1$H NMR (400MHz, $CDCl_3$) δ 8.68 (s, 1H), 8.39 (t, *J* = 8.4 Hz, 1H), 7.33 (dd, *J* = 8.8, 1.6 Hz, 1H), 7.19 (d, *J* = 5.2 Hz, 1H), 7.16 (s, 1H), 6.61 (dd, *J* = 16.8, 10.4 Hz, 1H), 6.42 (dt, *J* = 16.4, 2.4 Hz, 1H), 5.72 (dd, *J* = 10.4, 2.0 Hz, 1H), 5.07-5.02 (m, 1H), 4.70-4.65 (m, 1H), 4.09-4.03 (m, 1H), 4.02-3.95 (m, 3H), 3.94-3.83 (m, 3H), 3.70-3.65 (m, 1H), 3.00-2.97 (m, 2H), 2.36-2.23 (m, 1H), 2.18-2.15 (m, 1H), 1.92-1.87 (m, 2H); LC-MS: m/z = 559.3 $[M+H]^+$.

Example 30

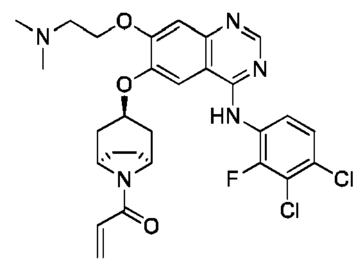

Synthetic route:

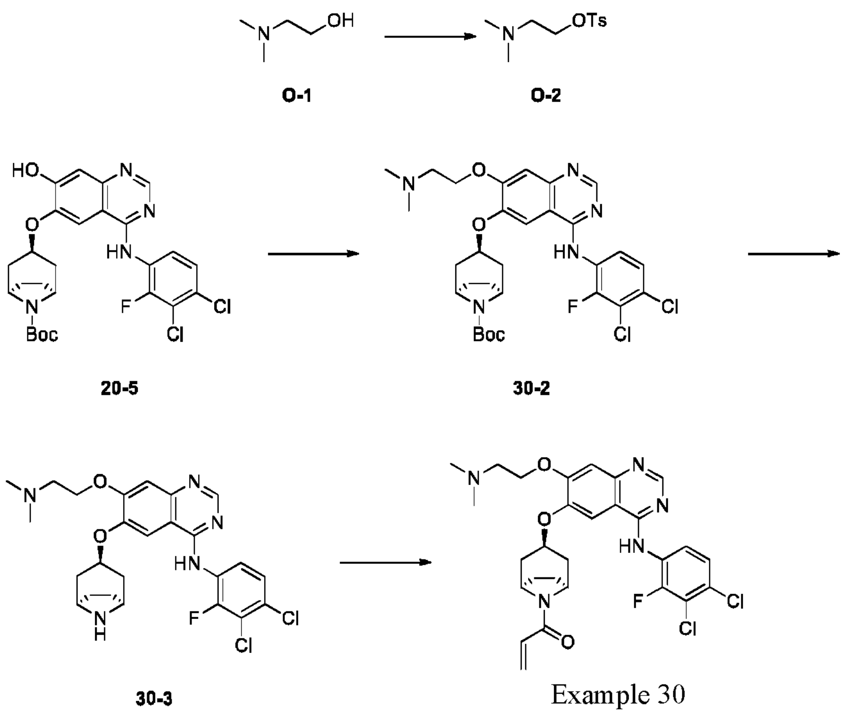

O-1 O-2

20-5 30-2

30-3 Example 30

The first step

Compound **O-1** (2.00 g, 22.44 mmol) was dissolved in pyridine (5 mL), and to the mixture was added p-toluenesulfonyl chloride (4.28 g, 22.44 mmol). The reaction solution was stirred at 10 °C for 16 hours. Ethyl acetate (50 mL) was added to the reaction solution and the mixture was filtered. The filtrate was concentrated to afford compound **O-2**. $^1$H NMR (400 MHz, $CDCl_3$) δ 7.49 (d, *J* = 7.6 Hz, 2H), 7.12 (d, *J* = 7.6 Hz, 2H), 3.98 (t, *J* = 6.4 Hz, 2H), 3.49 (t, *J*= 6.4 Hz, 2H), 2.76 (s, 3H), 2.30 (s, 6H). LC-MS: m/z = 244.0 $[M+H]^+$.

The second step

Compound **20-5** (200.0 mg, 364.02 μmol) was dissolved in N,N-dimethylformamide (3 mL), and to the mixture were added potassium carbonate (100.6 mg, 728.04 μmol) and compound **O-2** (177.2 mg, 728.04 μmol). The mixture was stirred at 70 °C for 16 hours. The reaction solution was cooled to 30 °C, diluted with water (10 mL), and extracted with ethyl acetate (10 mL x 3). The organic phases were combined, washed sequentially with water (30 mL x 2) and saturated brine (30 mL), dried over anhydrous sodium sulfate and filtered. The filtrate was concentrated under reduced pressure, and the residue was isolated by preparative thin-layer chromatography (dichloromethane:methanol = 10:1) to afford compound **30-2**. $^1$H NMR (400 MHz, $CDCl_3$) δ 8.70 (s, 1H), 8.53 (t, *J* = 8.4 Hz, 1H), 7.20-7.40 (m, 4H), 4.65-4.85 (m, 1H), 4.20-4.45 (m, 4H), 2.90-3.00 (m, 2H), 2.44 (s, 6H), 2.10-2.20 (m, 2H), 1.95-2.10 (m, 2H), 1.75-1.95 (m, 2H), 1.60-1.75 (m, 2H), 1.50 (s, 9H). LC-MS: m/z = 620.1 $[M+H]^+$.

The third step

The hydrochloride salt of compound **30-3** was obtained by referring to the eighth step of Example 20. LC-MS: *m/z* = 520.1 [M+H]+.

The fourth step

**Compound of example 30** was obtained by referring to the ninth step of Example 20. $^1$H NMR (400 MHz, DMSO-$d_6$) δ 9.68 (s, 1H), 8.40 (s, 1H), 7.86 (s, 1H), 7.55-7.70 (m, 2H), 7.26 (s, 1H), 6.77 (dd, $J_1$ = 16.8 Hz, $J_2$ = 10.4 Hz, 1H), 6.22 (d, *J* = 16.8 Hz 1H), 5.72 (d, *J* = 10.4 Hz 1H), 4.90-5.00 (m, 1H), 4.55-4.60 (m, 2H), 4.20-4.30 (m, 2H), 2.70-2.80 (m, 2H), 2.31(s, 6H), 1.80-2.10 (m, 6H), 1.60-1.70 (m, 1H), 1.45-1.60 (m, 1H). LC-MS: m/z = 574.1 [M+H]+.

Example 31

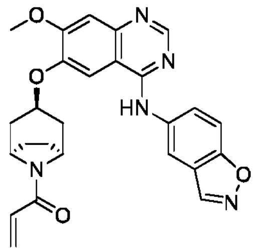

Synthetic route:

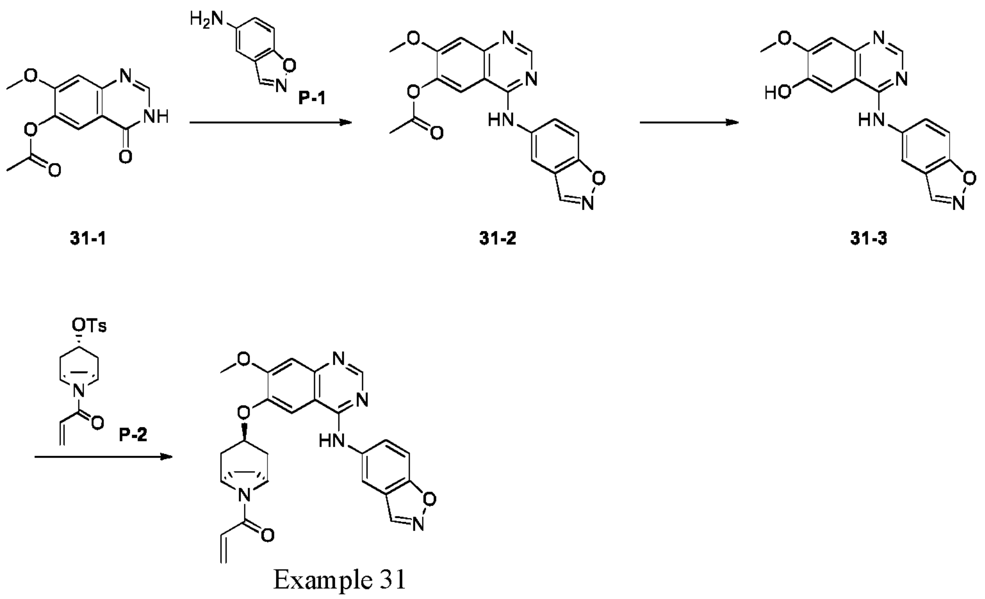


31-1 31-2 31-3

Example 31

The first step

Compound **31-1** (300.0 mg, 1.28 mmol) was dissolved in thionyl chloride (3.0 mL, 41.45 mmol), and to the mixture was added N,N-dimethylformamide (47.30 μL, 614.84 μmol). The reaction solution was heated to 80 °C and stirred for 1 hour. The reaction solution was concentrated under reduced pressure, and to the residue was added a solution of compound **P-1** (171.8 mg, 1.28 mmol) in isopropanol (10 mL) at room temperature. The mixture was heated to 90 °C and stirred for 1 hour. The reaction solution was cooled to 20 °C and filtered. The solid was washed with isopropanol (10 mL) and dried in the air to afford compound **31-2**. $^1$H NMR (400 MHz, DMSO-$d_6$) δ 11.51 (s, 1H), 9.35

(s, 1H), 8.90 (s, 1H), 8.72 (s, 1H), 8.23 (s, 1H), 7.85-8.00 (m, 2H), 7.50 (s, 1H), 4.03 (s, 3H), 2.41 (s, 3H). LC-MS: *m/z* = 351.0 $[M+H]^+$.

The second step

Compound **31-2** (400.0 mg, 1.14 mmol) was dissolved in ethanol (10 mL), and to the mixture was added a solution of sodium hydroxide (228.4 mg, 5.71 mmol) in water (2 mL). The reaction solution was stirred at 10 °C for 1 hour. To the reaction solution was added acetic acid (0.5 mL) and water (10 mL). The reaction solution was further stirred for 0.5 hour, and then filtered. The solid was washed with water (10 mL) and dried under reduced pressure to afford compound **31-3**. $^1H$ NMR (400 MHz, DMSO-$d_6$) δ 10.89 (s, 1H), 9.67 (s, 1H), 9.38 (s, 1H), 8.42 (s, 1H), 8.08 (d, *J* = 2.8 Hz,1H), 7.80-7.90 (m, 1H), 7.74 (s, 1H), 7.19 (s, 1H), 7.03 (d, *J*= 8.8 Hz, 1H), 3.97 (s, 3H). LC-MS: *m/z* = 309.0 $[M+H]^+$.

The third step

Compound **31-3** (100.0 mg, 324.37 μmol) was dissolved in N,N-dimethylacetamide (5 mL), and to the mixture were added potassium carbonate (89.7 mg, 648.74 μmol) and compound **P-2** (1034 mg, 308.15 μmol). The reaction solution was stirred at 70 °C for 16 hours. The reaction solution was cooled to 20 °C, diluted with water (30 mL), and extracted with ethyl acetate (20 mL x 3). The organic phases were combined, washed sequentially with water (30 mL x 3) and saturated brine (30 mL x 2), dried over anhydrous sodium sulfate, and filtered. The filtrate was concentrated under reduced pressure, and the residue was isolated by preparative thin-layer chromatography (dichloromethane:methanol = 15:1) to obtain **example 31.** $^1H$ NMR (400 MHz, DMSO-$d_6$) δ 9.68 (s, 1H), 9.46 (s, 1H), 8.44 (s, 1H), 8.23 (d, *J*= 2.4 Hz 1H), 8.00-8.10 (m, 1H), 7.76 (s, 1H), 7.50 (d, *J*= 9.6 Hz 1H), 6.77 (dd, $J_1$ = 16.8 Hz, $J_2$ =10.4 Hz, 1H), 6.22 (dd, $J_1$ = 16.8 Hz, $J_2$ = 2.4 Hz, 1H), 5.73 (dd, $J_1$ = 10.4 Hz, $J_2$ = 2.4 Hz, 1H), 4.90-5.10 (m, 1H), 4.55-4.65 (m, 2H), 3.98 (s, 3H), 2.15-2.35 (m, 2H), 1.80-2.00 (m, 4H), 1.60-1.70 (m, 1H), 1.45-1.55 (m, 1H). LC-MS: *m/z* = 472.1 $[M+H]^+$.

Example 32

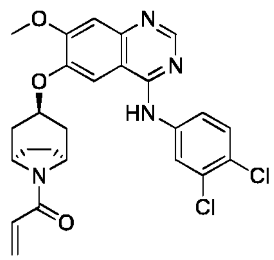

Synthetic route:

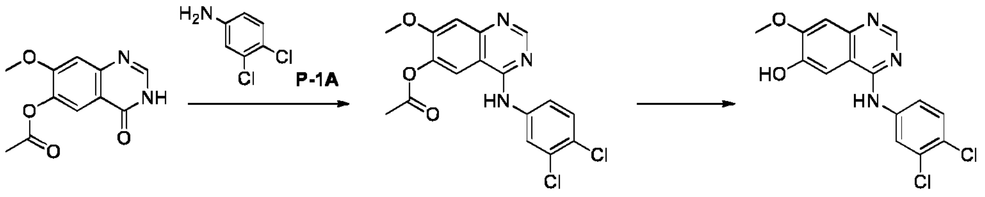


31-1 32-2 32-3

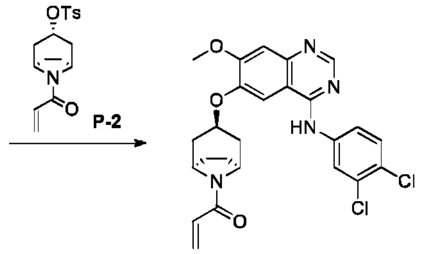


Example 32

The first step

Compound **32-2** was obtained by referring to the first step of Example 31. $^1$H NMR (400 MHz, DMSO-$d_6$) δ 11.21 (s, 1H), 8.97 (s, 1H), 8.71 (s, 1H), 8.18 (d, *J* = 2.0 Hz, 1H), 7.71-7.81 (m, 2H), 7.49 (s, 1H), 4.02 (s, 3H), 2.40 (s, 3H). LC-MS: *m/z* = 378.0 [M+H]$^+$.

The second step

Compound **32-3** was obtained by referring to the second step of example 31. $^1$H NMR (400 MHz, DMSO-$d_6$) δ 9.77 (s, 1H), 9.61 (s, 1H), 8.54 (s, 1H), 8.34 (d, *J* = 2.4 Hz, 1H), 7.80-7.90 (m, 1H), 7.81 (s, 1H), 7.61 (d, *J* = 8.8 Hz, 1H), 7.24 (s, 1H), 3.99 (s, 3H). LC-MS: *m/z* = 335.9 [M+H]$^+$.

The third step

**Compound of example 32** was obtained by referring to the third step of example 31. $^1$H NMR (400 MHz, DMSO-$d_6$) δ 9.62 (s, 1H), 8.58 (s, 1H), 8.32 (d, *J* = 2.8 Hz 1H), 7.85-7.95 (m, 2H), 7.68 (d, *J* = 8.8 Hz 1H), 7.24 (s, 1H), 6.77 (dd, $J_1$ = 16.8 Hz, $J_2$ =10.4 Hz, 1H), 6.22 (dd, $J_1$ = 16.8 Hz, $J_2$ = 2.4 Hz, 1H), 5.72 (dd, $J_1$ = 10.4 Hz, $J_2$ = 2.4 Hz, 1H), 4.90-5.10 (m, 1H), 4.60-4.65 (m, 2H), 3.92 (s, 3H), 2.20-2.40 (m, 2H), 1.80-2.10 (m, 4H), 1.60-1.70 (m, 1H), 1.40-1.50 (m, 1H). LC-MS: *m/z* = 499.1 [M+H]$^+$.

Example 33

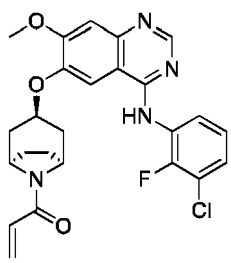

Synthetic route:

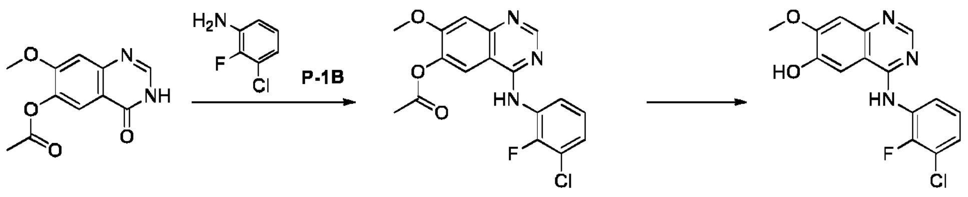


31-1 33-2 33-3

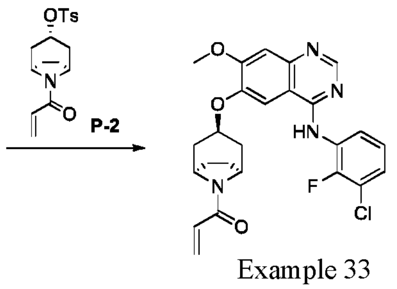


Example 33

The first step

Compound **33-2** was obtained by referring to the first step of example 31. LC-MS: *m/z* = 362.0 $[M+H]^+$.

The second step

Compound **33-3** was obtained by referring to the second step of example 31. $^1H$ NMR (400 MHz, DMSO-$d_6$) δ 9.76 (s, 1H), 9.50 (s, 1H), 8.35 (s, 1H), 7.66 (s, 1H), 7.42-7.60 (m, 2H), 7.20-7.30 (m, 1H), 7.21 (s, 1H), 3.98 (s, 3H). LC-MS: *m/z* = 319.9 $[M+H]^+$.

The third step

**Compound of example 33** was obtained by referring to the third step of example 31. $^1H$ NMR (400 MHz, DMSO-$d_6$) δ 9.57 (s, 1H), 8.38 (s, 1H), 7.78 (s, 1H), 7.50-7.65 (m, 2H), 7.25-7.35 (m, 1H), 7.21 (s, 1H), 6.77 (dd, $J_1$ = 16.8 Hz, $J_2$ =10.4 Hz, 1H), 6.22 (dd, $J_1$ = 16.8 Hz, $J_2$ = 2.0 Hz, 1H), 5.77 (dd, $J_1$ = 10.4 Hz, $J_2$ = 2.0 Hz, 1H), 4.90-5.10 (m, 1H), 4.55-4.65 (m, 2H), 3.98 (s, 3H), 2.20-2.40 (m, 2H), 1.85-2.10 (m, 4H), 1.60-1.70 (m, 1H), 1.45-1.55 (m, 1H). LC-MS: *m/z* = 483.0 $[M+H]^+$.

Example 34

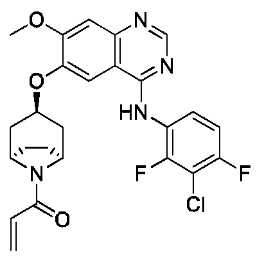

Synthetic route:

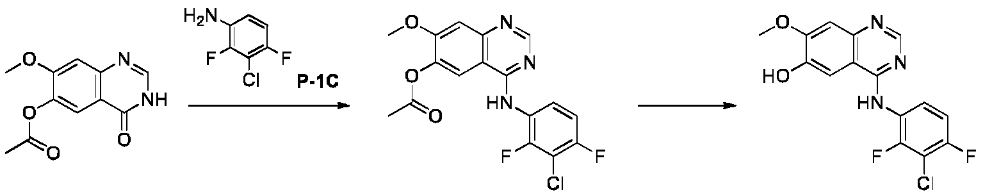


31-1 34-2 34-3

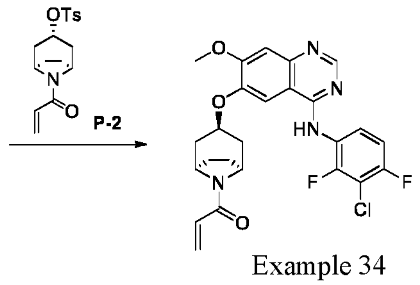


Example 34

The first step

Compound **34-2** was obtained by referring to the first step of example 31. LC-MS: $m/z$ = 380.0 $[M+H]^+$.

The second step

Compound **34-3** was obtained by referring to the second step of example 31. $^1$H NMR (400 MHz, DMSO-$d_6$) δ 9.76 (s, 1H), 9.49 (s, 1H), 8.33 (s, 1H), 7.65 (s, 1H), 7.50-7.60 (m, 1H), 7.32-7.40 (m, 1H), 7.21 (s, 1H), 3.98 (s, 3H). LC-MS: $m/z$ = 337.9 $[M+H]^+$.

The third step

**Compound of example 34** was obtained by referring to the third step of example 31. $^1$H NMR (400 MHz, DMSO-$d_6$) δ 9.60 (s, 1H), 8.37 (s, 1H), 7.78 (s, 1H), 7.56-7.65 (m, 1H), 7.38-7.50 (m, 1H), 7.21 (s, 1H), 6.77 (dd, $J_1$ = 16.8 Hz, $J_2$ = 10.4 Hz, 1H), 6.22 (dd, $J_1$ = 16.8 Hz, $J_2$ = 2.0 Hz, 1H), 5.72 (dd, $J_1$ = 10.4 Hz, $J_2$ = 2.0 Hz, 1H), 4.90-5.05 (m, 1H), 4.55-4.65 (m, 2H), 3.98 (s, 3H), 2.20-2.40 (m, 2H), 1.85-2.10 (m, 4H), 1.60-1.70 (m, 1H), 1.45-1.55 (m, 1H). LC-MS: $m/z$ = 501.1 $[M+H]^+$.

Example 35

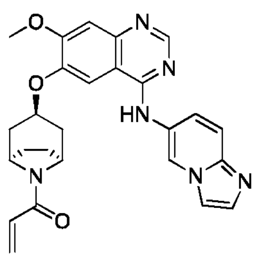

Synthetic route:

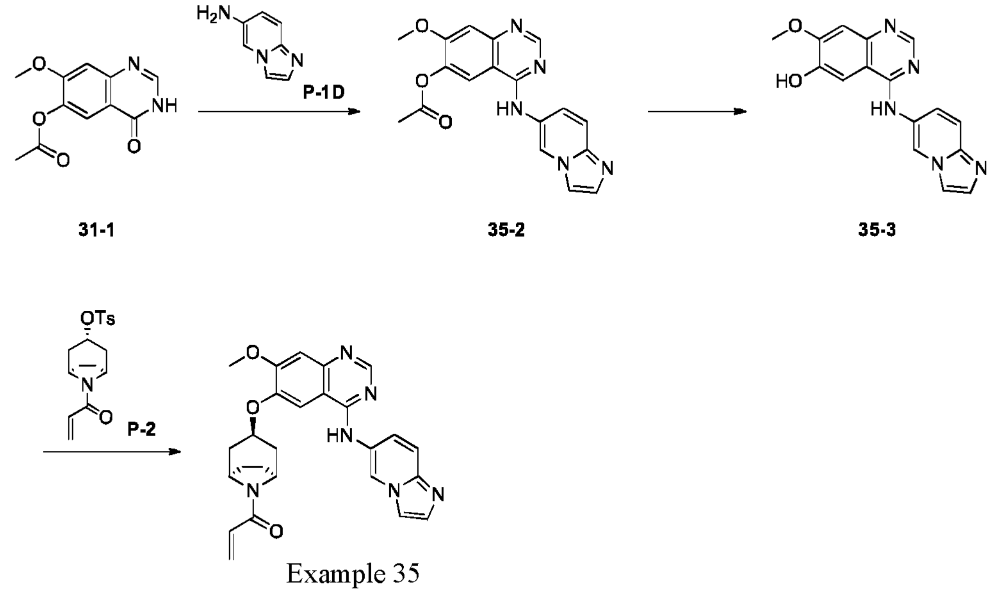


31-1 35-2 35-3

Example 35

The first step

Compound **35-2** was obtained by referring to the first step of example 31. LC-MS: $m/z$ = 350.0 $[M+H]^+$.

The second step

Compound **35-3** was obtained by referring to the second step of example 31. $^1H$ NMR (400 MHz, DMSO-$d_6$) $\delta$ 9.74 (s, 1H), 9.51 (s, 1H), 9.37 (s, 1H), 8.49 (s, 1H), 8.07 (s, 1H), 7.91 (s, 1H), 7.60-7.70 (m, 3H), 7.22 (s, 1H), 3.98 (s, 3H). LC-MS: $m/z$ = 307.9 $[M+H]^+$.

The third step

**Compound of example 35** was obtained by referring to the third step of example 31. $^1H$ NMR (400 MHz, DMSO-$d_6$) $\delta$ 9.68 (s, 1H), 9.27 (s, 1H), 8.49 (s, 1H), 8.00-8.10 (m, 2H), 7.55-7.70 (m, 3H), 7.21 (s, 1H), 6.77 (dd, $J_1$ = 16.8 Hz, $J_2$ =10.4 Hz, 1H), 6.22 (dd, $J_1$ = 16.8 Hz, $J_2$ = 2.0 Hz, 1H), 5.72 (dd, $J_1$ = 10.4 Hz, $J_2$ = 2.0 Hz, 1H), 5.10-5.15 (m, 1H), 4.55-4.65 (m, 2H), 3.98 (s, 3H), 2.20-2.40 (m, 2H), 1.80-2.10 (m, 4H), 1.60-1.70 (m, 1H), 1.40-1.50 (m, 1H). LC-MS: $m/z$ = 471.2 $[M+H]^+$.

Example 36

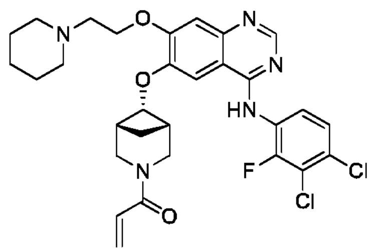

Synthetic route:

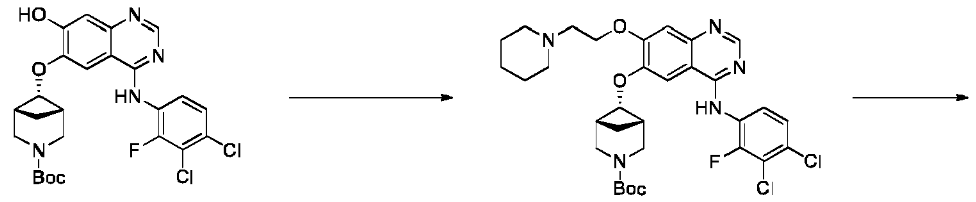

16-4 36-2

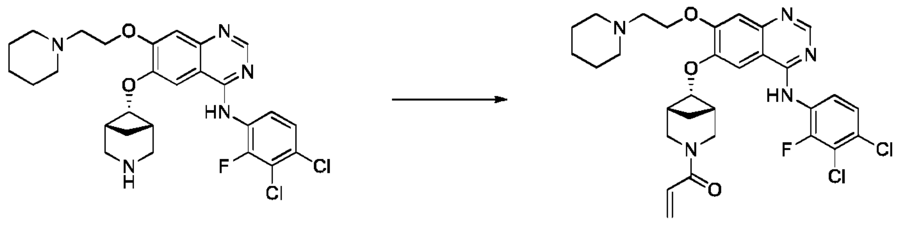

36-3 Example 36

The first step

Compound **16-4** (100.0 mg, 470.56 μmol) was dissolved in N,N-dimethylformamide (2 mL), and to the reaction solution was added NaH (60%, 22.4 mg, 560.34 μmol). The reaction solution was stirred at 0 °C for 0.5 hour, and a solution of compound 1-(2-bromoethyl)piperidine (55.1 mg, 373.56 μmol) in N,N-dimethylformamide (0.5 mL) was added. The reaction solution was stirred at 15 °C for 5 hours, and then diluted with water (10 mL), and extracted with ethyl acetate (5 mL x 3). The organic phases were combined, dried over anhydrous sodium sulfate, and filtered. The filtrate was concentrated under reduced pressure, and the residue was isolated by preparative thin-layer chromatography (dichloromethane:methanol = 7:1) to afford compound **36-2**.

The second step

The hydrochloride salt of compound **36-3** was obtained by referring to the second step of example 21.

The third step

**Compound of example 36** was obtained by referring to the ninth step of example 20. $^1$H NMR (400MHz, $CDCl_3$) δ 8.70 (s, 1H), 8.49 (t, *J* = 8.4 Hz, 1H), 7.34 (d, *J* = 8.8 Hz, 1H), 7.27-7.25 (m, 1H), 7.07 (s, 1H), 6.66-6.57 (m, 1H), 6.43 (d, *J* = 16.8 Hz, 1H), 5.73 (d, *J* = 10.0 Hz, 1H), 4.69-4.67 (m, 1H), 4.28-4.28 (m, 2H), 3.99-3.94 (m, 2H), 3.90-3.87 (m, 1H), 3.74-3.71 (m, 1H), 3.01-3.00 (m, 2H), 2.89 (m, 2H), 2.59 (m, 4H), 2.05-2.01 (m, 2H), 1.65 - 1.61 (m, 4H), 1.48-1.46 (m, 2H); LC-MS: m/z = 600.3 $[M+H]^+$.

Example 37

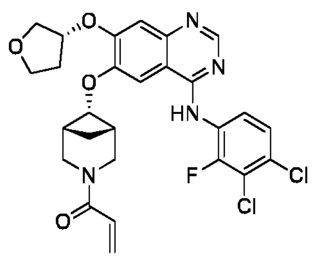

Synthetic route:

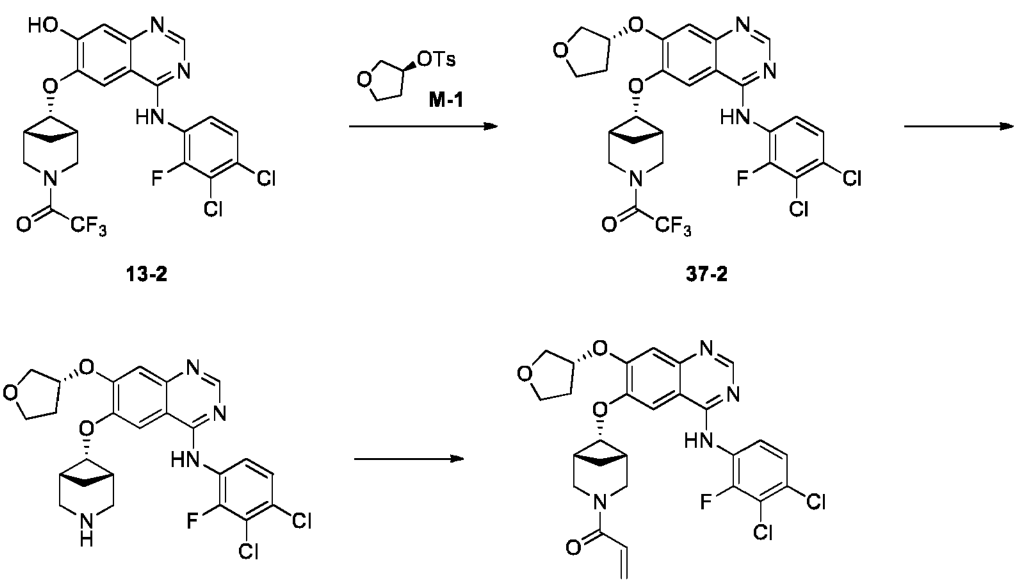


13-2 37-2

37-3 Example 37

The first step

Compound **13-2** (250.0 mg, 470.56 μmol) was dissolved in N,N-dimethylformamide (4 mL), and to the reaction solution were added potassium carbonate (130.0 mg, 941.11 μmol) and compound **M-1** (171.0 mg, 705.83 μmol). The reaction solution was stirred at 110 °C for 18 hours. The solvent was concentrated under reduced pressure, and the residue was isolated by preparative thin-layer chromatography (dichloromethane:methanol = 15:1) to afford compound **37-2**. LC-MS: m/z = 601.3 $[M+H]^+$.

The second step

Compound **37-2** (100.0 mg, 166.29 μmol) was dissolved in methanol (5 mL), and to the mixture was added potassium carbonate (68.9 mg, 498.86 μmol). The reaction solution was stirred at 40-50 °C for 3 hours, and then further stirred at 60 °C for 12 hours. The reaction solution was concentrated under reduced pressure, and the residue was isolated by preparative thin-layer chromatography (dichloromethane:methanol = 10:1) to afford compound **37-3**. LC-MS: m/z = 505.2 $[M+H]^+$.

The third step

Compound of example 37 was obtained by referring to the ninth step of example 20. $^1H$ NMR (400MHz, $CDCl_3$) δ 8.40 (s, 1H), 7.79 (s, 1H), 7.65-7.58 (m, 1H), 7.47 (d, *J* = 10.8 Hz, 1H), 7.18 (s, 1H), 6.84-6.73 (m, 1H), 6.36-6.29 (m, 1H), 5.82-5.75 (m, 1H), 5.19 (s, 1H), 4.09-4.03 (m, 1H), 3.99-3.87 (m, 5H), 3.84-3.78 (m, 1H), 3.60-3.75(m, 1H), 3.37 (s, 1H), 3.17-3.03 (m, 2H), 2.39-2.26 (m, 1H), 2.16 (s, 1H), 2.04-1.93 (s, 1H), 1.61 (d , *J* = 10.4 Hz, 1H). LC-MS: m/z = 559.3 $[M+H]^+$.

Example 38

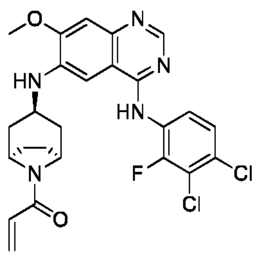

Synthetic route:

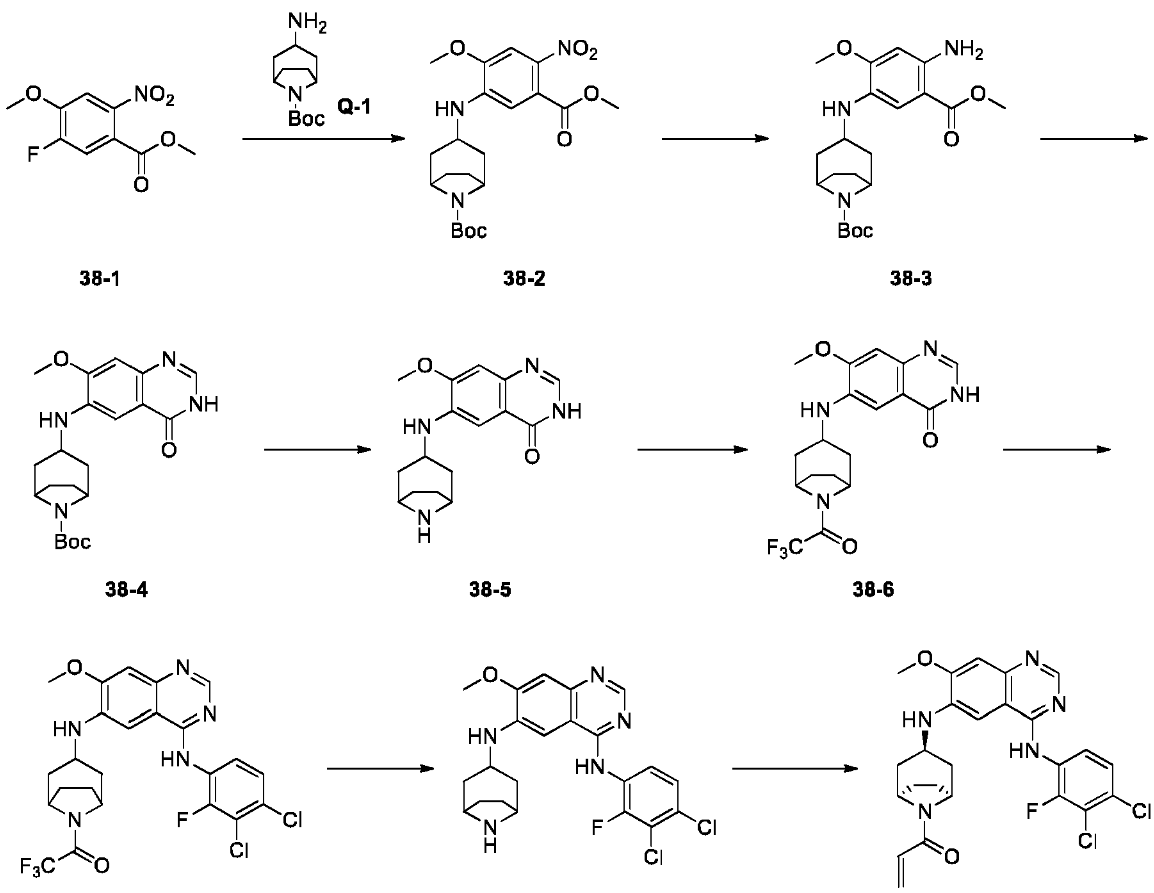


38-1 38-2 38-3

38-4 38-5 38-6

38-7 38-8 Example 38

The first step

Compound **38-1** (900.0 mg, 3.93 mmol) was dissolved in N,N-dimethylformamide (20 mL), and to the mixture were added potassium carbonate (1.09 g, 7.85 mmol) and compound **Q-1** (1.60 g, 7.07 mmol). The reaction solution was stirred at 75 °C for 16 hours. The reaction solution was cooled to 20 °C, diluted with water (30 mL), and extracted with ethyl acetate (30 mL x 2). The organic phases were combined, washed sequentially with water (40 mL x 2) and saturated brine (40 mL x 2), dried over anhydrous sodium sulfate, and filtered. The filtrate was concentrated under reduced pressure, and the residue was isolated by silica gel column chromatography (ethyl acetate:petroleum ether = 1:3) to afford compound **38-2**. $^1$H NMR (400 MHz, $CDCl_3$) δ 7.42 (s, 1H), 6.45 (s, 1H), 4.75 (d, *J* = 8.4 Hz, 1H), 4.10-4.40 (m, 2H), 3.87 (s, 3H), 3.86 (s, 3H), 3.75-3.80 (m, 1H), 1.90-2.10 (m, 4H), 1.50-1.70 (m, 4H), 1.41 (s, 9H). LC-MS: *m/z* = 458.1 [M+Na]$^+$.

The second step

Compound **38-2** (1.50 g, 3.44 mmol) was dissolved in methanol (30 mL), and to the mixture was added wet palladium/carbon (10%, 50.0 mg). The reaction solution was stirred at 20 °C under hydrogen atmosphere for 0.5 hour. The reaction solution was filtered, and the filtrate was concentrated under reduced pressure and dried to afford compound **38-3**. $^1$H NMR (400 MHz, $CDCl_3$) δ 7.09 (s, 1H), 6.10 (s, 1H), 5.36 (brs, 2H), 4.15-4.40 (m, 2H), 3.85 (s, 3H), 3.82 (s, 3H), 3.65-3.80 (m, 1H), 1.95-2.15 (m, 4H), 1.90-1.85 (m, 2H), 1.47 (s, 9H), 1.30-1.45 (m, 2H). LC-MS: *m/z* = 406.2 [M+H]$^+$.

The third step

Compound **38-3** (1.30 g, 3.21 mmol) and ammonium acetate (2.47 g, 32.06 mmol) were added to trimethyl orthoformate (31.29 g, 294.89 mmol). The reaction solution was stirred at 70 °C for 10 hours. The reaction solution was cooled to 20 °C, and concentrated under reduced pressure. The residue was slurried with water (10 mL) and filtered. The solid was dried to afford compound **38-4**. $^1$H NMR (400 MHz, DMSO-$d_6$) δ 7.83 (s, 1H), 7.08 (s, 1H), 7.00 (s, 1H), 5.17 (d, *J* = 8.4 Hz, 1H), 4.10-4.20 (m, 2H), 3.95-4.00(m, 1H), 3.92 (s, 3H), 1.90-2.00 (m, 4H), 1.75-1.90 (m, 2H), 1.50-1.60 (m, 2H), 1.43 (s, 9H). LC-MS: *m/z* = 401.1 [M+H]$^+$.

The fourth step

Compound **38-4** (1.20 g, 3.00 mmol) was added to a solution of hydrogen chloride in dioxane (4 N, 10 mL). The reaction solution was stirred at 0 °C for 0.4 hour. The reaction solution was concentrated under reduced pressure to obtain the hydrochloride salt of compound **38-5**. LC-MS: *m/z* = 301.0 [M+H]$^+$.

The fifth step

The hydrochloride salt of **38-5** (1.10 g, 3.27 mmol) and triethylamine (4.6 mL, 32.66 mmol) were dissolved in dichloromethane (30 mL) at 0 °C, and to the mixture was added trifluoroacetic anhydride (1.37 g, 6.53 mmol) dropwise. The reaction solution was stirred at 20 °C for 1 hour. The reaction solution was concentrated under reduced pressure, and the residue was slurried with water (40 mL) and filtered. The solid was dried to afford compound **38-6**. $^1$H NMR (400 MHz, DMSO-$d_6$) δ 11.90 (s, 1H), 7.80-7.90 (m, 1H), 7.13 (m, 1H), 7.00 (m, 1H), 5.28 (d, *J* = 9.2 Hz, 1H), 4.60-4.70 (m, 1H), 4.40-4.50 (m, 1H), 4.00-4.10 (m, 1H), 3.91 (s, 3H), 1.90-2.20 (m, 6H), 1.55-1.75 (m, 2H). LC-MS: *m/z* = 397.0 [M+H]$^+$.

The sixth step

Compound **38-6** (500.0 mg, 1.26 mmol) was dissolved in phosphorus oxychloride (24.1 mL, 260.22 mmol), and to the mixture was added N,N-dimethylformamide (92.2 mg, 1.26 mmol). The reaction solution was stirred at 100 °C for 5 hours. The reaction solution was concentrated under reduced pressure, and to the residue was added a solution of 3,4-dichloro-2-fluoroaniline (340.6 mg, 1.89 mmol) in isopropanol (5 mL). The reaction solution was stirred at 90 °C for 1 hour, and then cooled to 20 °C. The reaction solution was quenched by adding saturated sodium bicarbonate aqueous solution (10 mL), and extracted with ethyl acetate (10 mL x 2). The organic phases were combined, dried over anhydrous sodium sulfate, and filtered. The filtrate was concentrated under reduced pressure, and the residue was isolated by silica gel column chromatography (ethyl acetate:petroleum ether = 2:1) to afford compound **38-7**. $^1$H NMR (400 MHz, $CDCl_3$) δ 8.50-8.65 (m, 2H), 7.40-7.50 (m, 1H), 7.15-7.25 (m, 2H), 6.59 (s, 1H), 4.75-4.85 (m, 1H), 4.55-4.65 (m, 2H), 4.05-4.10 (m, 1H), 3.98 (s, 3H), 2.10-2.40 (m, 4H), 1.90-2.00 (m, 2H), 1.60-1.75 (m, 2H). LC-MS: *m/z* = 557.9 [M+H]$^+$.

The seventh step

Compound **38-7** (305.0 mg, 547.2 μmol) was dissolved in methanol (15 mL), and to the mixture was added potassium carbonate (378.2 mg, 2.74 mmol). The reaction solution was stirred at 50 °C for 14 hours. The reaction solution was concentrated under reduced pressure, and the residue was isolated using a silica gel preparation plate (dichloromethane:methanol = 10:1) to afford compound **38-8**. $^1$H NMR (400 MHz, DMSO-$d_6$) δ 9.45 (s, 1H), 8.28 (s, 1H), 7.67 (t, *J* = 8.0 Hz, 1H), 7.58 (d, *J* = 10.0 Hz, 1H), 7.20 (s, 1H), 7.14 (s, 1H), 5.17 (d, *J* = 8.4 Hz, 1H), 4.05-4.10 (m, 2H), 3.98-4.05 (m, 1H), 3.93 (s, 3H), 2.15-2.30 (m, 4H), 1.95-2.10 (m, 2H), 1.70-1.80 (m, 2H). LC-MS: *m/z* = 462.0 [M+H]$^+$.

The eighth step

Compound **38-8** (220.0 mg, 475.8 μmol) was dissolved in a mixed solvent of tetrahydrofuran (6 mL) and water

(6 mL) at 0 °C. To the mixture was added sodium bicarbonate (119.9 mg, 1.43 mmol), and added slowly acryloyl chloride (38.8 mg, 428.3 μmol) dropwise. The reaction solution was further stirred at 0 °C for 0.5 hour. The reaction solution was quenched by adding MeOH (1 mL) and concentrated under reduced pressure. The residue was isolated by preparative thin-layer chromatography (dichloromethane:methanol = 10:1) to obtain the **compound of example 38** ($R_f$= 0.3). $^1H$ NMR (400 MHz, DMSO-$d_6$) δ 9.35 (s, 1H), 8.24 (s, 1H), 7.66 (t, $J$ = 8.0 Hz, 1H), 7.57 (d, $J$ = 8.8 Hz, 1H), 7.14 (s, 1H), 7.06 (s, 1H), 6.72 (dd, $J_1$ = 16.8 Hz, $J_2$ = 10.4 Hz, 1H), 6.18 (dd, $J_1$ = 16.8 Hz, $J_2$=1.6 Hz, 1H), 5.68 (dd, $J_1$ = 10.4 Hz, $J_2$ = 1.6 Hz, 1H), 5.35 (d, $J$ = 9.2 Hz, 1H), 4.50-4.60 (m, 2H), 4.05-4.15 (m, 1H), 3.92 (s, 3H), 1.85-2.20 (m, 6H), 1.60-1.70 (m, 1H), 1.50-1.60 (m, 1H). LC-MS: $m/z$ = 538.0 [M+Na]$^+$.

Example 39

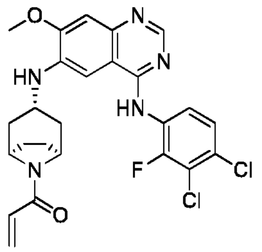

Synthetic route:

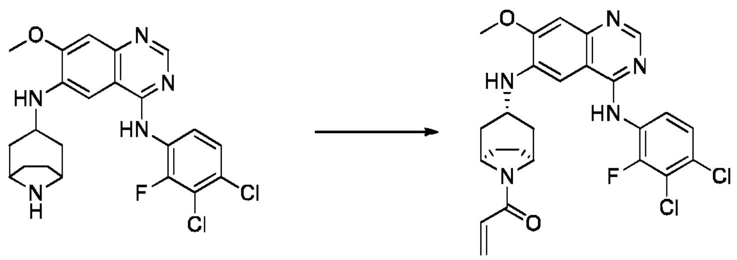

**38-8** Example 39

The first step

**Compound of example 39** was obtained by referring to the eighth step of example 38 ($R_f$ = 0.4). $^1H$ NMR (400 MHz, DMSO-$d_6$) δ 9.38 (s, 1H), 8.27 (s, 1H), 7.50-7.60 (m, 2H), 7.14 (s, 1H), 7.05 (s, 1H), 6.74 (dd, $J_1$ = 16.8 Hz, $J_2$ = 10.4 Hz, 1H), 6.18 (dd, $J_1$ = 16.8 Hz, $J_2$ = 2.0 Hz, 1H), 5.69 (dd, $J_1$ = 10.4 Hz, $J_2$ = 2.0 Hz, 1H), 5.47 (d, $J$ = 4.8 Hz, 1H), 4.50-4.60 (m, 2H), 4.01 (s, 3H), 3.80-3.90 (m, 1H), 2.20-2.30 (m, 1H), 1.85-2.15 (m, 7H). LC-MS: $m/z$ = 538.0 [M+Na]$^+$.

Example 40

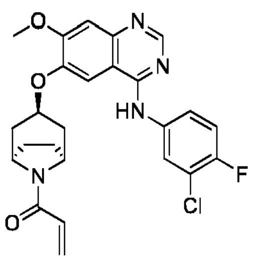

Synthetic route:

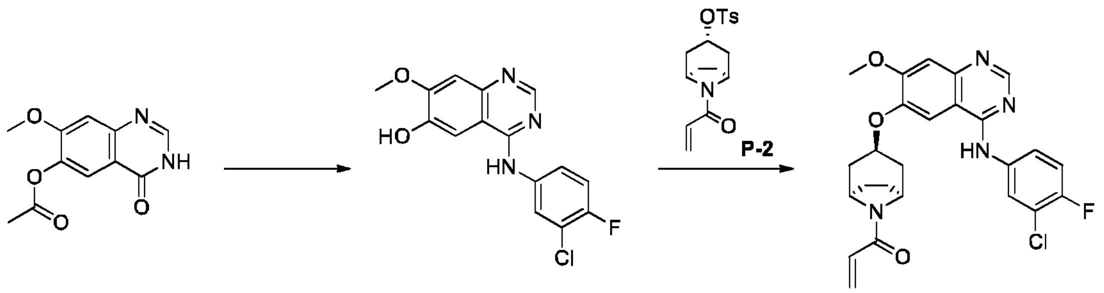


**40-1** **40-2** Example 40

The first step

Compound **40-1** (200.0 mg, 853.94 μmol) was dissolved in thionyl chloride (3 mL), and to the mixture was added N,N-dimethylformamide (6 μL 85.39 μmol). The reaction solution was heated to 80 °C and stirred for 1 hour. The reaction solution was concentrated under reduced pressure, and to the residue was added a solution of 3-chloro-4-fluoroaniline (130.0 mg, 893.09 μmol) in isopropanol (5 mL) at room temperature. The mixture was heated to 90 °C and stirred for 3 hours. The reaction solution was concentrated under reduced pressure, and to the residue was added ethyl acetate (15 mL) at room temperature. The suspension was stirred at room temperature for 1 hour and then filtered. The filter cake was dried in vacuo to afford compound **40-2**. LC-MS: *m/z* = 320.1 $[M+H]^+$.

The second step

Compound **40-2** (50.0 mg, 156.39 μmol) was dissolved in N,N-dimethylformamide (1 mL), and to the mixture were added cesium carbonate (100.0 mg, 306.92 μmol) and compound **P-2** (50.0 mg, 149.07 μmol). The reaction solution was stirred at 90 °C for 45 minutes. The reaction solution was cooled to 15 °C, poured into water (30 mL), and extracted with ethyl acetate (20 mL x 3). The organic phases were combined, washed sequentially with water (30 mL) and saturated brine (30 mL), dried over anhydrous sodium sulfate, and filtered. The filtrate was concentrated under reduced pressure, and the residue was isolated by preparative thin-layer chromatography (ethyl acetate:tetrahydrofuran = 4:1) to obtain the **compound of example 40**. $^1H$ NMR (400 MHz, DMSO-$d_6$) δ 9.68 (s, 1H), 8.51 (s, 1H), 8.19 (dd, $J_1$= 6.8 Hz, $J_2$= 2.8 Hz,, 1H), 7.95 (s, 1H), 7.78-7.84 (m, 1H), 7.46 (t, *J* = 9.2 Hz, 1H), 7.21 (s, 1H), 6.77 (dd, $J_1$= 16.4 Hz, $J_2$ = 10.4 Hz, 1H), 6.22 (dd, $J_1$= 16.4 Hz, $J_2$ = 2.4 Hz, 1H), 5.73 (dd, $J_1$ = 10.4 Hz, $J_2$ = 2.4 Hz, 1H), 4.97-5.11 (m, 1H), 4.58-4.63 (m, 2H), 3.92 (s, 3H), 2.30-2.38 (m, 1H), 2.20-2.27 (m, 1H), 2.00-2.09 (m, 2H), 1.84-1.97 (m, 2H), 1.65 (t, *J* = 10.4 Hz, 1H), 1.49 (t, *J* = 10.4 Hz, 1H). LC-MS: *m/z* = 483.1 $[M+H]^+$.

Example 41

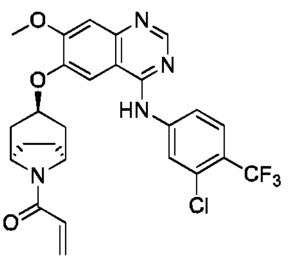

Synthetic route:

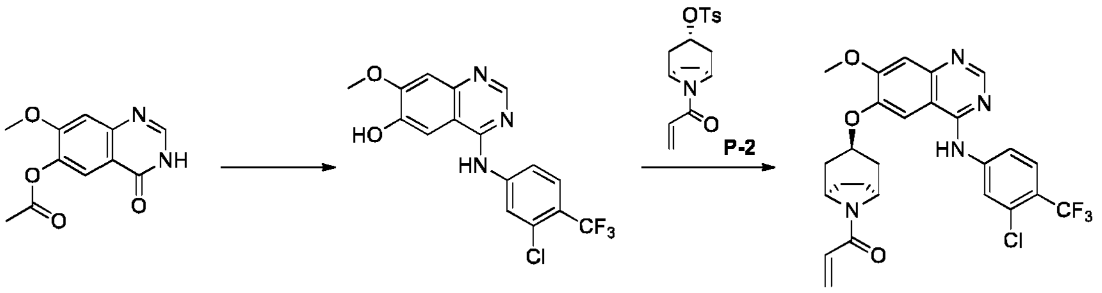


**40-1** **41-2** Example 41

The first step

Compound **41-2** was obtained by referring to the first step of example 40. $^1$H NMR (400 MHz, DMSO-*d*$_6$) δ 8.95 (s, 1H), 8.29 (s, 1H), 8.07 (s, 1H), 7.93-8.02 (m, 2H), 7.40 (s, 1H), 4.05 (s, 3H). LC-MS: *m*/*z* = 370.0 [M+H]$^+$.

The second step

**Compound of example 41** was obtained by referring to the second step of example 40. $^1$H NMR (400 MHz, DMSO-*d*$_6$) δ 9.82 (s, 1H), 8.65 (s, 1H), 8.44 (s, 1H), 8.04-8.09 (m, 1H), 7.87-7.94 (m, 2H), 7.28 (s, 1H), 6.77 (dd, $J_1$= 16.4 Hz, $J_2$ = 10.4 Hz, 1H), 6.22 (dd, $J_1$= 16.4 Hz, $J_2$ = 2.4 Hz, 1H), 5.73 (dd, $J_1$ = 10.4 Hz, $J_2$ = 2.4 Hz, 1H), 4.96-5.08 (m, 1H), 4.58-4.63 (m, 2H), 3.94 (s, 3H), 2.23-2.37 (m, 2H), 1.80-2.12 (m, 4H), 1.65 (t, $J$ = 10.4 Hz, 1H), 1.49 (t, $J$ = 10.4 Hz, 1H). LC-MS: *m*/*z* = 532.9 [M+H]$^+$.

Example 42

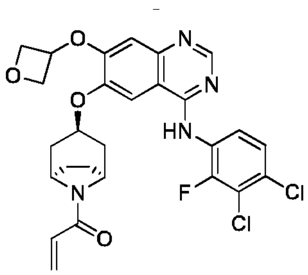

Synthetic route:

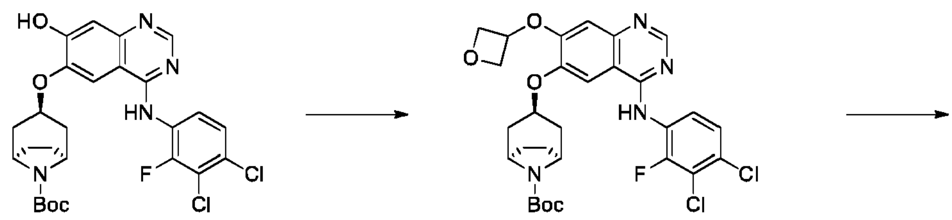

**20-5** **42-2**

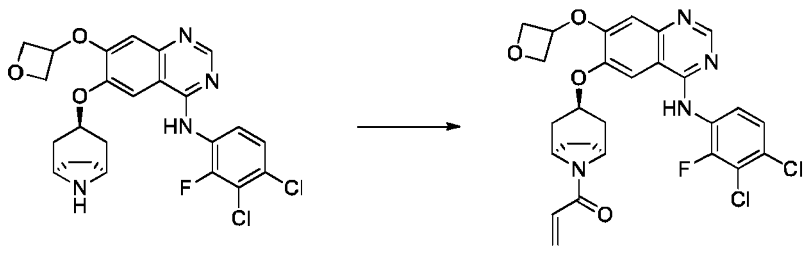

**42-3** Example 42

The first step

Compound **20-5** (200.0 mg, 364.02 μmol) was dissolved in N,N-dimethylformamide (2 mL), and to the mixture were added cesium carbonate (240.0 mg, 736.60 μmol), sodium iodide (4.0 mg, 26.69 μmol) and 3-iodooxetane (80.0 mg, 434.84 μmol). The reaction solution was stirred at 90 °C for 1 hour. The reaction solution was cooled to 15 °C, poured into water (30 mL), and extracted with ethyl acetate (20 mL × 3). The organic phases were combined, washed sequentially with water (30 mL) and saturated brine (30 mL), dried over anhydrous sodium sulfate, and filtered. The filtrate was concentrated under reduced pressure, and the residue was isolated by preparative thin-layer chromatography (ethyl acetate) to afford compound **42-2.** $^1$H NMR (400 MHz, $CDCl_3$) δ 8.63 (s, 1H), 8.48 (t, *J* = 8.8 Hz, 1H), 7.27 (dd, $J_1$ = 9.2 Hz, $J_2$ = 2.0 Hz, 1H), 7.18 (s, 1H), 6.76 (s, 1H), 5.28-5.32 (m, 1H), 4.99-5.05 (m, 2H), 4.68-4.80 (m, 3H), 4.21-4.40 (m, 2H), 2.10-2.15 (m, 2H), 2.00-2.06 (m, 2H), 1.82-1.96 (m, 2H), 1.58-1.64 (m, 2H), 1.45 (s, 9H). LC-MS: *m*/*z* = 605.3 $[M+H]^+$.

The second step

Compound **42-2** (400.0 mg, 709.92 μmol) was dissolved in dichloromethane (2mL), and to the mixture was added trifluoroacetic acid (1 mL). The reaction solution was reacted at 15 °C for 0.5 hour. The reaction solution was directly concentrated under reduced pressure to obtain the trifluoroacetate salt of compound **42-3.** LC-MS: *m*/*z* = 505.2 $[M+H]^+$.

The third step

The trifluoroacetate salt of compound **42-3** (50.0 mg, 98.94 μmol) was dissolved in a mixed solvent of tetrahydrofuran (2 mL) and water (2 mL) at 0 °C. To the mixture was added sodium bicarbonate (26.0 mg, 309.50 μmol), and added slowly acryloyl chloride (10.0 mg, 110.49 μmol) dropwise. The reaction solution was further stirred at 0 °C for 1 hour. The reaction solution was quenched by adding methanol (10 mL) and concentrated under reduced pressure. The residue was isolated by preparative thin-layer chromatography (ethyl acetate:tetrahydrofuran = 4:1) to obtain the **compound of example 42.** $^1$H NMR (400 MHz, DMSO-$d_6$) δ 9.72 (br s, 1H), 8.38 (s, 1H), 7.84 (s, 1H), 7.61 (s, 2H), 6.84-6.89 (m, 1H), 6.77 (dd, $J_1$= 16.4 Hz, $J_2$= 10.4 Hz, 1H), 6.22 (dd, $J_1$= 16.4 Hz, $J_2$= 2.4 Hz, 1H), 5.73 (dd, $J_1$= 10.4 Hz, $J_2$ = 2.4 Hz, 1H), 5.40-5.52 (m, 1H), 4.95-5.08 (m, 3H), 4.55-4.69 (m, 4H), 2.25-2.43 (m, 2H), 1.86-2.05 (m, 4H), 1.65 (t, *J* = 10.4 Hz, 1H), 1.49 (t, *J* = 10.4 Hz, 1H). LC-MS: *m*/*z* = 559.0 $[M+H]^+$.

Example 43

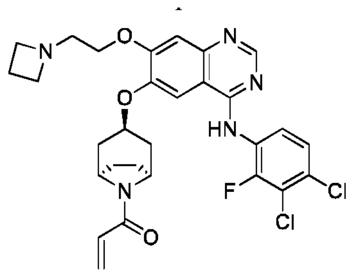

Synthetic route:

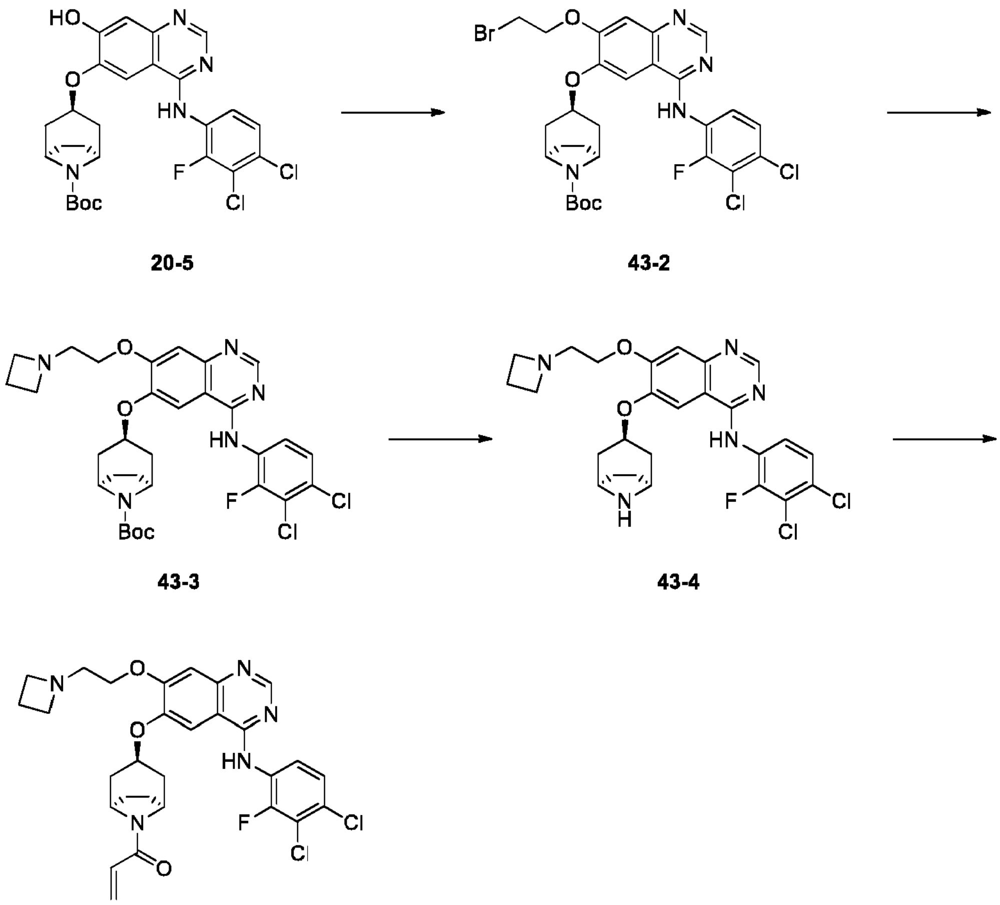

20-5

43-2

43-3

43-4

Example 43

The first step

Compound **20-5** (300.0 mg, 546.03 μmol) was dissolved in N,N-dimethylformamide (3 mL), and then to the mixture was added sodium hydride (60%, 45 mg, 1.12 mmol) in one portion at 0 °C. The reaction solution was stirred at 0 °C for 0.5 hour, and 1,2-dibromoethane (149.4 mg, 795.27 μmol) was added. The reaction solution was stirred at 15 °C for 11.5 hours, then quenched with water (50 mL) and extracted with ethyl acetate (50 mL × 3). The organic phases were combined, dried over anhydrous sodium sulfate, and filtered. The filtrate was concentrated under reduced pressure, and the residue was isolated by preparative thin-layer chromatography (ethyl acetate) to afford compound **43-2.** $^1$H NMR (400 MHz, $CDCl_3$) δ 8.64 (s, 1H), 8.46 (t, *J* = 8.53 Hz, 1H), 7.20-7.29 (m, 4H), 4.66-4.77 (m, 1H), 4.40-4.45 (m, 2H), 4.18-4.32 (m, 2H), 3.70-3.75 (m, 2H), 2.03-2.15 (m, 2H), 1.90-1.95 (m, 2H), 1.72-1.82 (m, 2H), 1.58-1.65 (m, 2H), 1.45 (s, 9H). LC-MS: *m/z* = 657.2 [M+H]$^+$.

The second step

Compound **43-2** (170.0 mg, 259.00 μmol) was dissolved in tetrahydrofuran (3mL), and to the mixture was added azetidine (42.3 mg, 740.88 μmol). The reaction solution was stirred at 50 °C for 1.5 hours, and then concentrated under reduced pressure to afford compound **43-3.** LC-MS: *m*/*z* = 632.2 $[M+H]^+$.

The third step

The trifluoroacetate salt of compound **43-4** was obtained by referring to the second step of example 42. LC-MS: *m*/*z* = 532.3 $[M+H]^+$.

The fourth step

**Compound of example 43** was obtained by referring to the third step of example 42. $^1$H NMR (400 MHz, DMSO-$d_6$) δ 9.63 (s, 1H), 8.39 (s, 1H), 7.80 (s, 1H), 7.56-7.67 (m, 2H), 7.19 (s, 1H), 6.77 (dd, $J_1$= 16.4 Hz, $J_2$= 10.4 Hz, 1H), 6.22 (dd, $J_1$= 16.4 Hz, $J_2$= 2.4 Hz, 1H), 5.73 (dd, $J_1$ = 10.4 Hz, $J_2$ = 2.4 Hz, 1H), 4.88-5.06 (m, 1H), 4.54-4.66 (m, 2H), 4.04-4.14 (m, 2H), 3.22-3.26 (m, 4H), 2.76-2.84 (m, 2H), 2.16-2.32 (m, 2H), 1.85-2.07 (m, 6H), 1.65 (t, *J* = 10.4 Hz, 1H), 1.49 (t, *J* = 10.4 Hz, 1H). LC-MS: *m*/*z* = 586.1 $[M+H]^+$.

Example 44

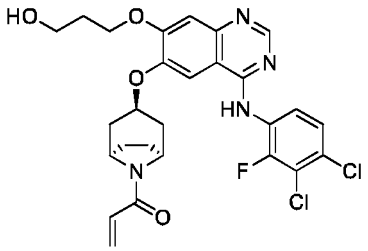

Synthetic route:

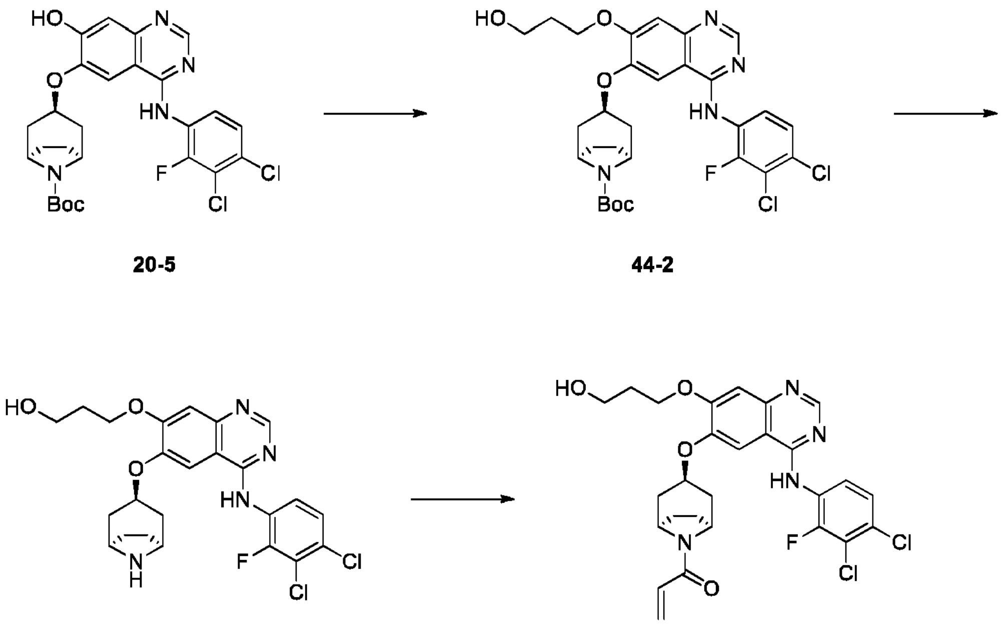

20-5

44-2

44-3

Example 44

The first step

Compound **44-2** was obtained by referring to the first step of example 43. LC-MS: *m/z* = 607.3 $[M+H]^+$.

The second step

Compound **44-2** (170.0 mg, 279.84 μmol) was dissolved in a solution of hydrogen chloride in ethyl acetate (4 N, 10 mL), and stirred at 20-25 °C for 15 minutes. The reaction solution was concentrated under reduced pressure to obtain the hydrochloride salt of compound **44-3.** LC-MS: m/z = 507.2 $[M+H]^+$.

The third step

**Compound of example 44** was obtained by referring to the third step of **example 42.** $^1$H NMR (400 MHz, DMSO-$d_6$) δ 9.63 (s, 1H), 8.37 (s, 1H), 7.80 (s, 1H), 7.46-7.68 (m, 2H), 7.21 (s, 1H), 6.77 (dd, $J_1$= 16.4 Hz, $J_2$= 10.4 Hz, 1H), 6.22 (dd, $J_1$= 16.4 Hz, $J_2$= 2.4 Hz, 1H), 5.73 (dd, $J_1$= 10.4 Hz, $J_2$ = 2.4 Hz, 1H), 4.82-4.94 (m, 1H), 4.57-4.62 (m, 2H), 4.16-4.24 (m, 2H), 3.53-3.64 (m, 2H), 2.16-2.32 (m, 2H), 1.75-2.10 (m, 6H), 1.65 (t, $J$ = 10.4 Hz, 1H), 1.49 (t, $J$ = 10.4 Hz, 1H). LC-MS: *m/z* = 560.9 $[M+H]^+$.

Example 45

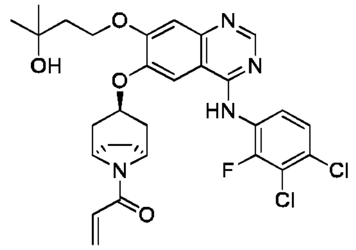

Synthetic route:

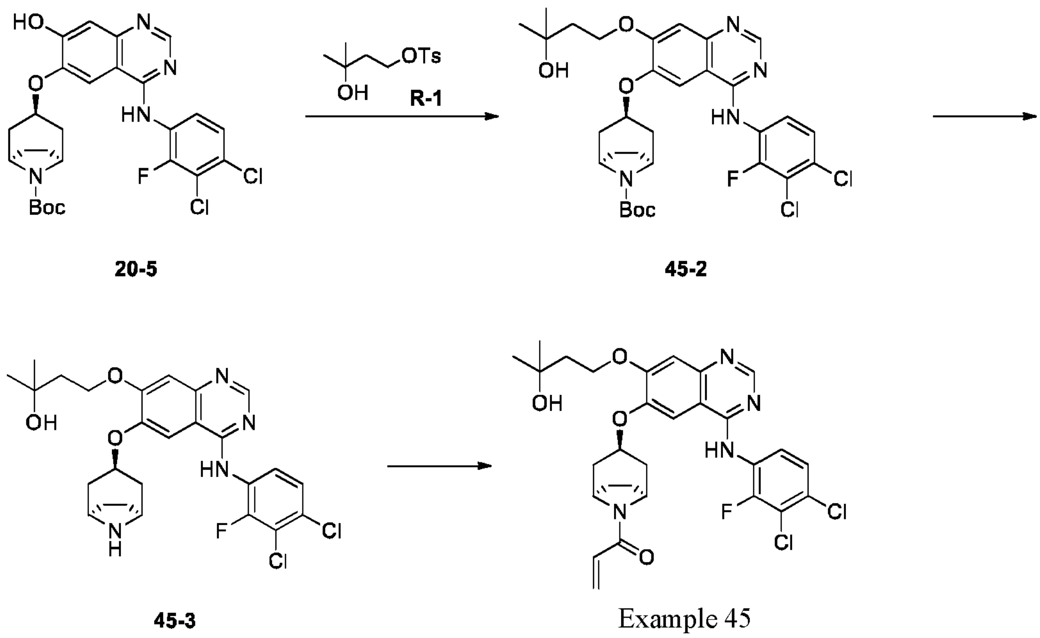


20-5

45-2

45-3

Example 45

The first step

Compound **45-2** was obtained by referring to the first step of example 42. $^1$H NMR (400 MHz, $CDCl_3$) δ 8.71 (s, 1H), 8.53 (t, *J* = 8.4 Hz, 1H), 7.27-7.37 (m, 3H), 7.17 (s, 1H), 4.71-4.84 (m, 1H), 4.28-4.44 (m, 3H), 2.14-2.22 (m, *J* = 7.8 Hz, 2H), 1.54-2.01 (m, 10H), 1.51 (s, 9H), 1.33 (s, 6H). LC-MS: *m*/*z* = 635.3 $[M+H]^+$.

The second step

The hydrochloride salt of compound **45-3** was obtained by referring to the second step of **example 44.** LC-MS: *m*/*z* = 535.3 $[M+H]^+$.

The third step

**Compound of example 45** was obtained by referring to the third step of **example 42.** $^1$H NMR (400 MHz, DMSO-$d_6$) δ 9.75 (s, 1H), 8.37 (s, 1H), 7.84 (s, 1H), 7.49-7.66 (m, 2H), 7.22 (s, 1H), 6.77 (dd, $J_1$ = 16.4 Hz, $J_2$ = 10.4 Hz, 1H), 6.22 (dd, $J_1$ = 16.4 Hz, $J_2$ = 2.4 Hz, 1H), 5.73 (dd, $J_1$ = 10.4 Hz, $J_2$ = 2.4 Hz, 1H), 4.88-5.03 (m, 1H), 4.55-4.64 (m, 2H), 4.18-4.27 (m, 2H), 2.17-2.39 (m, 2H), 1.82-2.07 (m, 6H), 1.65 (t, *J* = 10.4 Hz, 1H), 1.49 (t, *J* = 10.4 Hz, 1H), 1.17 (s, 6H). LC-MS: *m*/*z* = 589.3 $[M+H]^+$.

Example 46

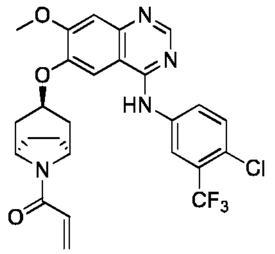

Synthetic route:

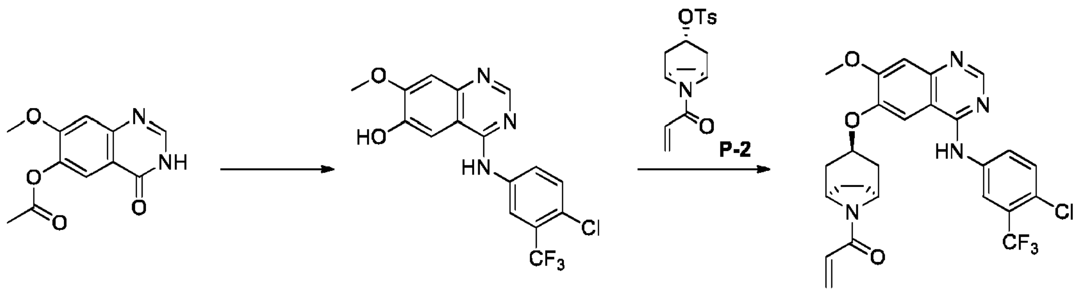


40-1 46-2 Example 46

The first step

Compound **46-2** was obtained by referring to the first step of example 40. $^1$H NMR (400 MHz, DMSO-$d_6$) δ 11.43 (s, 1H), 8.90 (s, 2H), 8.34 (d, *J* = 2.4 Hz, 1H), 8.19 (s, 1H), 8.15 (dd, $J_1$= 8.4 Hz, $J_2$= 2.4 Hz, 1H), 7.83 (d, *J*=8.4 Hz, 1H), 7.51 (s, 1H), 4.03 (s, 3H). LC-MS: *m*/*z* = 370.1 $[M+H]^+$.

The second step

**Compound of example 46** was obtained by referring to the second step of example 40. $^1$H NMR (400 MHz,

DMSO-$d_6$) δ 10.16 (s, 1H), 8.50-8.62 (m, 2H), 8.30-8.38 (m, 1H), 8.17 (s, 1H), 7.73 (d, $J$= 8.8 Hz, 1H), 7.23 (s, 1H), 6.77 (dd, $J_1$ = 16.4 Hz, $J_2$ = 10.4 Hz, 1H), 6.22 (dd, $J_1$ = 16.4 Hz, $J_2$ = 2.4 Hz, 1H), 5.73 (dd, $J_1$= 10.4 Hz, $J_2$= 2.4 Hz, 1H), 5.09-5.21 (m, 1H), 4.54-4.65 (m, 2H), 3.93 (s, 3H), 2.20-2.34 (m, 2H), 1.87-2.10 (m, 4H), 1.65 (t, $J$ = 10.4 Hz, 1H), 1.49 (t, $J$ = 10.4 Hz, 1H). LC-MS: $m/z$ = 533.1 $[M+H]^+$.

Example 47

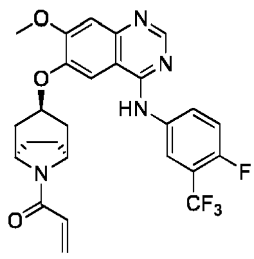

Synthetic route:

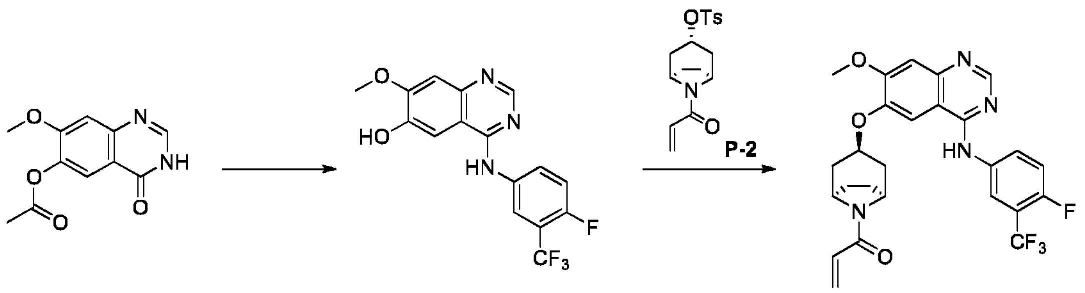


**40-1** **47-2** Example 47

The first step

Compound **47-2** was obtained by referring to the first step of example 40. LC-MS: $m/z$ = 354.1 $[M+H]^+$.

The second step

**Compound of example 47** was obtained by referring to the second step of example 40. $^1H$ NMR (400 MHz, DMSO-$d_6$) δ 9.81 (s, 1H), 8.52 (s, 1H), 8.29-8.38 (m, 1H), 8.17-8.25 (m, 1H), 7.95 (s, 1H), 7.56 (t, $J$ = 9.54 Hz, 1H), 7.23 (s, 1H), 6.77 (dd, $J_1$= 16.4 Hz, $J_2$= 10.4 Hz, 1H), 6.22 (dd, $J_1$= 16.4 Hz, $J_2$= 2.4 Hz, 1H), 5.73 (dd, $J_1$= 10.4 Hz, $J_2$= 2.4 Hz, 1H), 4.97-5.12 (m, 1H), 4.57-4.69 (m, 2H), 3.93 (s, 3H), 2.19-2.31 (m, 2H), 1.88-2.03 (m, 4H), 1.65 (t, $J$ = 10.4 Hz, 1H), 1.49 (t, $J$ = 10.4 Hz, 1H). LC-MS: $m/z$ = 517.1 $[M+H]^+$.

Example 48

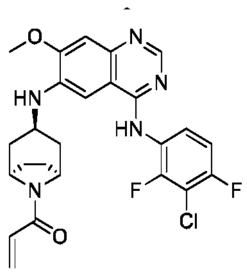

Synthetic route:

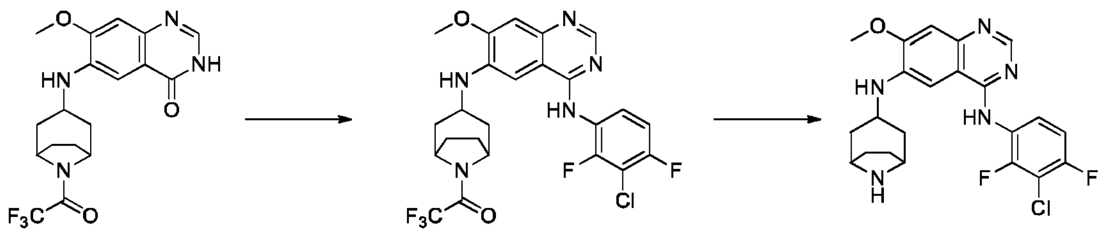

**38-6** **48-2** **48-3**

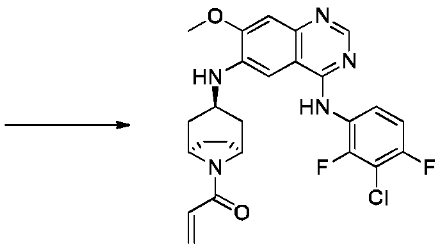

Example 48

The first step

Compound 48-2 (2.7 g) was obtained by referring to the sixth step of example 38. LC-MS: m/z = 542.2 [M+H]+.

The second step

Compound **48-3** (0.9 g) was obtained by referring to the seventh step of example 38. LC-MS: m/z = 446.1 [M+H]+.

The third step

Example 48 (160.0 mg) was obtained by referring to the eighth step of example 38. $^1$H NMR (400MHz, $CD_3OD$) D 8.23 (s, 1H), 7.61-7.52 (m, 1H), 7.27-7.17 (m, 2H), 7.09 (s, 1H), 6.75 (dd, $J_1$ = 12.8 Hz, $J_2$ = 10.4 Hz, 1H), 6.33 (dd, $J_1$ = 16.8 Hz, $J_2$ = 2.0 Hz, 1H), 5.79 (dd, $J_1$ = 6.4 Hz, $J_2$ = 2.0 Hz, 1H), 4.79-4.75 (m, 1H), 4.63-4.61 (m, 1H), 4.29-4.23 (m, 1H), 4.03 (s, 3H), 2.34-2.31 (m, 1H), 2.27-2.21 (m, 1H), 2.19-2.14 (m, 2H), 2.13-2.03 (m, 2H), 1.76-1.66 (m, 1H), 1.64-1.53 (m, 1H). LC-MS: m/z = 500.2 [M+H]+.

**Biochemical assay: in vitro evaluation**

**Assay example 1: evaluation of enzyme activity**

The purpose of this assay is to detect the in vitro inhibitory activity of compounds on HER1(ErbB 1), HER2(ErbB2), and HER4(ErbB4). The enzymes used in this assay are human ErbB1, ErbB2 and ErbB4. Eurofins Pharma Discovery Service provided the method for activity detection. The results of inhibitory activity of the test compounds on HER1, HER2, and HER4 are shown in Table 1.

Steps and method of the assay (96-well plate):

A buffer solution of the test compounds in a dilution of 5-fold (5μL), polypeptide substrate poly(Glu, Tyr) (4:1) (2.5μL), ErbB (4-20 ng, 2.5μL), $MnCl_2$(50 mM, 1.25 μL), $dH_2O$ (3.75 μL), and [γ-$^{33}$P]ATP (10 μL) were added, and incubated at 30 °C for 10 minutes. The reaction was terminated by adding 3% phosphoric acid. 10 μL of the sample was taken and transferred to Filtermate A. The filter disc was washed 3 times with 75 mM phosphoric acid and once with methanol. The filter disc was transferred to a sealed plastic bag and the scintillation mixture (4 mL) was added. The intensity of the emitted photon was detected by a scintillation luminescence counter. The CPM (times/min) of the enzyme sample was compared with the CPM of the internal control sample. The level of photon intensity reflected the degree of the tyrosine kinase activity.

**Table 1: results of the in vitro enzyme activity screening assay for compounds disclosed herein**

| Compound | **HER1 $IC_{50}$(nM)** | **HER2 $IC_{50}$ (nM)** | **HER4 $IC_{50}$ (nM)** |
|---|---|---|---|
| Example 1 | 2 | 3 | 10 |
| Example 3 | 2 | 5 | 17 |
| Example 4 | 2 | 3 | 13 |
| Example 5 | 9 | 24 | 157 |
| Example 6 | 2 | 4 | 17 |
| Example 7 | 40 | 69 | 61 |
| Example 8 | 6.0$^{b}$ | 5.5$^{b}$ | 3.5$^{b}$ |
| Example 9 | 9 | 13 | 8 |
| Example 13 | 5 | 8 | 7 |
| Example 18 | 9 | 12 | 5 |
| Example 20 | 18 | 11 | 10 |
| Example 21 | 12 | 5 | 3 |
| Example 22 | 12 | 7 | 3 |
| Example 23 | 8 | 4 | 2 |
| Example 24 | 10 | 6 | 3 |
| Example 25 | 6 | 3 | 6 |
| Example 26 | 8 | 5 | 4 |
| Example 27 | 16 | 5 | 3 |
| Example 32 | 15 | 5 | 3 |
| Example 33 | 7 | 6 | 3 |
| Example 34 | 5 | 4 | 2 |
| Example 38 | 8 | 5 | 3 |
| Example 40 | 11 | 5 | 2 |
| Example 44 | 5 | 4 | 3 |
| Example 45 | 8 | 7 | 10 |
| Example 48 | 9 | 11 | 4 |
| b: average of two tests. | | | |

Conclusion: the compounds disclosed herein have obvious inhibitory activity against HER1, HER2 and HER4.

**Assay example 2: evaluation of inhibitory activity on cell proliferation**

Purpose of the assay: to detect the inhibitory activity of the test compounds on cell proliferation.

Principle of the assay: the luciferase in a Cell-Titer-Glo reagent produces oxidized luciferin with luciferin, oxygen and ATP as reaction substrates, and releases energy in the form of light. Since the luciferase reaction requires ATP, the total amount of light generated by the reaction is proportional to the total amount of ATP that reflects cell viability.

Materials of the assay:

Cell line: NCI-N87 cell line (ATCC-CRL-5822), BT-474 cell line (ATCC-HTB-20), OE21 (ECACC-96062201)
Cell culture medium: (RPMI 1640 medium (Invitrogen#22400-105; 10% serum Invitrogen #10090148; L-glutamine 1×, Gibco#25030-081; penicillin-streptomycin Hyclone# SV30010)
Cell Titer-Glo® cell viability assay kit with luminescence (Promega #G7573)
384-well cell culture plate (Greiner # 781090)
Compound plate (LABCYTE # LP-0200)
$CO_2$ incubator (Thermo#371)
Vi-cell cell counter (Beckman Coulter)
Pipette (Eppendorf)
Pipetting tube (Greiner)
Pipetting gun (Eppendorf)
Multifunctional microplate reader (Envision Reader)
ECHO Liquid-handling workstation (Labcyte-ECHO555)

Steps and method of the assay:

2.1 Day 1:

According to the schematic diagram of the cell seed plate, cells were seeded in a 384- or 96-well plate at a density of 1000 cells per well and 25 μL per well, and 25 μL of PBS was added to the wells near edge of the place where no cells were seeded.

2.2 Day 0:

(1) The stock solution of the compounds has a concentration of 10 mM, and the compounds were diluted with DMSO to an initial concentration of 4 mM. The compounds were added to the plate with stock solution of compounds, 9 μL per well.
(2) The compounds were diluted with an ECHO liquid workstation, where 125 nL of compounds were added to each well of the cell plate, 125 nL of DMSO was added to each of the cell well in columns 2 and 23, and 125 nL of DMSO was added to each PBS well in columns 1 and 24.
(3) The cell plate was supplemented with 25 μL of medium per well, to a final volume of 50 μL per well; the compounds had a concentration of 1 μM, and was serially 3-fold diluted to obtain 10 concentrations, and the left and right wells were used in duplicate; the final concentration of DMSO was 0.25%.

2.3 After addition of the compounds, the cell plate was centrifuged at 1000 rpm for 1 min, and then placed in a 37 °C, 5% $CO_2$ incubator for incubation for 3 days.

2.4 Day 3:

The cell plate was taken out from the incubator and equilibrated at room temperature for 30 minutes. 25 μL of Cell-Titer-Glo reagent was added to each well, shaken for one minute to allow well mixed, and centrifuged at 1000 rpm for 1 minute. After 10 minutes, the plate was read on PerkinElmer Envision, and the fluorescence reading time was set to 0.2 second.

Assay results: the assay results are shown in Table 2.

CTG assay of OE19 cells

OE19 cells with saturation of 80%-90% were digested with trypsin, centrifuged, resuspended and counted. The

concentration of cells was adjusted to 90 ml/well. OE19 cells were added to a 96-well cell culture plate to 8000 OE19 cells per well. The cells were cultured in a cell incubator containing 5% $CO_2$ at 37 °C overnight.

The stock solution of the test compounds was serially 3-fold diluted with DMSO to a total of 10 concentrations. Before the start of the assay, the serially diluted test compounds were further diluted to 10 × compound solutions (the compound with a highest concentration contains 1% DMSO) with cell culture medium under sterile conditions. The prepared 10 × compound solutions were added to the cell culture plate, and the 10 μL compound solution was added to each well. In this way, the final concentrations of staurosporine as the standard control and all the test compounds were obtained starting from 10 μM as the initial concentration in a 3-fold serial dilution to get 10 test concentrations.

After 72 hours of cell culture, the 96-well cell culture plate was taken out. Cell Titer Glo reagent was added at 50 ml/well, mixed, centrifuged, and incubated at room temperature in the dark for 10 minutes. The cell plate was placed in Envision for reading.

The inhibition rate was calculated according to original data as follows:

$$\text{Inhibition \%} = (\text{ZPE - sample detection value})/(\text{ZPE-HPE}) \times 100\%$$

The well containing 10 mM staurosporine on DAY3 (the first day of the assay is the day when compounds were added, and DAY3 is the day when reading is perfomed) was used as the HPE (100% inhibition control) well, and the well containing 0.1% DMSO on DAY3 was used as the ZPE (0% inhibition control) well. Each concentration of test compounds was tested in duplicate.

Non-linear regression analysis was performed using the processed data and GraphPad Prism 6 analysis software to obtain a dose-response curve, and the half-inhibitory concentration ($IC_{50}$) of the test compounds on OE19 cells was calculated. The assay results are shown in Table 2.

**Table 2: results of the screening assay of inhibitory activity of compounds disclosed herein against cell proliferation in vitro**

| Compound | BT-474 cell | NCI-N87 cell | OE21 cell[c] | OE19 cell[c] |
|---|---|---|---|---|
| | $IC_{50}$ (nM) | $IC_{50}$ (nM) | $IC_{50}$ (nM) | $IC_{50}$ (nM) |
| Example 1 | ND | < 1.5[d] | ND | ND |
| Example 7 | ND | 1.5[d] | ND | ND |
| Example 8 | 0.39[b] | 0.28[b] | 0.47 | 0.48 |
| Example 9 | 2.1[d] | 0.9[d] | ND | ND |
| Example 13 | 2.0[d] | 1.6[d] | ND | ND |
| Example 18 | 2.4[d] | 1.3[d] | ND | ND |
| Example 20 | ND | 0.6 | ND | ND |
| Example 21 | 0.46 | 0.35 | ND | ND |
| Example 23 | ND | 0.14 | ND | ND |
| Example 24 | ND | 0.17 | ND | ND |
| Example 25 | ND | 0.19 | ND | ND |
| Example 26 | ND | 0.24 | ND | ND |
| Example 27 | ND | 0.31 | ND | ND |
| Example 32 | ND | 0.29 | ND | ND |
| Example 34 | ND | 0.42 | 0.27 | 0.001 |
| Example 38 | ND | 0.46 | 0.52 | 1.20 |
| Example 40 | ND | 0.34 | ND | ND |
| Example 42 | ND | 0.92 | ND | ND |
| Example 44 | ND | 0.33 | ND | ND |
| Example 45 | ND | 0.69 | ND | ND |

(continued)

| Compound | BT-474 cell | NCI-N87 cell | OE21 cell[c] | OE19 cell[c] |
|---|---|---|---|---|
| | $IC_{50}$ (nM) | $IC_{50}$ (nM) | $IC_{50}$ (nM) | $IC_{50}$ (nM) |
| Example 48 | ND | ND | 0.17 | 0.24 |
| ND: not tested; b: average of two tests; d: initial dilution concentration of compounds is 10 μM | | | | |

Conclusion: the compounds disclosed herein have obvious inhibitory activity on the proliferation of NCI-N87, BT-474, OE21 and OE19 cells.

**Assay Example 3: In Vivo Pharmacodynamics Study in BALB/c Nude Mouse Model with Subcutaneous Xenograft Tumor of Human Gastric Cancer NCI-N87 Cells**

Purpose of the assay: to evaluate the in vivo efficacy of compounds to be tested on subcutaneous xenograft tumor of human gastric cancer NCI-N87 cells in a BALB/c nude mouse model.

Animals of the assay: female BALB/c nude mice, 6-8 weeks old, weighted 18-22 grams; supplier: shanghai sippr bk laboratory animals Ltd.

Method and steps of the assay:

3.1 cell culture

Human gastric cancer NCI-N87 cells were cultured in monolayer in vitro. The culture conditions were RPMI-1640 medium plus 10% fetal bovine serum, 100 U/mL penicillin, 100 μg/mL streptomycin and 2 mM glutamine, 37 °C, 5 % $CO_2$. The passaging was performed by the conventional digestion with trypsin-EDTA twice a week. When the cell saturation was 80%-90%, the cells were collected, counted and inoculated.

3.2 Tumor cell inoculation (tumor inoculation)

0.2 mL ($10 \times 10^6$ cells) of NCI-N87 cells were inoculated subcutaneously on the right back of each nude mouse (PBS + Matrigel, 1:1). When the average tumor volume reached 145 $mm^3$, administration to each group was started.

3.3 Formulation of test compounds

The test compounds were formulated into a 0.3 mg/mL clear solution in the solvents of 10% NMP (N-methylpyrrolidone) + 10% ethylene glycol stearate + 80% water.

3.4 Tumor measurement and assay index

The assay index is to investigate whether tumor growth is inhibited, delayed or cured. Tumor diameter was measured twice a week with a vernier caliper. The calculation formula of tumor volume is: $V = 0.5a \times b^2$, wherein a and b represent the long and short diameters of tumor, respectively.

The antitumor efficacy of the compounds were evaluated by TGI (%) or relative tumor proliferation rate T/C (%). TGI (%) reflects the tumor growth inhibition rate. TGI (%) is calculated as follows: TGI (%) = [1-(average tumor volume at the end of administration for a certain treatment group - average tumor volume at the beginning of administration for this treatment group)/(average tumor volume at the end of treatment for a solvent control group - average tumor volume at the beginning of administration for this solvent control group)] × control group.

Relative tumor proliferation rate T/C (%): the calculation formula is as follows: T/C % = TRTV/CRTV (TRTV: RTV for the treatment group; CRTV: RTV for the negative control group). Relative tumor volume (RTV) is calculated based on tumor measurement results. The calculation formula is $RTV = V_t/V_0$, wherein $V_0$ is the average tumor volume measured at the beginning of administration (i.e., $d_0$) to each group, Vt is the average tumor volume at a certain measurement, and the data for TRTV and CRTV were taken on the same day.

The tumor weight was measured after the assay is over and the T/C weight percentage was calculated. T weight and C weight represent the tumor weight of the administration group and the vehicle control group, respectively.

3.5 Statistical analysis

Statistical analysis included the mean and standard error (SEM) of tumor volume at each time point in each group. The treatment group showed the best therapeutic effect at the end of the assay, i.e., on the twenty-first day after administration. Therefore, based on this data, statistical analysis was performed to assess the difference between the groups. The comparison between two groups was analyzed by T-test, and the comparison among three or more groups was analyzed by one-way ANOVA. If the F value shows a significant difference, a test should be carried out by Games-Howell method. If the F value does not show a significant difference, an analysis should be carried out by Dunnet (2-sided) method. All data analysis was carried out with SPSS 17.0. $p<0.05$ was considered a significant difference.

3.6 Assay results

The body weight of test animals was used as a reference index for indirect determination of drug toxicity. In this model, all administration groups (except the Poziotinib group) did not show significant weight loss. In the Poziotinib group, on the 7th day after administration, 2 mice showed more than 20% of body weight loss, 3 mice showed more than 15% of body weight loss, and 1 mouse showed more than 10% of body weight loss. On the 8th day after administration, 2 mice were euthanized, and the remaining mice gradually recovered from lowering the dose of administration, and no morbidity or death was observed. The results of drug efficacy were shown in Table 3.

3.7 Conclusion of assay and discussion

Compared with the vehicle group, the control group Poziotinib and Example 1, Example 7 and Example 8 disclosed herein all exhibited excellent tumor growth inhibitory effects, in which TGI was 115.6%, 103.98%, 92.63% and 119.71%, respectively. Moreover, the safety of Example 1, Example 7 and Example 8 disclosed herein on the weight index of model animals is significantly better than that of the control compound Poziotinib.

**Table 3: evaluation of the anti-tumor efficacy of compounds disclosed herein on human gastric cancer NCI-N87 xenograft model**

| (calculated based on tumor volume on day 21 after administration) | | | | |
|---|---|---|---|---|
| Group | Tumor volume (mm3)[a] (Day 21) | T/C[b](%) | TGI[b](%) | *P value*[c] |
| Vehicle group | 727±120 | -- | -- | -- |
| Example 1 (3 mg/kg) | 122±11 | 16.62 | 103.98 | *0.050* |
| Example 7 (3 mg/kg) | 188±9 | 26.10 | 92.63 | *0.079* |
| Example 8<br>3 mg/kg (D0-7)+1 mg/kg (D8-21) | 30±7 | 4.19 | 119.71 | *0.028* |
| Poziotinib<br>3 mg/kg (D0-7)+1 mg/kg (D8-21) | 54±12 | 7.55 | 115.60 | *0.032* |

Note:
"--" means no calculation was required.
a. Mean ± SEM.
b. Tumor growth inhibition was calculated by T/C and TGI (TGI (%) = $[1-(T_{21}-T_0)/(V_{21}-V_0)]\times100$).
c. The p value was based on tumor volume.

**Assay Example 4: In Vivo Pharmacodynamics Study in BALB/c Nude Mouse Model with Subcutaneous Xenograft Tumor of Human Gastric Cancer NCI-N87 Cells**

Purpose of the assay: to evaluate the in vivo efficacy of test compounds disclosed herein on subcutaneous xenograft tumor of human gastric cancer NCI-N87 cells in a BALB/c nude mouse model.

Animals of the assay: female BALB/c nude mice, 6-8 weeks old, weighted 18-22 grams; supplier: Shanghai Ling Chang Biotechnology Co., Ltd.

Method and steps of the assay:

4.1 cell culture

Human gastric cancer NCI-N87 cells were cultured in monolayer in vitro. The culture conditions were RPMI-1640 medium plus 10% fetal bovine serum, 100 U/mL penicillin, 100 U/mL streptomycin and 2 mM glutamine, 37 °C, 5 % $CO_2$. The passaging was performed by conventional digestion with trypsin-EDTA twice a week. When the cell saturation was 80%-90%, the cells were collected, counted and inoculated.

4.2 Tumor cell inoculation (tumor inoculation)

0.2 mL ($10 \times 10^6$) of NCI-N87 cells (PBS + Matrigel, 1:1) were inoculated subcutaneously on the right back of each mouse. When the average tumor volume reached 158 $mm^3$, administration to each group was started.

4.3 Formulation of test compounds

The test compounds were formulated into a 0.04 mg/mL and 0.08 mg/mL clear solution in and the solvents of 10% NMP (N-methylpyrrolidone) + 10% ethylene glycol stearate + 80% water.

4.4 Tumor measurement and assay index

The assay index is to investigate whether tumor growth is inhibited, delayed or cured. Tumor diameter was measured twice a week with a vernier caliper. The calculation formula of tumor volume is: $V = 0.5a \times b^2$, wherein a and b represent the long and short diameters of tumor, respectively.

The antitumor efficacy of the compounds were evaluated by TGI (%) or tumor proliferation rate T/C (%). TGI (%) reflects the tumor growth inhibition rate. TGI (%) is calculated as follows: TGI (%) = [1-(average tumor volume at the end of administration for a certain treatment group - average tumor volume at the beginning of administration for this treatment group)/(average tumor volume at the end of treatment for a solvent control group - average tumor volume at the beginning of administration for this solvent control group)] × 100%.

Tumor proliferation rate T/C (%): the calculation formula is as follows: T/C (%) = average tumor volume at the end of administration for a certain treatment group/average tumor volume at the end of treatment for the solvent control group × 100%.

4.5 Statistical analysis

Statistical analysis included the mean and standard error (SEM) of tumor volume at each time point in each group (See Table 5-1 for specific data). The treatment group showed the best therapeutic effect at the end of the assay, i.e., on the twenty-eight day after. Therefore, based on this data, statistical analysis was performed to assess the difference between the groups. The comparison between two groups was analyzed by T-test, and the comparison among three or more groups was analyzed by one-way ANOVA. After testing, F value showed significant difference, and thus a test was carried out by Games-Howell method. All data analysis was carried out with SPSS 17.0. $p<0.05$ was considered a significant difference. 4.6 Assay results

4.6.1 Mortality, morbidity and body weight changes

The body weight of test animals was used as a reference index for indirect determination of drug toxicity. In this model, the body weight of mice in the treatment group has a downward trend, and there was no other morbidity or death.

4.6.2 Evaluation index of antitumor efficacy

**Table 4: evaluation of anti-tumor efficacy of compounds disclosed herein in subcutaneous xenograft tumor model of human gastric cancer NCI-N87 cells**

(calculated based on the tumor volume on the 28th day after administration)

| Group | Dose | Tumor volume (mm3)a (Day 28) | T/C[b](%) | TGI[b](%) | *P value*[c] |
|---|---|---|---|---|---|
| Vehicle group | -- | 626 ± 60 | -- | -- | -- |
| Poziotinib | 0.8 mg/kg | 28 ± 8 | 4.47 | 127.78 | *0.002* |
| Poziotinib | 0.4 mg/kg | 84 ± 9 | 13.42 | 115.81 | *0.003* |
| Example 38 | 0.8 mg/kg | 15 ± 3 | 2.40 | 130.56 | *0.002* |
| Example 38 | 0.4 mg/kg | 47 ± 13 | 7.51 | 123.72 | *0.002* |

Note:
a. Mean + SEM.
b. Tumor growth inhibition was calculated by T/C and TGI (TGI (%) = [1-(T28-T0)/(V28-V0)] ×100).
c. The p value was calculated based on tumor volume.

4.7 Conclusion of assay and discussion

In the assay, in vivo efficacy of compounds disclosed herein in subcutaneous xenograft tumor model of human gastric cancer NCI-N87 cells was evaluated. Compared with the vehicle control group, example 38@0.8 mg/kg of the treatment group was comparable to the reference compound Poziotinib@0.8 mg/kg regarding the drug efficacy, and both exhibited significant tumor inhibiting effects; in the low-dose group, example 38 was comparable to or better than the reference compound Poziotinib regarding the drug efficacy.

**Assay Example 5: In Vivo Pharmacodynamics Study in BALB/c Nude Mouse Model with Subcutaneous Xenograft Tumor of Human Gastric Cancer NCI-N87 Cells**

Purpose of the assay: to evaluate the in vivo efficacy of test compounds disclosed herein on subcutaneous xenograft tumor of human gastric cancer NCI-N87 cells in a BALB/c nude mouse model.

Animals of the assay: female BALB/c nude mice, 6-8 weeks old, weighing 18-22 grams; supplier: shanghai sippr bk laboratory animals Ltd.

Method and steps of the assay:

5.1 cell culture

Human gastric cancer NCI-N87 cells were cultured in monolayer in vitro. The culture conditions were RPMI-1640 medium plus 10% fetal bovine serum, 100 U/mL penicillin, 100 μg/mL streptomycin, 37 °C, 5 % $CO_2$. The passaging was performed by conventional digestion with trypsin-EDTA twice a week. When the cell saturation was 80%-90%, the cells were collected, counted and inoculated.

5.2 Tumor cell inoculation (tumor inoculation)

0.2 mL ($10 \times 10^6$) of NCI-N87 cells (PBS + Matrigel, 1:1) were inoculated subcutaneously on the right back of each mouse. When the average tumor volume reached 156 $mm^3$, administration to each group was started.

5.3 Formulation of test compounds

The test compounds were formulated into a 0.05 mg/mL clear solution in the solvents of 10% NMP (N-methylpyrrolidone) + 10% ethylene glycol stearate + 80% water. 5.4 Tumor measurement and assay index

The assay index is to investigate whether tumor growth is inhibited, delayed or cured. Tumor diameter was measured twice a week with a vernier caliper. The calculation formula of tumor volume is: $V = 0.5a \times b^2$, wherein a and b represent the long and short diameters of tumor, respectively.

The antitumor efficacy of the compounds were evaluated by TGI (%) or tumor proliferation rate T/C (%). TGI (%) reflects the tumor growth inhibition rate. TGI (%) is calculated as follows: TGI (%) = [1-(average tumor volume at

the end of administration for a certain treatment group - average tumor volume at the beginning of administration for this treatment group)/(average tumor volume at the end of treatment for a solvent control group - average tumor volume at the beginning of administration for this solvent control group)] × 100%.

Tumor proliferation rate T/C (%): the calculation formula is as follows: T/C (%) = average tumor volume at the end of administration for a certain treatment group/average tumor volume at the end of treatment for the solvent control group × 100%.

5.5 Statistical analysis

Statistical analysis included the mean and standard error (SEM) of tumor volume at each time point in each group (See Table 5-1 for specific data). The treatment group showed the best therapeutic effect on at the end of the assay, i.e., the twenty-eight day after administration. Therefore, based on this data, statistical analysis was performed to assess the difference between the groups. The comparison between two groups was analyzed by T-test, and the comparison among three or more groups was analyzed by one-way ANOVA. After testing, F value showed significant difference, and thus a test was carried out by Games-Howell method. All data analysis was carried out with SPSS 17.0. $p<0.05$ was considered a significant difference.

5.6 Assay results

5.6.1 Mortality, morbidity and body weight changes

The body weight of test animals was used as a reference index for indirect determination of drug toxicity. In this model, the body weight of mice in the treatment group has a downward trend, and there was no other morbidity or death.

4.6.2 Evaluation index of antitumor efficacy

**Table 5: evaluation of anti-tumor efficacy of compounds disclosed herein in subcutaneous xenograft tumor model of human gastric cancer NCI-N87 cells**

| (calculated based on the tumor volume on the 21th day after administration) | | | | | |
|---|---|---|---|---|---|
| Group | Dose | Tumor volume ($mm^3$)[a] (Day 21) | T/C[b] (%) | TGI[b](%) | *P value*[c] |
| Vehicle group | -- | 631 ± 107 | -- | -- | -- |
| Example 38 | 0.5 mg/kg | 63 ± 10 | 10.69 | 119.41 | *0.007* |
| Control compound 2 | 0.5 mg/kg | 541 ± 88 | 89.35 | 18.83 | *0.796* |

Note:
a. Mean + SEM.
b. Tumor growth inhibition was calculated by T/C and TGI (TGI (%) = [1-(T21-T0)/(V21-V0)] ×100).
c. The p value was calculated based on tumor volume.

4.7 Conclusion of assay and discussion

In the assay, in vivo efficacy of the compounds disclosed herein and control compound 2 in subcutaneous xenograft tumor model of human gastric cancer NCI-N87 cells was evaluated. Compared with the solvent control group, Example 38@0.5 mg/kg of the treatment group exhibited significant tumor inhibiting effect, in which the tumor inhibiting rate was T/C = 10.69 %, TGI = 119.41 %, and p = 0.007; at the same dose, the efficacy of control compound 2 was not obvious, in which T/C = 89.35 %, and TGI = 18.83 %; the efficacy of the compounds disclosed herein was significantly better than that of the control compound 2.

**Assay Example 6: In vivo pharmacodynamics study in BALB/c nude mouse model with subcutaneous xenograft tumor of human esophageal carcinoma OE21 cells**

Purpose of the assay: to evaluate the in vivo efficacy of the compounds to be tested on subcutaneous xenograft tumor of human esophageal carcinoma OE21 cells in a BALB/c nude mouse model.

Animals of the assay: female BALB/c nude mice, 6-8 weeks old, weighing 17-23 grams; supplier: shanghai sippr bk laboratory animals Ltd.

Method and steps of the assay:

6.1 cell culture

Human esophageal carcinoma OE21 cells were cultured in monolayer in vitro. The culture conditions were RPMI-1640 medium plus 10% fetal bovine serum, 100 U/mL penicillin, 100 U/mL streptomycin and 2 mM glutamine, 37 °C, 5 % $CO_2$. The passaging was performed by conventional digestion with trypsin-EDTA twice a week. When the cell saturation was 80%-90%, the cells were collected, counted and inoculated.

6.2 Tumor cell inoculation (tumor inoculation)

0.1 ml (20.1) OE21 cells (PBS) were inoculated subcutaneously on the right back of each mouse. When the average tumor volume reached 112 $mm^3$, administration to each group was started.

6.3 Formulation of test compounds

The test compound was formulated into a 0.15 mg/mL to 0.3 mg/mL clear or suspension solution in the solvents of 10% NMP + 10% ethylene glycol stearate + 80% water. 6.4 Tumor measurement and assay index

The assay index is to investigate whether tumor growth is inhibited, delayed or cured. Tumor diameter was measured twice a week with vernier caliper. The calculation formula of tumor volume is: $V = 0.5a \times b^2$, wherein a and b represent the long and short diameters of tumor, respectively.

The antitumor efficacy of the compounds were evaluated by TGI (%) or relative tumor proliferation rate T/C (%). TGI (%) reflects the tumor growth inhibition rate. TGI (%) is calculated as follows: TGI (%) = [(1-(average tumor volume at the end of administration for a certain treatment group - average tumor volume at the beginning of administration for this treatment group))/(average tumor volume at the end of treatment for a solvent control group - average tumor volume at the beginning of administration for this solvent control group)] × 100%.

Relative tumor proliferation rate T/C (%): the calculation formula is as follows: T/C (%) = average tumor volume at the end of administration for a certain treatment group/average tumor volume at the end of treatment for the solvent control group × 100%. 6.5 Statistical analysis

Statistical analysis included the mean and standard error (SEM) of tumor volume at each time point in each group. One-way ANOVA was used for comparison among three or more groups. If the F value showed significant difference, multiple comparison should be made after ANOVA analysis. SPSS 17.0 was used for all data analysis. $p<0.05$ is considered a significant difference.

6.6 Assay results

6.6.1 Mortality, morbidity and body weight changes

In this model, animals in the Poziotinib@2/1.5 mg/kg, Example 8/Example 38@2/0.5 mg/kg, and Example 38@2/1.5 mg/kg administration groups all showed varying degrees of body weight loss and dandruff; in the Poziotinib@2/1.5 mg/kg administration group, from the 6th and 7th day after the administration to each group, 1 mouse showed more than 15% of body weight loss and 3 mice showed more than 15% of body weight loss, and their administration was discontinued; moreover, mice in all groups had dark yellow urine, which suggested toxicity; starting from the 3rd day after administration, in the test group, Example 8@2 mg/kg was replaced with Example 38@0.5 mg/kg, the dose of Poziotinib@2 mg/kg was reduced to 1.5 mg/kg, and the dose of Example 38@2 mg/kg was reduced to 1.5 mg/kg. After the dose adjustment, the body weight of each group of animals recovered significantly (Figure 1).

6.6.2 Evaluation index of antitumor efficacy

**Table 6: evaluation of anti-tumor efficacy of compounds disclosed herein on OE21 xenograft tumor model (calculated based on tumor volume on day 21 after administration)**

| Group | Dose | Tumor volume (mm3)a (Day 21) | T/C[b] (%) | TGI[b] (%) | *P value*[c] |
|---|---|---|---|---|---|
| Vehicle group | | 948±124 | -- | -- | -- |
| Poziotinib | 2/1.5 mg/kg | 57±7 | 6.05 | 106.65 | *0.008* |
| Example 8/Example 38 | 2/0.5 mg/kg | 56±15 | 5.89 | 106.87 | *0.008* |
| Example 38 | 2/1.5 mg/kg | 55±13 | 5.82 | 106.65 | *0.008* |

Note:
a. Mean + SEM.
b. Tumor growth inhibition was calculated by T/C and TGI by referring to Assay Example 4.
c. The p value was calculated based on the tumor volume on the 21st day after administration using One-Way ANOVA statistical method by comparing with the vehicle group.

6.7 Conclusion of assay and discussion

In this assay, in vivo efficacy of compounds disclosed herein and the reference compound Poziotinib in the OE21 xenograft tumor model was evaluated. On the 21st day after the administration, the tumor volume of tumor-bearing mice in the solvent control group reached 948 $mm^3$. The test substance Poziotinib@2/1.5 mg/kg, Example 8/Example 38@2/0.5 mg/kg and Example 38@2/1.5 mg/kg group had a significant tumor inhibiting effect compared with the solvent control group. The tumor inhibition rates were: T/C = 6.05 %, TGI = 106.65 %, p = 0.008 (Poziotinib@2/1.5 mg/kg group); T/C = 5.89 %, TGI = 106.87 %, p = 0.008 (Example 8/Example 38@2/0.5 mg/kg group); T/C = 5.82 %, TGI = 106.65 %, p = 0.008 (Example 38@2/1.5 mg/kg group), respectively. The efficacy of low-dose 0.5 mpk of Example 38 was comparable to that of high-dose 1.5 mpk of reference compound Poziotnib and Example 38.

Example 38 has significant tumor inhibiting effect, and its efficacy is comparable to that of the reference compound Poziotinib. However, in the high-dose group of the reference compound, the body weight loss of mice was significant. Three mice showed more than 15% of body weight loss, and their administration was discontinued. Moreover, mice in all groups had dark yellow urine, which suggested toxicity. Example 38 had little effect on the body weight of mice, without discontinuing the administration and without the toxicity suggestion of dark yellow urine, suggesting that its tolerability was significantly better than that of the reference compound. Compared with the reference compound Poziotinib, Example 38 had lower effective dose, better tolerability, and superior safety window.

**Assay Example 7: assay to study pharmacokinetics in mice**

Purpose of the assay: this assay aims to investigate in vivo plasma pharmacokinetics of the example compounds disclosed herein and the reference compound in female BALB/c mice after the single intravenous injection and intragastric administration thereof.

Animals of the assay: female BALB/c nude mice, 7-9 weeks old, weighted 17-23 grams; supplier: shanghai sippr bk laboratory animals Ltd.

Sample collection: at each time point, a 0.03 mL blood sample was collected from the saphenous vein of the test animal by puncture, and the actual blood collection time was recorded. All blood samples were added into commercial EDTA-K2 anticoagulation tubes with a specification of 1.5 mL (the supplier was Jiangsu KANGJIAN Medical Apparatus Co., Ltd.). Within half an hour after the blood sample was collected, the blood sample was centrifuged at 3000g at 4 °C for 10 minutes, and the supernatant plasma was drawn, quickly placed in dry ice, and stored in a refrigerator at -80 °C for LC-MS/MS analysis.

Data analysis: Plasma concentrations were processed using a non-compartmental model of WinNonlin™Version6.3 (Pharsight, MountainView, CA) pharmacokinetic software, and pharmacokinetic parameters $C_{max}$, $T_{max}$, $T_{1/2}$, and $AUC_{0-last}$ were calculated using a linear log trapezoid method.

**Table 7: Comparison of PK results of the compounds disclosed herein and the reference compound**

| | $C_{max}$ (nM) | $T_{max}$ (h) | $T_{1/2}$ (h) | $AUC_{0-last}$(nM.h) |
|---|---|---|---|---|
| Example 38 | 10600 | 2.0 | 3.2 | 122154 |

(continued)

| | $C_{max}$ (nM) | $T_{max}$ (h) | $T_{1/2}$ (h) | $AUC_{0-last}$(nM.h) |
|---|---|---|---|---|
| Poziotinib | 17267 | 0.25 | 2.3 | 97870 |
| Control compound 2 | 15300 | 2.0 | 1.5 | 82021 |
| $C_{max}$: peak concentration; $T_{max}$: time to reach peak concentration; $T_{1/2}$: the time required to clear half of the compound; $AUC_{0-last}$: integrated area of concentration in the time from 0 to the last sampling. | | | | |

The results of pharmacokinetic studies in mice showed that the compounds disclosed herein had a longer half-life than that of the reference compound, and had significantly better oral plasma exposure than that of the reference compound Poziotinib and the control compound 2.

**Assay Example 8: evaluation of inhibition of CYP enzyme activity**

The results of the compounds disclosed herein and the reference compound Poziotinib in the assay of CYP enzyme activity in human liver microsome were shown in the following table (Table 8).

**Table 8: comparison of results of the compounds disclosed herein and the reference compound on the inhibition of CYP enzyme**

| | CYP1A2 | CYP2C9 | CYP2C19 | CYP2D6 | CYP3A4 |
|---|---|---|---|---|---|
| Example 38 | >50 | 16 | 11 | 6 | >50 |
| Poziotinib | >50 | 8 | 12 | 1 | >50 |

The results of inhibition of CYP activity of human liver microsome by the compounds disclosed herein showed that example 38 and Poziotinib had no inhibitory activity on CYP1A2 and CYP3A4. The inhibitory activity of example 38 on CYP2C19 was comparable to that of the reference compound Poziotinib, and the activity of example 38 on CYP2C9 and CYP2D6 was improved, which was 2 times and 6 times better than that of the reference compound, respectively. In a comprehensive comparison, example 38 had improved activity on CYP and was superior to that of the reference compound, so the risk of drug-drug interaction was lower.

## Claims

1. A compound of Formula (I), a stereoisomer thereof or a pharmaceutically acceptable salt thereof,

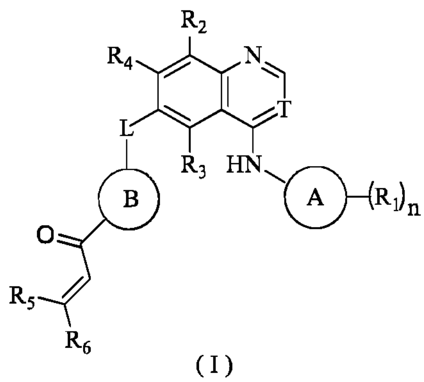

(I)

wherein,

T is selected from the group consisting of N and CR';
each $R_1$ is independently selected from the group consisting of H, F, Cl, Br, I, OH, $NH_2$, CN, $C_{1-3}$ alkyl and $C_{1-3}$ alkoxy; wherein the $C_{1-3}$ alkyl or $C_{1-3}$ alkoxy is optionally substituted with 1, 2 or 3 substituents independently selected from the group consisting of F, Cl, Br, I, OH, $NH_2$ and CN;

$R_2$ and $R_3$ are each independently selected from the group consisting of H, F, Cl, Br, I, OH, $NH_2$ and $C_{1-3}$ alkyl, wherein the $C_{1-3}$ alkyl is optionally substituted with 1, 2 or 3 substituents independently selected from the group consisting of F, Cl, Br and I;

$R_4$ is selected from the group consisting of $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O-, wherein the $C_{1-6}$ alkyl, $C_{1-6}$ alkoxy, 3- to 7-membered heterocycloalkyl-O-, 3- to 7-membered heterocycloalkyl-$C_{1-6}$ alkyl-O-, $C_{3-6}$ cycloalkyl-O- and $C_{3-6}$ cycloalkyl-$C_{1-6}$ alkyl-O- are each independently optionally substituted with 1, 2, or 3 R;

$R_5$ and $R_6$ are each independently selected from the group consisting of H, $C_{1-3}$ alkyl and 3- to 7-membered heterocycloalkyl, wherein the $C_{1-3}$ alkyl and 3- to 7-membered heterocycloalkyl are optionally substituted with 1, 2, or 3 R;

L is selected from the group consisting of -O-, -$NR^a$-, -$CR^{b1}R^{b2}$-, -$CR^{b1}R^{b2}$-O-, and -$CR^{b1}R^{b2}$-NH-;

$R^a$ is H;

alternatively, $R_4$ is connected to $R^a$ to form a 5- to 7-membered heterocycloalkyl group optionally substituted with 1, 2, 3 or 4 R';

ring A is selected from the group consisting of phenyl and 5- to 10-membered heteroaryl;

ring B is selected from the group consisting of

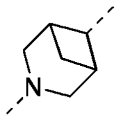 , 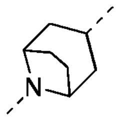 , 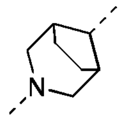 ,

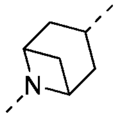 , 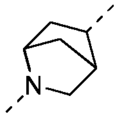 , 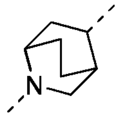 , 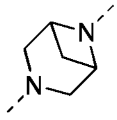 and 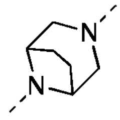 ;

n is selected from the group consisting of 1, 2, 3, 4 and 5;

each R is independently selected from the group consisting of F, Cl, Br, I, OH, $NH_2$, CN, $NR^{c1}R^{c2}$, $C_{1-3}$ alkyl, $C_{1-3}$ alkoxy, $C_{1-3}$ alkyl-NH-C(=O)-, $C_{1-3}$ alkylthio and 3- to 7-membered heterocycloalkyl, wherein the $C_{1-3}$ alkyl, $C_{1-3}$ alkoxy, $C_{1-3}$ alkyl-NH-C(=O)-, $C_{1-3}$ alkylthio and 3- to 7-membered heterocycloalkyl are optionally substituted with 1, 2, or 3 R';

R' is each independently selected from the group consisting of F, Cl, Br, I, OH, $NH_2$, CN and $C_{1-3}$ alkyl;

$R^{b1}$ and $R^{b2}$ are each independently selected from the group consisting of H, F, Cl, Br, I and $C_{1-3}$ alkyl;

$R^{c1}$ and $R^{c2}$ are each independently selected from the group consisting of H and $C_{1-3}$ alkyl;

the 5- to 7-membered heterocycloalkyl, 5- to 10-membered heteroaryl and 3- to 7-membered heterocycloalkyl each contains 1, 2, 3 or 4 heteroatoms or heteroatom groups independently selected from the group consisting of N, -O-, -S- and -NH-.

**2.** The compound according to claim 1, a stereoisomer thereof or a pharmaceutically acceptable salt thereof, wherein

each R is independently selected from the group consisting of F, Cl, Br, I, OH, $NH_2$, CN, 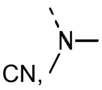 , Me, Et,

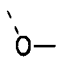 , 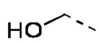 , 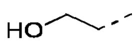 , 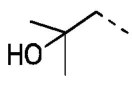 , 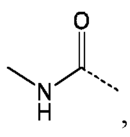 ,

piperidinyl, pyrrolidinyl, azetidinyl, morpholinyl and oxetanyl, wherein the Me, Et, 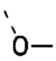, piperidinyl, pyrrolidinyl, azetidinyl, morpholinyl and oxetanyl are optionally substituted with 1, 2 or 3 substituents independently selected from the group consisting of F, Cl, Br, I, OH, $NH_2$ and $C_{1-3}$ alkyl;

preferably,

wherein each R is independently selected from the group consisting of F, Cl, Br, I, OH, $NH_2$, CN, 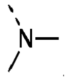, Me, Et,

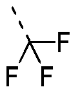, 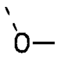, 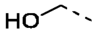, 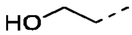, 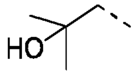, 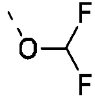, 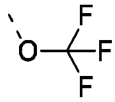,

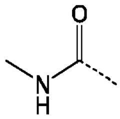, 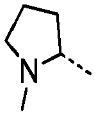, 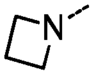, 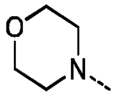 and 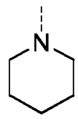.

3. The compound according to claim 1 or 2, a stereoisomer thereof or a pharmaceutically acceptable salt thereof, wherein each $R_1$ is independently selected from the group consisting of H, F, Cl, Br, I, OH, $NH_2$, CN, Me and 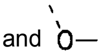, wherein the Me and 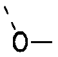 are optionally substituted with 1, 2 or 3 substituents independently selected from the group consisting of F, Cl, Br, I, OH, $NH_2$ and CN;
preferably,
wherein each $R_1$ is independently selected from the group consisting of H, F, Cl, Br, I, OH, $NH_2$, CN, Me, $CH_2F$, $CHF_2$, $CF_3$,

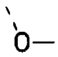 and 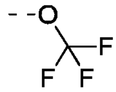.

4. The compound according to any one of claims 1 to 3, a stereoisomer thereof or a pharmaceutically acceptable salt thereof, wherein $R_2$ and $R_3$ are each independently selected from the group consisting of H, F, Cl, Br, I, OH, $NH_2$, Me and $CF_3$.

5. The compound according to any one of claims 1 to 4, a stereoisomer thereof or a pharmaceutically acceptable salt thereof, wherein $R_4$ is selected from the group consisting of

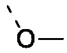, 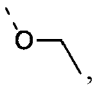, 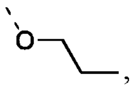,

piperidinyl-$C_{1-3}$ alkyl-O-, oxetanyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O-, wherein the

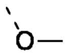, 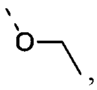, 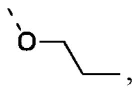,

piperidinyl-$C_{1-3}$ alkyl-O-, oxetanyl-O-, morpholinyl-$C_{1-3}$ alkyl-O-, azetidinyl-O-, tetrahydrofuranyl-O-, cyclopropyl-O-, pyrrolidinyl-$C_{1-3}$ alkyl-O- and azetidinyl-$C_{1-3}$ alkyl-O- are optionally substituted with 1, 2, or 3 R;
preferably,
wherein $R_4$ is selected from the group consisting of

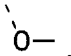, 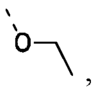, 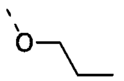,

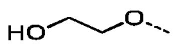, 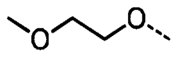, 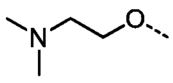, 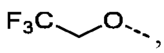, 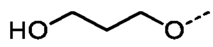, 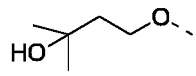,

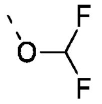, 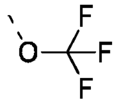, 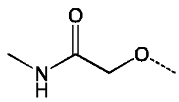, 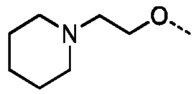, 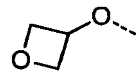, 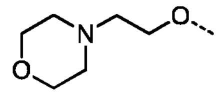,

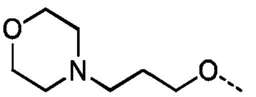, 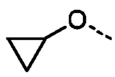, 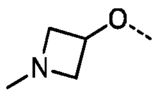, 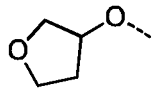, 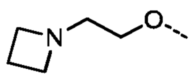

and

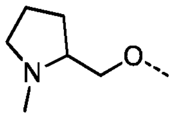.

6. The compound according to any one of claims 1 to 5, a stereoisomer thereof or a pharmaceutically acceptable salt thereof, wherein $R_5$ and $R_6$ are each independently selected from the group consisting of H, Me, Et and

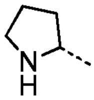,

wherein the Me, Et and

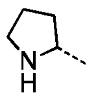

are optionally substituted with 1, 2, or 3 R;
preferably,
wherein $R_5$ and $R_6$ are each independently selected from the group consisting of H,

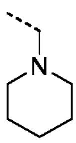, 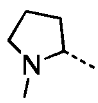 and 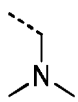.

7. The compound according to any one of claims 1 to 6, a stereoisomer thereof or a pharmaceutically acceptable salt thereof, wherein T is selected from the group consisting of N and C(CN).

8. The compound according to any one of claims 1 to 7, a stereoisomer thereof or a pharmaceutically acceptable salt thereof, wherein L is selected from the group consisting of -O-, -NH-, $-CH_2-$, $-CH_2-O-$ and $-CH_2-NH-$.

9. The compound according to any one of claims 1 to 8, a stereoisomer thereof or a pharmaceutically acceptable salt thereof, wherein the moiety

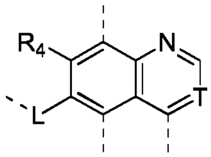

is selected from the group consisting of

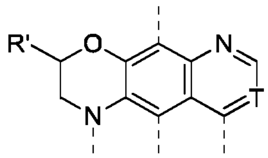 , 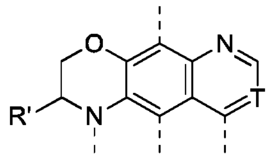 ,

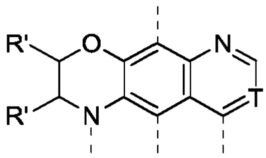 , 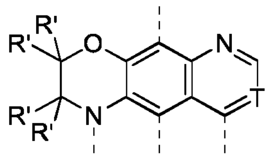 and 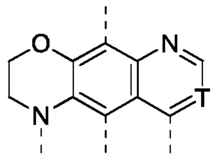 ;

preferably, wherein the moiety

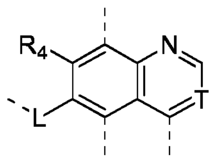 is 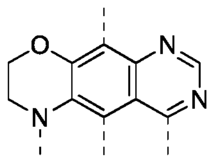 .

**10.** The compound according to any one of claims 1 to 9, a stereoisomer thereof or a pharmaceutically acceptable salt thereof, wherein the ring A is selected from the group consisting of phenyl, azaindenyl, benzo[*b*]thienyl, imidazo[1,2-*a*]pyridyl and benzo[*d*]isoxazolyl;
preferably,

wherein the moiety

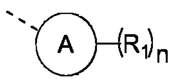

is selected from the group consisting of

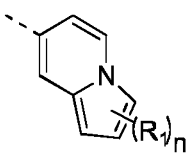 , 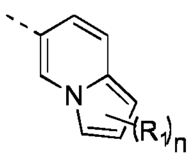 , 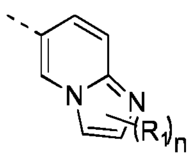 , 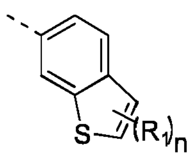 , 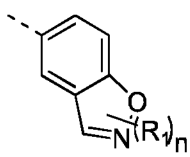

and

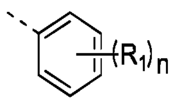 ;

preferably,
wherein the moiety

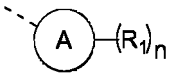

is selected from the group consisting of

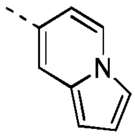,

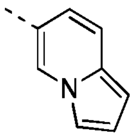, 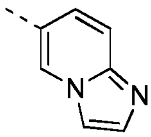, 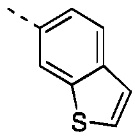, 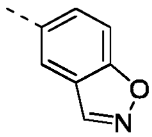, 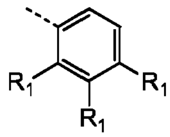, 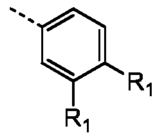

and

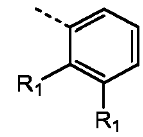;

preferably,
wherein the moiety

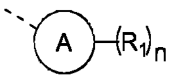

is selected from the group consisting of

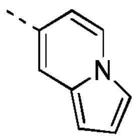,

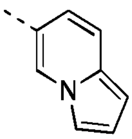, 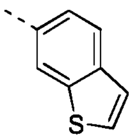, 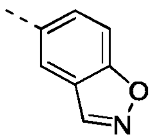, 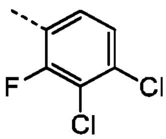, 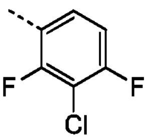, 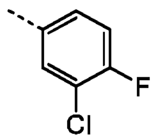, 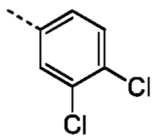,

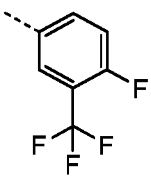, 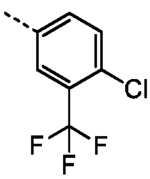, 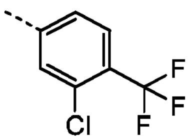, 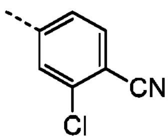, 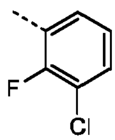 and 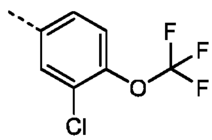.

**11.** The compound according to any one of claims 1 to 10, a stereoisomer thereof or a pharmaceutically acceptable

salt thereof, wherein the ring B is selected from the group consisting of

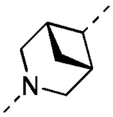 , 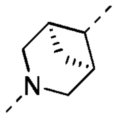 , 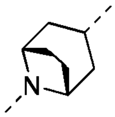 , 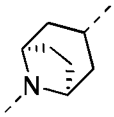 , 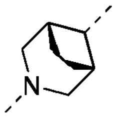 , 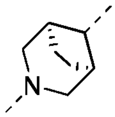 , 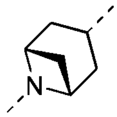 ,

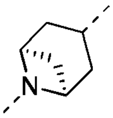 , 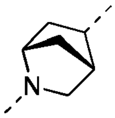 , 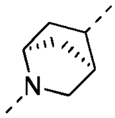 , 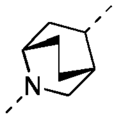 , 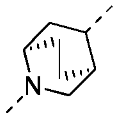 , 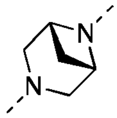 , 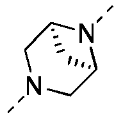 , 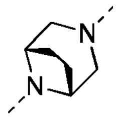 ,

and

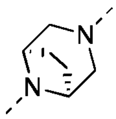 ;

preferably, wherein the moiety

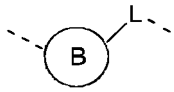

is selected from the group consisting of

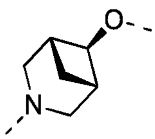 ,

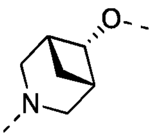 , 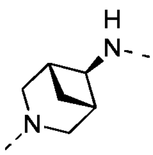 , 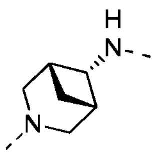 , 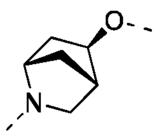 , 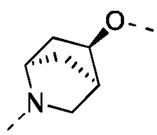 , 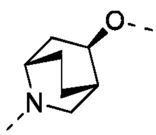 ,

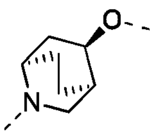 , 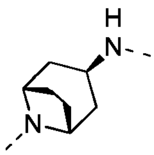 , 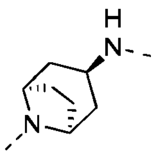 , 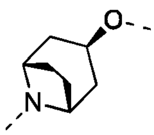 and 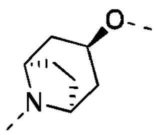 .

**12.** The compound according to any one of claims 1 to 11, a stereoisomer thereof or a pharmaceutically acceptable salt thereof, wherein the compound is selected from the group consisting of:

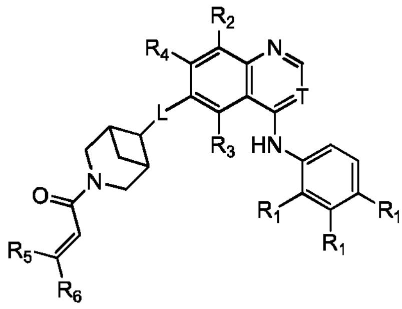

(II -1),

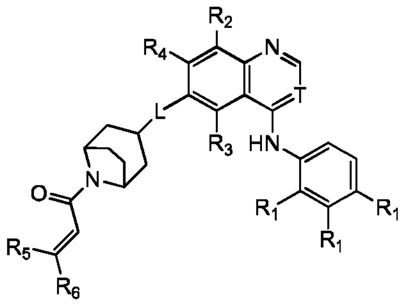

(II -2),

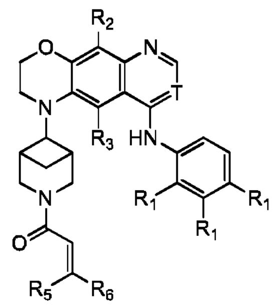

(II -3),

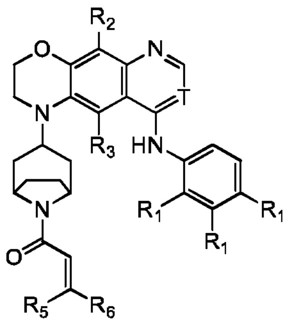

(II -4),

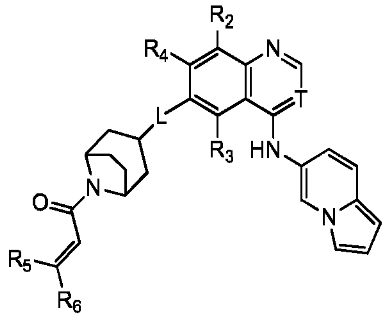

(II -5),

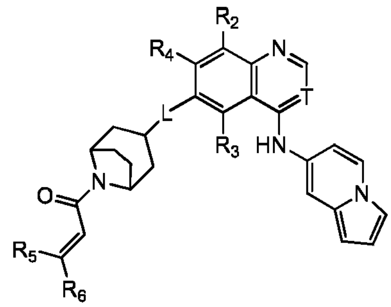

(II -6),

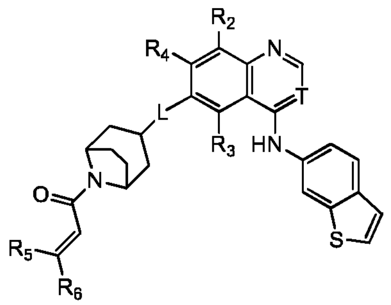

(II -7),

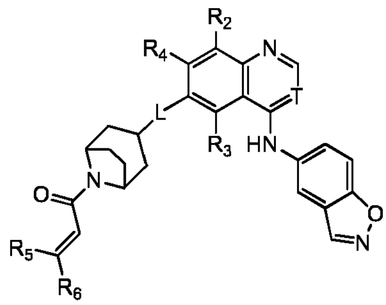

(II -8),

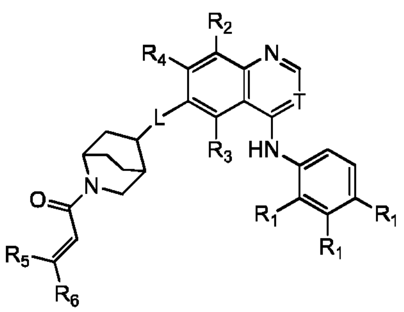

(II -9),

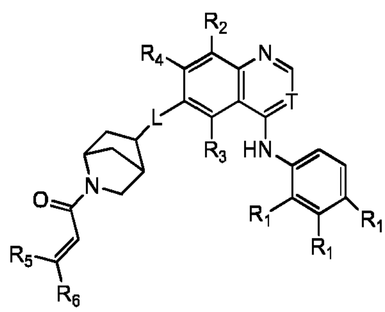

(II -10)

and

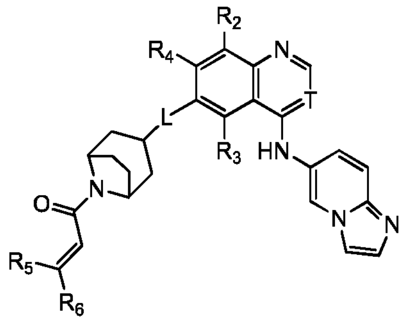

(II -11) ,

wherein T, L, $R_1$, $R_2$, $R_3$, $R_4$, $R_5$ and $R_6$ are as defined in claims 1 to 11; preferably, wherein the compound is selected from the group consisting of:

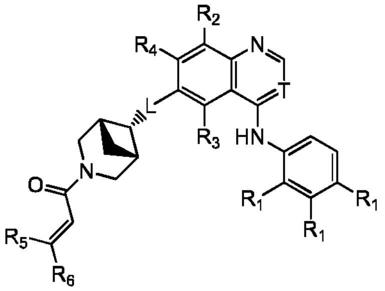

( III - 1) ,

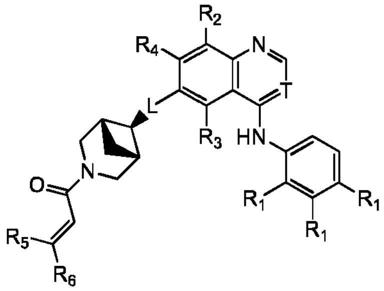

( III - 2) ,

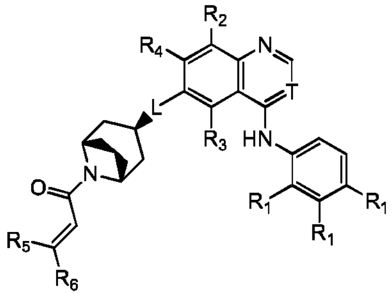

( III - 3) ,

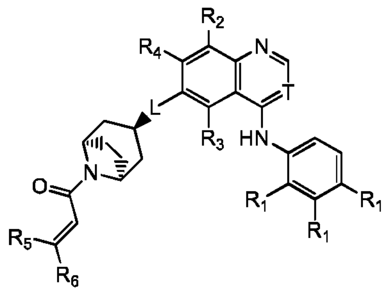

( III - 4) ,

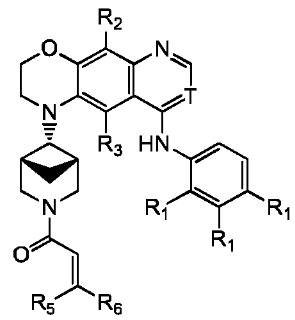

(III - 5) ,

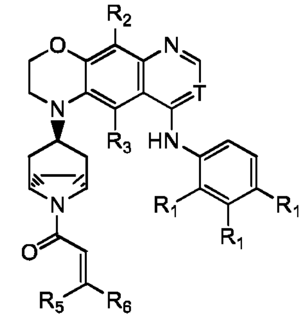

( III - 6) ,

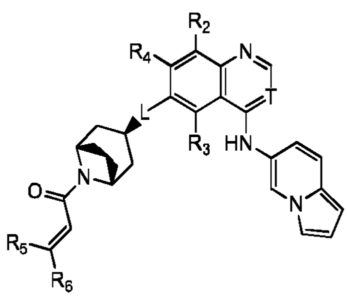

(III - 7) ,

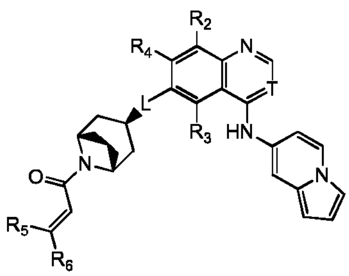

(III - 8) ,

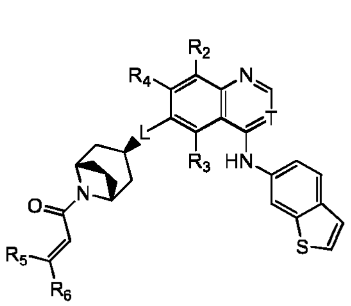

(III-9) ,

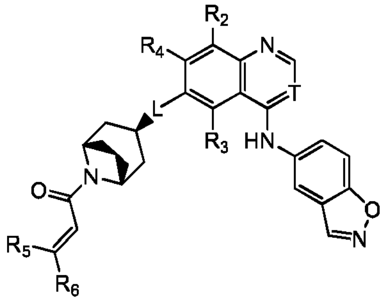

(III-10) ,

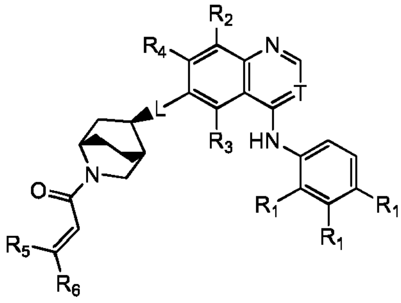

(III-11) ,

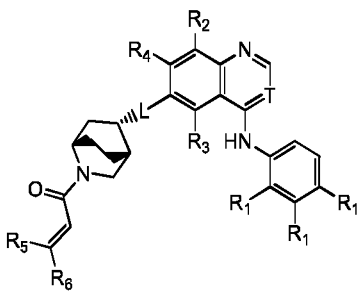

(III-12) ,

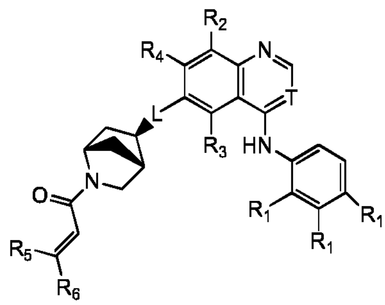

(III-13) ,

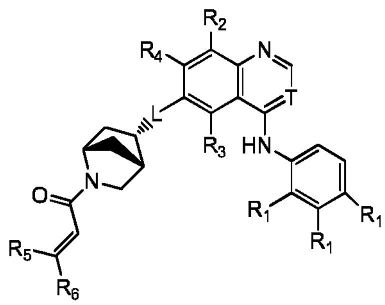

(III-14) ,

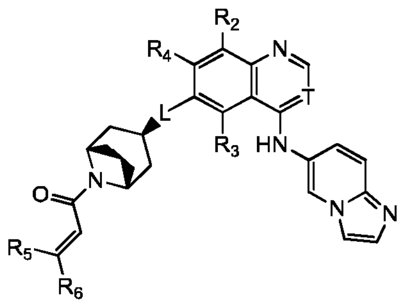

(III-15) , and

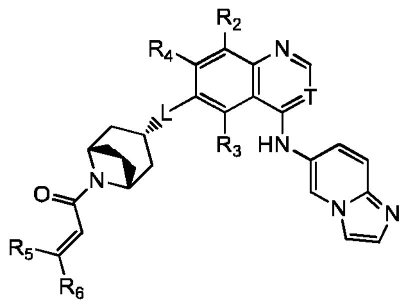

(III-16) ,

wherein T, L, $R_1$, $R_2$, $R_3$, $R_4$, $R_5$ and $R_6$ are as defined in claims 1 to 11.

**13.** The compound according to claim 1, a stereoisomer thereof or a pharmaceutically acceptable salt thereof, wherein the compound is selected from the group consisting of:

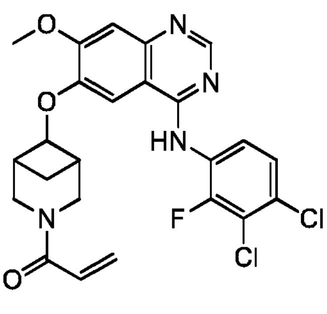

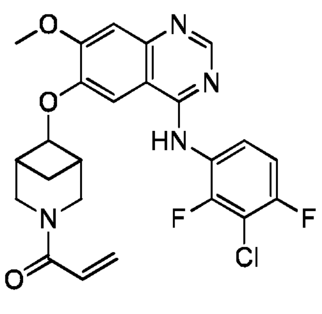

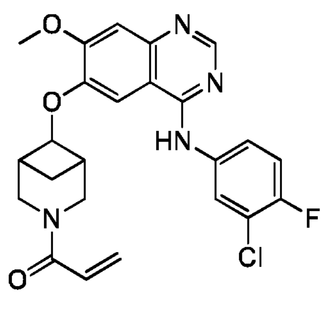

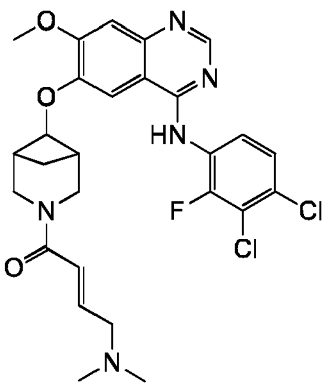

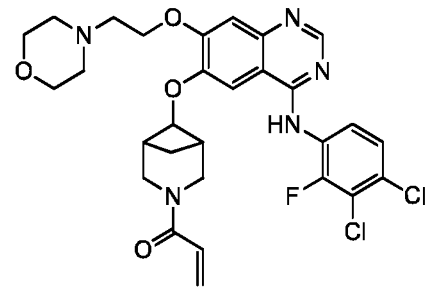

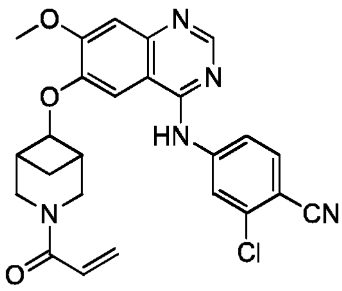

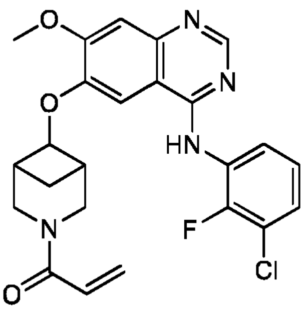

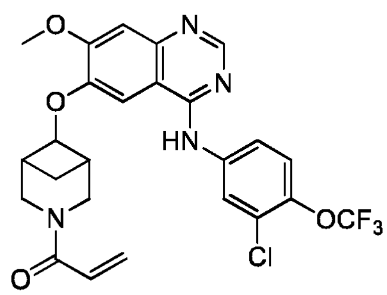

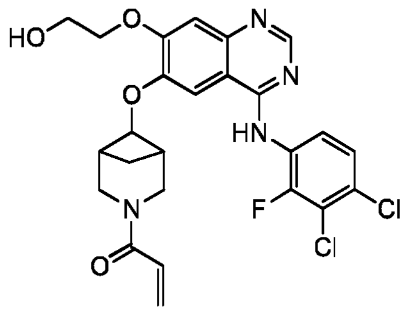

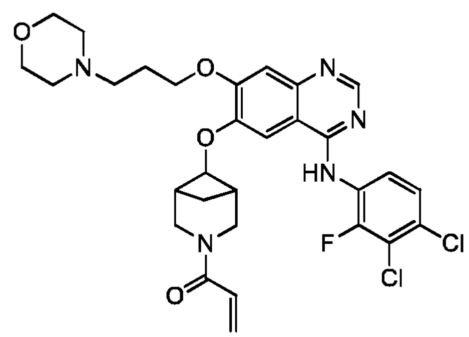

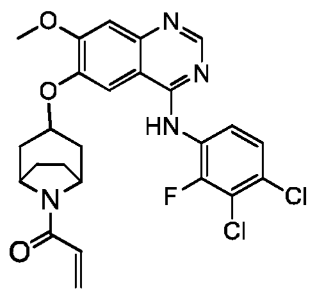

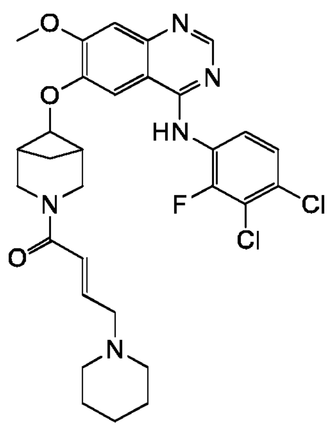

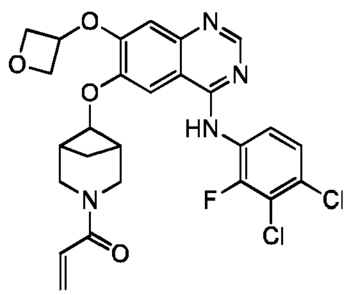

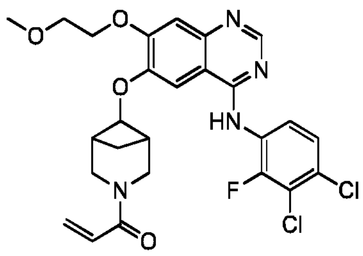

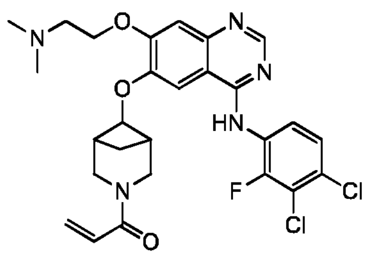

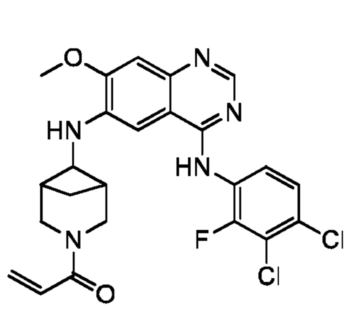
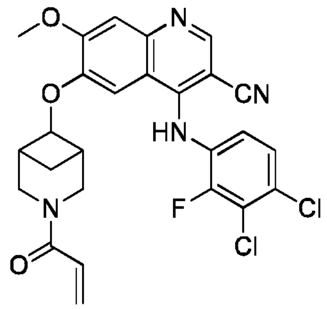
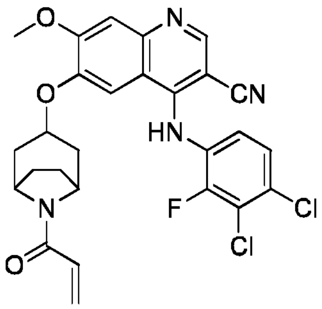

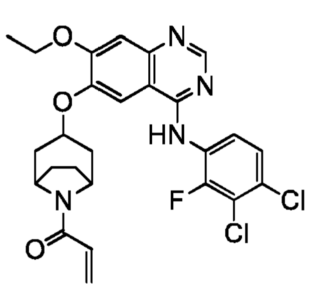
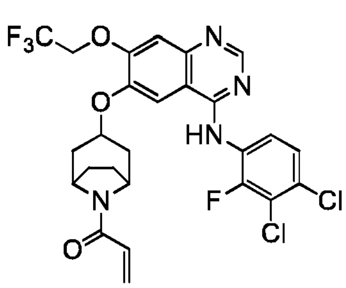
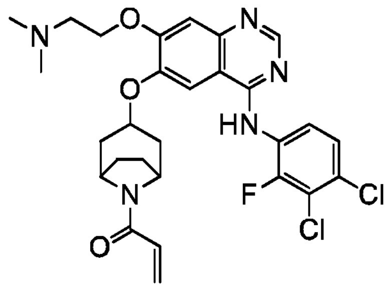

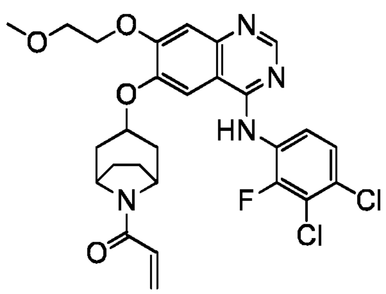
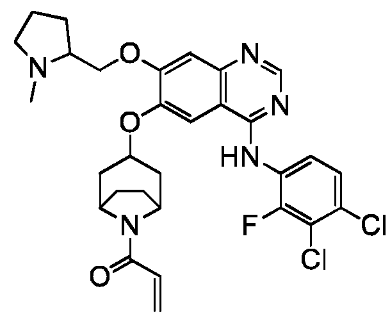
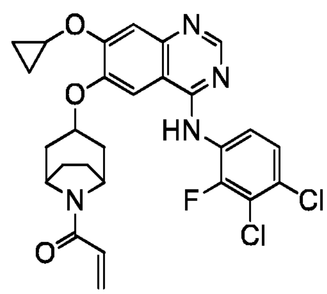

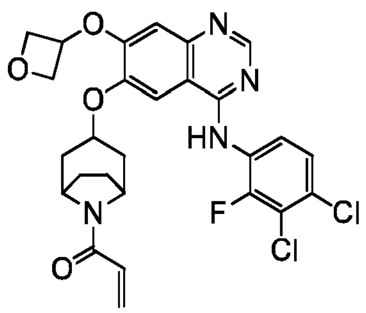
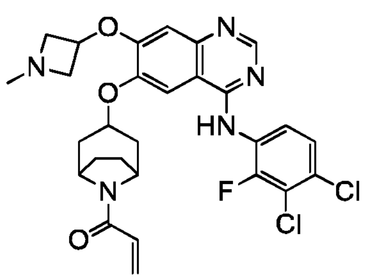
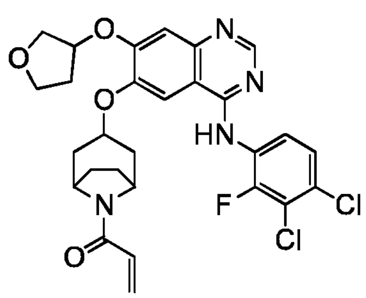

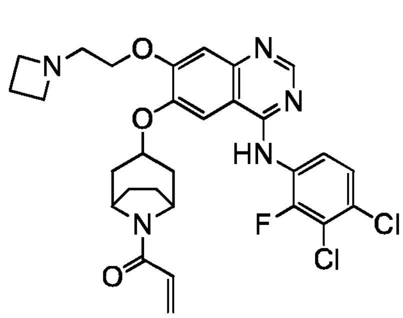
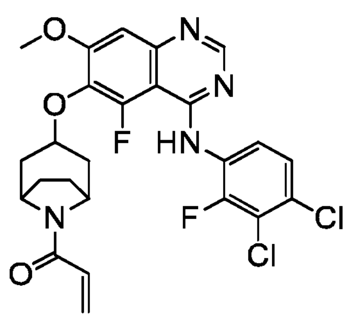
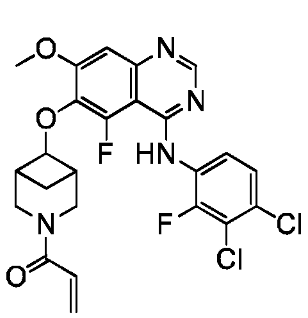

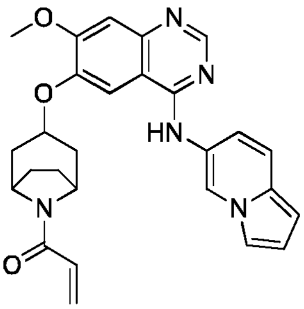
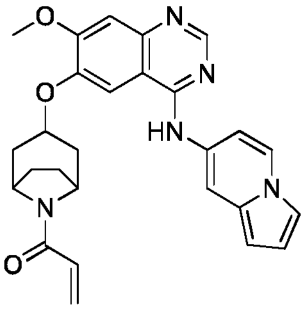
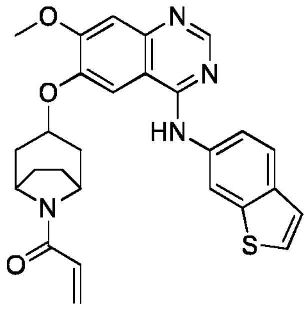

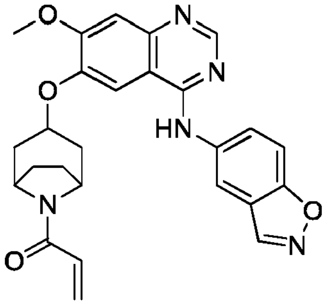
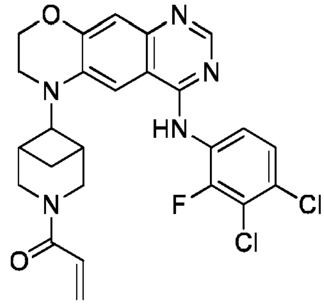

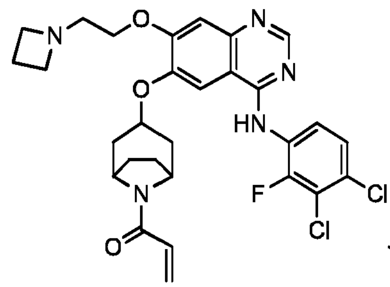
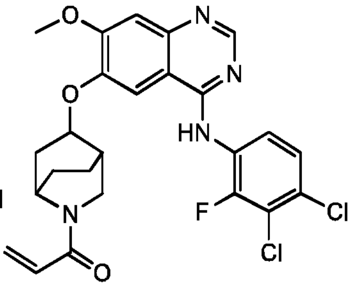
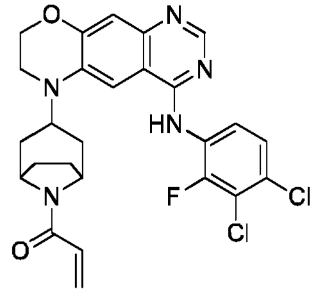

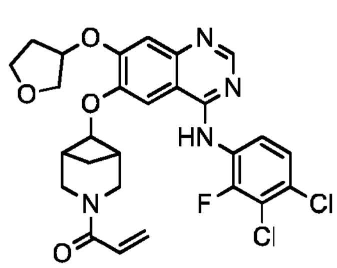
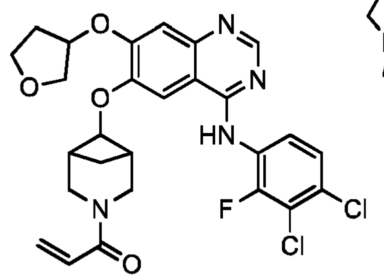
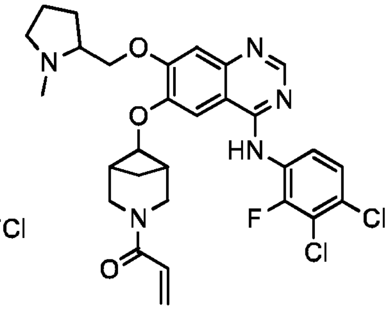

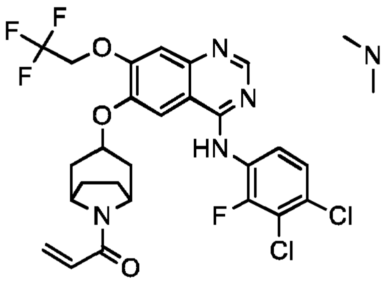
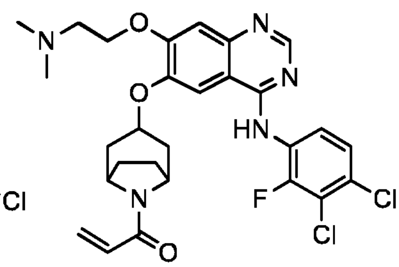
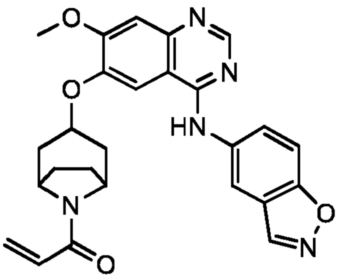

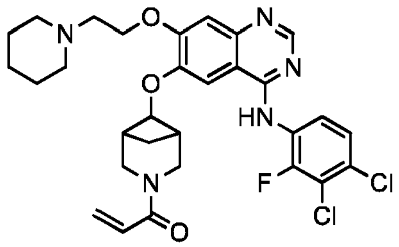
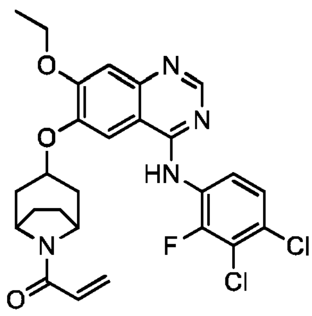
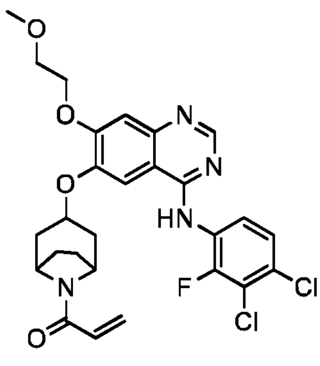

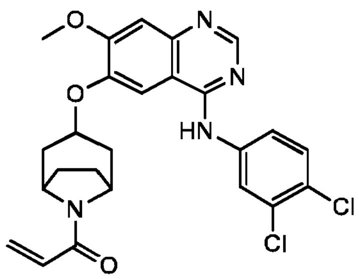
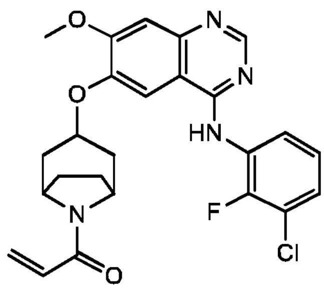
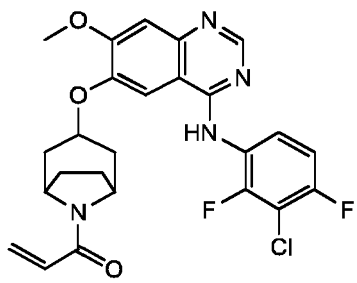

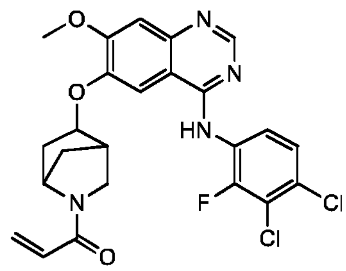
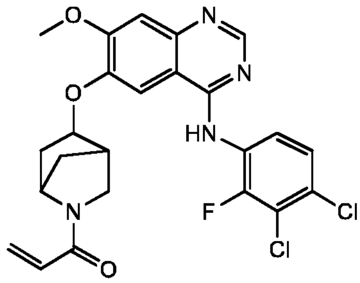
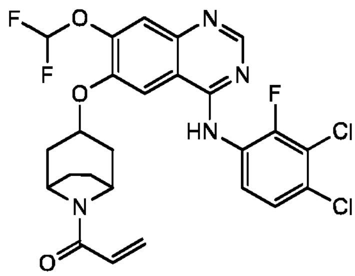

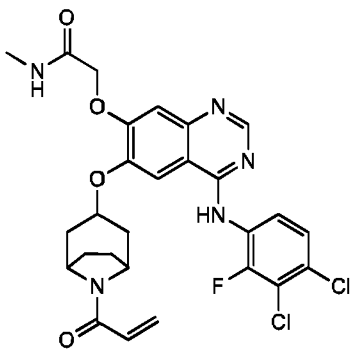
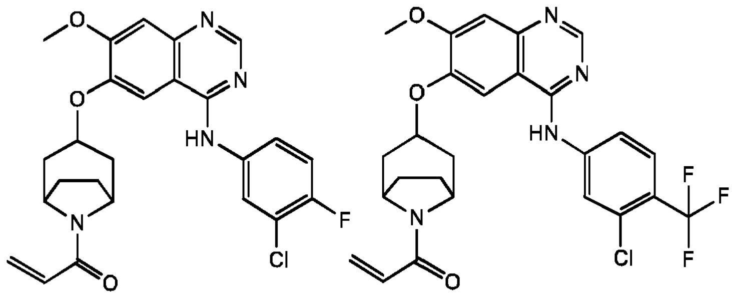

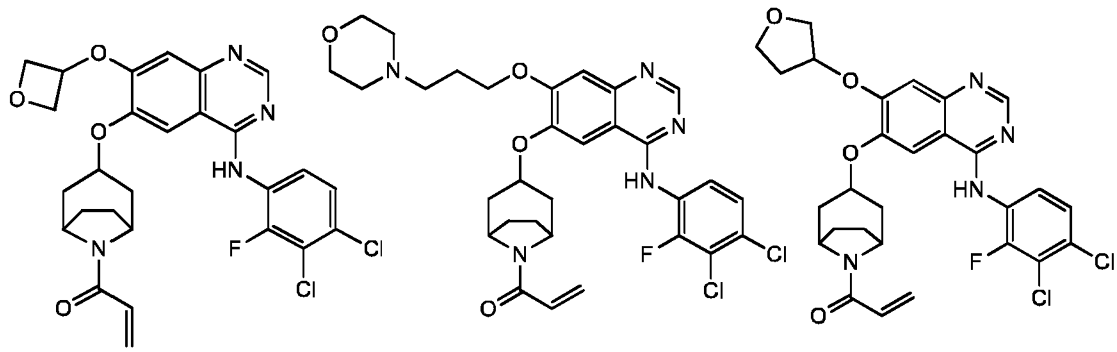

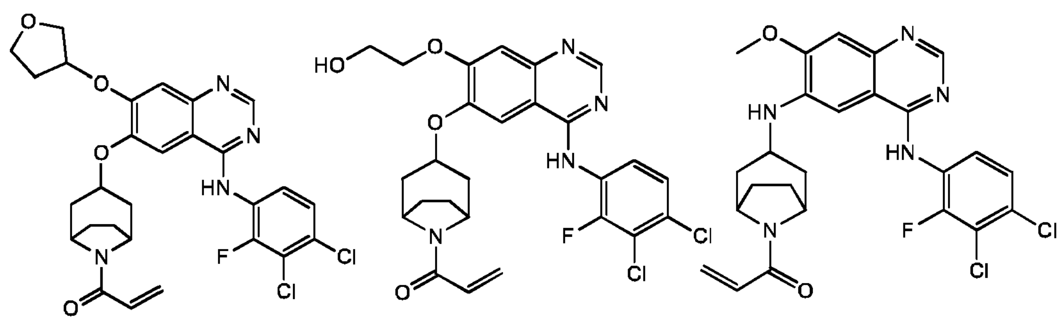

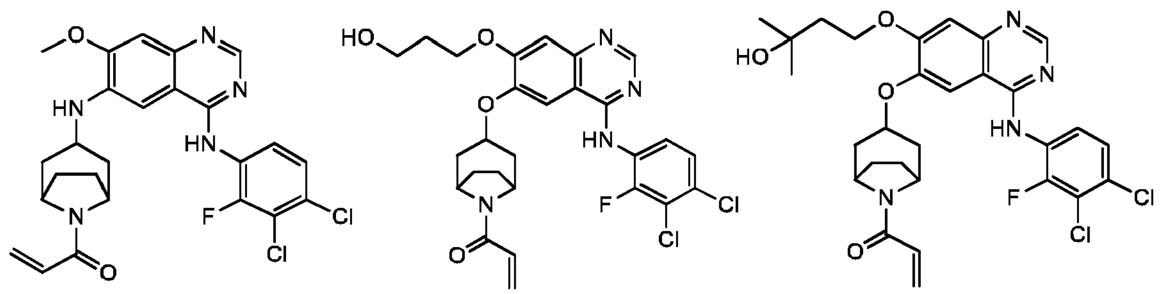

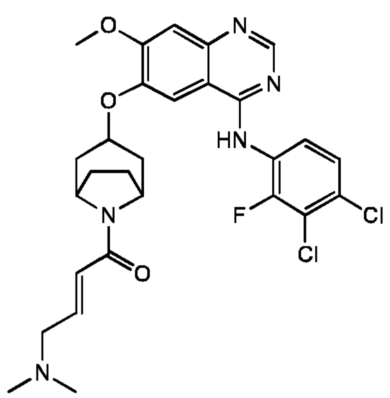
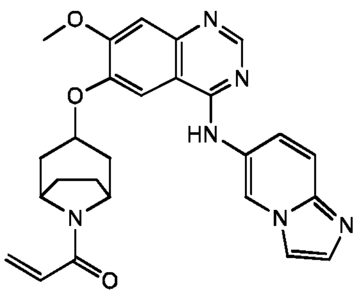
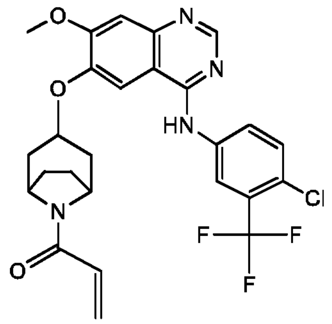

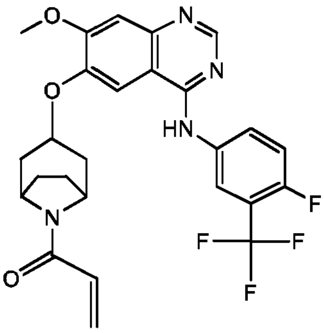 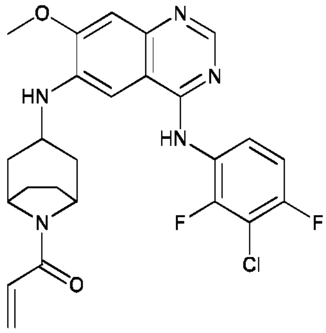 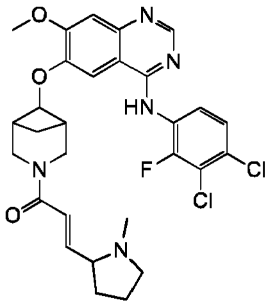 ;

preferably,
the compound is selected from the group consisting of:

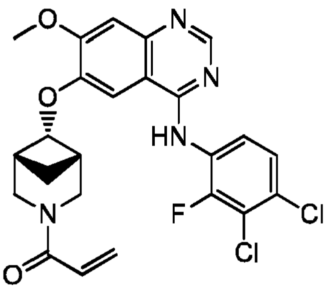 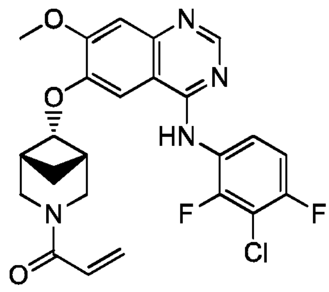 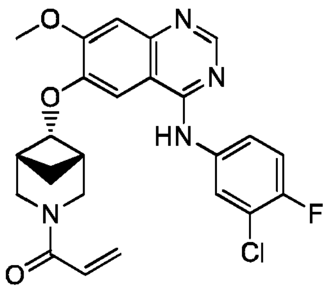

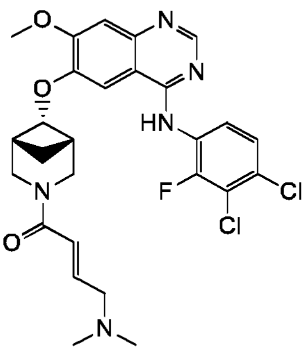 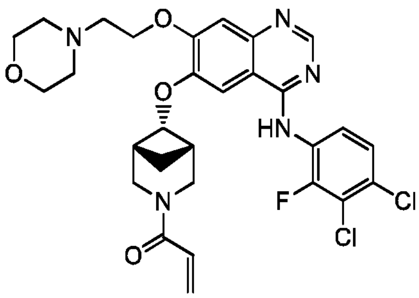 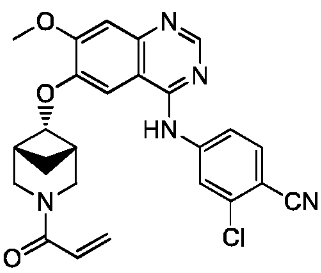

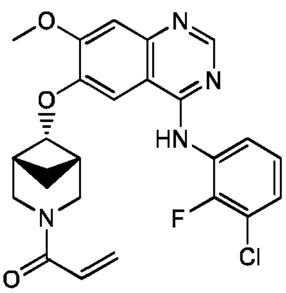 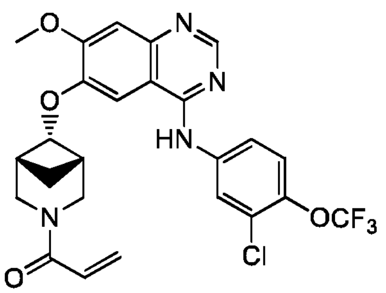 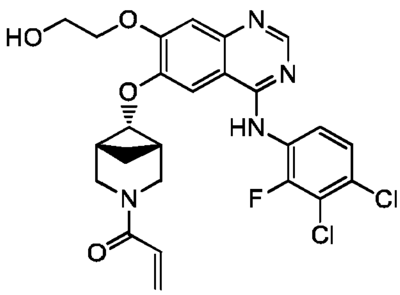

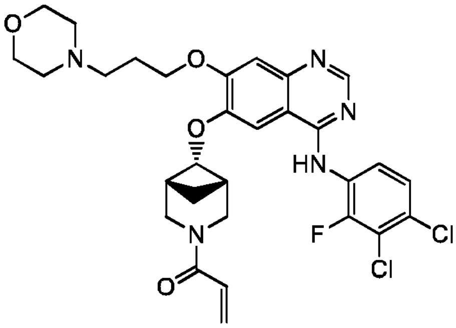
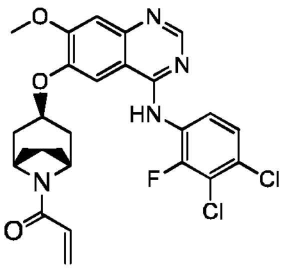
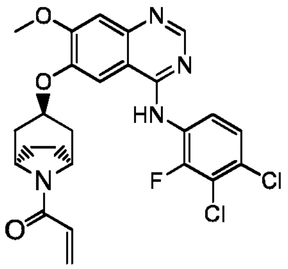

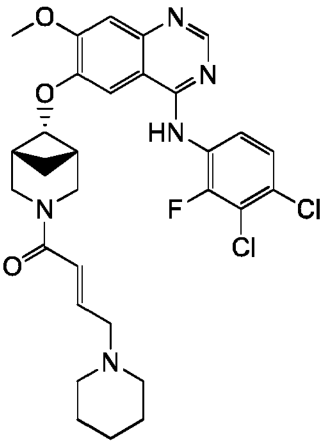
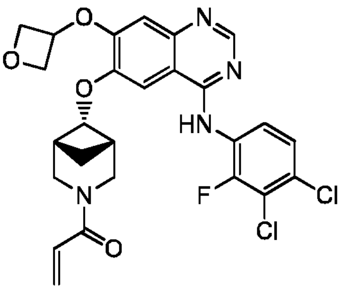
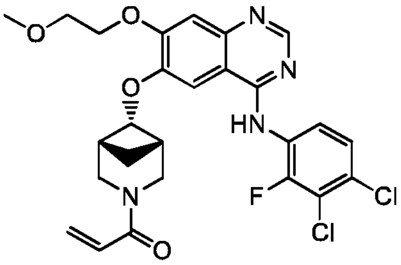

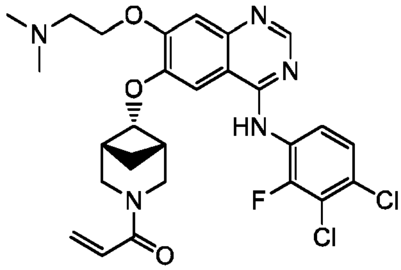
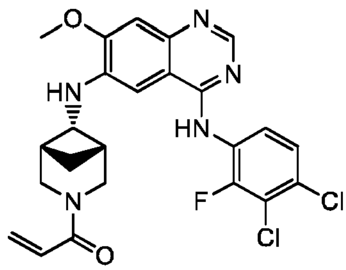
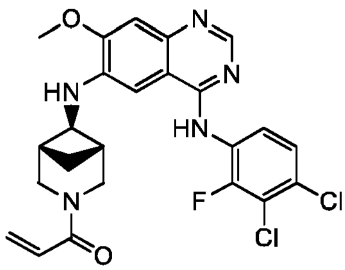

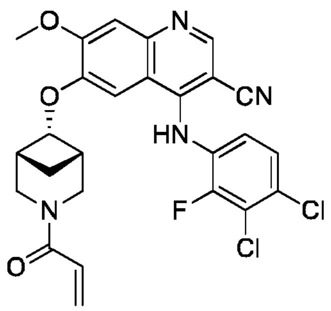
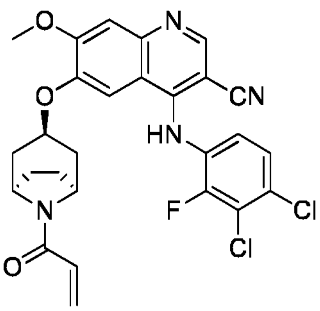
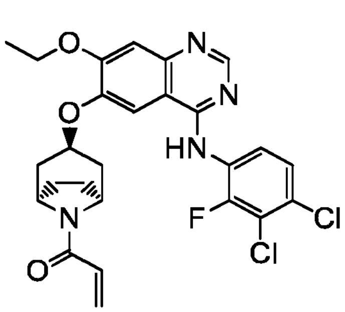

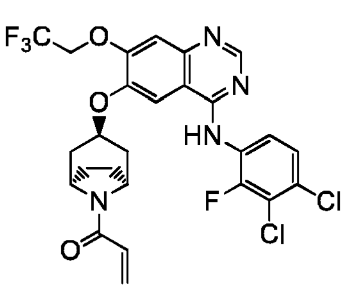
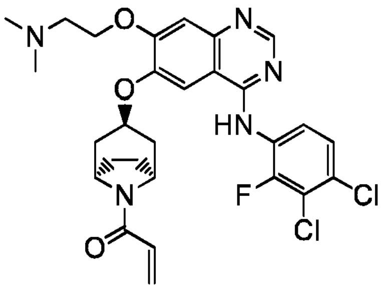
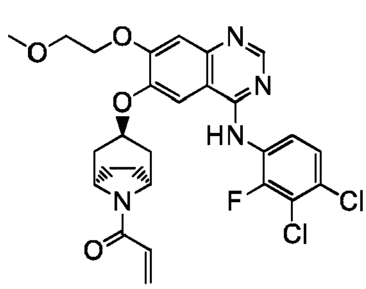

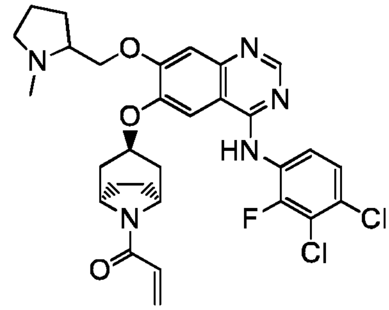
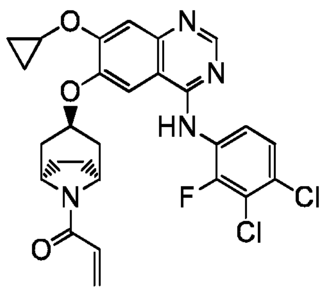
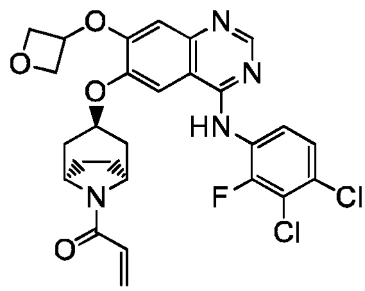

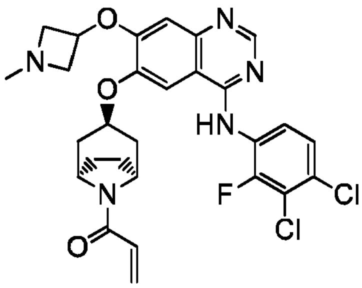
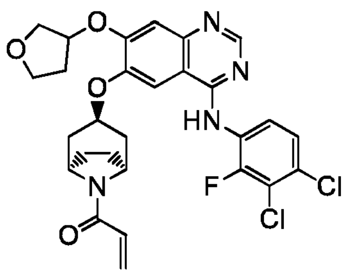
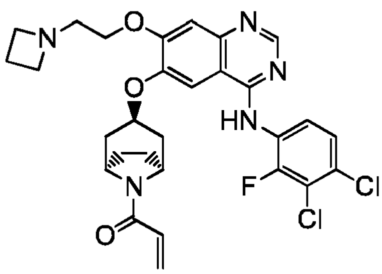

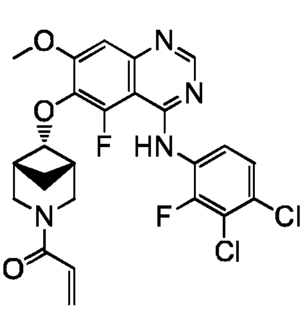
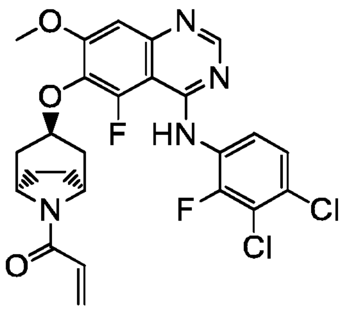
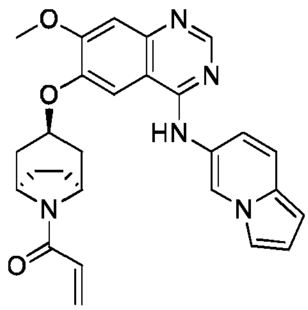

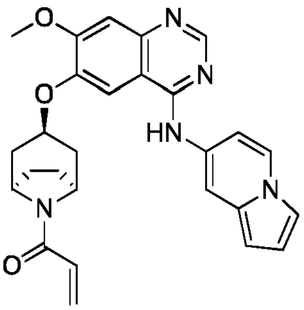
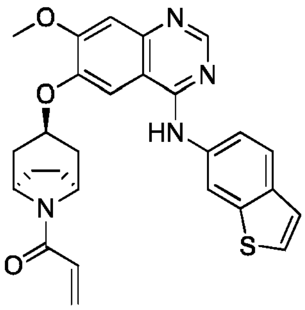
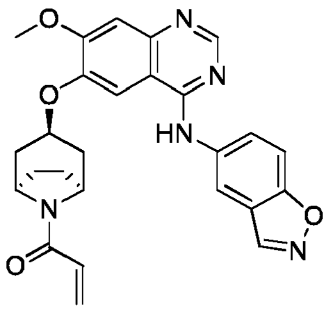

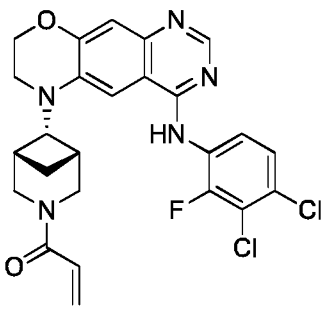
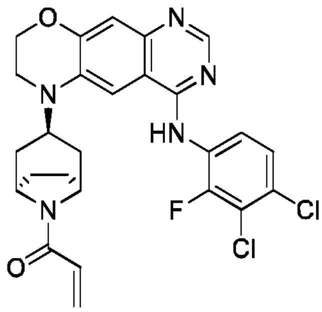
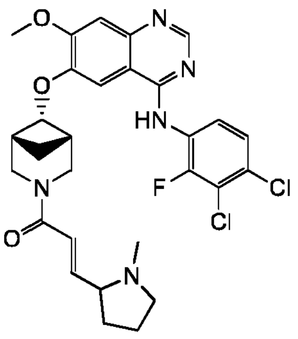
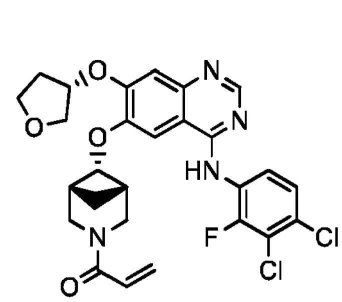
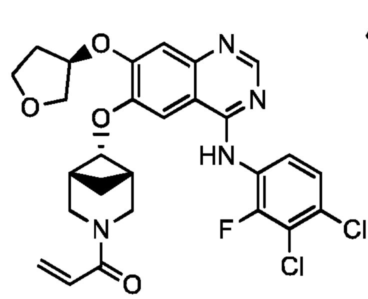
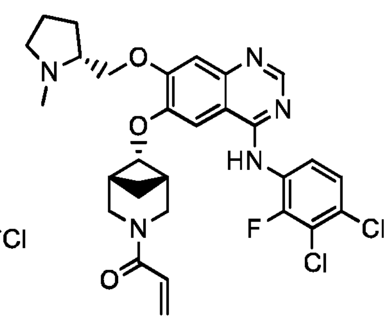
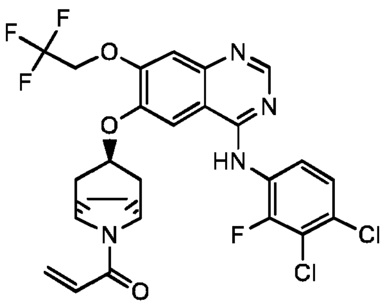
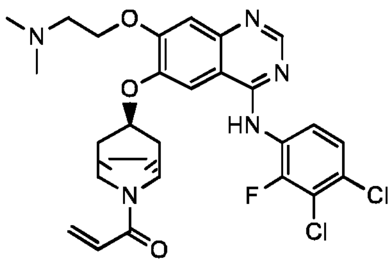
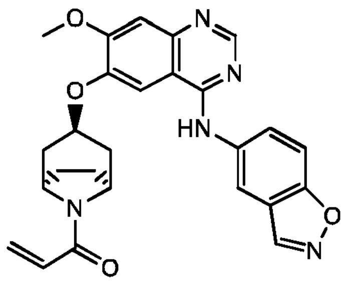
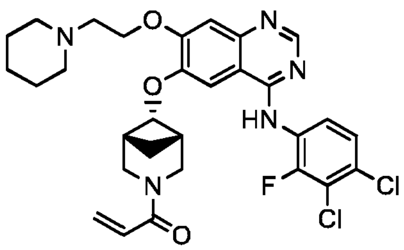
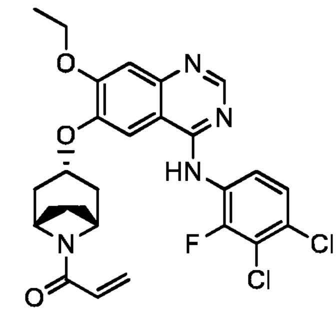
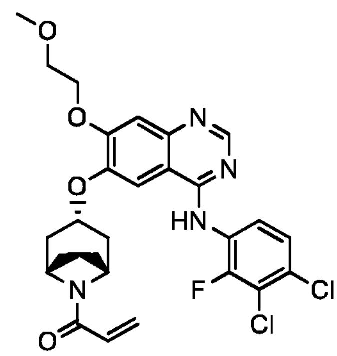
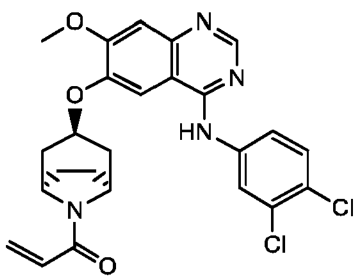
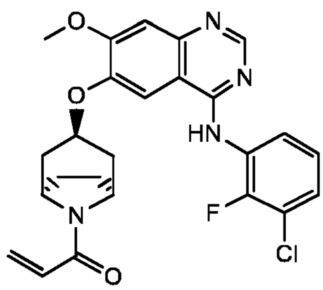
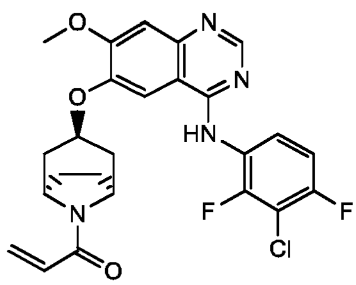

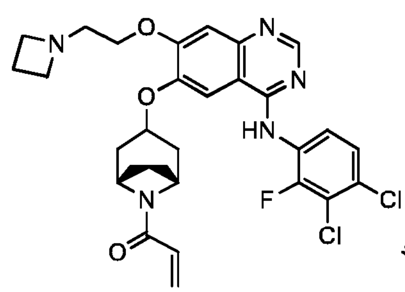
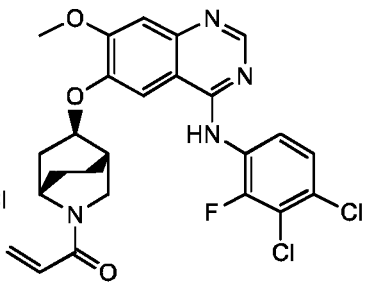
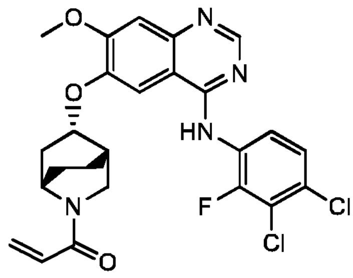

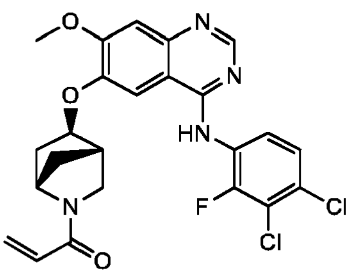
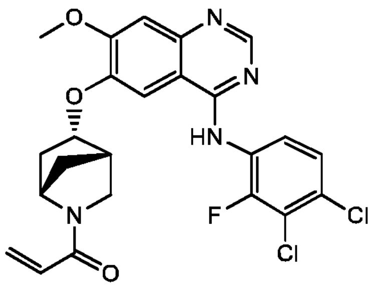
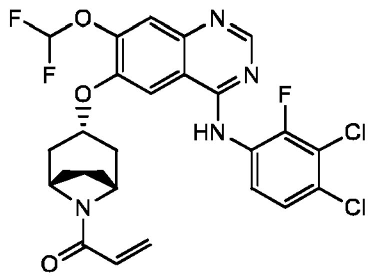

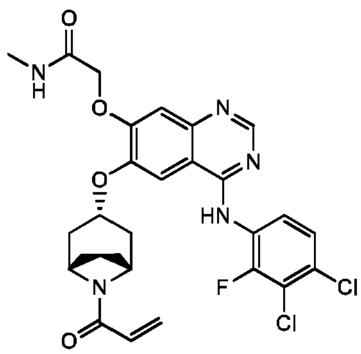
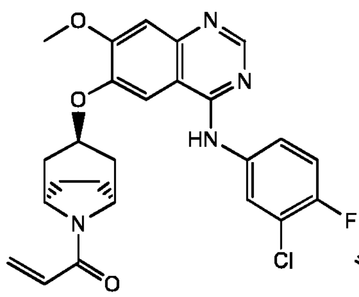
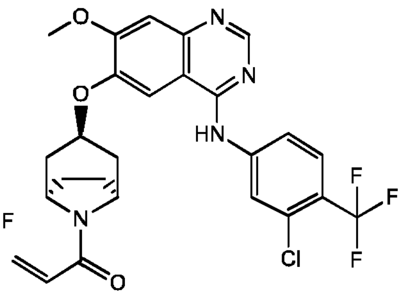

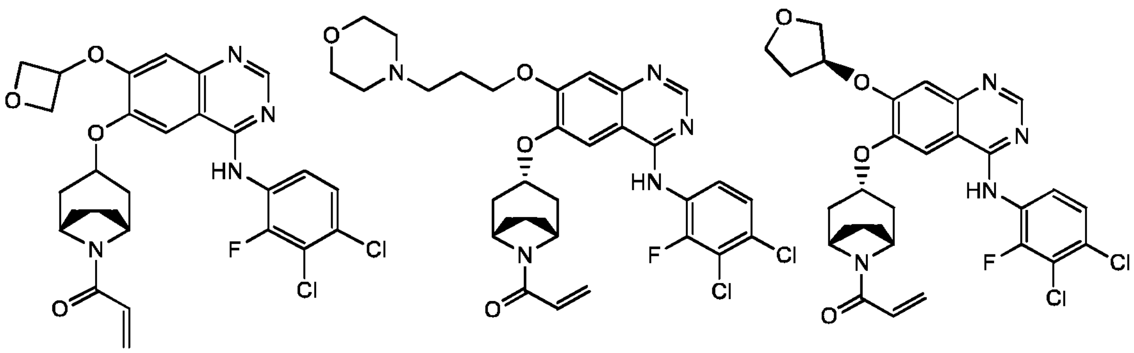

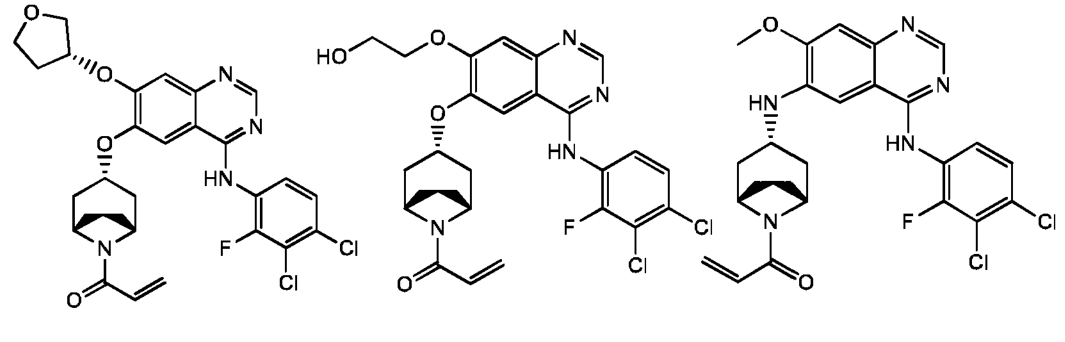

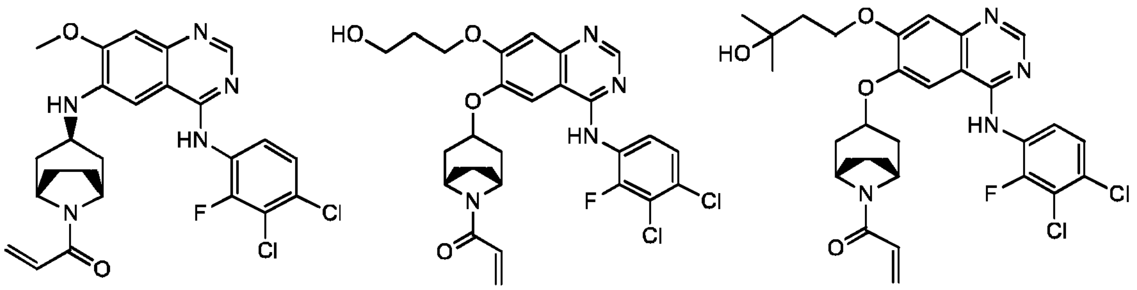

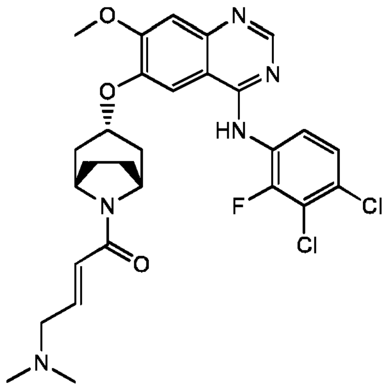

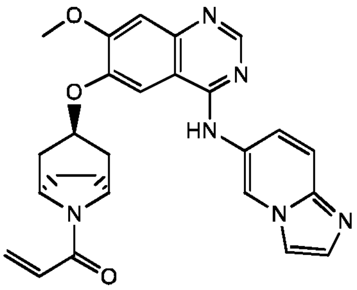

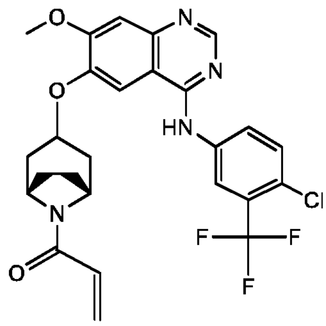

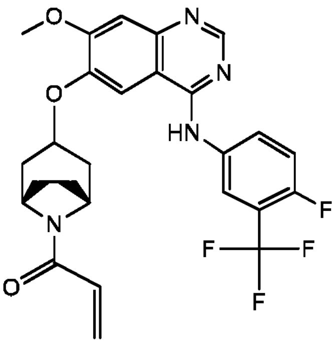

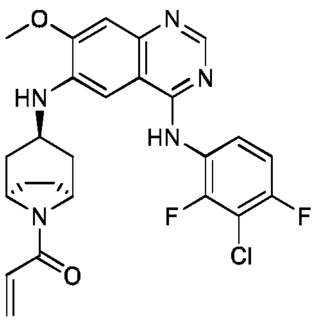

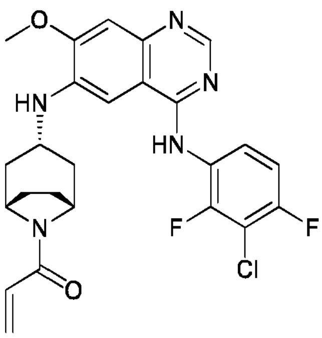

.

14. A pharmaceutical composition, comprising a therapeutically effective amount of the compound according to any one of claims 1 to 13, a stereoisomer thereof or a pharmaceutically acceptable salt thereof, as an active ingredient, and pharmaceutically acceptable carrier(s).

15. A compound according to any one of claims 1 to 13, a stereoisomer thereof or a pharmaceutically acceptable salt thereof, or the pharmaceutical composition according to claim 14, for use in a method of treatment of cancer associated with Pan-HER tyrosine kinase.

## Patentansprüche

1. Verbindung der Formel (I), ein Stereoisomer davon oder ein pharmazeutisch unbedenkliches Salz davon,

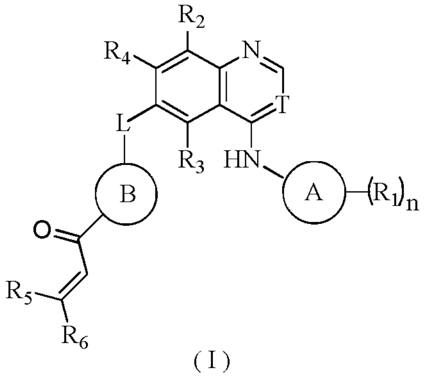

(I)

wobei

T aus der Gruppe bestehend aus N und CR' ausgewählt ist; $R_1$ jeweils unabhängig aus der Gruppe bestehend aus H, F, Cl, Br, I, OH, $NH_2$, CN, $C_{1-3}$-Alkyl und $C_{1-3}$-Alkoxy ausgewählt ist; wobei das $C_{1-3}$-Alkyl oder $C_{1-3}$-Alkoxy gegebenenfalls durch 1, 2 oder 3 Substituenten, die unabhängig aus der Gruppe bestehend aus F, Cl, Br, I, OH, $NH_2$ und CN ausgewählt sind, substituiert ist;

$R_2$ und $R_3$ jeweils unabhängig aus der Gruppe bestehend aus H, F, Cl, Br, I, OH, $NH_2$ und $C_{1-3}$-Alkyl ausgewählt sind, wobei das $C_{1-3}$-Alkyl gegebenenfalls durch 1, 2 oder 3 Substituenten, die unabhängig aus der Gruppe bestehend aus F, Cl, Br und I ausgewählt sind, substituiert ist; $R_4$ aus der Gruppe bestehend aus $C_{1-6}$-Alkyl, $C_{1-6}$-Alkoxy, 3-bis 7-gliedrigem Heterocycloalkyl-O-, 3- bis 7-gliedrigem Heterocycloalkyl-$C_{1-6}$-alkyl-O-, $C_{3-6}$-Cycloalkyl-O- und $C_{3-6}$-Cycloalkyl-$C_{1-6}$-alkyl-O- ausgewählt ist, wobei das $C_{1-6}$-Alkyl, $C_{1-6}$-Alkoxy, 3- bis 7-gliedrige Heterocycloalkyl-O-, 3- bis 7-gliedrige Heterocycloalkyl-$C_{1-6}$-alkyl-O-, $C_{3-6}$-Cycloalkyl-O- und $C_{3-6}$-Cycloalkyl-$C_{1-6}$-alkyl-O- jeweils unabhängig gegebenenfalls durch 1, 2 oder 3 R substituiert sind;

$R_5$ und $R_6$ jeweils unabhängig aus der Gruppe bestehend aus H, $C_{1-3}$-Alkyl und 3- bis 7-gliedrigem Heterocycloalkyl ausgewählt sind, wobei das $C_{1-3}$-Alkyl und 3- bis 7-gliedrige Heterocycloalkyl gegebenenfalls durch 1, 2 oder 3 R substituiert sind;

L aus der Gruppe bestehend aus -O-, -$NR^a$-, -$CR^{b1}R^{b2}$-, - $CR^{b1}R^{b2}$-O- und -$CR^{b1}R^{b2}$-NH- ausgewählt ist;

$R^a$ für H steht;

alternativ dazu $R_4$ mit $R^a$ unter Bildung einer 5- bis 7-gliedrigen Heterocycloalkylgruppe, die gegebenenfalls durch 1, 2, 3 oder 4 R' substituiert ist, verbunden ist; Ring A aus der Gruppe bestehend aus Phenyl und 5- bis 10-gliedrigem Heteroaryl ausgewählt ist;

Ring B aus der Gruppe bestehend aus

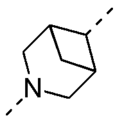 , 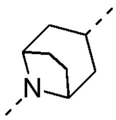 ,

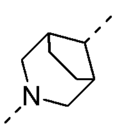 , 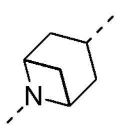 , 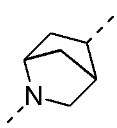 , 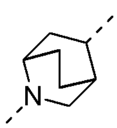 , 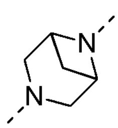 und 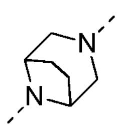

ausgewählt ist;

n aus der Gruppe bestehend aus 1, 2, 3, 4 und 5 ausgewählt ist;

R jeweils unabhängig aus der Gruppe bestehend aus F, Cl, Br, I, OH, $NH_2$, CN, $NR^{c1}R^{c2}$, $C_{1-3}$-Alkyl, $C_{1-3}$-Alkoxy, $C_{1-3}$-Alkyl-NH-C(=O)-, $C_{1-3}$-Alkylthio und 3- bis 7-gliedrigem Heterocycloalkyl ausgewählt ist, wobei das $C_{1-3}$-Alkyl, $C_{1-3}$-Alkoxy, $C_{1-3}$-Alkyl-NH-C(=O)-, $C_{1-3}$-Alkylthio und 3- bis 7-gliedrige Heterocycloalkyl gegebenenfalls durch 1, 2, oder 3 R' substituiert sind;

R' jeweils unabhängig aus der Gruppe bestehend aus F, Cl, Br, I, OH, $NH_2$, CN und $C_{1-3}$-Alkyl ausgewählt ist;

$R^{b1}$ und $R^{b2}$ jeweils unabhängig aus der Gruppe bestehend aus H, F, Cl, Br, I und $C_{1-3}$-Alkyl ausgewählt sind;

$R^{c1}$ und $R^{c2}$ jeweils unabhängig aus der Gruppe bestehend aus H und $C_{1-3}$-Alkyl ausgewählt sind;

das 5- bis 7-gliedrige Heterocycloalkyl, 5- bis 10-gliedrige Heteroaryl und 3- bis 7-gliedrige Heterocycloalkyl

jeweils 1, 2, 3 oder 4 Heteroatome bzw. Heteroatomgruppen, die unabhängig aus der Gruppe bestehend aus N, -O-, -S- und -NH- ausgewählt sind, enthalten.

2. Verbindung nach Anspruch 1, ein Stereoisomer davon oder ein pharmazeutisch unbedenkliches Salz davon, wobei R jeweils unabhängig aus der Gruppe bestehend aus F, Cl, Br, I, OH, $NH_2$, CN,

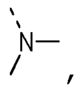 ,

Me , Et,

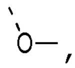 , 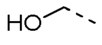 , 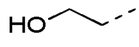 ,

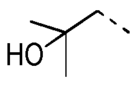 , 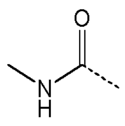 ,

Piperidinyl, Pyrrolidinyl, Azetidinyl, Morpholinyl und Oxetanyl ausgewählt ist, wobei das Me, Et,

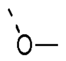 ,

Piperidinyl, Pyrrolidinyl, Azetidinyl, Morpholinyl und Oxetanyl gegebenenfalls durch 1, 2 oder 3 Substituenten, die unabhängig aus der Gruppe bestehend aus F, Cl, Br, I, OH, $NH_2$ und $C_{1-3}$-Alkyl ausgewählt sind, substituiert sind; vorzugsweise
wobei R jeweils unabhängig aus der Gruppe bestehend aus F, Cl, Br, I, OH, $NH_2$, CN,

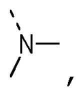 ,

Me, Et,

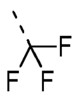 , 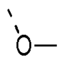 , 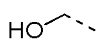

, 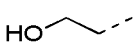 , 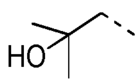 , 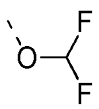 , 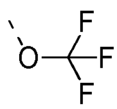 , 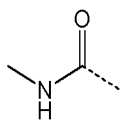 , 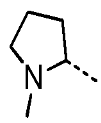 , 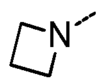

, 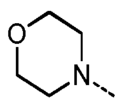 und 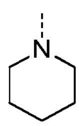

ausgewählt ist.

**3.** Verbindung nach Anspruch 1 oder 2, ein Stereoisomer davon oder ein pharmazeutisch unbedenkliches Salz davon, wobei $R_1$ jeweils unabhängig aus der Gruppe bestehend aus H, F, Cl, Br, I, OH, $NH_2$, CN, Me und 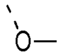 ausgewählt ist, wobei das Me und 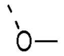 gegebenenfalls durch 1, 2 oder 3 Substituenten, die unabhängig aus der Gruppe bestehend aus F, Cl, Br, I, OH, $NH_2$ und CN ausgewählt sind, substituiert sind;

vorzugsweise

wobei $R_1$ jeweils unabhängig aus der Gruppe bestehend aus H, F, Cl, Br, I, OH, $NH_2$, CN, Me, $CH_2F$, $CHF_2$, $CF_3$, 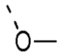 und

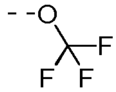

ausgewählt ist.

**4.** Verbindung nach einem der Ansprüche 1 bis 3, ein Stereoisomer davon oder ein pharmazeutisch unbedenkliches Salz davon, wobei $R_2$ und $R_3$ jeweils unabhängig aus der Gruppe bestehend aus H, F, Cl, Br, I, OH, $NH_2$, Me und $CF_3$ ausgewählt sind.

**5.** Verbindung nach einem der Ansprüche 1 bis 4, ein Stereoisomer davon oder ein pharmazeutisch unbedenkliches Salz davon, wobei $R_4$ aus der Gruppe bestehend aus

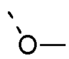, 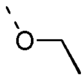, 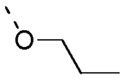,

Piperidinyl-$C_{1-3}$-alkyl-O-, Oxetanyl-O-, Morpholinyl-$C_{1-3}$-alkyl-O-, Azetidinyl-O-, Tetrahydrofuranyl-O-, Cyclopropyl-O-, Pyrrolidinyl-$C_{1-3}$-alkyl-O- und Azetidinyl-$C_{1-3}$-alkyl-O- ausgewählt ist, wobei das

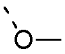, 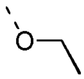, 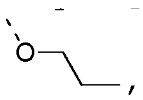,

Piperidinyl-$C_{1-3}$-alkyl-O-, Oxetanyl-O-, Morpholinyl-$C_{1-3}$-alkyl-O-, Azetidinyl-O-, Tetrahydrofuranyl-O-, Cyclopropyl-O-, Pyrrolidinyl-$C_{1-3}$-alkyl-O- und Azetidinyl-$C_{1-3}$-alkyl-O- gegebenenfalls durch 1, 2 oder 3 R substituiert sind;

vorzugsweise

wobei $R_4$ aus der Gruppe bestehend aus

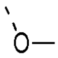, 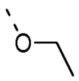, 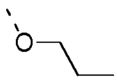,

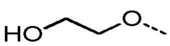, 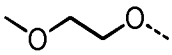, 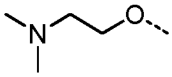, 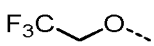, 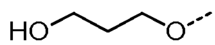,

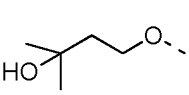, 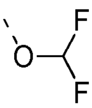, 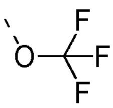, 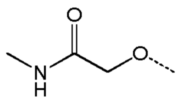, 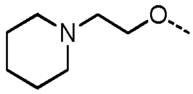,

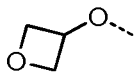, 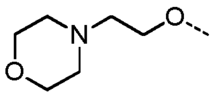, 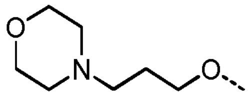, 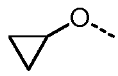, 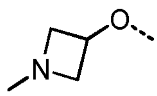,

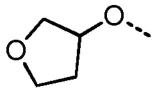, 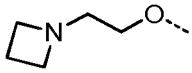 und 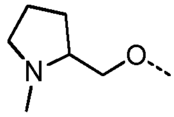

ausgewählt ist.

6. Verbindung nach einem der Ansprüche 1 bis 5, ein Stereoisomer davon oder ein pharmazeutisch unbedenkliches Salz davon, wobei $R_5$ und $R_6$ jeweils unabhängig aus der Gruppe bestehend aus H, Me, Et und

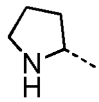

ausgewählt sind, wobei das Me, Et und

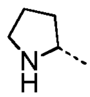

gegebenenfalls durch 1, 2 oder 3 R substituiert sind; vorzugsweise wobei $R_5$ und $R_6$ jeweils unabhängig aus der Gruppe bestehend aus H,

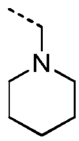, 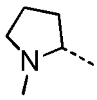 und 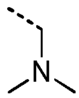

ausgewählt sind.

7. Verbindung nach einem der Ansprüche 1 bis 6, ein Stereoisomer davon oder ein pharmazeutisch unbedenkliches Salz davon, wobei T aus der Gruppe bestehend aus N und C(CN) ausgewählt ist.

8. Verbindung nach einem der Ansprüche 1 bis 7, ein Stereoisomer davon oder ein pharmazeutisch unbedenkliches Salz davon, wobei L aus der Gruppe bestehend aus -O-, -NH-, $-CH_2-$, $-CH_2-O-$ und $-CH_2-NH-$ausgewählt ist.

9. Verbindung nach einem der Ansprüche 1 bis 8, ein Stereoisomer davon oder ein pharmazeutisch unbedenkliches Salz davon, wobei die Gruppierung

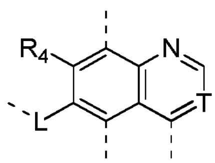

aus der Gruppe bestehend aus

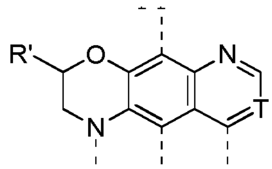,

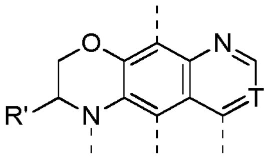, 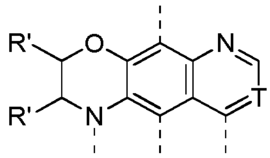, 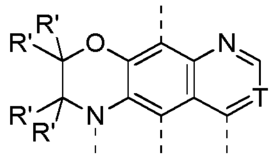

und

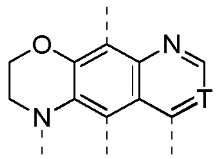

ausgewählt ist;
vorzugsweise wobei es sich bei der Gruppierung

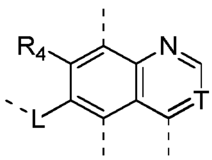

um

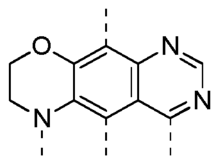

handelt.

**10.** Verbindung nach einem der Ansprüche 1 bis 9, ein Stereoisomer davon oder ein pharmazeutisch unbedenkliches Salz davon, wobei der Ring A aus der Gruppe bestehend aus Phenyl, Azaindenyl, Benzo[*b*]-thienyl, Imidazo[1,2-a]pyridyl und Benzo[d]isoxazolyl ausgewählt ist;
vorzugsweise

wobei die Gruppierung

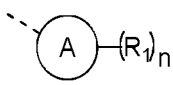

aus der Gruppe bestehend aus

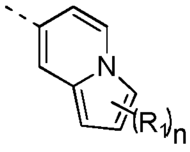, 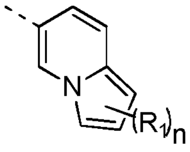, 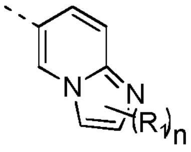, 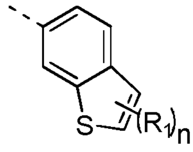,

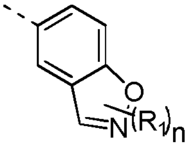 und 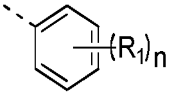

ausgewählt ist; vorzugsweise wobei die Gruppierung

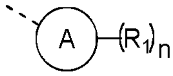

aus der Gruppe bestehend aus

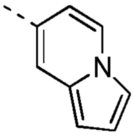, 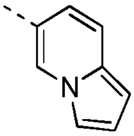, 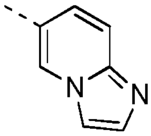, 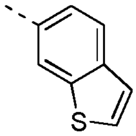, 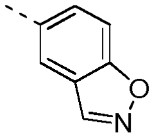,

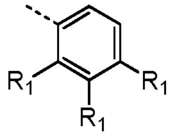, 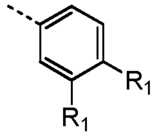 und 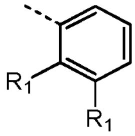

ausgewählt ist; vorzugsweise wobei die Gruppierung

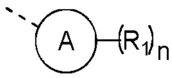

aus der Gruppe bestehend aus

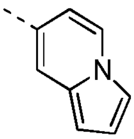, 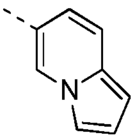, 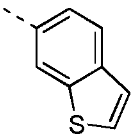, 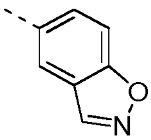, 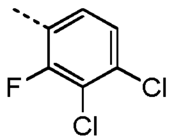,

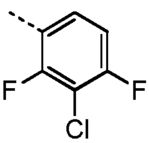, 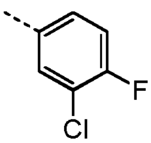, 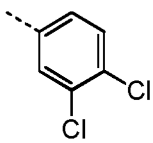, 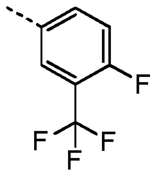, 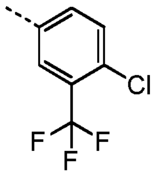,

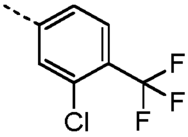, 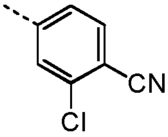, 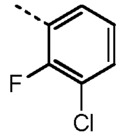 und 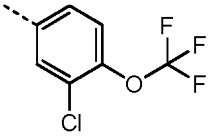

ausgewählt ist.

11. Verbindung nach einem der Ansprüche 1 bis 10, ein Stereoisomer davon oder ein pharmazeutisch unbedenkliches Salz davon, wobei der Ring B aus der Gruppe bestehend aus

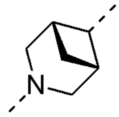, 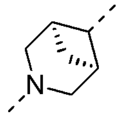, 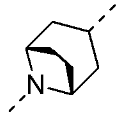, 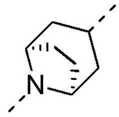,

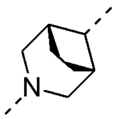, 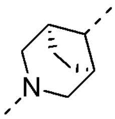, 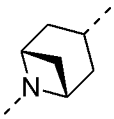, 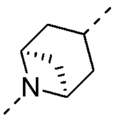, 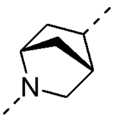, 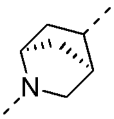, 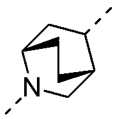,

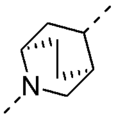, 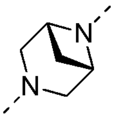, 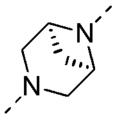, 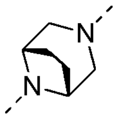 und 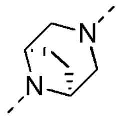

ausgewählt ist; vorzugsweise wobei die Gruppierung

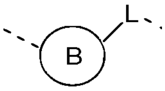

aus der Gruppe bestehend aus

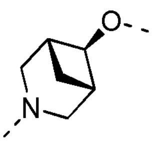, 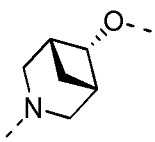, 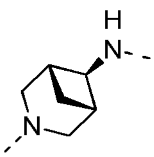, 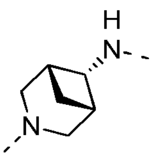, 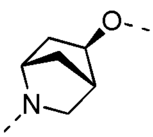,

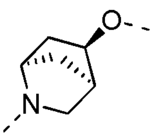, 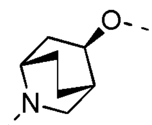, 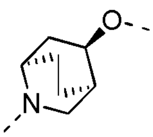, 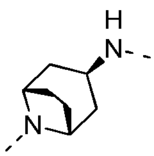, 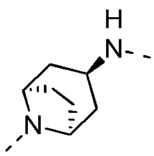,

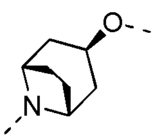 und 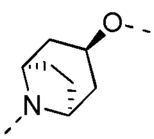

ausgewählt ist.

**12.** Verbindung nach einem der Ansprüche 1 bis 11, ein Stereoisomer davon oder ein pharmazeutisch unbedenkliches Salz davon, wobei die Verbindung aus der Gruppe bestehend aus:

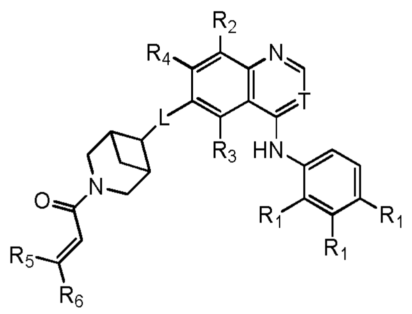

(II -1) ,

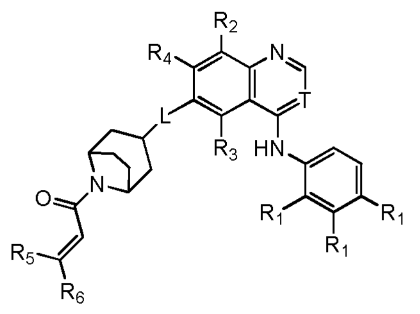

(II -2) ,

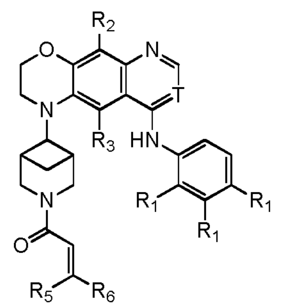

(II -3) ,

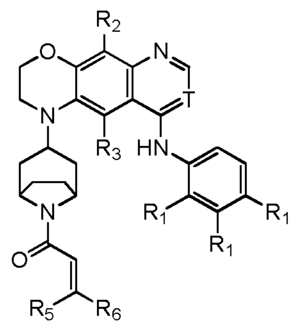

(II -4) ,

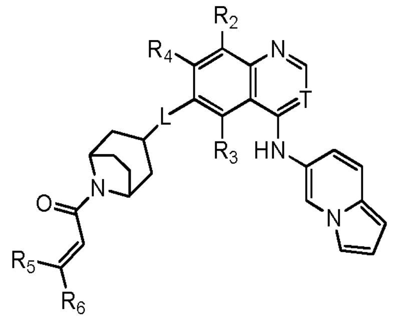

(II -5) ,

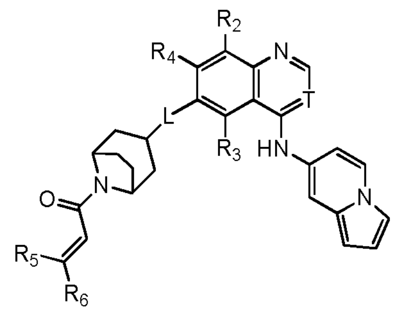

(II -6) ,

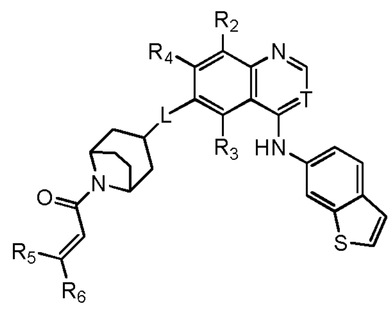

(II -7) ,

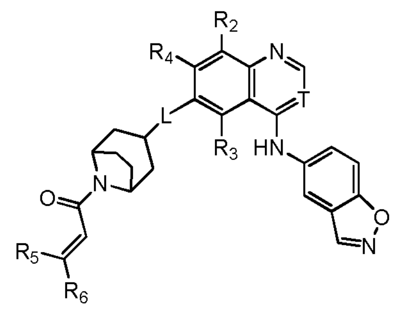

(II -8) ,

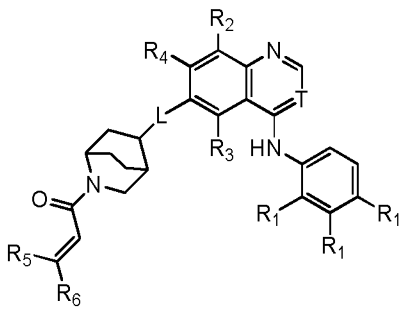

(II -9) ,

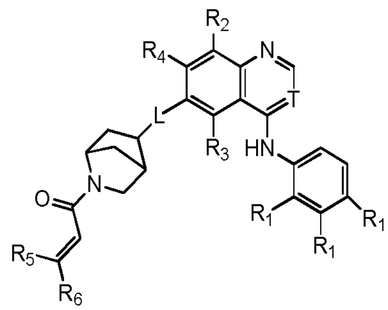

(II -10)

und

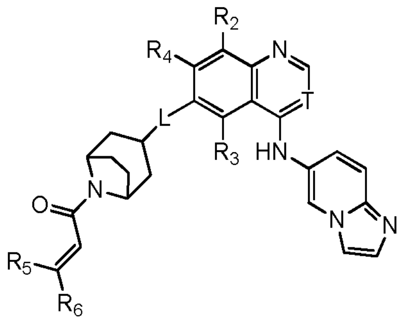

(II -11)

ausgewählt ist, wobei T, L, $R_1$, $R_2$, $R_3$, $R_4$, $R_5$ und $R_6$ wie in den Ansprüchen 1 bis 11 definiert sind; vorzugsweise

wobei die Verbindung aus der Gruppe bestehend aus:

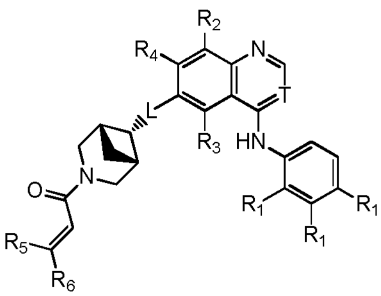

( III -1) ,

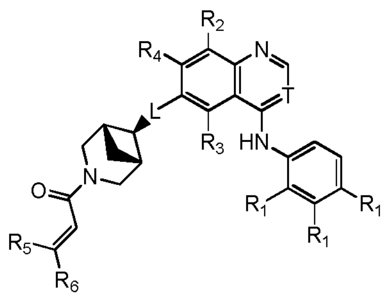

( III -2) ,

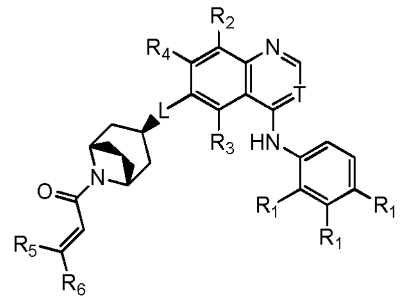

( III -3) ,

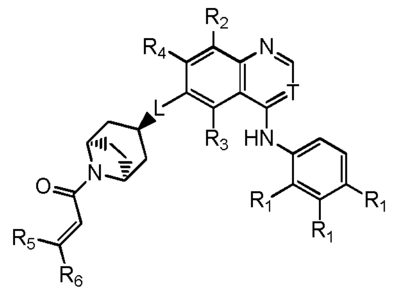

( III -4) ,

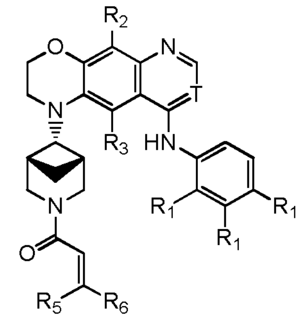

(III -5) ,

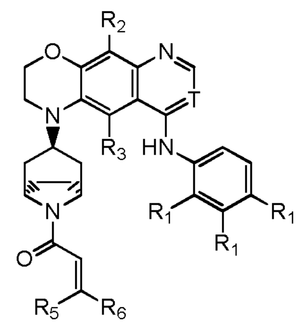

( III -6) ,

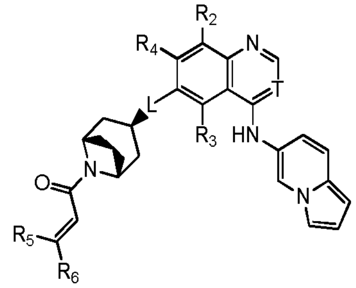

(III -7) ,

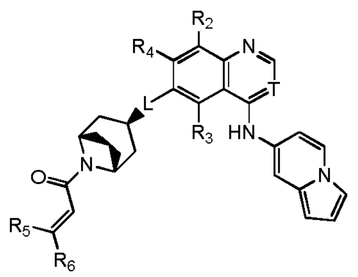

(III -8) ,

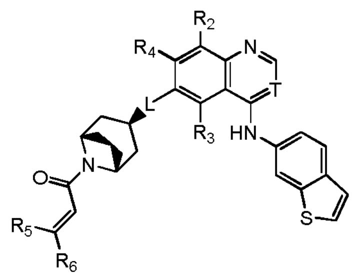

(III -9) ,

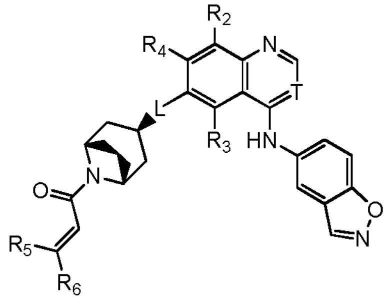

(III -10) ,

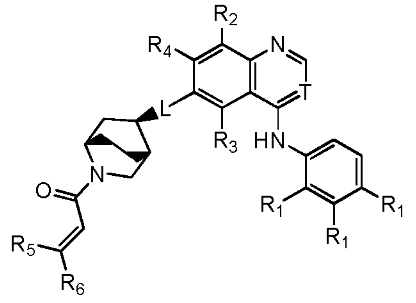

(III -11) ,

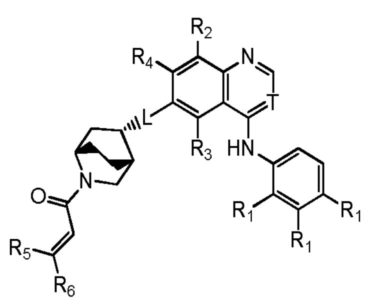

(III-12) ,

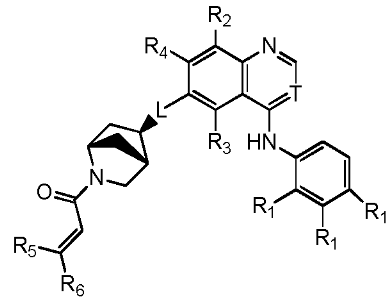

(III-13) ,

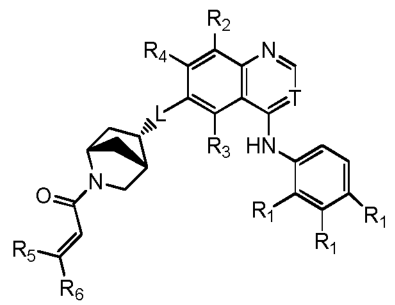

(III-14) ,

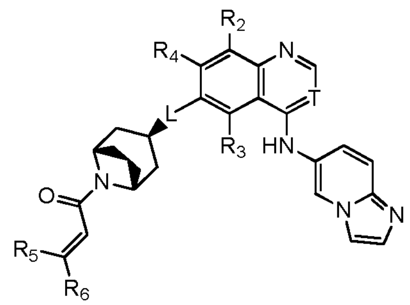

(III-15)

und

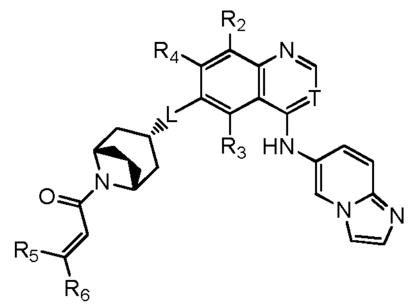

(III-16)

ausgewählt ist, wobei T, L, $R_1$, $R_2$, $R_3$, $R_4$, $R_5$ und $R_6$ wie in den Ansprüchen 1 bis 11 definiert sind.

**13.** Verbindung nach Anspruch 1, ein Stereoisomer davon oder ein pharmazeutisch unbedenkliches Salz davon, wobei die Verbindung aus der Gruppe bestehend aus:

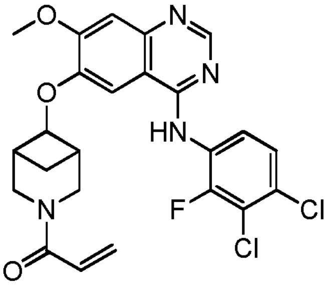

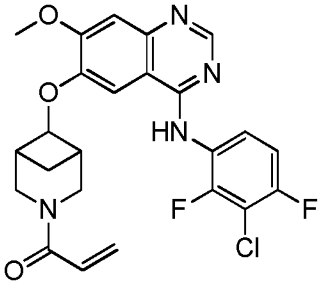

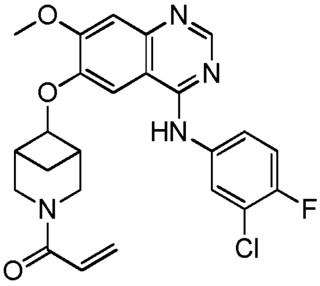

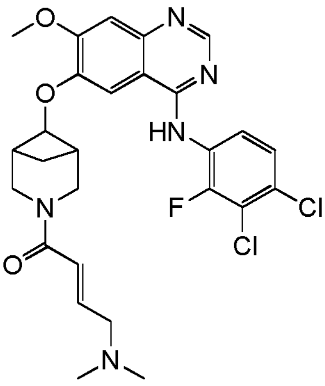
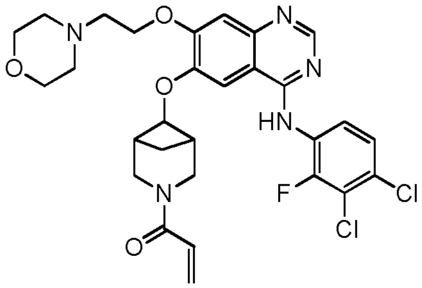

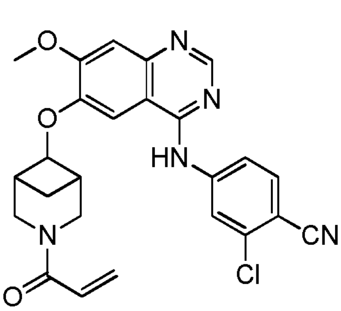
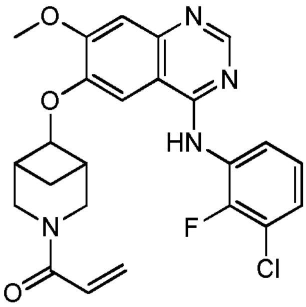
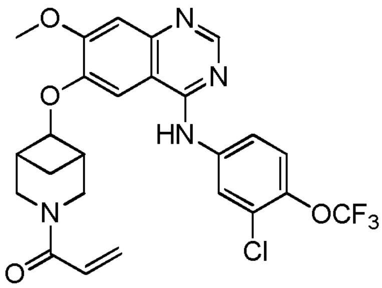

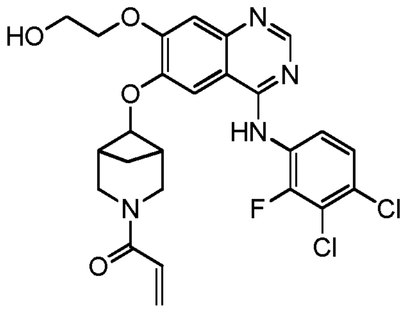

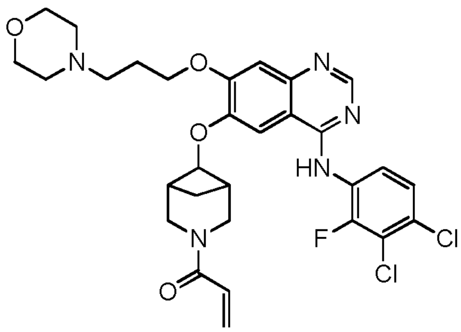
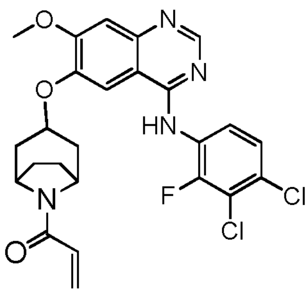

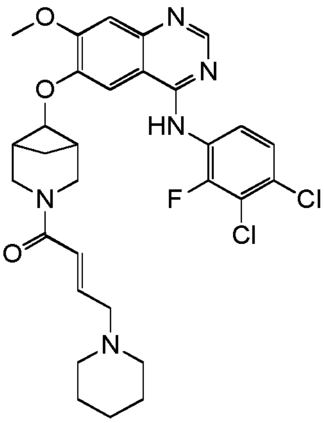

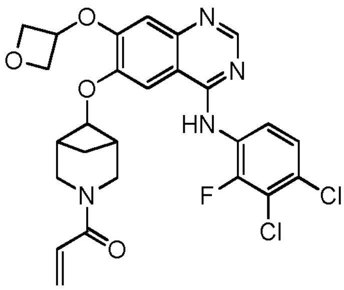

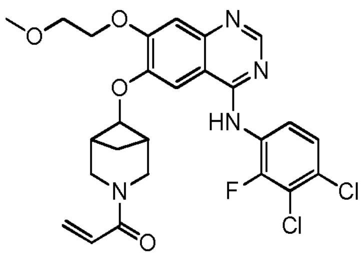

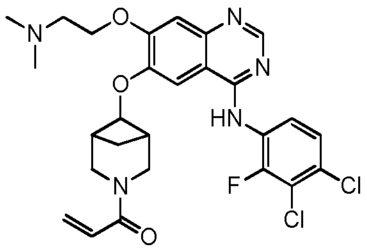

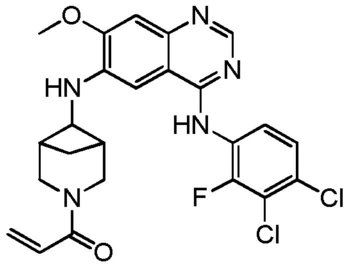

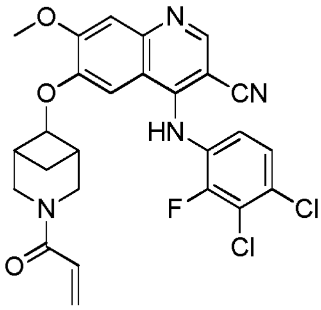

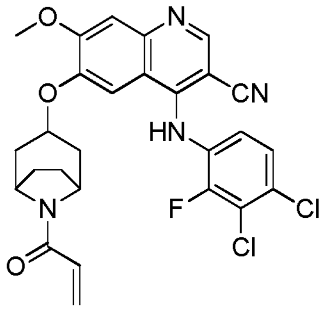

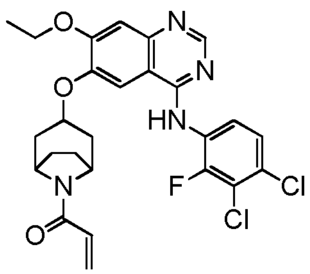

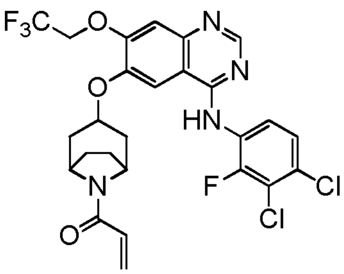

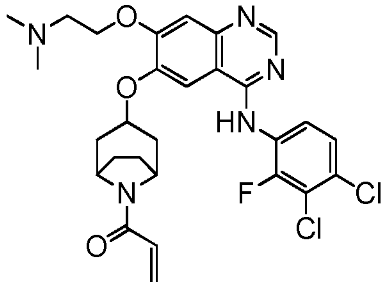

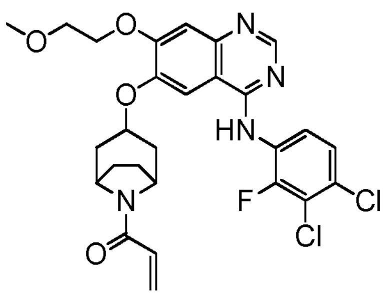

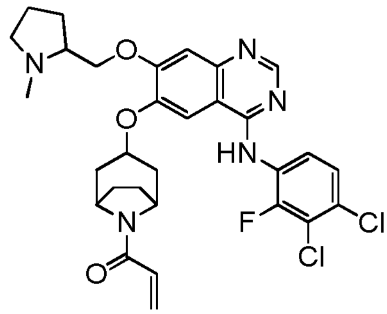

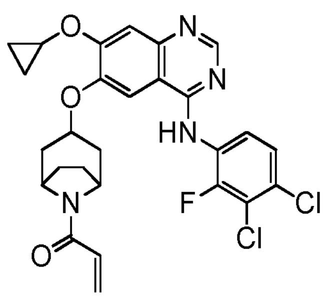

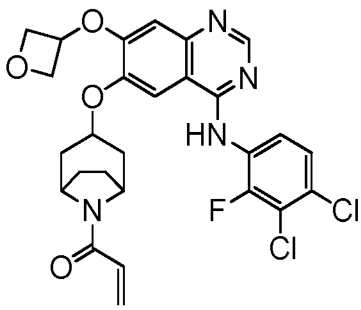

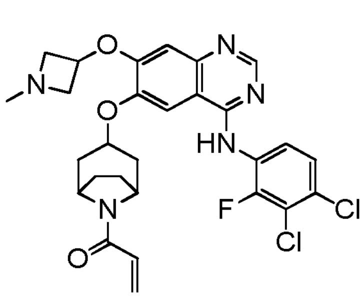

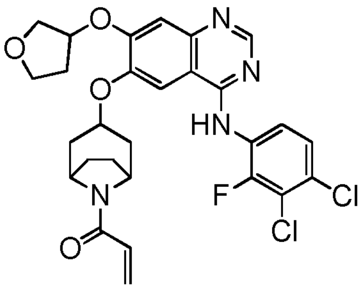
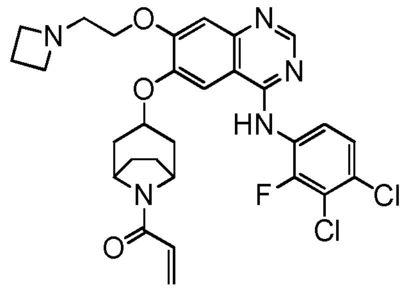

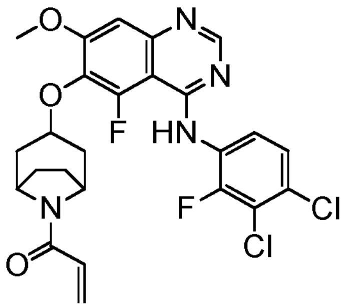
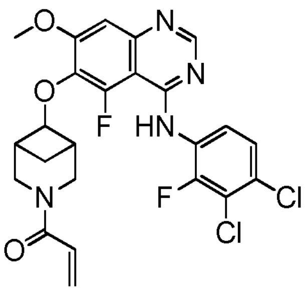

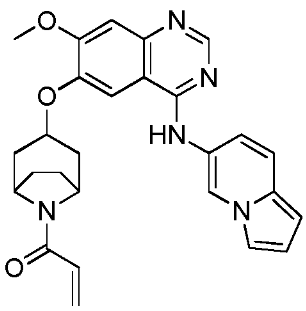
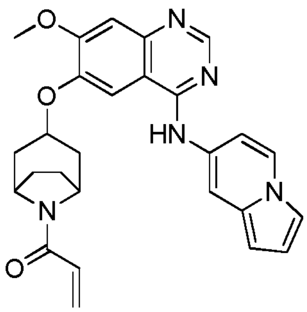
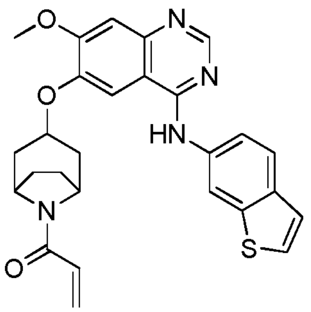

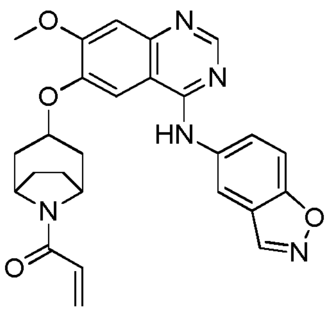
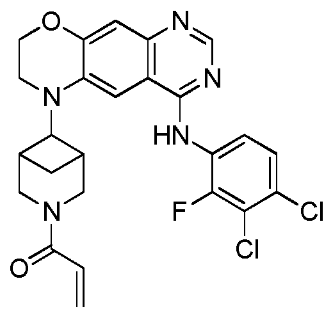

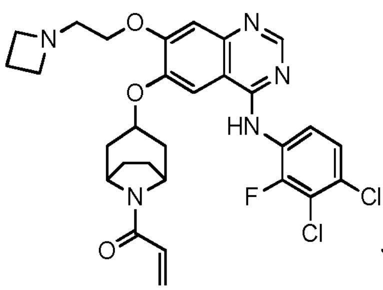
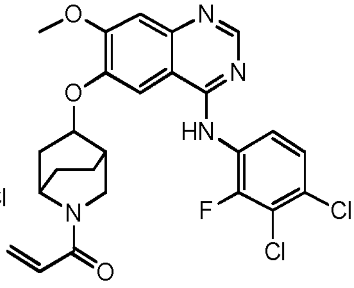
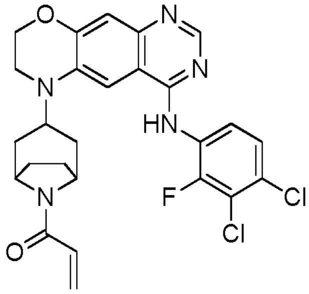

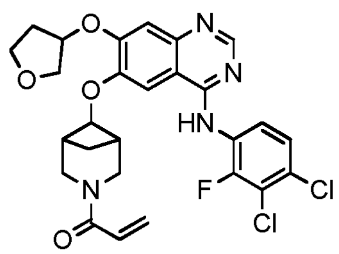
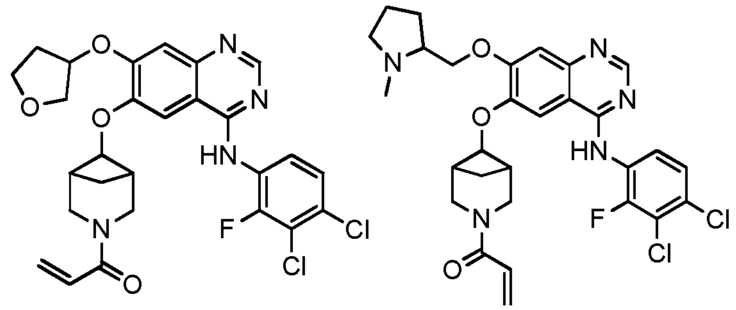
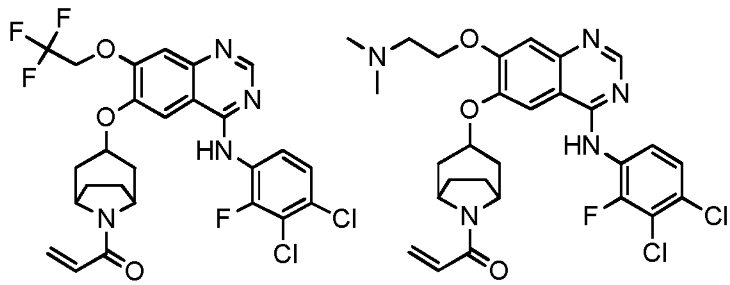
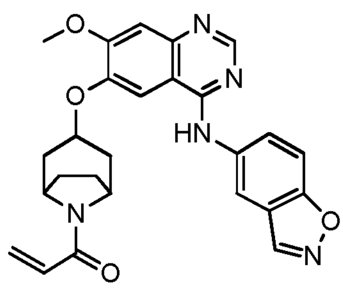
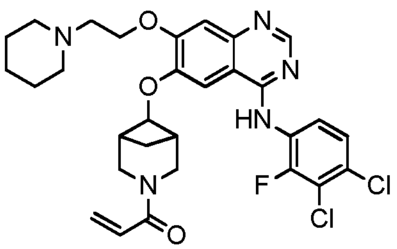
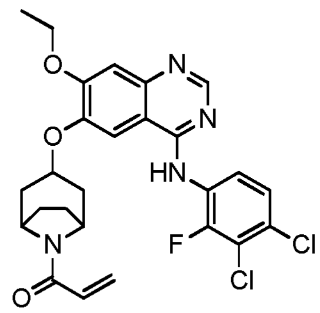
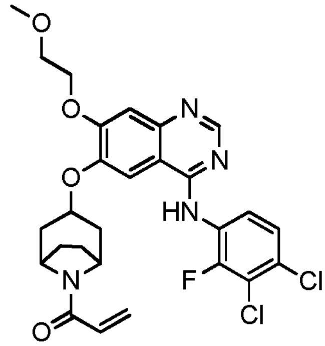
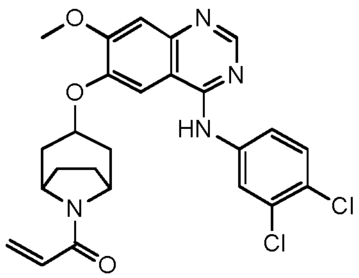
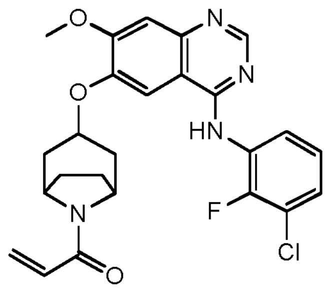
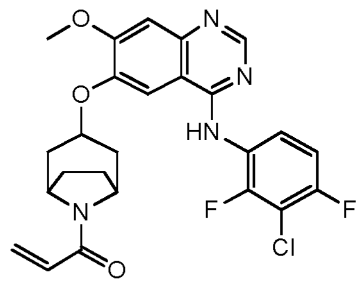
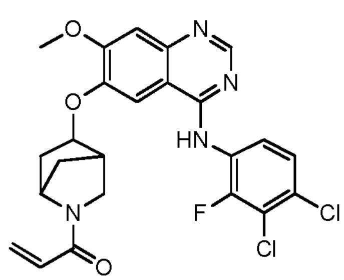
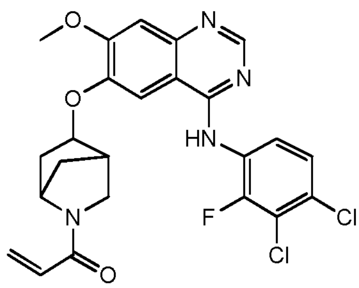
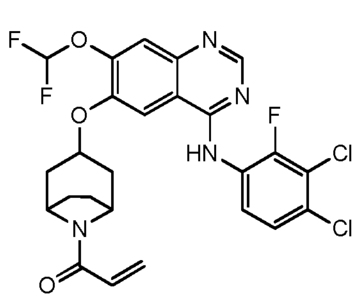

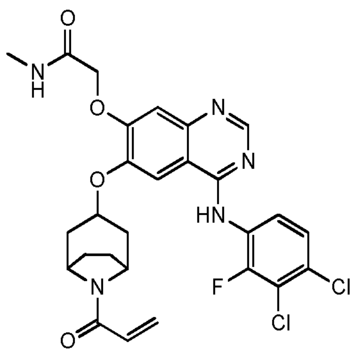

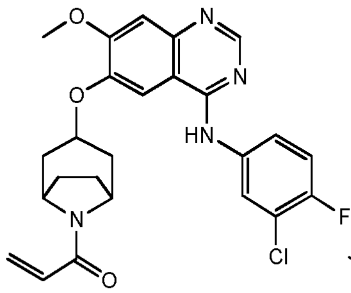

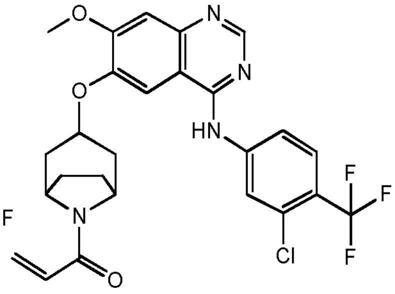

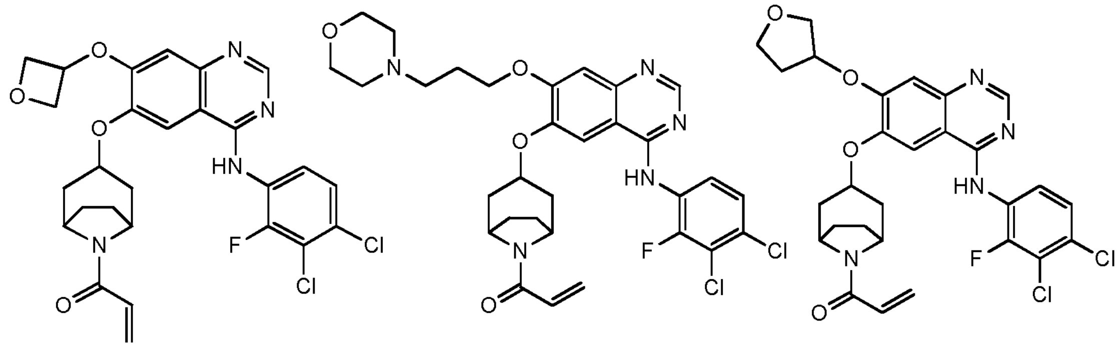

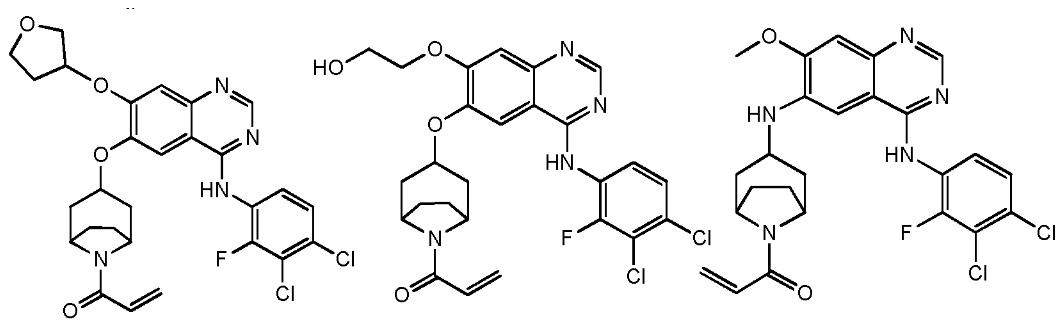

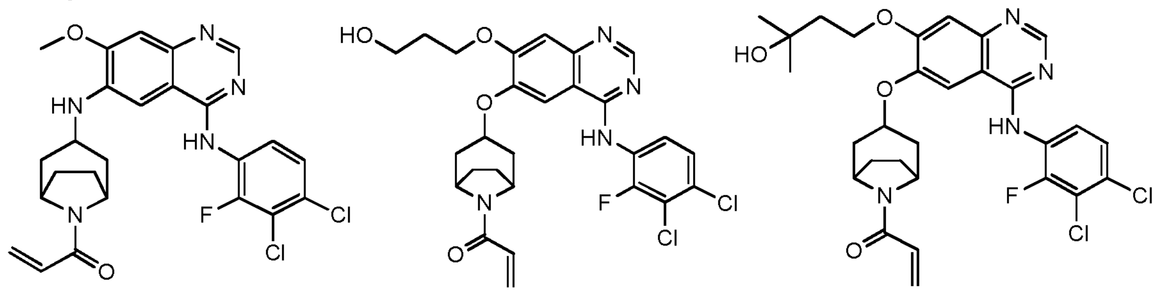

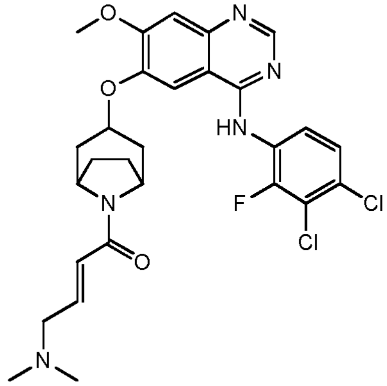

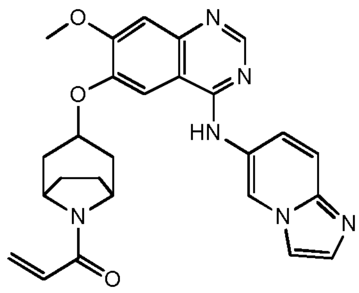

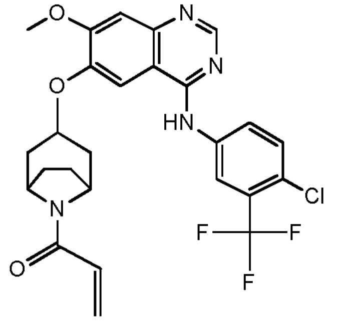

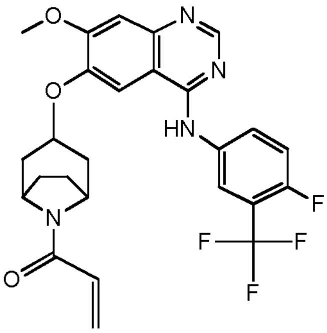
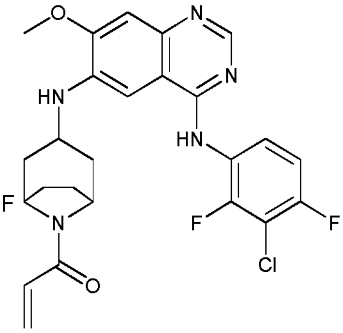
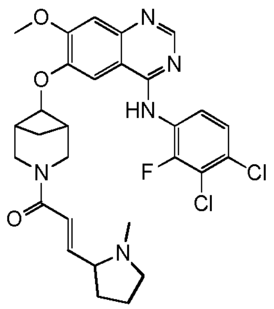

ausgewählt ist;
vorzugsweise
die Verbindung aus der Gruppe bestehend aus:

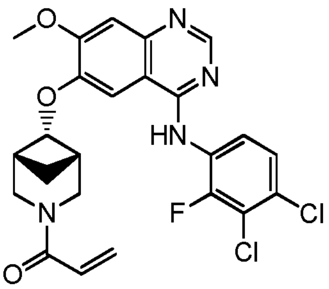
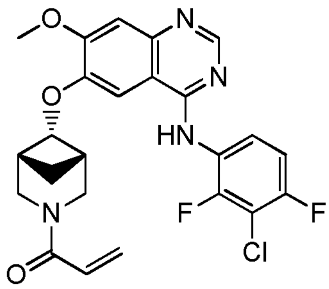
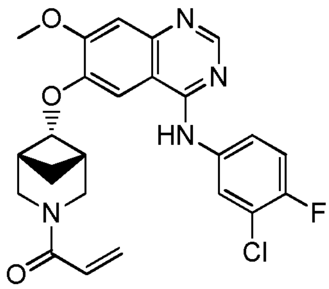

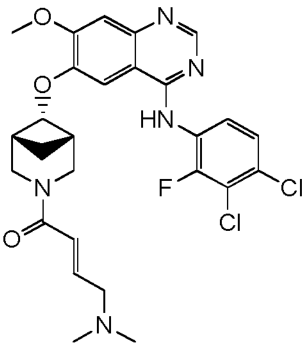
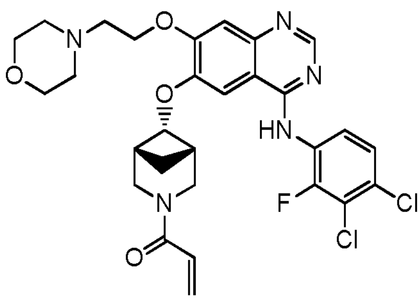
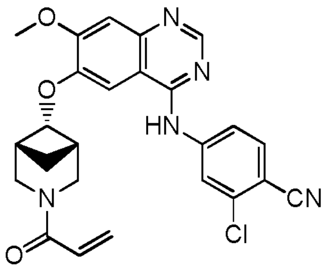

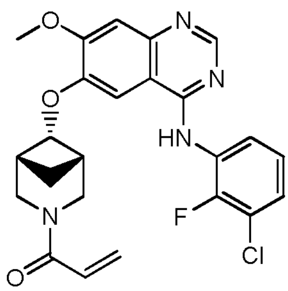
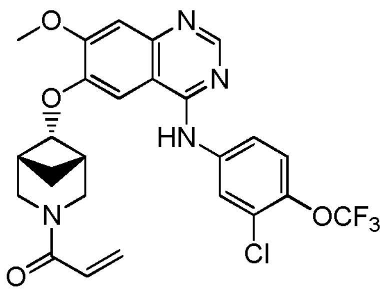

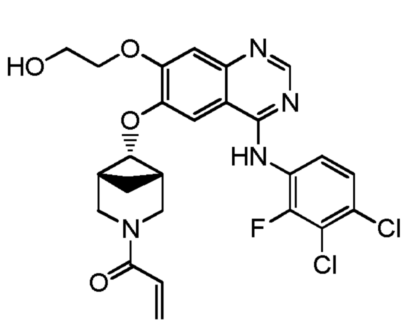

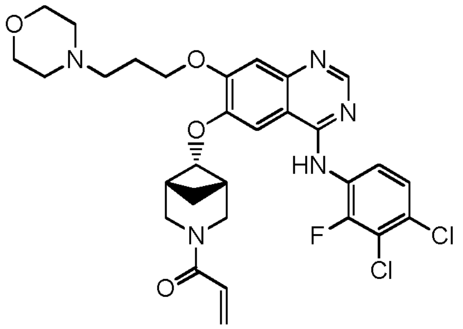

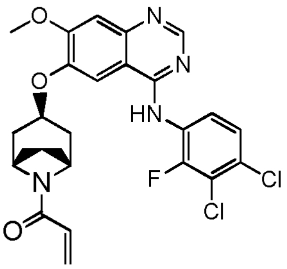

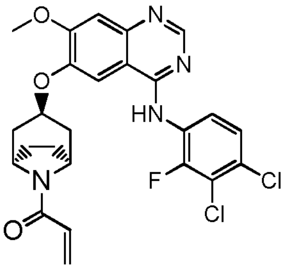

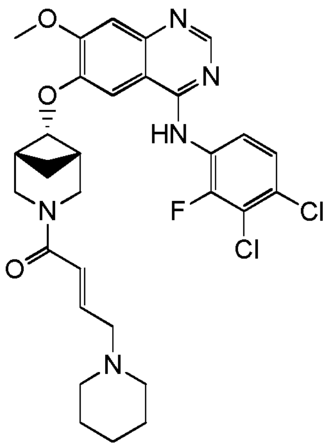

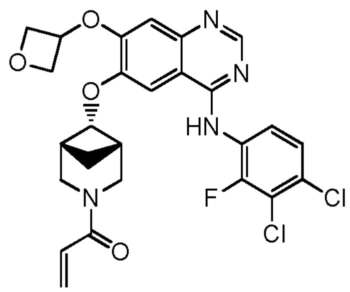

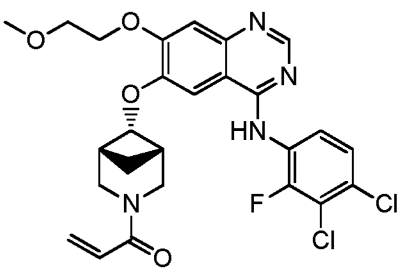

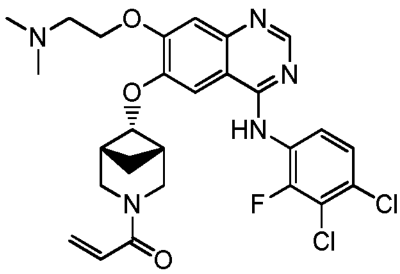

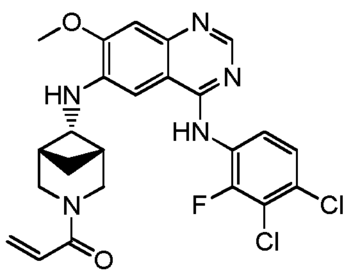

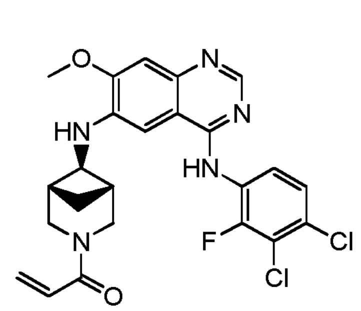

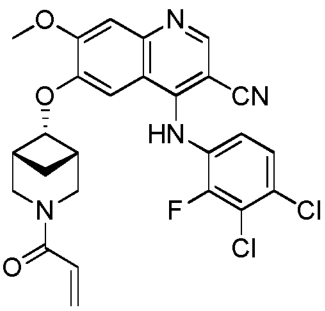

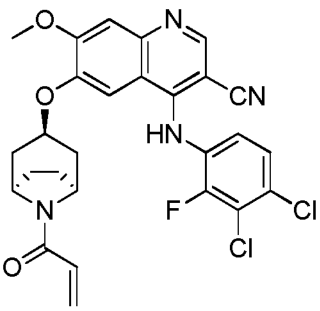

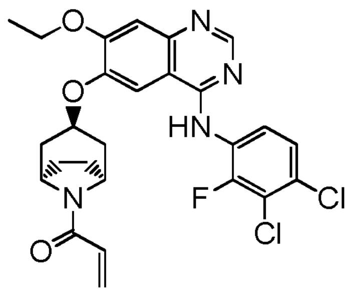

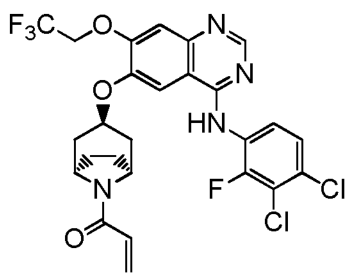

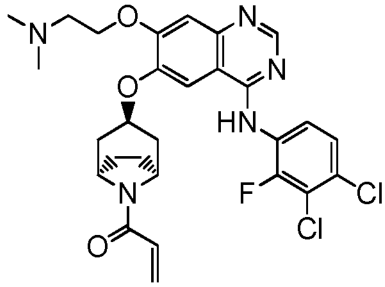

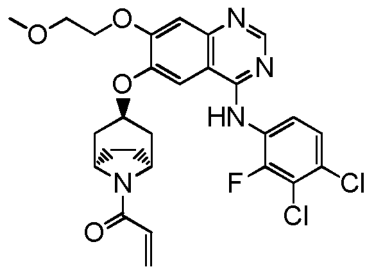

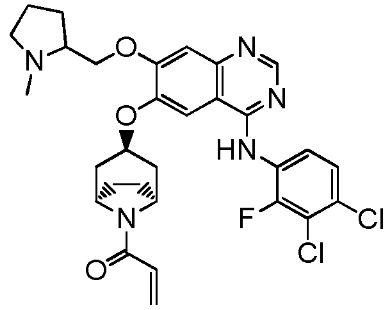

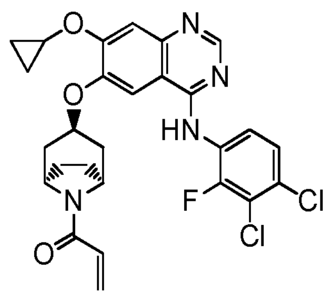

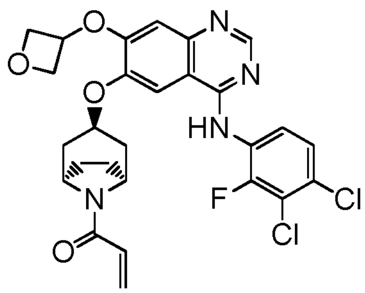

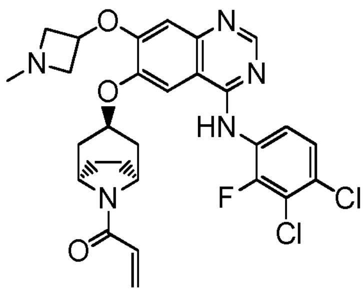

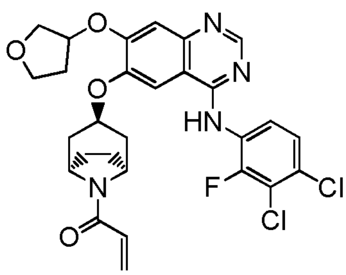

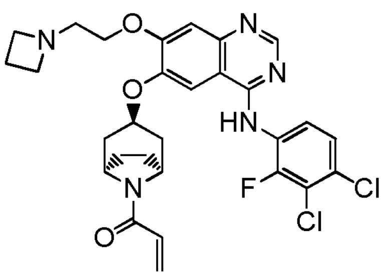

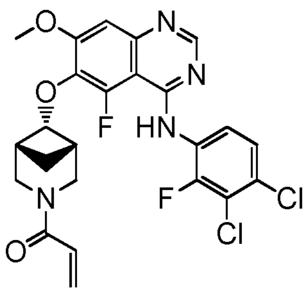

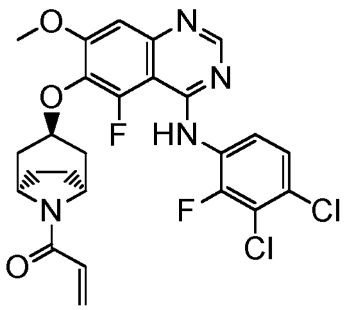

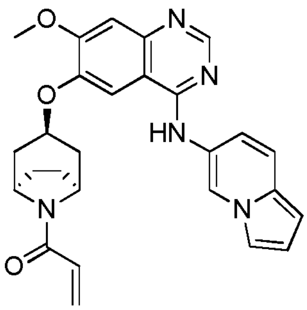

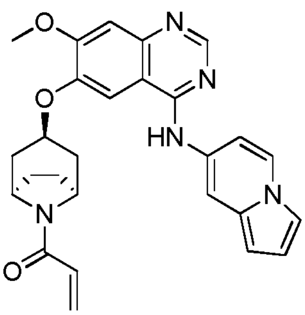

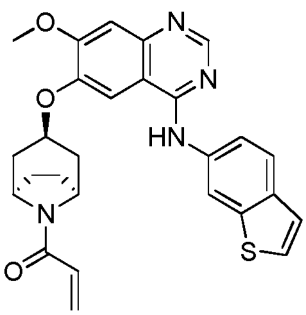

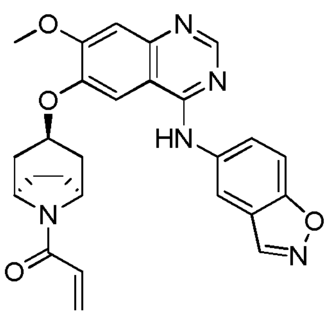

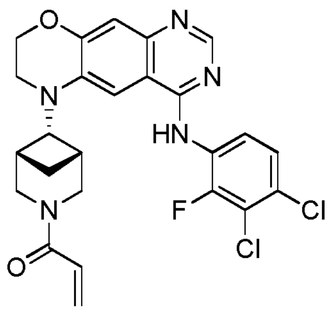
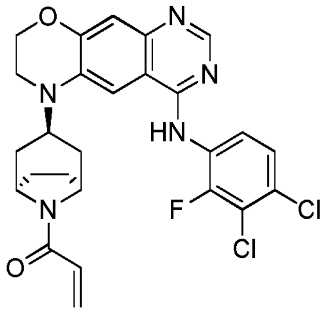
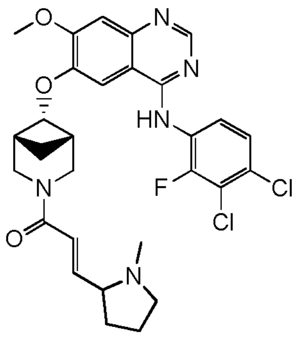

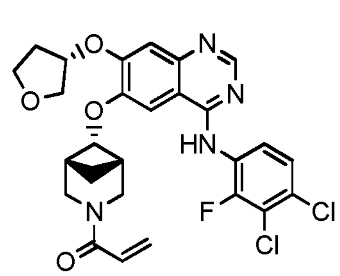
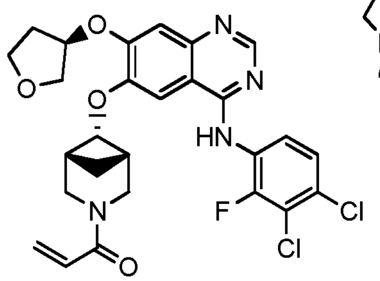
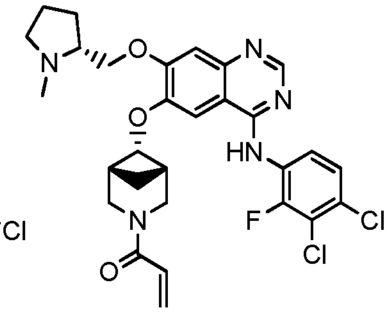

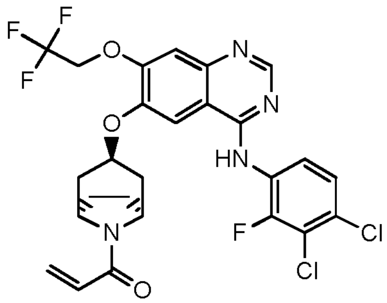
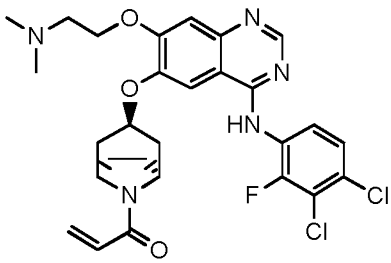
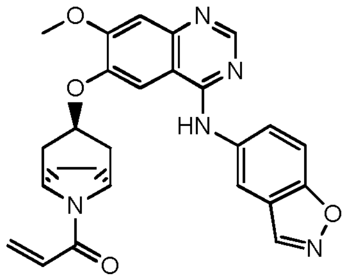

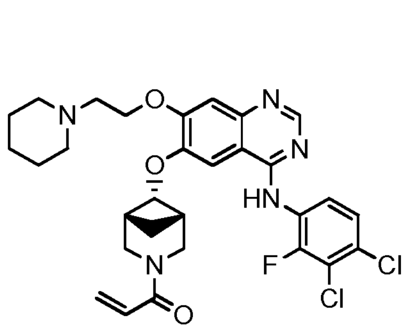
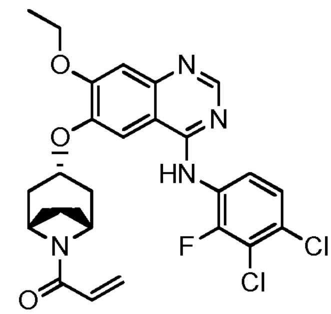
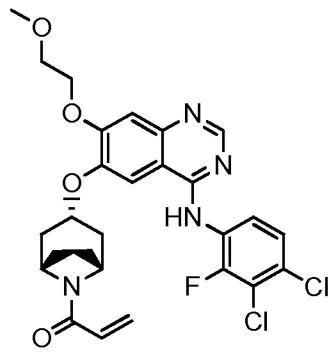

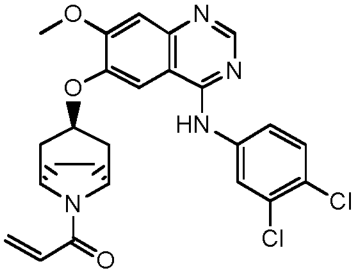
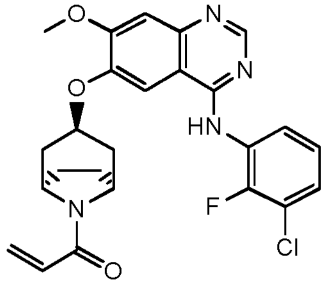
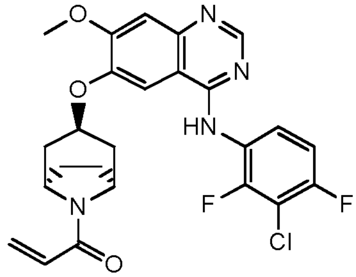

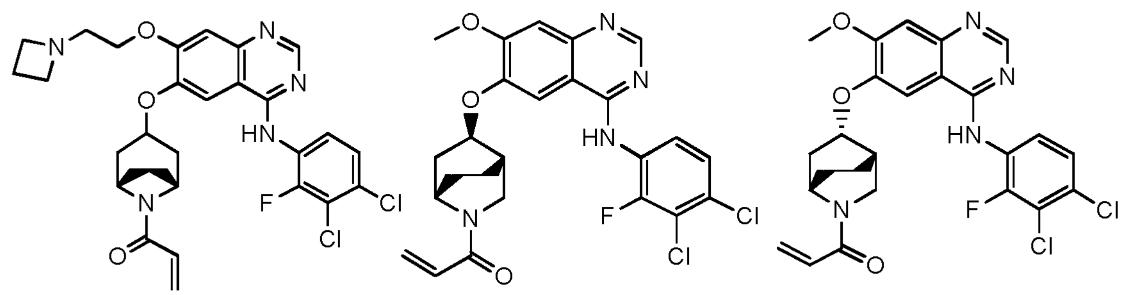

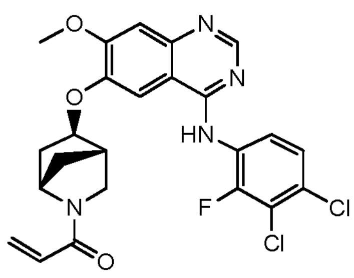

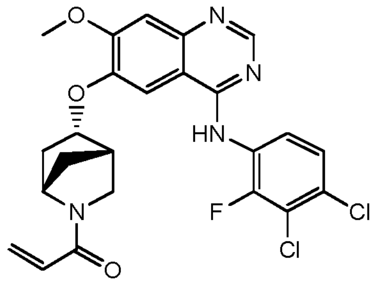

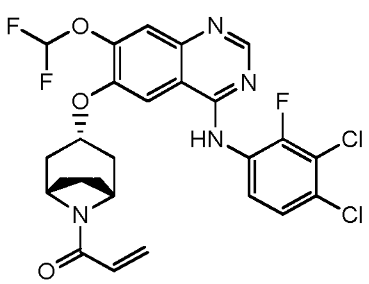

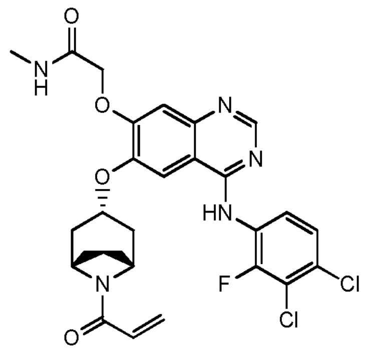

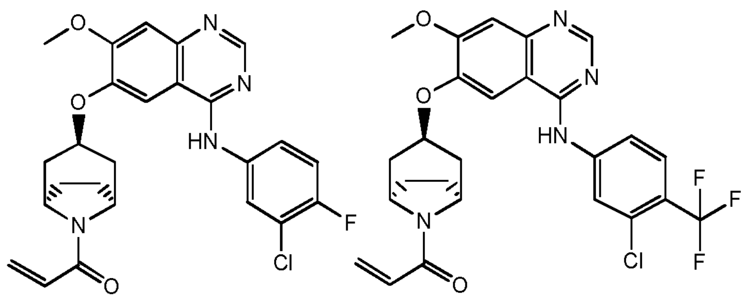

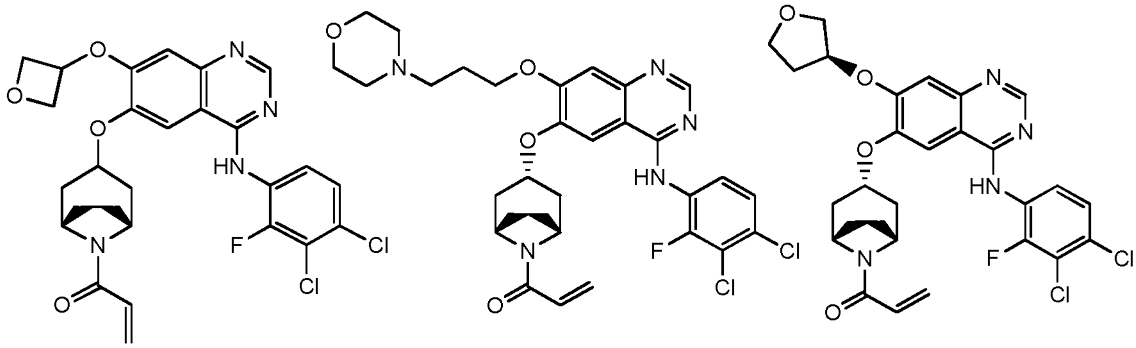

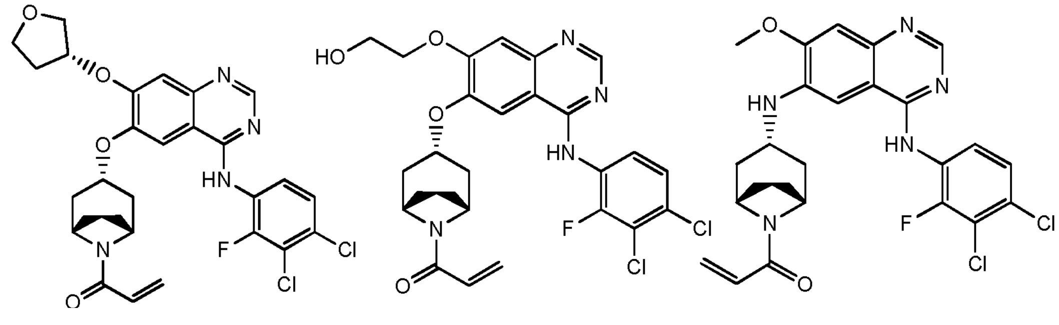

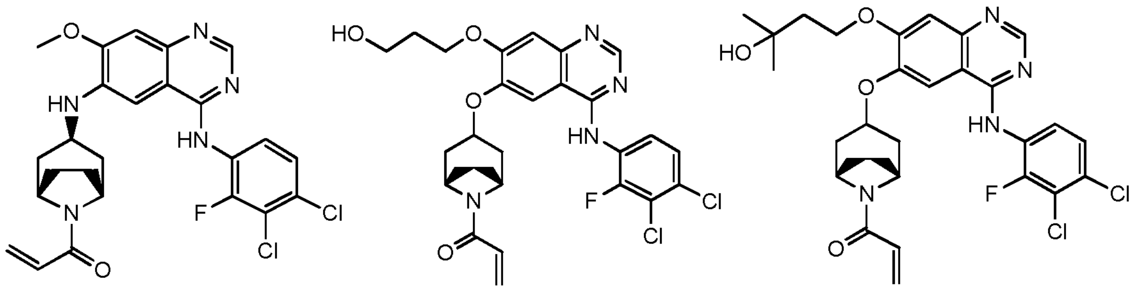

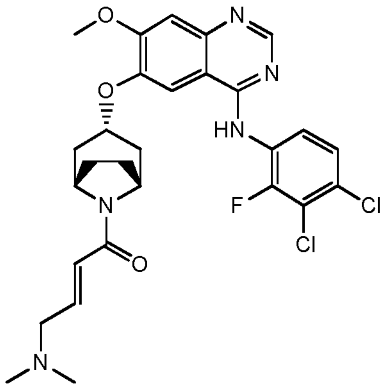

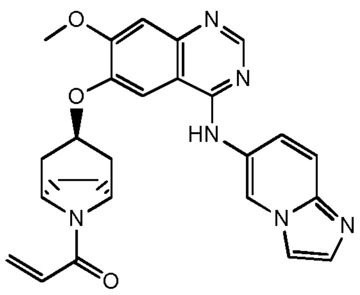

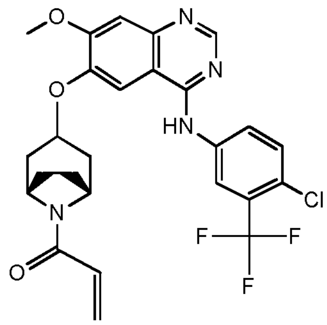

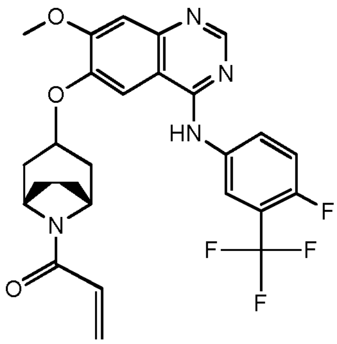

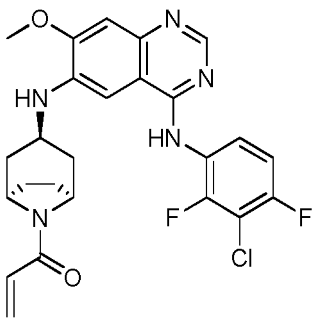

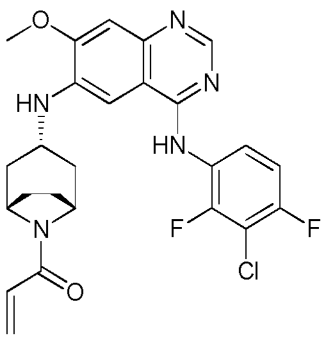

ausgewählt ist.

14. Pharmazeutische Zusammensetzung, umfassend eine therapeutisch wirksame Menge der Verbindung nach einem der Ansprüche 1 bis 13, ein Stereoisomer davon oder ein pharmazeutisch unbedenkliches Salz davon als Wirkstoff und einen oder mehrere pharmazeutisch unbedenkliche Träger.

15. Verbindung nach einem der Ansprüche 1 bis 13, ein Stereoisomer davon oder ein pharmazeutisch unbedenkliches Salz davon oder pharmazeutische Zusammensetzung nach Anspruch 14 zur Verwendung bei einem Verfahren zur Behandlung von Krebs, der mit Pan-HER-Tyrosinkinase assoziiert ist.

**Revendications**

1. Composé de formule (I), stéréoisomère correspondant ou sel pharmaceutiquement acceptable correspondant,

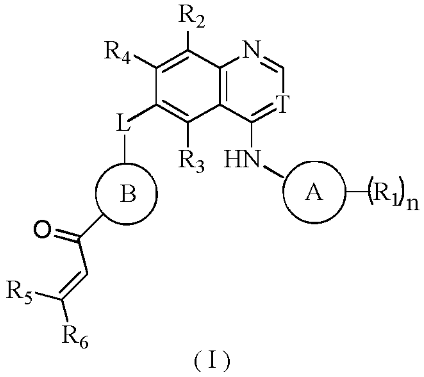

(I)

T étant choisi dans le groupe constitué par N et CR' ;

chaque $R_1$ étant indépendamment choisi dans le groupe constitué par H, F, Cl, Br, I, OH, $NH_2$, CN, $C_{1-3}$ alkyle et $C_{1-3}$ alcoxy ; le $C_{1-3}$ alkyle ou $C_{1-3}$ alcoxy étant éventuellement substitué par 1, 2 ou 3 substituants indépendamment choisis dans le groupe constitué par F, Cl, Br, I, OH, $NH_2$ et CN ;

$R_2$ et $R_3$ étant chacun indépendamment choisis dans le groupe constitué par H, F, Cl, Br, I, OH, $NH_2$ et $C_{1-3}$ alkyle, le $C_{1-3}$ alkyle étant éventuellement substitué par 1, 2 ou 3 substituants indépendamment choisis dans le groupe constitué par F, Cl, Br et I ;

$R_4$ étant choisi dans le groupe constitué par $C_{1-6}$ alkyle, $C_{1-6}$ alcoxy, hétérocycloalkyle à 3 à 7 chaînons-O-, hétérocycloalkyle à 3 à 7 chaînons-$C_{1-6}$ alkyl-O-, $C_{3-6}$ cycloalkyl-O- et $C_{3-6}$ cycloalkyl-$C_{1-6}$ alkyl-O-, les $C_{1-6}$ alkyle, $C_{1-6}$ alcoxy, hétérocycloalkyle à 3 à 7 chaînons-O-, hétérocycloalkyle à 3 à 7 chaînons-$C_{1-6}$ alkyl-O-, $C_{3-6}$ cycloalkyl-O- et $C_{3-6}$ cycloalkyl-$C_{1-6}$ alkyl-O- étant chacun indépendamment éventuellement substitués par 1, 2, ou 3 R ;

$R_5$ et $R_6$ étant chacun indépendamment choisis dans le groupe constitué par H, $C_{1-3}$ alkyle et hétérocycloalkyle à 3 à 7 chaînons, les $C_{1-3}$ alkyle et hétérocycloalkyle à 3 à 7 chaînons étant éventuellement substitués par 1, 2, ou 3 R ;

L étant choisi dans le groupe constitué par -O-, $-NR^a-$, $-CR^{b1}R^{b2}-$, $-CR^{b1}R^{b2}-O-$, et $-CR^{b1}R^{b2}-NH-$ ;

$R^a$ étant H ;

en variante, $R_4$ étant relié à $R^a$ pour former un groupe hétérocycloalkyle à 5 à 7 chaînons éventuellement substitué par 1, 2, 3 ou 4 R' ;

le cycle A étant choisi dans le groupe constitué par phényle et hétéroaryle à 5 à 10 chaînons ;

le cycle B étant choisi dans le groupe constitué par

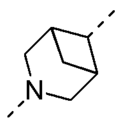, 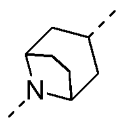, 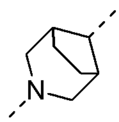, 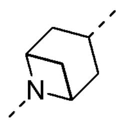, 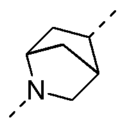, 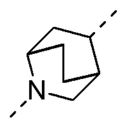, 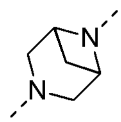,

et

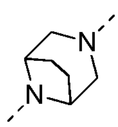 ;

n étant choisi dans le groupe constitué par 1, 2, 3, 4 et 5 ;

chaque R étant indépendamment choisi dans le groupe constitué par F, Cl, Br, I, OH, $NH_2$, CN, $NR^{c1}R^{c2}$, $C_{1-3}$ alkyle, $C_{1-3}$ alcoxy, $C_{1-3}$ alkyl-NH-C (=O) -, $C_{1-3}$ alkylthio et hétérocycloalkyle à 3 à 7 chaînons, les $C_{1-3}$ alkyle, $C_{1-3}$ alcoxy, $C_{1-3}$ alkyl-NH-C (=O) -, $C_{1-3}$ alkylthio et hétérocycloalkyle à 3 à 7 chaînons étant éventuellement substitués par 1, 2, ou 3 R' ;

R' étant chacun indépendamment choisi dans le groupe constitué par F, Cl, Br, I, OH, $NH_2$, CN et $C_{1-3}$ alkyle ;

$R^{b1}$ et $R^{b2}$ étant chacun indépendamment choisis dans le groupe constitué par H, F, Cl, Br, I et $C_{1-3}$ alkyle ;

$R^{c1}$ et $R^{c2}$ étant chacun indépendamment choisis dans le groupe constitué par H et $C_{1-3}$ alkyle ; l'hétérocycloalkyle à 5 à 7 chaînons, hétéroaryle à 5 à 10 chaînons et hétérocycloalkyle à 3 à 7 chaînons contenant

chacun 1, 2, 3 ou 4 hétéroatomes ou groupes d'hétéroatomes indépendamment choisis dans le groupe constitué par N, -O-, -S- et -NH-.

**2.** Composé selon la revendication 1, stéréoisomère correspondant ou sel pharmaceutiquement acceptable correspondant, chaque R étant indépendamment choisi dans le groupe constitué par F, Cl, Br, I, OH, $NH_2$, CN,

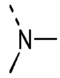 ,

Me , Et,

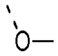 , 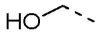 , 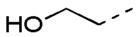 , 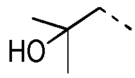 , 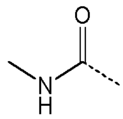 ,

pipéridinyle, pyrrolidinyle, azétidinyle, morpholinyle et oxétanyle, les Me, Et, 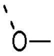, pipéridinyle, pyrrolidinyle, azétidinyle, morpholinyle et oxétanyle étant éventuellement substitués par 1, 2 ou 3 substituants indépendamment choisis dans le groupe constitué par F, Cl, Br, I, OH, $NH_2$ et $C_{1-3}$ alkyle ; préférablement,
chaque R étant indépendamment choisi dans le groupe constitué par F, Cl, Br, I, OH, $NH_2$, CN,

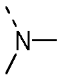 ,

Me, Et,

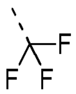 , 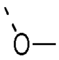 , 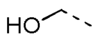 , 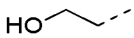 , 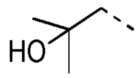 , 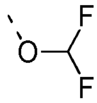 , 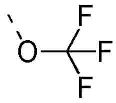 , 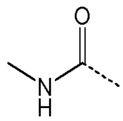

, 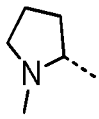 , 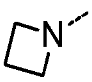 , 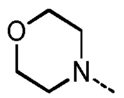 et 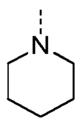 .

**3.** Composé selon la revendication 1 ou 2, stéréoisomère correspondant ou sel pharmaceutiquement acceptable correspondant, chaque $R_2$ étant indépendamment choisi dans le groupe constitué par H, F, Cl, Br, I, OH, $NH_2$, CN,

Me et 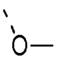, les Me et 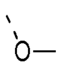 étant éventuellement substitués par 1, 2 ou 3 substituants indépendamment choisis dans le groupe constitué par F, Cl, Br, I, OH, $NH_2$ et CN ; préférablement,
chaque $R_1$ étant indépendamment choisi dans le groupe constitué par H, F, Cl, Br, I, OH, $NH_2$, CN, Me, $CH_2F$, $CHF_2$, $CF_3$,

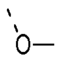 et 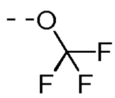 .

**4.** Composé selon l'une quelconque des revendications 1 à 3, stéréoisomère correspondant ou sel pharmaceutique-

ment acceptable correspondant, $R_2$ et $R_3$ étant chacun indépendamment choisis dans le groupe constitué par H, F, Cl, Br, I, OH, $NH_2$, Me et $CF_3$.

5. Composé selon l'une quelconque des revendications 1 à 4, stéréoisomère correspondant ou sel pharmaceutiquement acceptable correspondant, $R_4$ étant choisi dans le groupe constitué par

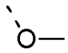, 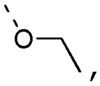, 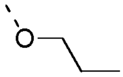,

pipéridinyl-$C_{1-3}$ alkyl-O-, oxétanyl-0-, morpholinyl-$C_{1-3}$ alkyl-O-, azétidinyl-O-, tétrahydrofuranyl-O-, cyclopropyl-O-, pyrrolidinyl-$C_{1-3}$ alkyl-O- et azétidinyl-$C_{1-3}$ alkyl-O-, les

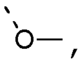, 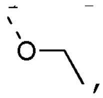,

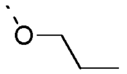,

pipéridinyl-$C_{1-3}$ alkyl-O-, oxétanyl-0-, morpholinyl-$C_{1-3}$ alkyl-O-, azétidinyl-O-, tétrahydrofuranyl-O-, cyclopropyl-O-, pyrrolidinyl-$C_{1-3}$ alkyl-O- et azétidinyl-$C_{1-3}$ alkyl-O- étant éventuellement substitués par 1, 2, ou 3 R ; préférablement,
$R_4$ étant choisi dans le groupe constitué par

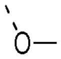, 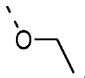,

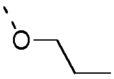, 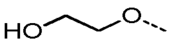, 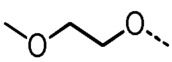, 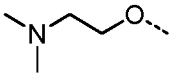, 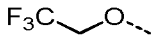,

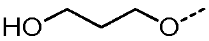, 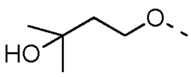, 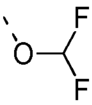, 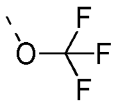, 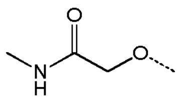,

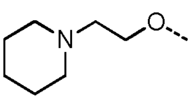, 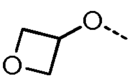, 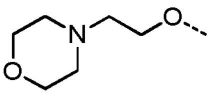, 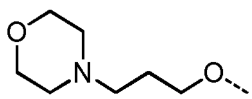, 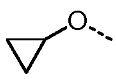

, 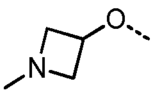, 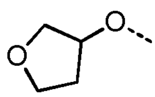, 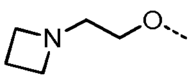 et 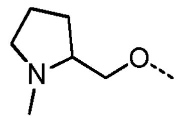.

6. Composé selon l'une quelconque des revendications 1 à 5, stéréoisomère correspondant ou sel pharmaceutiquement acceptable correspondant, $R_5$ et $R_6$ étant chacun indépendamment choisis dans le groupe constitué par H, Me, Et et

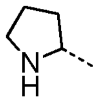,

les Me, Et et

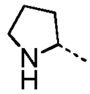

étant éventuellement substitués par 1, 2, ou 3 R ;
préférablement,
$R_5$ et $R_6$ étant chacun indépendamment choisis dans le groupe constitué par H,

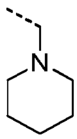, 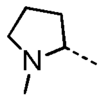 et 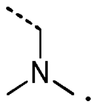.

7. Composé selon l'une quelconque des revendications 1 à 6, stéréoisomère correspondant ou sel pharmaceutiquement acceptable correspondant, T étant choisi dans le groupe constitué par N et C(CN).

8. Composé selon l'une quelconque des revendications 1 à 7, stéréoisomère correspondant ou sel pharmaceutiquement acceptable correspondant, L étant choisi dans le groupe constitué par -O-, -NH-, $-CH_2-$, $-CH_2-O-$ et $-CH_2-NH-$.

9. Composé selon l'une quelconque des revendications 1 à 8, stéréoisomère correspondant ou sel pharmaceutiquement acceptable correspondant, le groupement

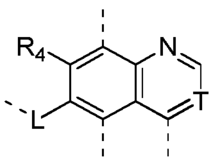

étant choisi dans le groupe constitué par

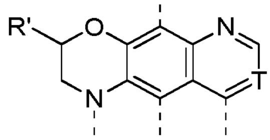,

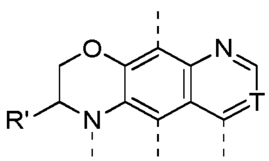, 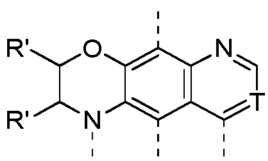, 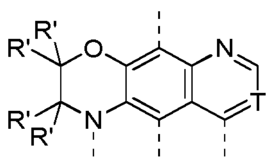

et

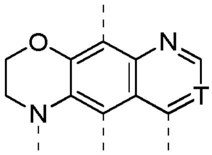

;

préférablement, le groupement

,

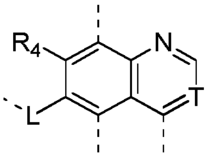

étant

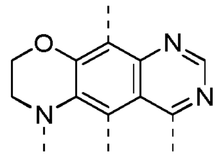

.

**10.** Composé selon l'une quelconque des revendications 1 à 9, stéréoisomère correspondant ou sel pharmaceutiquement acceptable correspondant, le cycle A étant choisi dans le groupe constitué par phényle, azaindényle, benzo[b]thiényle, imidazo[1,2-a]pyridinyle et benzo[d]isoxazolyle ;
préférablement,

le groupement

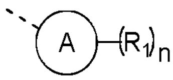

étant choisi dans le groupe constitué par

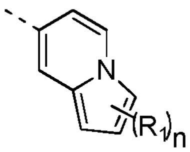 , 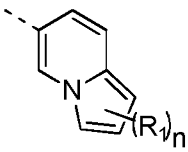 , 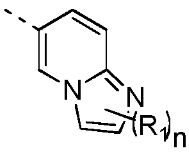 ,

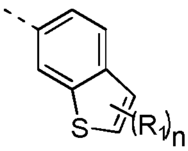 , 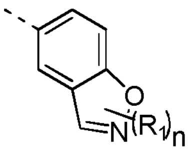 et 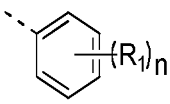 ;

préférablement,
le groupement

,

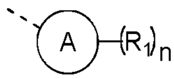

étant choisi dans le groupe constitué par

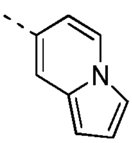 , 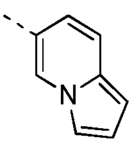 , 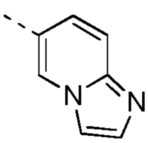 , 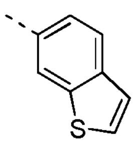 ,

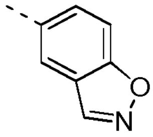 , 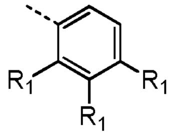 , 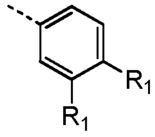 et 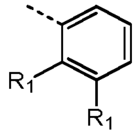 ;

préférablement,
le groupement

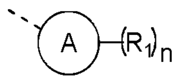

étant choisi dans le groupe constitué par

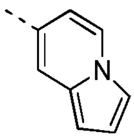 , 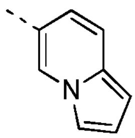 , 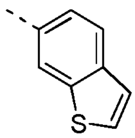 , 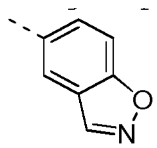 ,

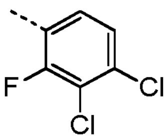 , 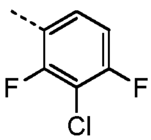 , 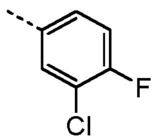 , 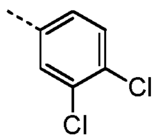 , 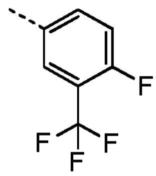 ,

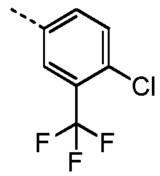 , 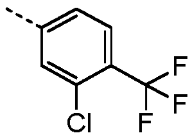 , 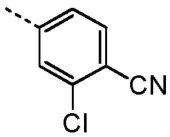 , 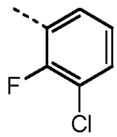 et 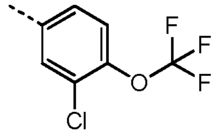 .

**11.** Composé selon l'une quelconque des revendications 1 à 10, stéréoisomère correspondant ou sel pharmaceutiquement acceptable correspondant, le cycle B étant choisi dans le groupe constitué par

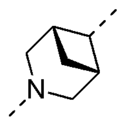 , 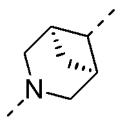 , 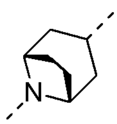 , 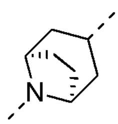 , 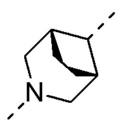 , 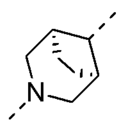 , 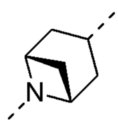 , 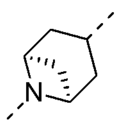 , 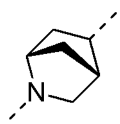 , 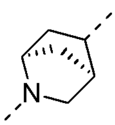 , 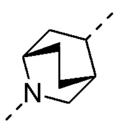 , 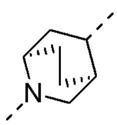 , 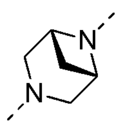 , 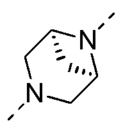 , 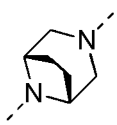

et

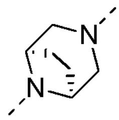;

préférablement, le groupement

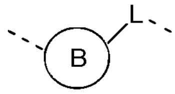

étant choisi dans le groupe constitué par

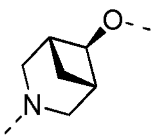, 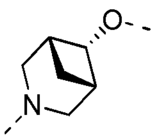, 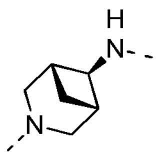, 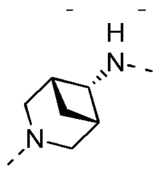,

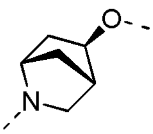, 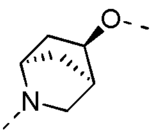, 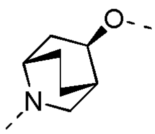, 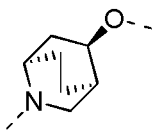, 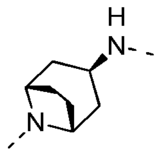,

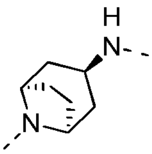, 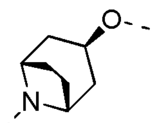 et 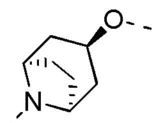.

**12.** Composé selon l'une quelconque des revendications 1 à 11, stéréoisomère correspondant ou sel pharmaceutiquement acceptable correspondant, le composé étant choisi dans le groupe constitué par :

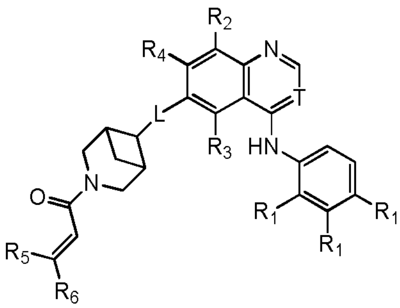

( II -1) ,

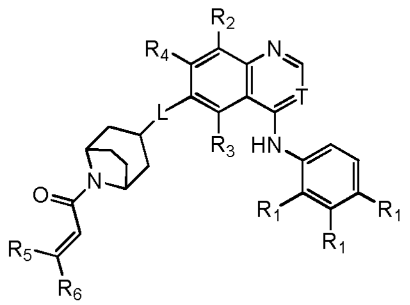

( II -2) ,

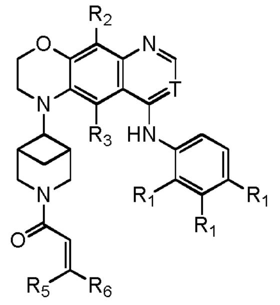

(II -3) ,

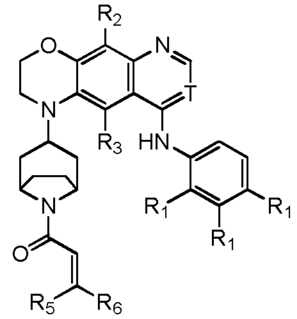

( II -4) ,

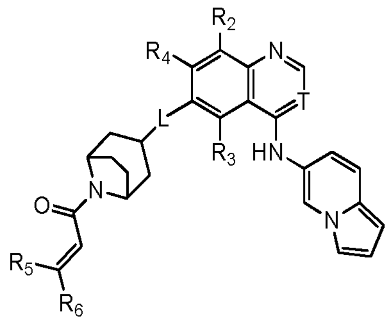

(II -5) ,

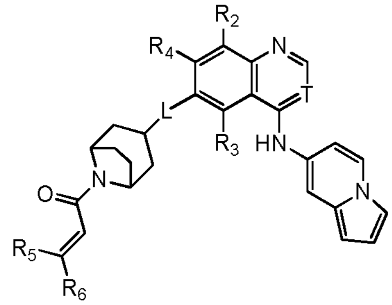

(II -6) ,

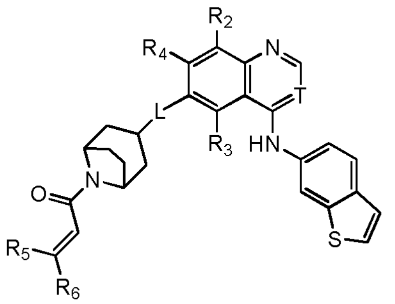

(II -7) ,

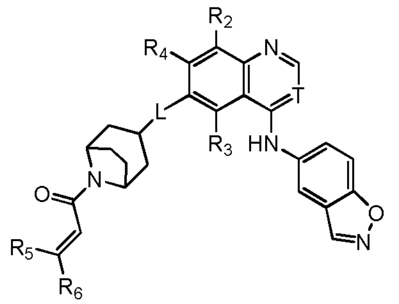

(II -8) ,

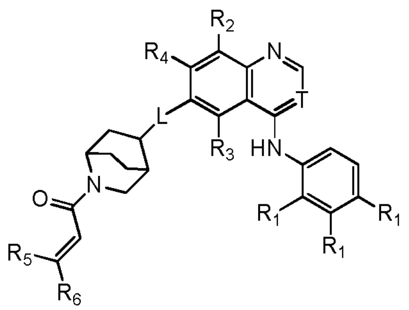

(II -9) ,

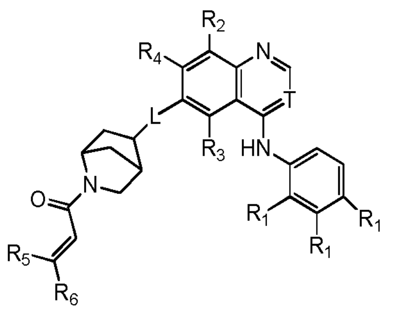

(II -10)

et

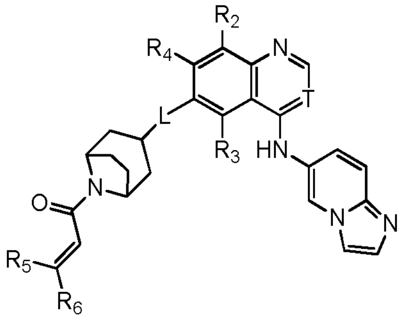

(II -11) ,

T, L, $R_1$, $R_2$, $R_3$, $R_4$, $R_5$ et $R_6$ étant tels que définis dans les revendications 1 à 11 ; préférablement,
le composé étant choisi dans le groupe constitué par :

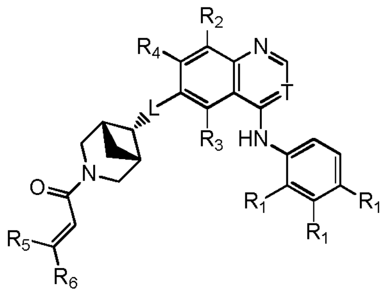

( III -1) ,

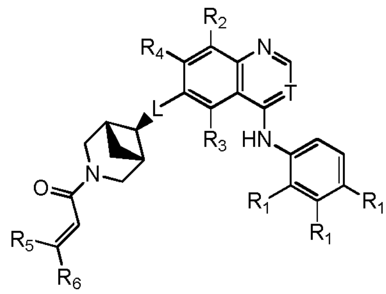

( III -2) ,

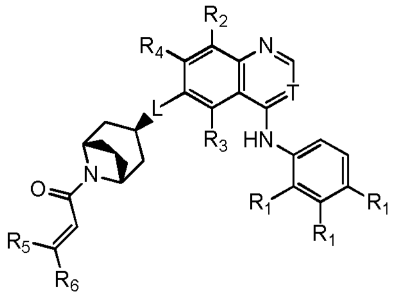

( III -3) ,

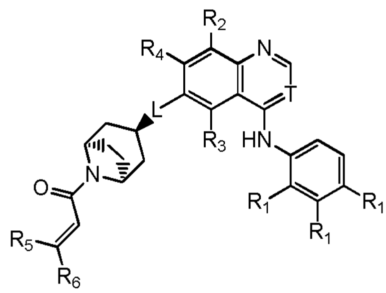

( III -4) ,

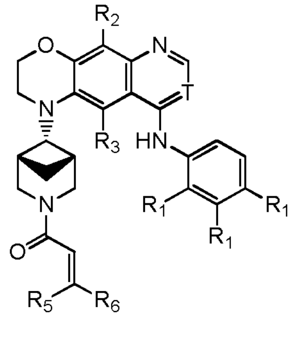

(III -5) ,

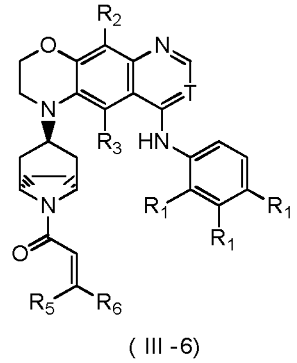

( III -6) ,

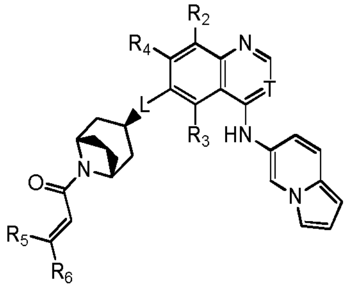

(III -7) ,

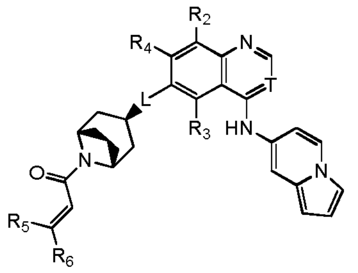

(III -8) ,

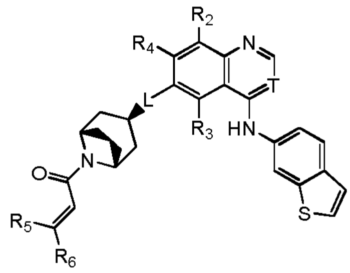

(III -9) ,

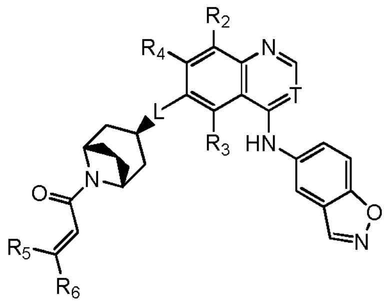

(III -10) ,

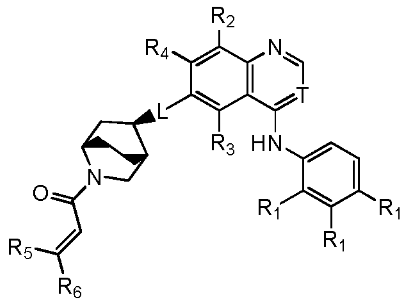

(III -11) ,

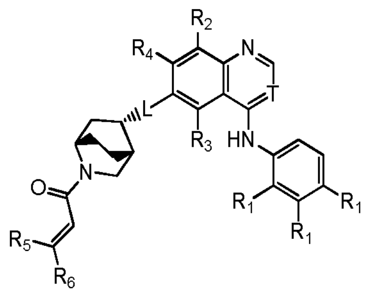

(III -12) ,

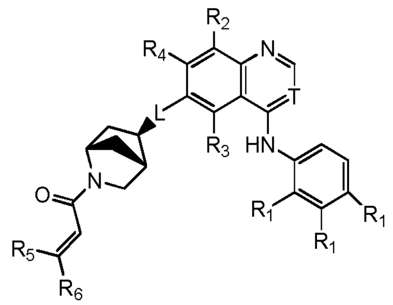

(III -13) ,

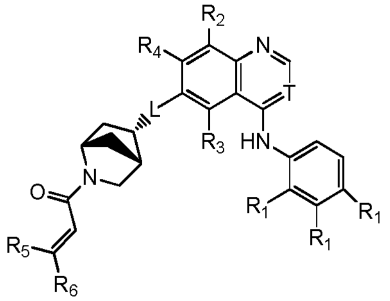

(III -14) ,

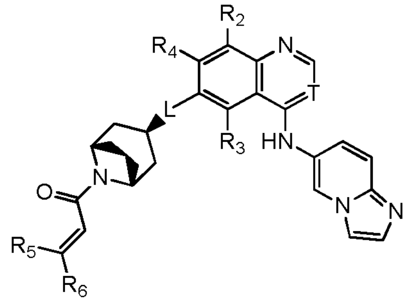

(III -15) ,

et

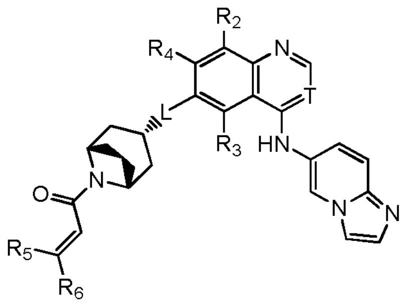

(III -16) ,

T, L, $R_1$, $R_2$, $R_3$, $R_4$, $R_5$ et $R_6$ étant tels que définis dans les revendications 1 à 11.

**13.** Composé selon la revendication 1, stéréoisomère correspondant ou sel pharmaceutiquement acceptable correspondant, le composé étant choisi dans le groupe constitué par :

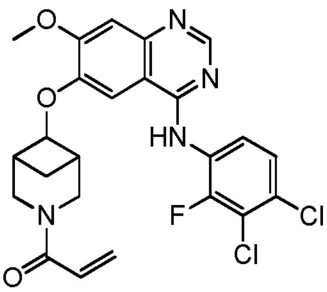
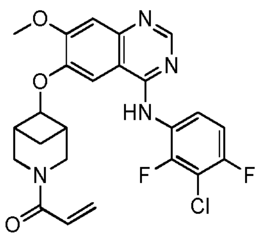
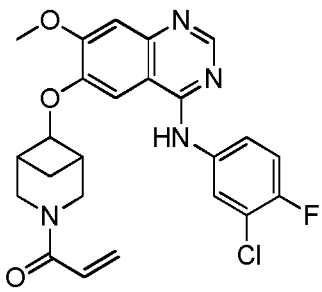

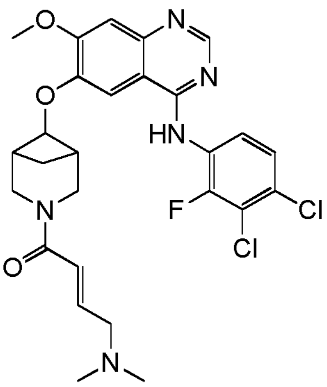
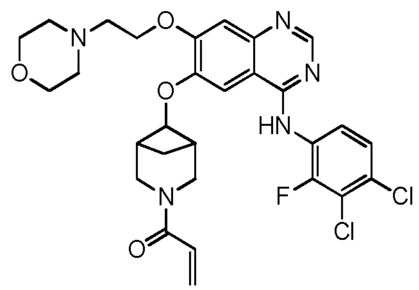

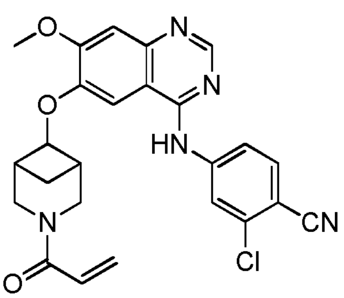
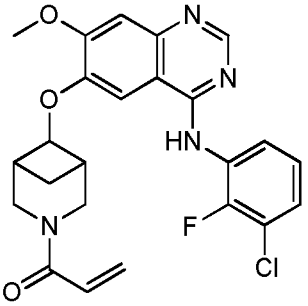
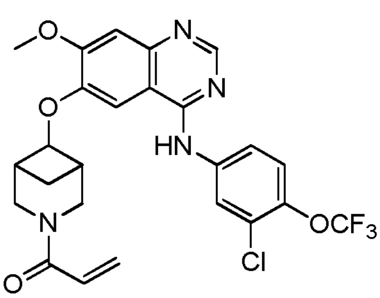

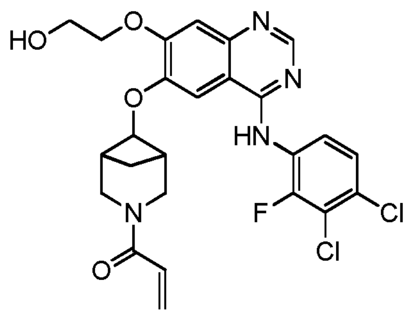

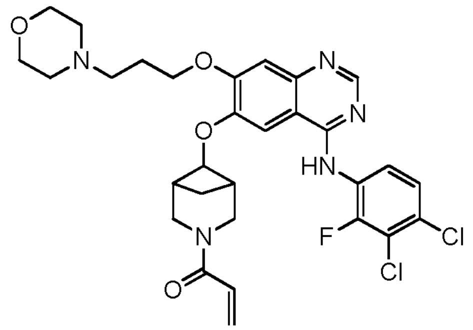

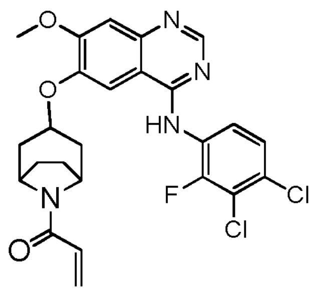

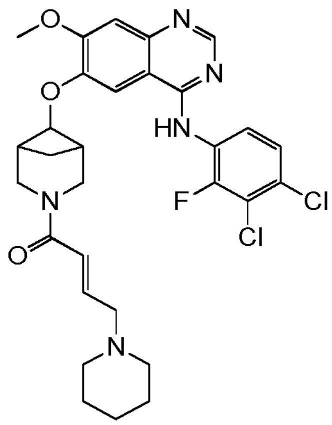

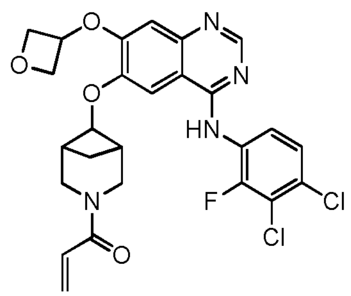

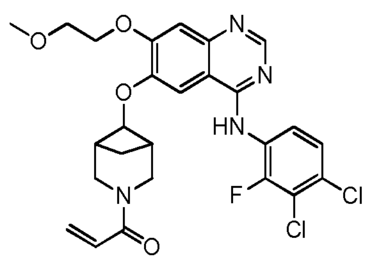

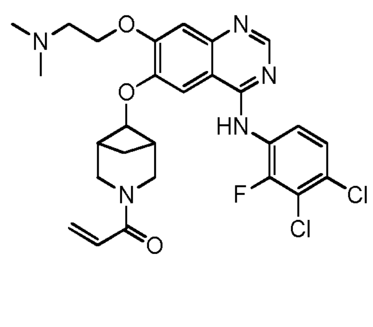

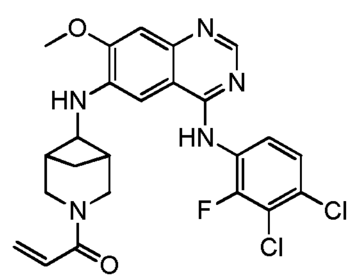

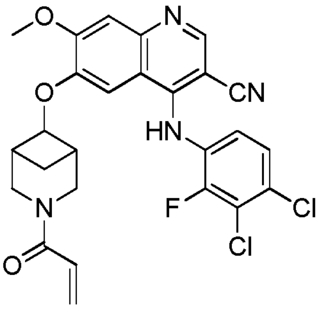

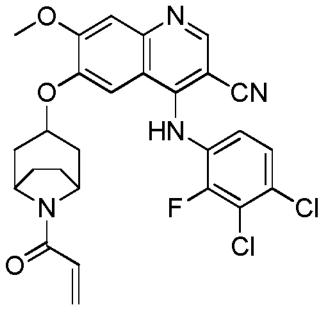

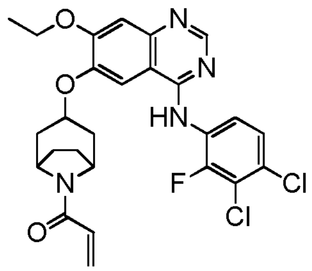

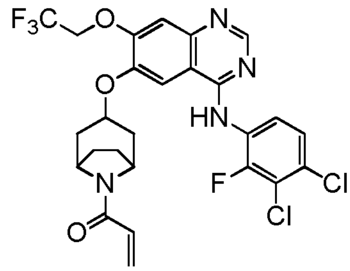

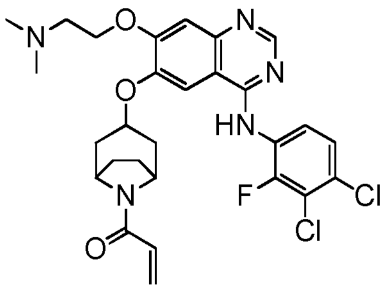

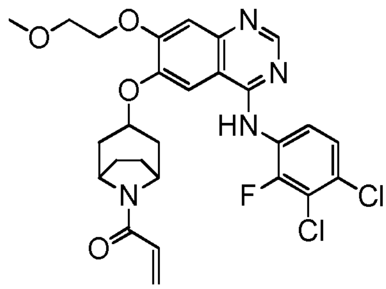

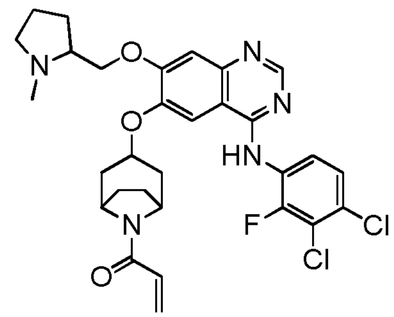

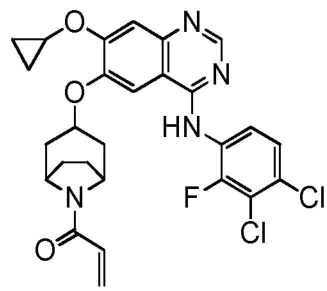

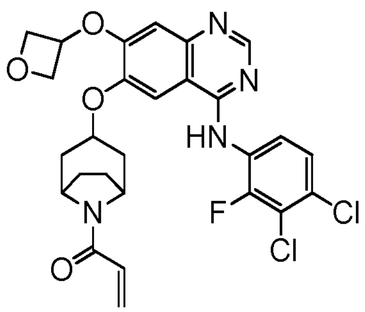

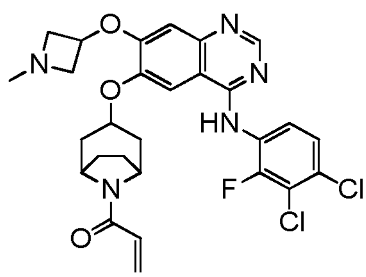

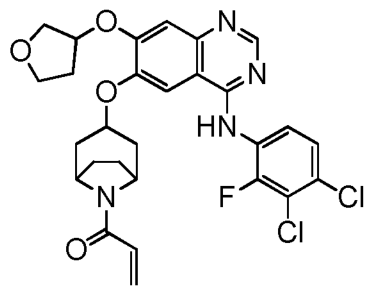

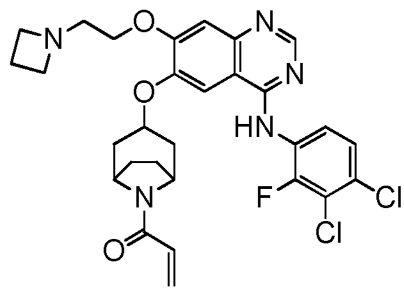

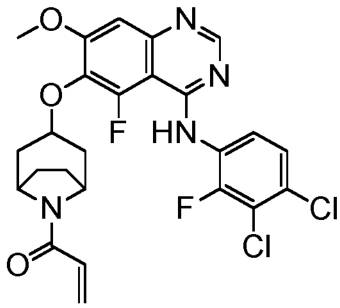

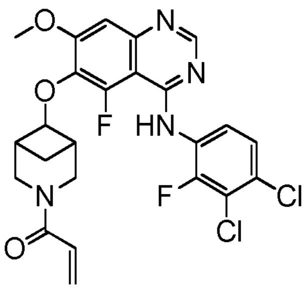

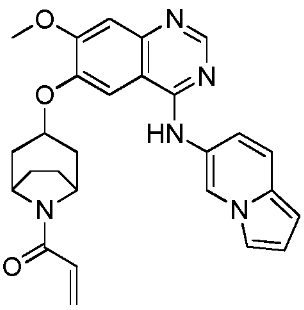

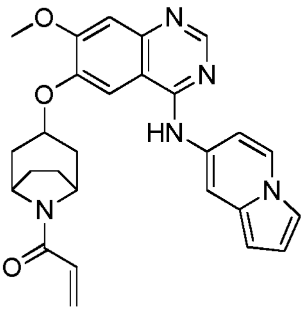

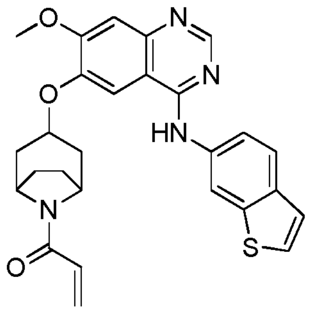

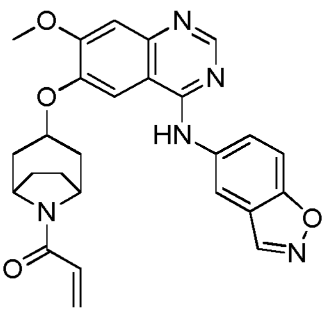

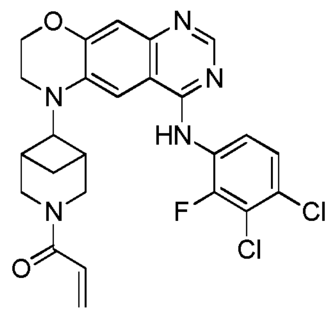

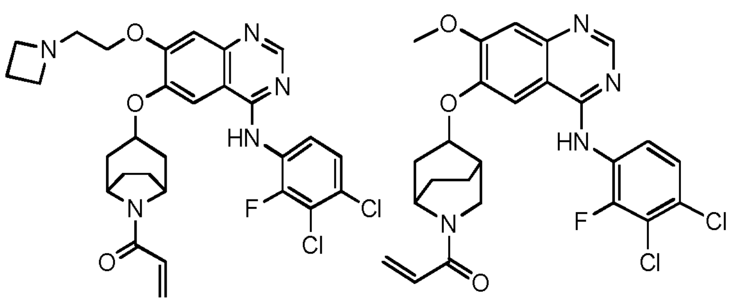

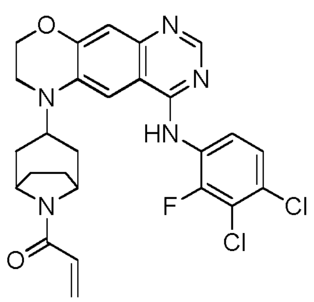

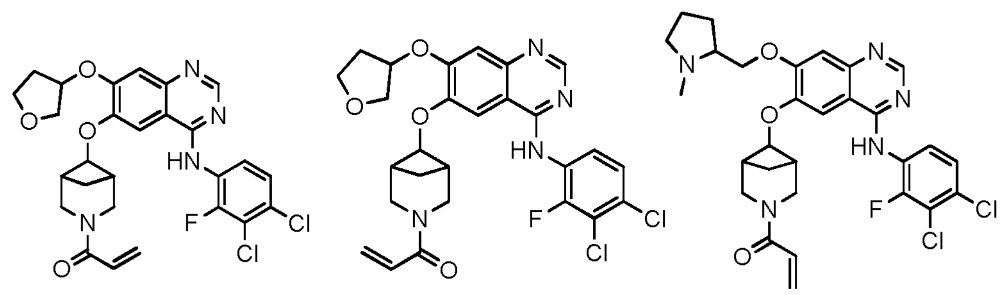

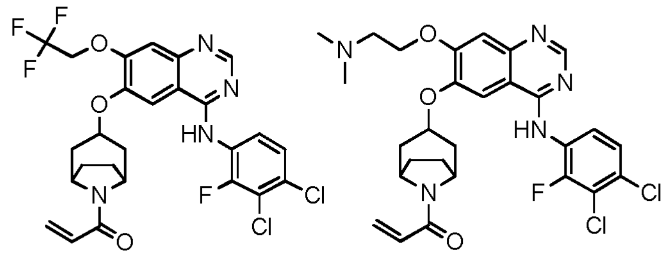

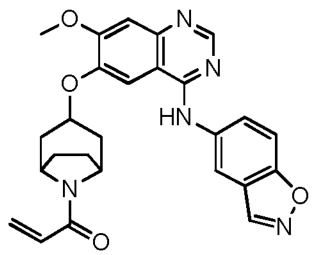

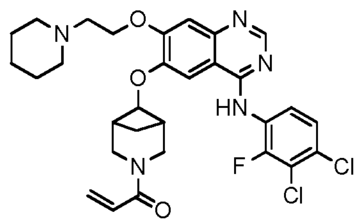

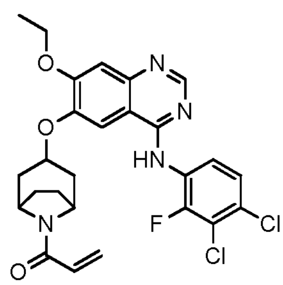

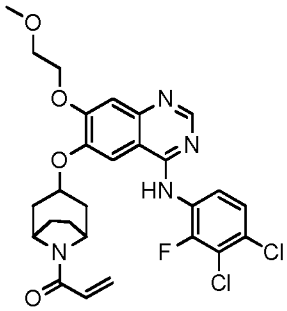

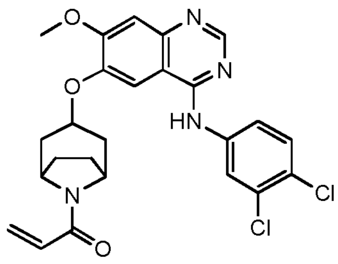

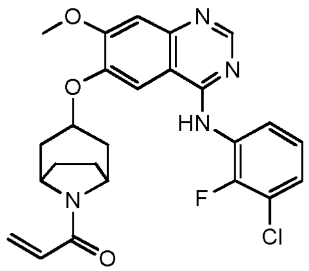

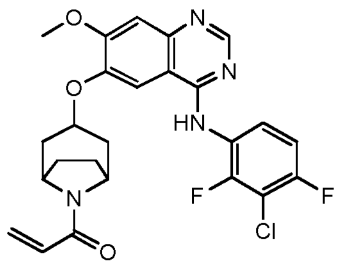

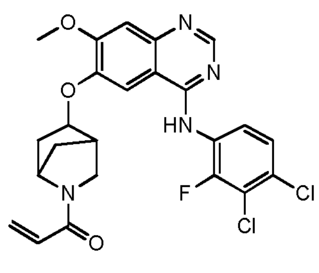

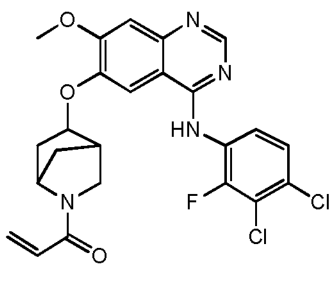

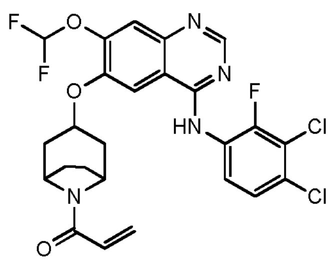

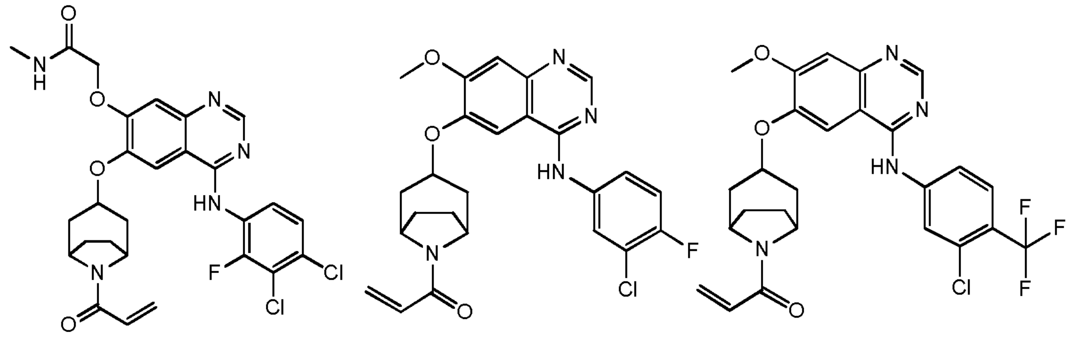

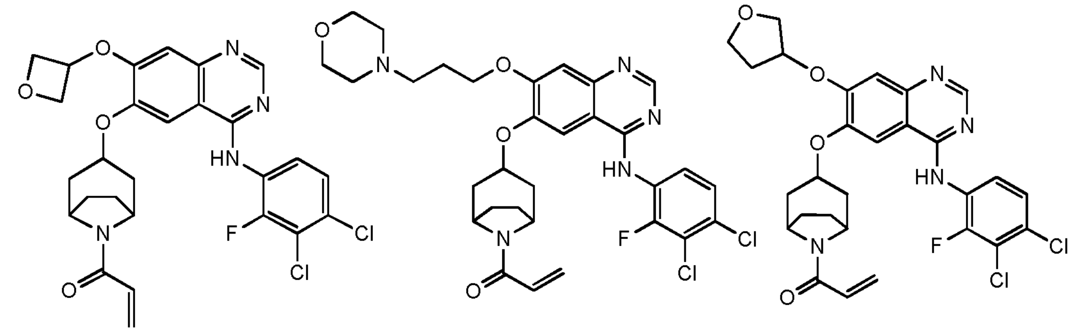

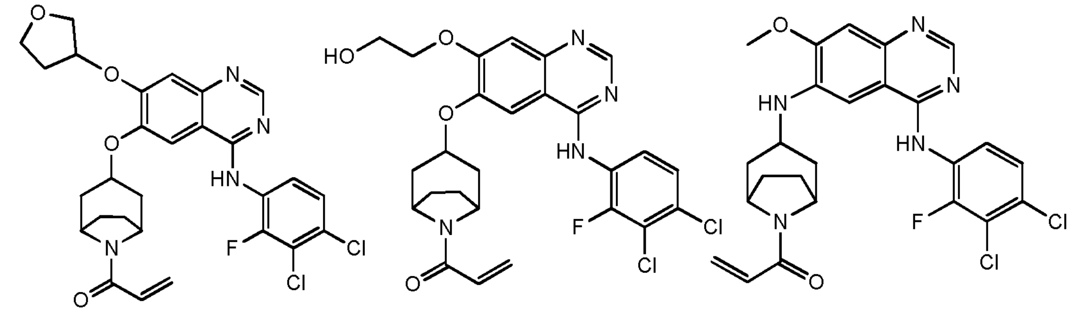

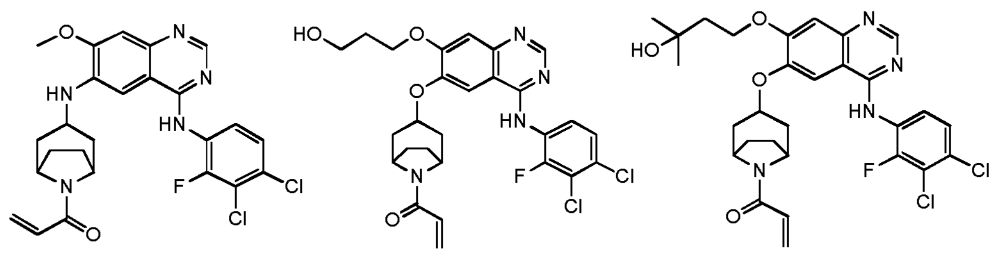

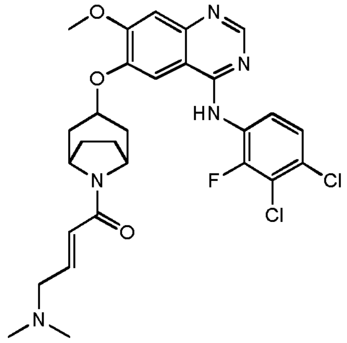

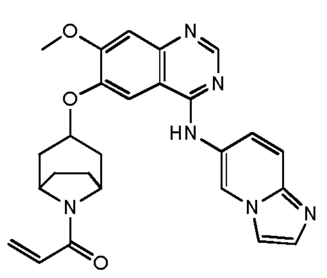

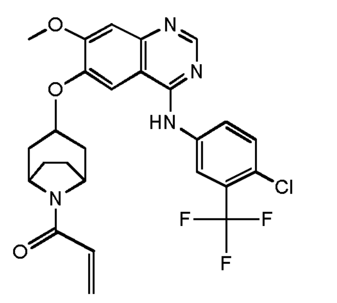

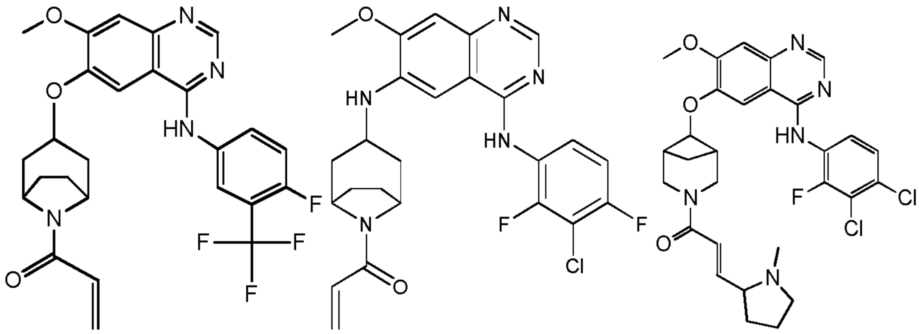

;

préférablement,
le composé étant choisi dans le groupe constitué par :

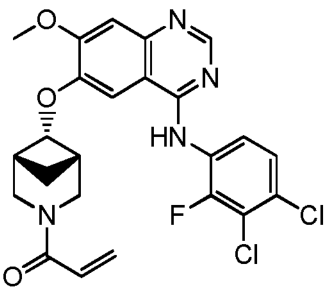

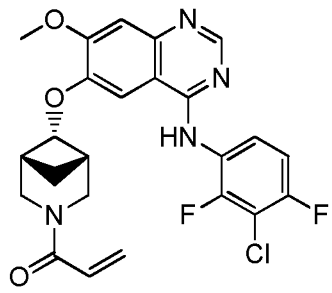

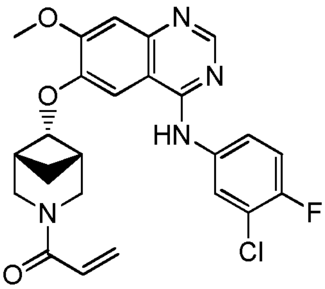

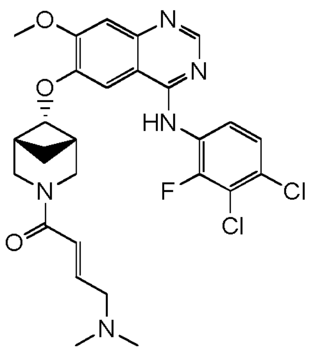

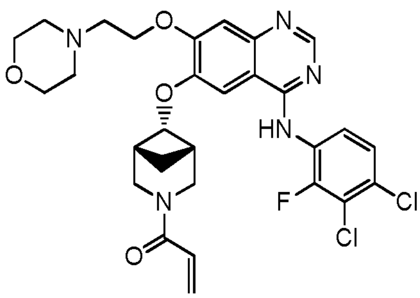

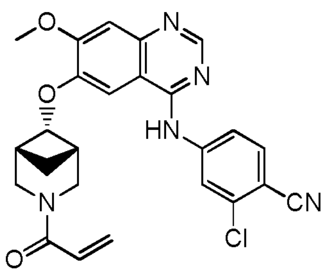

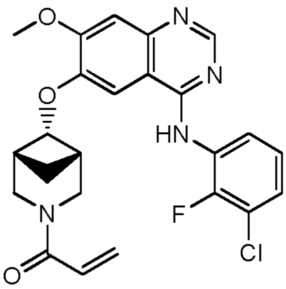

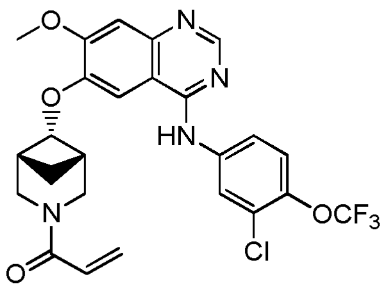

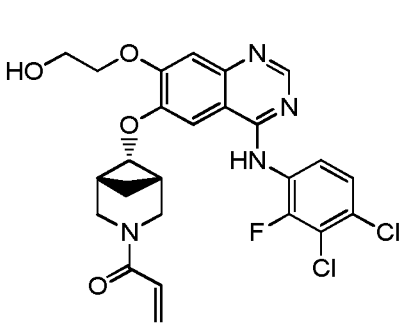

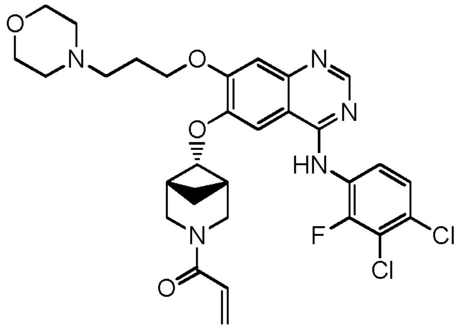

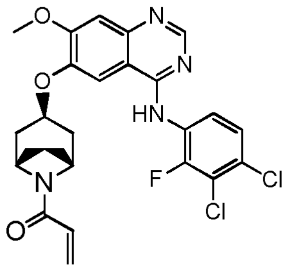

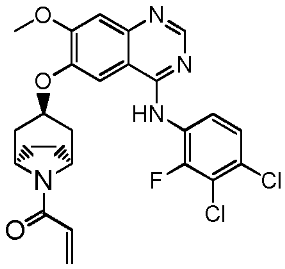

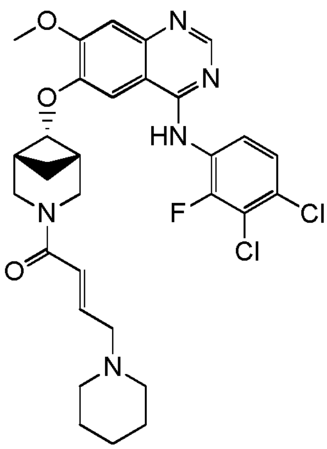

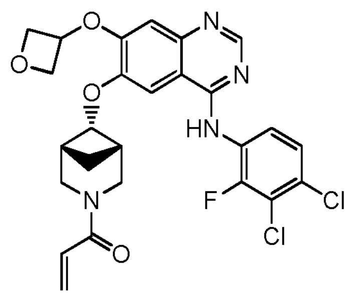

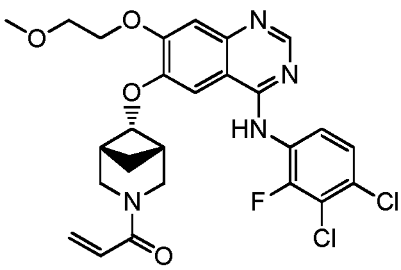

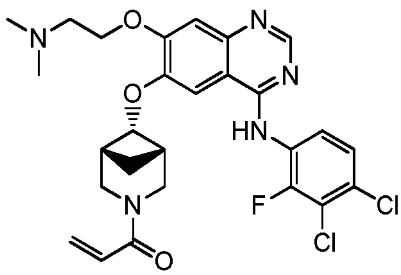

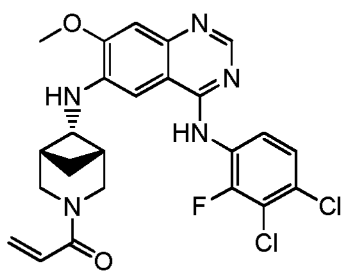

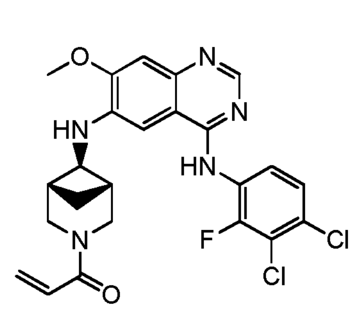
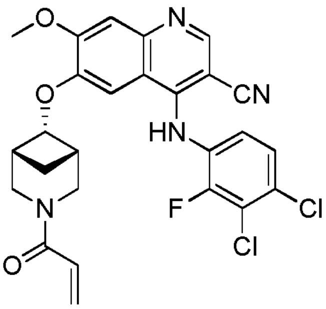
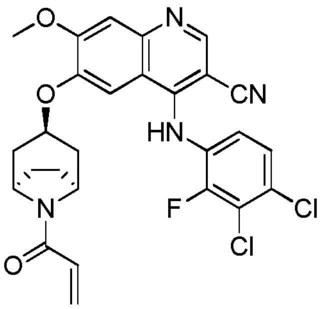

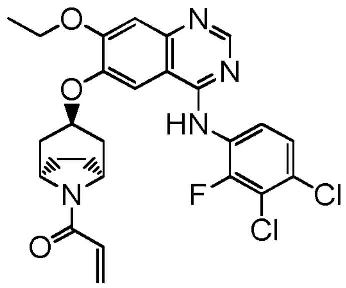

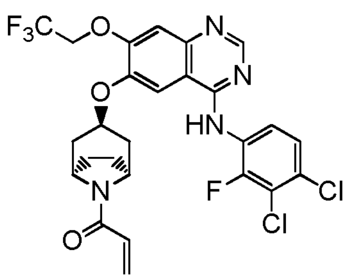
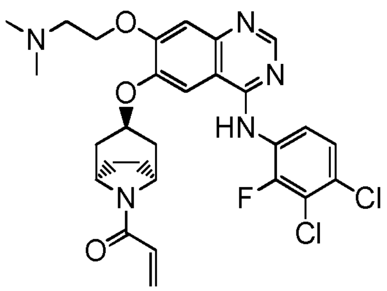

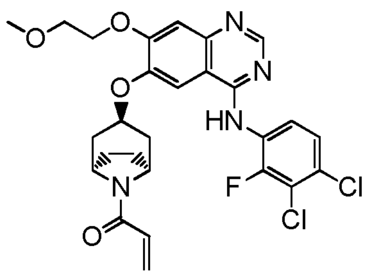

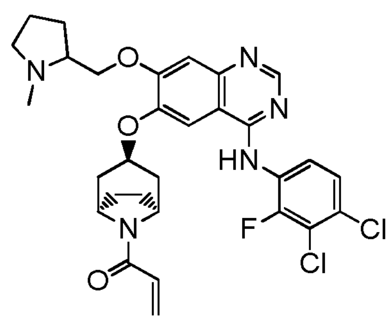
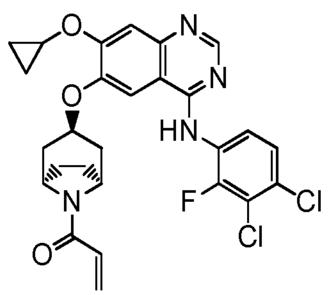

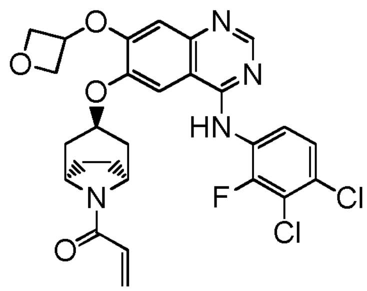

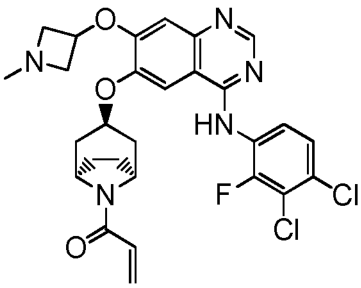

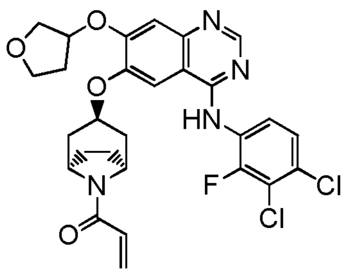

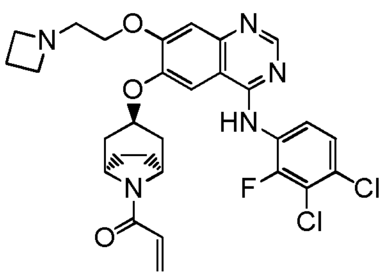

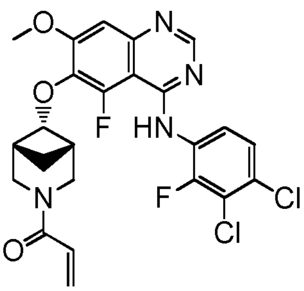

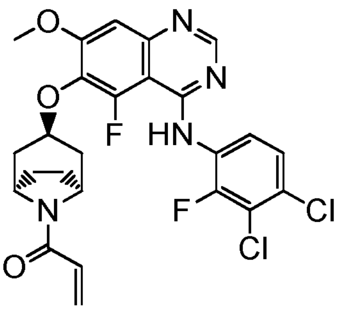

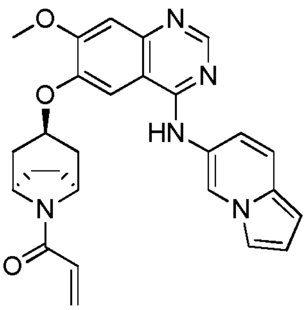

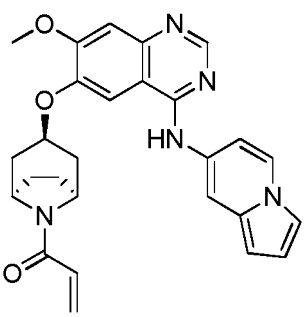

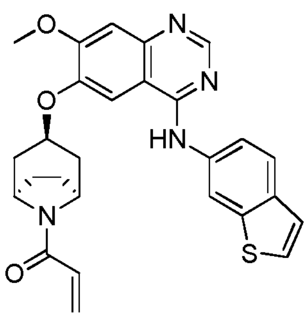

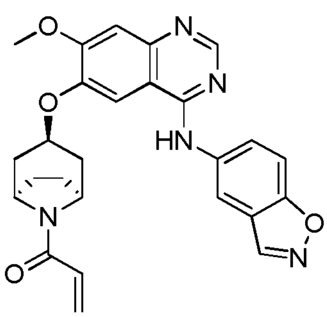

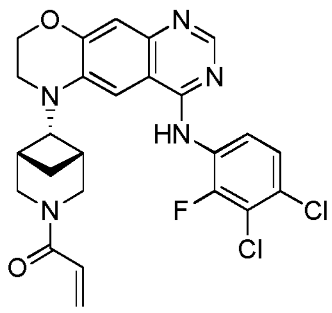
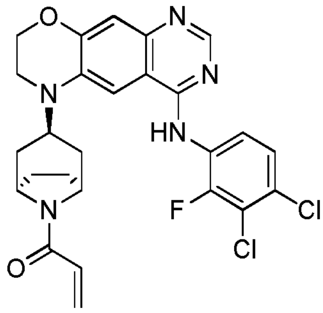
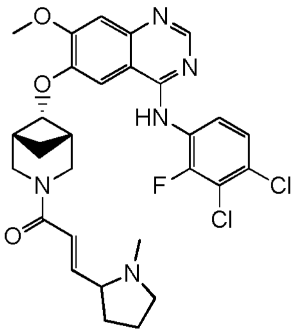

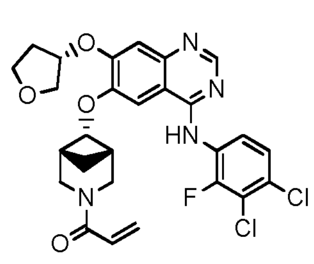
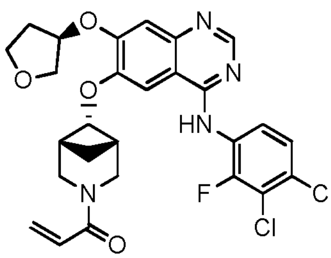
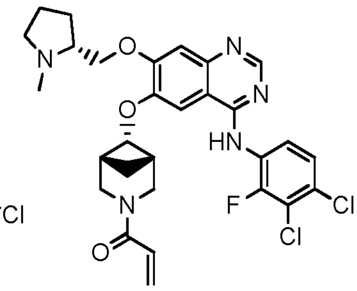

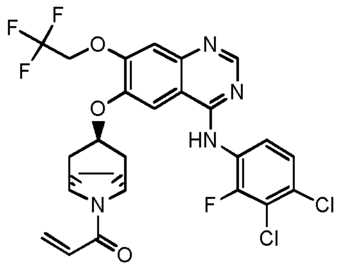
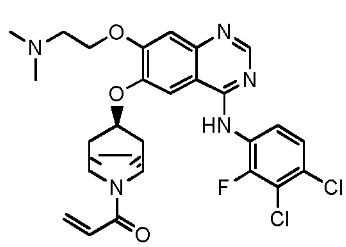
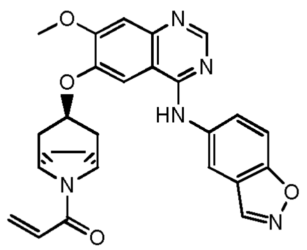

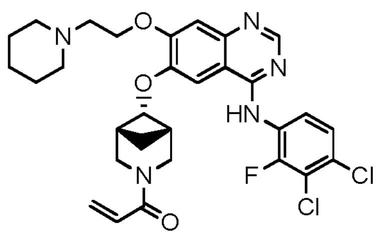
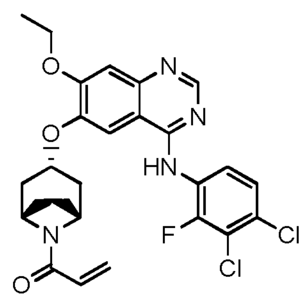
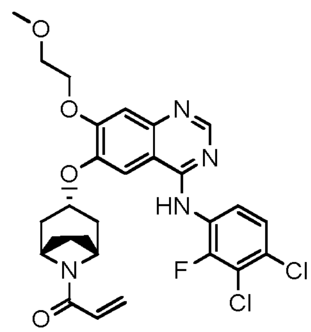

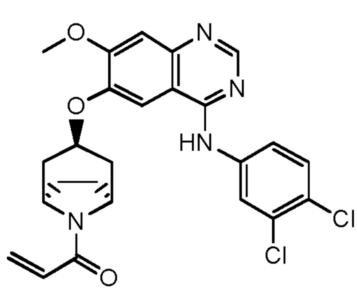
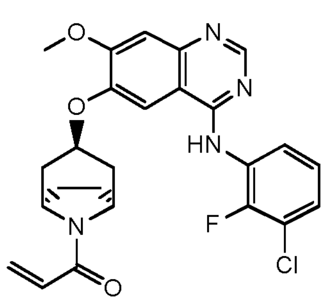
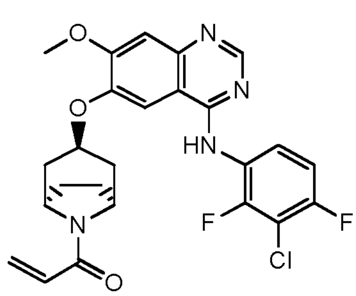

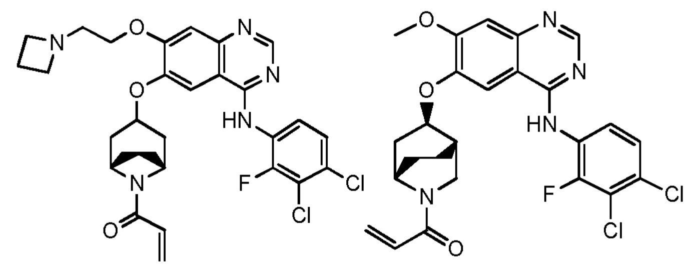

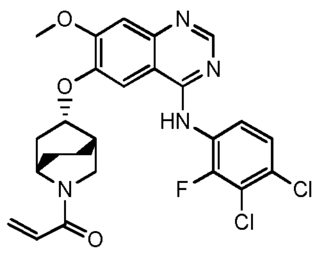

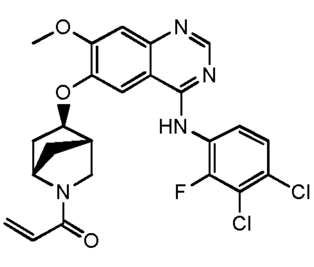

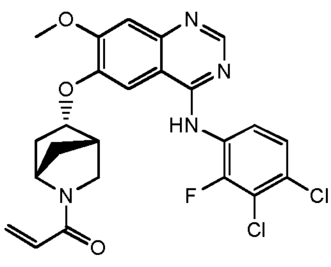

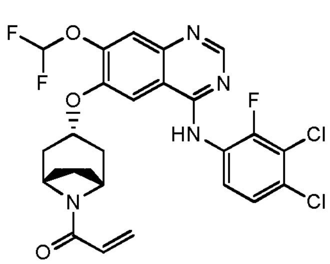

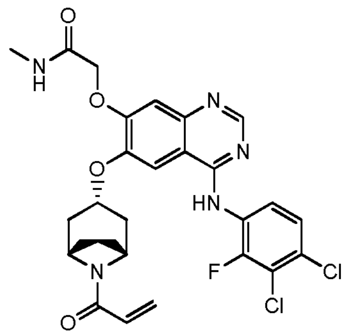

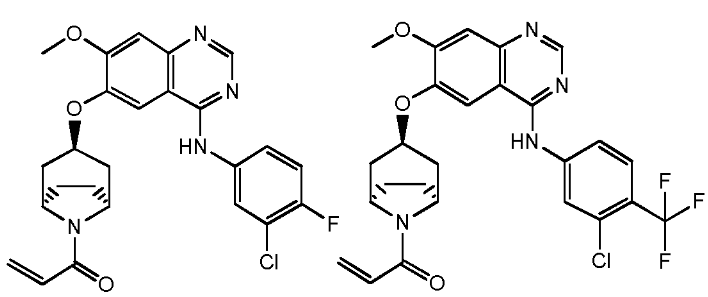

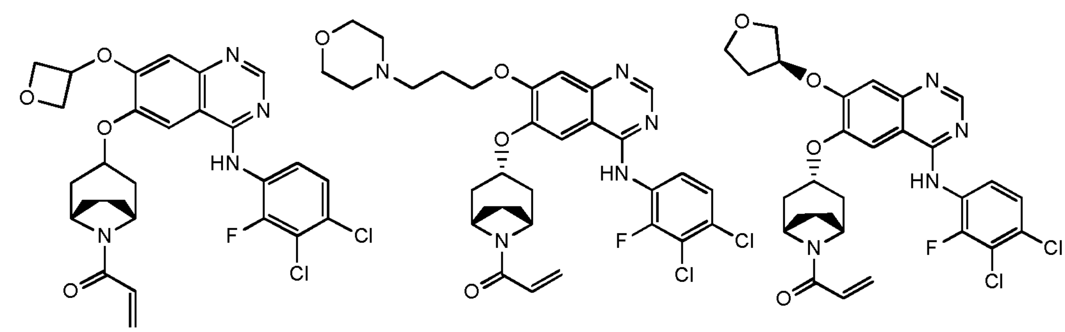

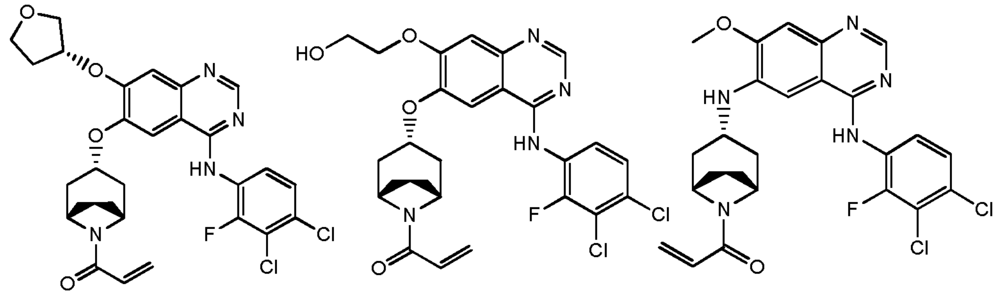

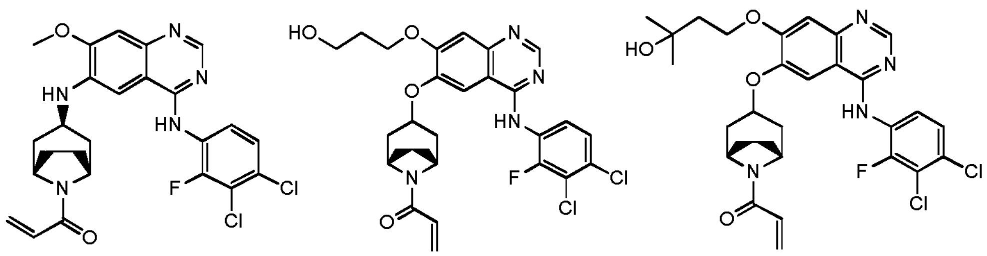

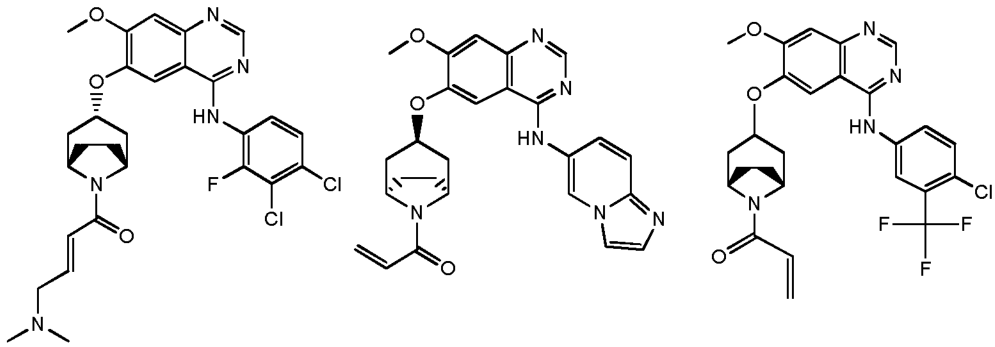

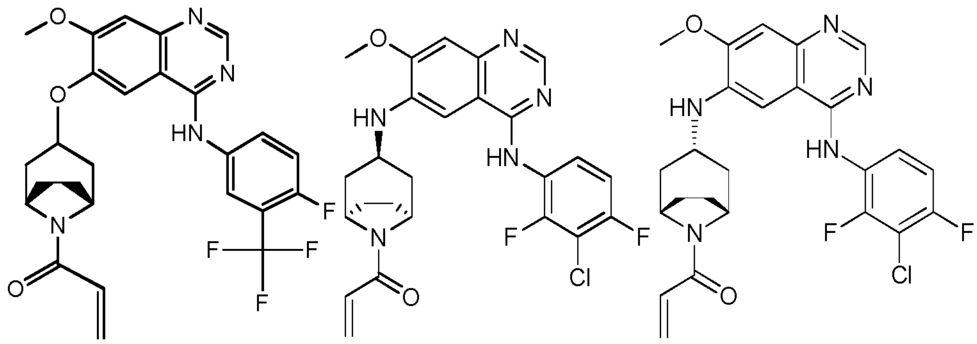

.

**14.** Composition pharmaceutique, comprenant une quantité thérapeutiquement efficace du composé selon l'une quelconque des revendications 1 à 13, d'un stéréoisomère correspondant ou d'un sel pharmaceutiquement acceptable correspondant, en tant qu'un ingrédient actif, et un ou plusieurs supports pharmaceutiquement acceptables.

**15.** Composé selon l'une quelconque des revendications 1 à 13, stéréoisomère correspondant ou sel pharmaceutiquement acceptable correspondant, ou composition pharmaceutique selon la revendication 14, pour une utilisation dans un procédé de traitement d'un cancer associé à la tyrosine kinase Pan-HER.

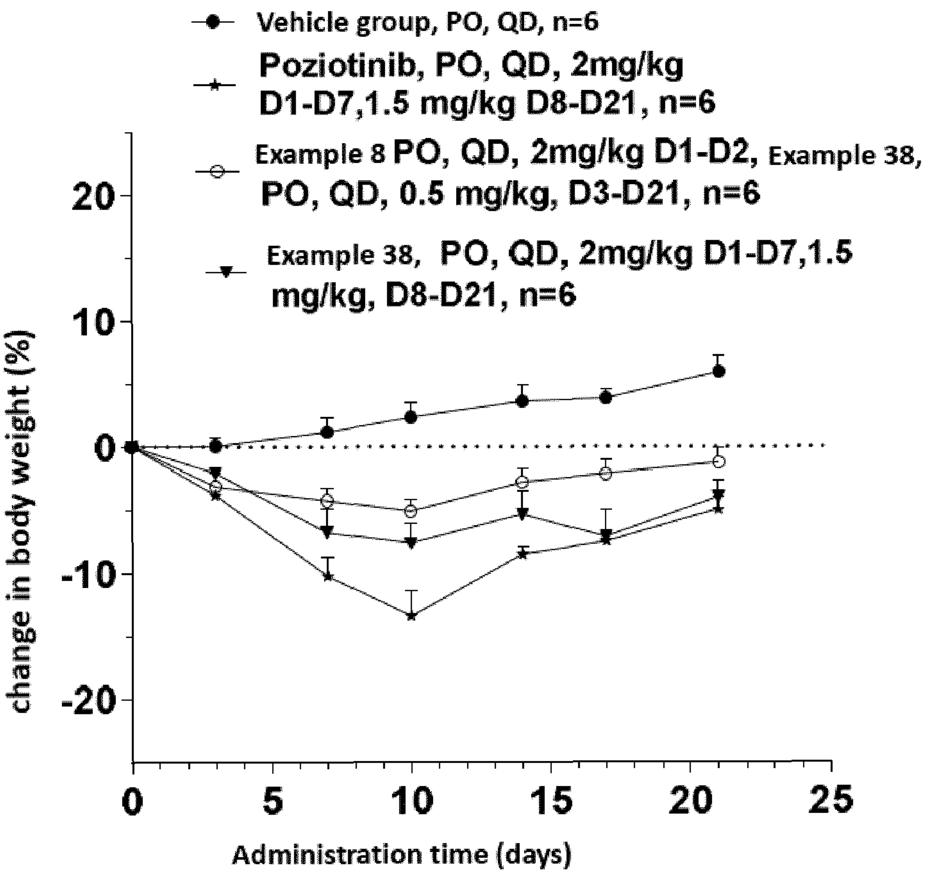
Vehicle group, PO, QD, n=6
Poziotinib, PO, QD, 2mg/kg D1-D7,1.5 mg/kg D8-D21, n=6
Example 8 PO, QD, 2mg/kg D1-D2, Example 38, PO, QD, 0.5 mg/kg, D3-D21, n=6
Example 38, PO, QD, 2mg/kg D1-D7,1.5 mg/kg, D8-D21, n=6
20
10
0
-10
-20
change in body weight (%)
0
5
10
15
20
25
Administration time (days)

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201711376757
- CN 201810225742
- CN 201810576529
- WO 2008150118 A
- WO 2015043515 A
- CN 104513229 A
- CN 101679384 A
- CN 106279128 A
- WO 2017107985 A1
- WO 2015154725 A1

### Non-patent literature cited in the description

- **BASELGA. J.** *Oncologist,* 2002, vol. 7, 2-8